# EUROPEAN PATENT SPECIFICATION

(11) **EP 3 480 213 B1**
(45) Date of publication and mention of the grant of the patent: **13.11.2019**
(21) Application number: 18212801.7
(22) Date of filing: 15.06.2015
(51) Int. Cl.: C07K 14/74, C12N 15/62

(54) **SYNTAC POLYPEPTIDES AND USES THEREOF**
SYNTAC-POLYPEPTIDE UND VERWENDUNGEN DAVON
POLYPEPTIDES SYNTAC ET LEURS UTILISATIONS

(30) Priority: 18.06.2014 US 201462013715 P
(43) Date of publication of application: 08.05.2019
(62) Divisional of application: 15809277.5
(73) Proprietor: Albert Einstein College of Medicine, Bronx, NY 10461 (US)
(72) Inventor: SEIDEL, Ronald, D., III, Nattick, MA 01760 (US); CHAPARRO, Rodolfo, J., Bronx, NY 10461 (US); HILLERICH, Brandan, S., Arlington, MA 02474 (US); GARFORTH, Scott, J., Bronx, NY 10464 (US); ALMO, Steven, C., Pelham, NY 10803 (US)
(74) Representative: Mewburn Ellis LLP

(56) References cited:
- WO-A2-02/072631
- WO-A2-2010/037395
- US-A1- 2012 264 161

## Description

### INTRODUCTION

Throughout this application various publications are referred to in square brackets. Full citations for these references may be found at the end of the

The rapid progress over the past decade in the development of high throughput technologies for clinically relevant biomarker discovery has been paralleled by the stepwise development and application of biologies, drugs in which the active substance is produced by or extracted from a biological source (e.g., monoclonal antibodies, therapeutic proteins, and peptides), and has revolutionized the treatment of immune-borne conditions. However, current biologic therapies are prompting safety regulatory actions at double the rate of their synthetic counterparts (17% for biologies, 8.5% synthetics) [1]. This is thought to manifest from the mode of action of these immune-modulating biologies: *global* immunosuppression in the case of autoimmunity (e.g., Humira [2]) and *global* immunostimulation for the treatment of cancers (e.g., Yervoy [3]). These treatments do not adequately restrict immunomodulation to pathogenically relevant cells and as a result, predispose patients to potentially *deadly* infections and a host of troubling side effects [4-6]. Further, the moderate efficacy and safety profiles of these drugs [7] has elicited a recent trend toward targeted therapeutics. First generation "targeted" biologics direct their effects on more restricted T cell subsets (e.g., antibodies and protein therapeutics such as anti-4-1BB, anti-CD27, LAG-3, and TIM-3) [8-11]. However, like previous therapies, these "1^{st}-gen" efforts remain unable to target only disease-relevant cells.

At the core of the molecular events comprising an adaptive immune response is the engagement of the T cell receptor (TCR) with a small peptide antigen non-covalently presented by a major histocompatibility complex (MHC) molecule. This represents the immune system's *targeting* mechanism and is a requisite molecular interaction for T cell activation and effector function. Following epitope-specific cell targeting, the recruited T cells are activated through general engagement of costimulatory molecules found on the antigen presenting cell. Both signals are required to drive T cell specificity and activation or inhibition. Importantly, during T cell development, a genomic editing process results in the expression of a *unique* TCR on every T cell [12], whereas the costimulatory molecule is generally expressed on all T cells (or large T cell 'subsets'). Current approaches rely almost exclusively on the general engagement of the costimulatory molecule, resulting in "global therapies". These global immunotherapies are incredibly potent but indiscriminately target T cells leading to significant toxicity. If costimulatory molecules could preferentially bind to T cells bearing disease-relevant TCRs, their potency would advance from a liability to a strength.

There exist a number of approaches for T cell modulation, which include the use of soluble costimulatory molecules generally expressed as Fc fusions or antibodies directed at costimulatory molecules capable of blocking costimulatory function [13, 14], antibody-drug conjugates (ADCs) [15], bi-specific antibodies (BsAbs) [16, 17] and free peptide antigens [18]. Notably, ADCs (often referred to as magic bullets) promise the targeted delivery of toxins (or other drug payloads) directly to pathologic cells. However ADCs currently suffer from a lack of preferred biomarkers for antibody targeting and poor internalization rates as only ∼1.5% of the administered dose is found inside tumor cells, with internalization often being required for cell killing. Bispecific antibodies provide an attractive opportunity to combine additive and synergistic effects of multiple mAbs, and can be used to bridge tumor cells with T cells [17], and therefore do not require internalization to illicit a response. Although bispecific antibodies have been developed to have bivalent interactions with two different antigens [19], these constructs still lack modularity and suffer reduced affinity compared to the parental mAb [20]. Adoptive T cell (CAR-T) therapy partially addresses these issues, and is an attractive alternative to the traditional therapies described above [21]. CAR-T uses genetically modified primary T cells bearing chimeric antigen receptors (CARs) on their surface: patient's T cells are extracted, purified and genetically modified to target tumor specific antigens through the use CARs. The CAR generally has an external single chain variable domain (an antibody fragment) that targets pathologic cells but harbors traditional costimulatory molecule cytoplasmic domains. Once the engineered T cells bind to target antigen, the internal stimulatory domains provide the necessary signals for the T cell to become fully active. In this fully active state, the T cells can more effectively proliferate and attack cancer cells. Tempering this response so as to avoid cytokine release syndrome and associated side effects, along with scalability issues (e.g., the significant expense and difficulty associated with the T-cell extraction and modification) currently prevent this technology from entering mainstream use [22].

Biologies, also known as biopharmaceuticals, are drugs in which the active substance is produced by or extracted from a biological source (in contrast to "small-molecule" drugs). Biologics are relatively recent additions to the global therapeutic market, being for the most part recombinant proteins produced through genetic engineering; these include monoclonal antibodies, therapeutic proteins, and peptides. Most of the currently marketed biologic drugs are used to relieve patients suffering from chronic diseases, such as cancer, diabetes, cardiovascular diseases, infertility and cystic fibrosis. The global biologics market was valued at $163 billion in 2012 and is expected to reach $252 billion by 2017 supporting a five-year compound annual growth rate of 9%. Driving this growth is the need for a more extensive drug pipeline, identification of attractive targets against challenging diseases and a push to pursue follow-on biologics (biosimilars, generic biologies) exemplified by the recent introduction of an abbreviated FDA approval pathway.

The present invention addresses the need for precision therapeutics for immuno-oncology and autoimmunity - tailored therapeutics that clonally target only the disease-related T cells for upregulation (e.g., in the case of cancer) or suppression (e.g., in the case of autoimmunity) as opposed to the global and "pseudo-targeted" modulators currently on the market or in development.

### SUMMARY

This invention provides a multimeric polypeptide comprising a heterodimeric polypeptide comprising
(a) a first polypeptide comprising, in order from N-terminus to C-terminus:
   i) a peptide epitope; and
   ii) a first major class II histocompatibility complex (MHC) polypeptide;
b) a second polypeptide comprising a second class II MHC polypeptide, and
c) at least one immunomodulatory polypeptide,
wherein the first and/or the second polypeptide comprises the at least one immunomodulatory polypeptide,
wherein the peptide epitope is an epitope of a self antgen, and
optionally, wherein the multimeric polypeptide comprises an immunoglobulin (Ig) Fc polypeptide or a non-Ig scaffold..

The invention further provides a method of selectively inhibiting the activity and/or reducing the number of a self-reactive T cell, the method comprising contacting the T cell *in vitro* with the multimeric polypeptide of the invention, wherein said contacting selectively inhibits the activity and/or reduces the number of the self-reactive T cells.

The invention also provides a composition comprising the multimeric polypeptide of the invention and a pharmaceutically acceptable excipient.

This disclosure also provides recombinant polypeptide comprising a sequence of amino acids identical to a first B2M leader sequence contiguous with a candidate epitope peptide contiguous with a first amino acid linker sequence contiguous with a sequence of amino acids identical to a human native B2M peptide sequence contiguous with a second amino acid linker sequence contiguous with a second B2M leader sequence contiguous with a T cell modulatory domain peptide sequence contiguous with a third amino acid linker contiguous with a sequence of amino acids identical to a MHC heavy chain contiguous with a sequence of amino acids identical to an immunoglobulin Fc domain.

Also provided is a method of inhibiting a T cell clone which recognizes an epitope peptide comprising contacting a T cell of the clone with a recombinant peptide as described herein, wherein the recombinant peptide comprises the epitope peptide and comprises a T cell modulatory domain which is an inhibitory domain, in an amount effective to inhibit a T cell clone.

Also provided is a method of treating an autoimmune disorder by inhibiting a self-reactive T cell clone which recognizes an epitope peptide comprising contacting a T cell of the clone with a recombinant peptide as described herein, wherein the recombinant peptide comprises the epitope peptide and comprises a T cell modulatory domain which is an inhibitory domain, in an amount effective to treat an autoimmune disorder.

Also provided is a method of stimulating a T cell clone which recognizes an epitope peptide comprising contacting a T cell of the clone with a recombinant peptide as described herein, wherein the recombinant peptide comprises the epitope peptide and comprises a T cell modulatory domain which is an stimulatory domain, in an amount effective to stimulate a T cell clone.

Also provided is a method of treating a cancer by stimulating a T cell clone which recognizes an epitope peptide on a cancer comprising contacting a T cell of the clone with a recombinant peptide as described herein, wherein the recombinant peptide comprises the epitope peptide and comprises a T cell modulatory domain which is an stimulatory domain, in an amount effective to treat the cancer.

Also provided is a recombinant polypeptide construct comprising (i) a candidate epitope peptide bound by a first amino acid linker sequence contiguous with a sequence of amino acids comprising a sequence identical to a human native B2M peptide sequence contiguous with a second amino acid linker sequence contiguous with a T cell modulatory domain peptide, wherein (i) is bound by one, or more than one, disulfide bond to (ii) a sequence of amino acids having the sequence of a MHC heavy chain contiguous with a third amino acid linker sequence contiguous with a sequence of amino acids identical to an immunoglobulin Fc domain.

Also provided is recombinant polypeptide construct comprising (i) a candidate epitope peptide bound by a first amino acid linker sequence contiguous with a sequence of amino acids comprising a sequence identical to a human native B2M peptide sequence, wherein (i) is bound by one, or more than one, disulfide bond to (ii) a T cell modulatory domain peptide contiguous with a second amino acid linker sequence contiguous with a sequence of amino acids having the sequence of a MHC heavy chain contiguous a third amino acid linker sequence contiguous with a sequence of amino acids identical to an immunoglobulin Fc domain.

Also provided is a protein comprising two of the recombinant polypeptide constructs described herein joined by one or more disulfide bonds between the respective immunoglobulin Fc domains thereof.

Also provided is a protein comprising two of the recombinant polypeptide constructs described herein joined by one or more disulfide bonds between the respective immunoglobulin Fc domains thereof.

This disclosure provides an isolated suspension-adapted cell transduced by or transfected with a heterologous nucleic acid comprising, in 5' to 3' order a sequence encoding a recombinant polypeptide as described herein.

The present disclosure provides a recombinant polypeptide comprising a sequence of amino acids identical to a first B2M leader sequence contiguous with a candidate epitope peptide contiguous with a first amino acid linker sequence contiguous with a sequence of amino acids identical to a human native B2M peptide sequence contiguous with a second amino acid linker sequence contiguous with a T cell modulatory domain peptide sequence contiguous with a third amino acid linker contiguous with a second B2M leader sequence contiguous with a sequence of amino acids identical to a MHC heavy chain contiguous with a sequence of amino acids identical to an immunoglobulin Fc domain. In some cases, the candidate epitope comprises 7-20 amino acids. In some cases, the third amino acid linker is self-cleaving. In some cases, the second amino acid linker is self-cleaving. In some cases, the self-cleaving peptide is a viral 2A peptide or has the sequence thereof. In some cases, the first and/or second B2M leader sequence has the sequence of human B2M leader sequence. In some cases, the MHC heavy chain is a human MHC heavy chain. In some cases, the MHC heavy chain is an MHC I molecule. In some cases, the MHC heavy chain is an HLA-A02:01. In some cases, the MHC heavy chain is an MHC II molecule. In some cases, the immunoglobulin Fc domain is an IgG Fc domain. In some cases, the immunoglobulin Fc domain is an IgA Fc domain. In some cases, the immunoglobulin Fc domain is an IgM Fc domain. In some cases, the immunoglobulin Fc domain is a human immunoglobulin Fc domain. In some cases, the immunoglobulin Fc domain is an IgG1 Fc domain. In some cases, the recombinant polypeptide comprises a His-8 tag contiguous with the C-terminal thereof. In some cases, the T cell modulatory domain is an inhibitory domain. In some cases, the T cell modulatory domain is a stimulating domain. In some cases, the T cell modulatory domain is an antibody, and antibody fragment, a peptide ligand, a T cell costimulatory peptide, a cytokine or a toxin. In some cases, the T cell modulatory domain comprises a PD-L1 peptide, the Ig variable domain of a PD-L1 peptide, the T cell modulatory domain comprises 4-1BBL, the T cell modulatory domain comprises B7-1W88A, or the T cell modulatory domain comprises anti-CD28 single chain Fv. In some cases, the recombinant polypeptide comprises a mutation in a human native B2M peptide sequence thereof and in the Heavy Chain sequence thereof so as to effect a disulfide bond between the B2M peptide sequence and Heavy Chain sequence.

The present disclosure provides a recombinant polypeptide comprising a sequence of amino acids identical to a first B2M leader sequence contiguous with a candidate epitope peptide contiguous with a first amino acid linker sequence contiguous with a sequence of amino acids identical to a human native B2M peptide sequence contiguous with a second amino acid linker sequence contiguous with a second B2M leader sequence contiguous with a T cell modulatory domain peptide sequence contiguous with a third amino acid linker contiguous with a sequence of amino acids identical to a MHC heavy chain contiguous with a sequence of amino acids identical to an immunoglobulin Fc domain. In some cases, the candidate epitope comprises 7-20 amino acids. In some cases, the third amino acid linker is self-cleaving. In some cases, the second amino acid linker is self-cleaving. In some cases, the self-cleaving peptide is a viral 2A peptide or has the sequence thereof. In some cases, the first and/or second B2M leader sequence has the sequence of human B2M leader sequence. In some cases, the MHC heavy chain is a human MHC heavy chain. In some cases, the MHC heavy chain is an MHC I molecule. In some cases, the MHC heavy chain is an HLA-A02:01. In some cases, the MHC heavy chain is an MHC II molecule. In some cases, the immunoglobulin Fc domain is an IgG Fc domain. In some cases, the immunoglobulin Fc domain is an IgA Fc domain. In some cases, the immunoglobulin Fc domain is an IgM Fc domain. In some cases, the immunoglobulin Fc domain is a human immunoglobulin Fc domain. In some cases, the immunoglobulin Fc domain is an IgG1 Fc domain. In some cases, the recombinant polypeptide comprises a His-8 tag contiguous with the C-terminal thereof. In some cases, the T cell modulatory domain is an inhibitory domain. In some cases, the T cell modulatory domain is a stimulating domain. In some cases, the T cell modulatory domain is an antibody, and antibody fragment, a peptide ligand, a T cell costimulatory peptide, a cytokine or a toxin. In some cases, the T cell modulatory domain comprises a PD-L1 peptide, the Ig variable domain of a PD-L1 peptide, the T cell modulatory domain comprises 4-1BBL, the T cell modulatory domain comprises B7-1W88A, or the T cell modulatory domain comprises anti-CD28 single chain Fv. In some cases, the recombinant polypeptide comprises a mutation in a human native B2M peptide sequence thereof and in the Heavy Chain sequence thereof so as to effect a disulfide bond between the B2M peptide sequence and Heavy Chain sequence.

In some cases, the recombinant polypeptide comprises a mutation in a human native B2M peptide sequence thereof and in the Heavy Chain sequence thereof so as to effect a disulfide bond between the B2M peptide sequence and Heavy Chain sequence. In some cases, the Heavy Chain sequence is an HLA and wherein the disulfide bond links one of the following pairs of residues: B2M residue 12, HLA residue 236; B2M residue 12, HLA residue 237; B2M residue 8, HLA residue 234; B2M residue 10, HLA residue 235; B2M residue 24, HLA residue 236; B2M residue 28, HLA residue 232; B2M residue 98, HLA residue 192; B2M residue 99, HLA residue 234; B2M residue 3, HLA residue 120; B2M residue 31, HLA residue 96; B2M residue 53, HLA residue 35; B2M residue 60, HLA residue 96; B2M residue 60, HLA residue 122; B2M residue 63, HLA residue 27; B2M residue Arg3, HLA residue Gly120; B2M residue His31, HLA residue Gln96; B2M residue Asp53, HLA residue Arg35; B2M residue Trp60, HLA residue Gln96; B2M residue Trp60, HLA residue Asp122; B2M residue Tyr63, HLA residue Tyr27; B2M residue Lys6, HLA residue Glu232; B2M residue Gln8, HLA residue Arg234; B2M residue Tyr10, HLA residue Pro235; B2M residue Ser11, HLA residue Gln242; B2M residue Asn24, HLA residue Ala236; B2M residue Ser28, HLA residue Glu232; B2M residue Asp98, HLA residue His192; and B2M residue Met99, HLA residue Arg234.

In some cases, the recombinant polypeptide comprises a mutation in a human native B2M peptide sequence thereof and in the Heavy Chain sequence thereof so as to effect a disulfide bond between the B2M peptide sequence and Heavy Chain sequence. In some cases, the Heavy Chain sequence is an HLA and wherein the disulfide bond links one of the following pairs of residues: first linker position Gly 2, Heavy Chain (HLA) position Tyr 84; Light Chain (B2M) position Arg 12, HLA Ala236; and/or B2M residue Arg12, HLA residue Gly237.

In some cases, the T cell modulatory domain is an inhibitory domain. In some cases, the T cell modulatory domain is a stimulating domain. In some cases, the T cell modulatory domain is an antibody, and antibody fragment, a peptide ligand, a T cell costimulatory peptide, a cytokine or a toxin. In some cases, the T cell modulatory domain comprises a PD-L1 peptide, the Ig variable domain of a PD-L1 peptide, the T Cell modulatory domain comprises 4-1BBL, the T Cell modulatory domain comprises B7-1W88A, or the T cell modulatory domain comprises anti-CD28 single chain Fv.

The present disclosure provides a nucleic acid encoding any of the recombinant polypeptides described above, or elsewhere herein. The present disclosure provides a cell transformed with a nucleic acid encoding any of the recombinant polypeptides described above, or elsewhere herein.

The present disclosure provides a method of inhibiting a T cell clone which recognizes an epitope peptide comprising contacting a T cell of the clone with a recombinant peptide of any of described above, or elsewhere herein, wherein the recombinant peptide comprises the epitope peptide and comprises a T cell modulatory domain which is an inhibitory domain, in an amount effective to inhibit a T cell clone.

The present disclosure provides a method of treating an autoimmune disorder by inhibiting a self-reactive T cell clone which recognizes an epitope peptide comprising contacting a T cell of the clone with a recombinant peptide described above, or elsewhere herein, wherein the recombinant peptide comprises the epitope peptide and comprises a T cell modulatory domain which is an inhibitory domain, in an amount effective to treat an autoimmune disorder.

The present disclosure provides a method of stimulating a T cell clone which recognizes an epitope peptide comprising contacting a T cell of the clone with a recombinant peptide described above, or elsewhere herein, wherein the recombinant peptide comprises the epitope peptide and comprises a T cell modulatory domain which is an stimulatory domain, in an amount effective to stimulate a T cell clone.

The present disclosure provides a method of treating a cancer by stimulating a T cell clone which recognizes an epitope peptide on a cancer comprising contacting a T cell of the clone with a recombinant peptide described above, or elsewhere herein, wherein the recombinant peptide comprises the epitope peptide and comprises a T cell modulatory domain which is an stimulatory domain, in an amount effective to treat the cancer.

The present disclosure provides a recombinant polypeptide construct comprising (i) a candidate epitope peptide bound by a first amino acid linker sequence contiguous with a sequence of amino acids comprising a sequence identical to a human native B2M peptide sequence contiguous with a second amino acid linker sequence contiguous with a T cell modulatory domain peptide, wherein (i) is bound by one, or more than one, disulfide bond to (ii) a sequence of amino acids having the sequence of a MHC heavy chain contiguous with a third amino acid linker sequence contiguous with a sequence of amino acids identical to an immunoglobulin Fc domain. The present disclosure provides a protein comprising two of the recombinant polypeptide constructs joined by one or more disulfide bonds between the respective immunoglobulin Fc domains thereof.

The present disclosure provides a recombinant polypeptide construct comprising (i) a candidate epitope peptide bound by a first amino acid linker sequence contiguous with a sequence of amino acids comprising a sequence identical to a human native B2M peptide sequence, wherein (i) is bound by one, or more than one, disulfide bond to (ii) a T cell modulatory domain peptide contiguous with a second amino acid linker sequence contiguous with a sequence of amino acids having the sequence of a MHC heavy chain contiguous a third amino acid linker sequence contiguous with a sequence of amino acids identical to an immunoglobulin Fc domain. The present disclosure provides a protein comprising two of the recombinant polypeptide constructs joined by one or more disulfide bonds between the respective immunoglobulin Fc domains thereof.

The present disclosure provides multimeric polypeptides comprising at least a first polypeptide and a second polypeptide, where the first polypeptide comprises, in order from N-terminus to C-terminus: i) an epitope; and ii) a first major histocompatibility complex (MHC) polypeptide; and where the second polypeptide comprises, in order from N-terminus to C-terminus: i) a second MHC polypeptide; and ii) an immunoglobulin (Ig) Fc polypeptide, where the multimeric polypeptide comprises an immunomodulatory domain at the C-terminus of the first polypeptide or at the N-terminus of the second polypeptide. The present disclosure provides nucleic acids comprising nucleotide sequences encoding the multimeric polypeptide. The present disclosure provides recombinant expression vectors comprising the nucleic acids. The present disclosure provides genetically modified host cells, where the genetically modified host cells are genetically modified with a nucleic acid of the present disclosure or a recombinant expression vector of the present disclosure. The present disclosure provides compositions, including pharmaceutical compositions, comprising the multimeric polypeptides. The present disclosure provides methods of modulating an activity of a T cell, the methods involving contacting the T cell with a multimeric polypeptide of the present disclosure. The present disclosure provides methods of treatment involving administering to an individual in need thereof an effective amount of a multimeric polypeptide of the present disclosure. The present disclosure provides a container comprising a multimeric polypeptide of the present disclosure, or a composition (e.g., a pharmaceutical composition) comprising a multimeric polypeptide of the present disclosure.

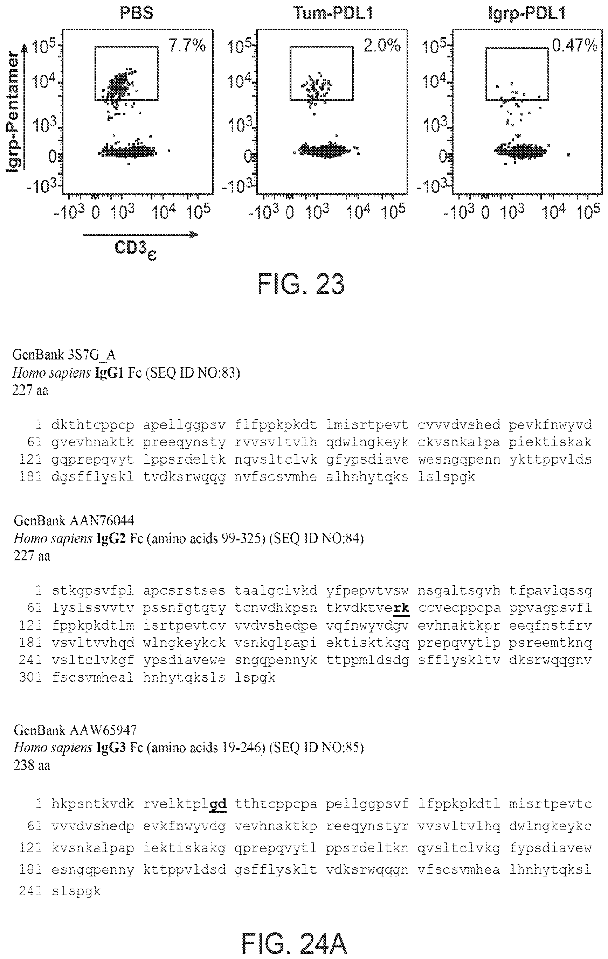

The present disclosure provides a multimeric polypeptide comprising: a) a first polypeptide comprising, in order from N-terminus to C-terminus: i) an epitope; and ii) a first MHC polypeptide; and b) a second polypeptide comprising, in order from N-terminus to C-terminus: i) an immunomodulatory domain; iii) a second MHC polypeptide; and ii) an Ig Fc polypeptide. The present disclosure provides a multimeric polypeptide comprising: a) a first polypeptide comprising, in order from N-terminus to C-terminus: i) an epitope; ii) a first MHC polypeptide; and iii) an immunomodulatory domain; and b) a second polypeptide comprising, in order from N-terminus to C-terminus: i) a second MHC polypeptide; and ii) an immunoglobulin (Ig) Fc polypeptide. In some cases, the first MHC polypeptide is a β2-microglobulin polypeptide; and wherein the second MHC polypeptide is an MHC class I heavy chain polypeptide. In some cases, the β2-microglobulin polypeptide comprises an amino acid sequence having at least 85% amino acid sequence identity to the amino acid sequence set forth in SEQ ID NO:4. In some cases, the MHC class I heavy chain polypeptide is an HLA-A, an HLA-B, or an HLA-C, heavy chain. In some cases, the MHC class I heavy chain polypeptide comprises an amino acid sequence having at least 85%, at least 90%, at least 95%, or 100%, amino acid sequence identity to the amino acid sequence set forth in SEQ ID NO:5. In some cases, the first MHC polypeptide is an MHC Class II alpha chain polypeptide; and wherein the second MHC polypeptide is an MHC class II beta chain polypeptide. In some cases, the epitope is a T-cell epitope. In some cases, the Ig Fc polypeptide is an IgG1 Fc polypeptide, an IgG2 Fc polypeptide, an IgG3 Fc polypeptide, an IgG4 Fc polypeptide, an IgA Fc polypeptide, or an IgM Fc polypeptide. In some cases, the Ig Fc polypeptide comprises an amino acid sequence having at least 85%, at least 90%, at least 95%, or 100%, amino acid sequence identity to an amino acid sequence depicted in FIG. 24A-24C. In some cases, the first polypeptide and the second polypeptide are non-covalently associated. In some cases, the first polypeptide and the second polypeptide are covalently linked. In some cases, the covalent linkage is via a disulfide bond. In some cases, the first MHC polypeptide or a linker between the epitope and the first MHC polypeptide comprises an amino acid substitution to provide a first Cys residue, and the second MHC polypeptide comprises an amino acid substitution to provide a second Cys residue, and wherein the disulfide linkage is between the first and the second Cys residues. In some cases, the multimeric polypeptide comprises a first linker interposed between the epitope and the first MHC polypeptide. In some cases, the immunomodulatory polypeptide is selected from a 4-1BBL polypeptide, a B7-1 polypeptide; a B7-2 polypeptide, an ICOS-L polypeptide, an OX-40L polypeptide, a CD80 polypeptide, a CD86 polypeptide, a PD-L1 polypeptide, a FasL polypeptide, and a PD-L2 polypeptide. In some cases, the first polypeptide or the second polypeptide comprises 2 or more immunomodulatory polypeptides. In some cases, the 2 or more immunomodulatory polypeptides are in tandem. In some cases, the multimeric polypeptide comprises a third polypeptide, wherein the third polypeptide comprises an immunomodulatory polypeptide comprising an amino acid sequence having at least 90% amino acid sequence identity to the immunomodulatory polypeptide of the first polypeptide. In some cases, the third polypeptide is covalently linked to the first polypeptide. In some cases, wherein the second polypeptide comprises, in order from N-terminus to C-terminus: i) the second MHC polypeptide; ii) the immunoglobulin (Ig) Fc polypeptide; and iii) an affinity tag.

The present disclosure provides a multimeric polypeptide comprising: a) a first polypeptide comprising, in order from N-terminus to C-terminus: i) an epitope; ii) a first MHC polypeptide; and b) a second polypeptide comprising, in order from N-terminus to C-terminus: i) a second MHC polypeptide; and ii) optionally an Ig Fc polypeptide or a non-Ig scaffold, wherein the multimeric polypeptide comprises optionally an immunoglobulin (Ig) Fc polypeptide or a non-Ig scaffold, wherein the multimeric polypeptide comprises one or more immunomodulatory domains, wherein the one or more immunomodulatory domain is: A) at the C-terminus of the first polypeptide; B) at the N-terminus of the second polypeptide; C) at the C-terminus of the second polypeptide; or D) at the C-terminus of the first polypeptide and at the N-terminus of the second polypeptide. In some cases, a multimeric polypeptide comprises a single immunomodulatory polypeptide. In some cases, a multimeric polypeptide comprises two immunomodulatory polypeptides (e.g., two copies of the same immunomodulatory polypeptide). In some cases, a multimeric polypeptide comprises three immunomodulatory polypeptides (e.g., three copies of the same immunomodulatory polypeptide). In some cases, a multimeric polypeptide comprises four immunomodulatory polypeptides (e.g., four copies of the same immunomodulatory polypeptide). In some cases, a multimeric polypeptide comprises a single immunomodulatory polypeptide. In some cases, a multimeric polypeptide comprises two immunomodulatory polypeptides (e.g., two copies of the same immunomodulatory polypeptide). In some cases, a multimeric polypeptide comprises three immunomodulatory polypeptides (e.g., three copies of the same immunomodulatory polypeptide). In some cases, a multimeric polypeptide comprises four immunomodulatory polypeptides (e.g., four copies of the same immunomodulatory polypeptide). In some cases, the multimeric polypeptide comprises: a) a first polypeptide comprising, in order from N-terminus to C-terminus: i) an epitope; ii) a first MHC polypeptide; and iii) an immunomodulatory domain; and b) a second polypeptide comprising, in order from N-terminus to C-terminus: i) a second MHC polypeptide; and ii) an Ig Fc polypeptide. In some cases, the multimeric polypeptide comprises: a) a first polypeptide comprising, in order from N-terminus to C-terminus: i) an epitope; and ii) a first MHC polypeptide; and b) a second polypeptide comprising, in order from N-terminus to C-terminus: i) an immunomodulatory domain; iii) a second MHC polypeptide; and ii) an immunoglobulin (Ig) Fc polypeptide. In some cases, the multimeric polypeptide comprises: a) a first polypeptide comprising, in order from N-terminus to C-terminus: i) an epitope; and ii) a first MHC polypeptide; and b) a second polypeptide comprising, in order from N-terminus to C-terminus: i) a second MHC polypeptide; and ii) an Ig Fc polypeptide; and iii) an immunomodulatory domain. In some cases, the multimeric polypeptide comprises: a) a first polypeptide comprising, in order from N-terminus to C-terminus: i) an epitope; and ii) a first MHC polypeptide; and b) a second polypeptide comprising, in order from N-terminus to C-terminus: i) a second MHC polypeptide; and ii) an immunomodulatory domain. In some cases, the multimeric polypeptide comprises: a) a first polypeptide comprising, in order from N-terminus to C-terminus: i) an epitope; and ii) a first MHC polypeptide; and b) a second polypeptide comprising, in order from N-terminus to C-terminus: i) an immunomodulatory domain; and ii) a second MHC polypeptide. In some cases, the multimeric polypeptide comprises: a) a first polypeptide comprising, in order from N-terminus to C-terminus: i) an epitope; ii) a first MHC polypeptide; and iii) an immunomodulatory domain; and b) a second polypeptide comprising, in order from N-terminus to C-terminus: i) a second MHC polypeptide. In some cases, the non-Ig scaffold is an XTEN polypeptide, a transferrin polypeptide, an Fc receptor polypeptide, an elastin-like polypeptide, a silk-like polypeptide, or a silk-elastin-like polypeptide. In some cases, the first MHC polypeptide is a β2-microglobulin polypeptide; and wherein the second MHC polypeptide is an MHC class I heavy chain polypeptide. In some cases, the β2-microglobulin polypeptide comprises an amino acid sequence having at least 85% amino acid sequence identity to the amino acid sequence set forth in SEQ ID NO:4. In some cases, the MHC class I heavy chain polypeptide is an HLA-A, an HLA-B, or an HLA-C heavy chain. In some cases, the MHC class I heavy chain polypeptide comprises an amino acid sequence having at least 85% amino acid sequence identity to the amino acid sequence set forth in SEQ ID NO:5. In some cases, the first MHC polypeptide is an MHC Class II alpha chain polypeptide; and wherein the second MHC polypeptide is an MHC class II beta chain polypeptide. In some cases, the epitope is a T-cell epitope. In some cases, the multimeric polypeptide comprises an Fc polypeptide, and wherein the Ig Fc polypeptide is an IgG1 Fc polypeptide, an IgG2 Fc polypeptide, an IgG3 Fc polypeptide, an IgG4 Fc polypeptide, an IgA Fc polypeptide, or an IgM Fc polypeptide. In some cases, the Ig Fc polypeptide comprises an amino acid sequence having at least 85%, at least 90%, at least 95%, at least 98%, or at least 100%, amino acid sequence identity to an amino acid sequence depicted in FIG. 24A-24C. In some cases, the first polypeptide and the second polypeptide are non-covalently associated. In some cases, the first polypeptide and the second polypeptide are covalently linked. In some cases, the covalent linkage is via a disulfide bond. In some cases, the first MHC polypeptide or a linker between the epitope and the first MHC polypeptide comprises an amino acid substitution to provide a first Cys residue, and the second MHC polypeptide comprises an amino acid substitution to provide a second Cys residue, and wherein the disulfide linkage is between the first and the second Cys residues. In some cases, the multimeric polypeptide comprises a first linker interposed between the epitope and the first MHC polypeptide. In some cases, the immunomodulatory polypeptide is selected from a 4-1BBL polypeptide, a B7-1 polypeptide; a B7-2 polypeptide, an ICOS-L polypeptide, an OX-40L polypeptide, a CD80 polypeptide, a CD86 polypeptide, a PD-L1 polypeptide, a FasL polypeptide, and a PD-L2 polypeptide. In some cases, the multimeric polypeptide comprises 2 or more immunomodulatory polypeptides. In some cases, the 2 or more immunomodulatory polypeptides are in tandem. In some cases, the multimeric polypeptide comprises a third polypeptide, wherein the third polypeptide comprises an immunomodulatory polypeptide comprising an amino acid sequence having at least 90% amino acid sequence identity to the immunomodulatory polypeptide of the first polypeptide or the second polypeptide. In some cases, the third polypeptide is covalently linked to the first polypeptide. In some cases, the second polypeptide comprises, in order from N-terminus to C-terminus: i) the second MHC polypeptide; ii) the Ig Fc polypeptide; and iii) an affinity tag.

The present disclosure provides a nucleic acid comprising nucleotide sequences encoding the polypeptide chains of a multimeric polypeptide of the present disclosure; in some cases, the nucleic acid is present in a recombinant expression vector. The present disclosure provides a nucleic acid comprising a nucleotide sequence encoding a recombinant polypeptide, i) wherein the recombinant polypeptide comprises, in order from N-terminus to C-terminus: a) an epitope; b) a first MHC polypeptide; c) an immunomodulatory polypeptide; d) a proteolytically cleavable linker or a ribosome skipping signal; e) a second MHC polypeptide; and f) an immunoglobulin (Ig) Fc polypeptide or a non-Ig-based scaffold; or ii) wherein the recombinant polypeptide comprises, in order from N-terminus to C-terminus: a) an epitope; b) a first MHC polypeptide; c) a proteolytically cleavable linker or a ribosome skipping signal; d) an immunomodulatory polypeptide; e) a second MHC polypeptide; and f) an Ig Fc polypeptide or a non-Ig-based scaffold. In some cases, the first MHC polypeptide is a β2-microglobulin polypeptide; and wherein the second MHC polypeptide is an MHC class I heavy chain polypeptide. In some cases, the β2-microglobulin polypeptide comprises an amino acid sequence having at least 85% amino acid sequence identity to the amino acid sequence set forth in SEQ ID NO:4. In some cases, the MHC class I heavy chain polypeptide is an HLA-A, HLA-B, or HLA-C heavy chain. In some cases, the MHC class I heavy chain polypeptide comprises an amino acid sequence having at least 85% amino acid sequence identity to the amino acid sequence set forth in SEQ ID NO:5. In some cases, the first MHC polypeptide is an MHC Class II alpha chain polypeptide; and wherein the second MHC polypeptide is an MHC class II beta chain polypeptide. In some cases, the epitope is a T-cell epitope. In some cases, the Ig Fc polypeptide is an IgG1 Fc polypeptide, an IgG2 Fc polypeptide, an IgG3 Fc polypeptide, an IgG4 Fc polypeptide, an IgA Fc polypeptide, or an IgM Fc polypeptide. In some cases, the Ig Fc polypeptide comprises an amino acid sequence having at least 85% amino acid sequence identity to an amino acid sequence depicted in Figures 24A-24C. In some cases, the immunomodulatory polypeptide is selected from a 4-1BBL polypeptide, a B7-1 polypeptide; a B7-2 polypeptide, an ICOS-L polypeptide, an OX-40L polypeptide, a CD80 polypeptide, a CD86 polypeptide, a PD-L1 polypeptide, a FasL polypeptide, and a PD-L2 polypeptide. In some cases, the immunomodulatory polypeptide is selected from a CD7, CD30L, CD40, CD70, CD83, HLA-G, MICA, MICB, HVEM, lymphotoxin beta receptor, 3/TR6, ILT3, ILT4, and HVEM. In some cases, the proteolytically cleavable linker or ribosome skipping signal comprises an amino acid sequence selected from: a) LEVLFQGP (SEQ ID NO:37); b) ENLYTQS (SEQ ID NO:34); c) a furin cleavage site; d) LVPR (SEQ ID NO:36); e) GSGATNFSLLKQAGDVEENPGP (SEQ ID NO:64); f) GSGEGRGSLLTCGDVEENPGP (SEQ ID NO:65); g) GSGQCTNYALLKLAGDVESNPGP (SEQ ID NO:66); and h) GSGVKQTLNFDLLKLAGDVESNPGP (SEQ ID NO:67). In some cases, the recombinant polypeptide comprises, in order from N-terminus to C-terminus: a) a first leader peptide; b) the epitope; c) the first MHC polypeptide; d) the immunomodulatory polypeptide; e) the proteolytically cleavable linker or ribosome skipping signal; f) a second leader peptide; g) the second MHC polypeptide; and h) the immunoglobulin (Ig) Fc polypeptide. In some cases, the first leader peptide and the second leader peptide is a β2-M leader peptide. In some cases, the nucleotide sequence is operably linked to a transcriptional control element. In some cases, the transcriptional control element is a promoter that is functional in a eukaryotic cell. In some cases, the first MHC polypeptide or a linker between the epitope and the first MHC polypeptide comprises an amino acid substitution to provide a first Cys residue, and the second MHC polypeptide comprises an amino acid substitution to provide a second Cys residue, and wherein the first and the second Cys residues provide for a disulfide linkage between the first MHC polypeptide and the second MHC polypeptide. The present disclosure provides a recombinant expression vector comprising any one of the nucleic acids described above or elsewhere herein. In some cases, the recombinant expression vector is a viral vector. In some cases, the recombinant expression vector is a non-viral vector. The present disclosure provides a host cell genetically modified with a recombinant expression vector as described above and elsewhere herein. In some cases, the host cell is *in vitro.* In some cases, the host cell is genetically modified such that the cell does not produce an endogenous MHC β2-microglobulin polypeptide. In some cases, the host cell is a T lymphocyte.

The present disclosure provides a composition comprising: a) a first nucleic acid comprising a nucleotide sequence encoding a first polypeptide comprising, in order from N-terminus to C-terminus: i) an epitope; ii) a first MHC polypeptide; and iii) an immunomodulatory domain; and b) a first nucleic acid comprising a nucleotide sequence encoding a second polypeptide comprising, in order from N-terminus to C-terminus: i) a second MHC polypeptide; and ii) an Ig Fc polypeptide or a non-Ig-based scaffold. The present disclosure provides a composition comprising: a) a first nucleic acid comprising a nucleotide sequence encoding a first polypeptide comprising, in order from N-terminus to C-terminus: i) an epitope; and ii) a first MHC polypeptide; and b) a first nucleic acid comprising a nucleotide sequence encoding a second polypeptide comprising, in order from N-terminus to C-terminus: i) an immunomodulatory domain ii) a second MHC polypeptide; and iii) an Ig Fc polypeptide. In some cases, the first and/or the second nucleic acid is present in a recombinant expression vector. The present disclosure provides a host cell genetically modified with a nucleic acid composition described above or elsewhere herein.

The present disclosure provides a method of producing a multimeric polypeptide as described above or elsewhere herein, the method comprising: a) culturing a host cell as described above or elsewhere herein *in vitro* in a culture medium under conditions such that the host cell synthesizes the multimeric polypeptide; and b) isolating the multimeric polypeptide from the host cell and/or from the culture medium. In some cases, the second polypeptide comprises an affinity tag, and wherein said isolating comprises contacting the multimeric polypeptide produced by the cell with a binding partner for the affinity tag, wherein the binding partner is immobilized, thereby immobilizing the multimeric polypeptide. In some cases, the method comprises eluting the immobilized multimeric polypeptide.

The present disclosure provides a method of selectively modulating the activity of an epitope-specific T cell, the method comprising contacting the T cell with a multimeric polypeptide as describe above or elsewhere herein, wherein said contacting selectively modulates the activity of the epitope-specific T cell. In some cases, the immunomodulatory polypeptide is an activating polypeptide, and wherein the multimeric polypeptide activates the epitope-specific T cell. In some cases, the immunomodulatory polypeptide is an inhibiting polypeptide, and wherein the multimeric polypeptide inhibits the epitope-specific T cell. In some cases, the contacting is carried out *in vitro.* In some cases, the contacting is carried out *in vivo.*

The present disclosure provides a method of selectively modulating the activity of an epitope-specific T cell in an individual, the method comprising administering to the individual an effective amount of a multimeric polypeptide as described above or elsewhere herein effective to selectively modulate the activity of an epitope-specific T cell in an individual. In some cases, the immunomodulatory polypeptide is an activating polypeptide, and wherein the multimeric polypeptide activates the epitope-specific T cell. In some cases, the epitope is a cancer-associated epitope, and wherein said administering selectively increases the activity of a T cell specific for the cancer-associate epitope. In some cases, the immunomodulatory polypeptide is an inhibitory polypeptide, and wherein the multimeric polypeptide inhibits activity of the epitope-specific T cell. In some cases, the epitope is a self-epitope, and wherein said administering selectively inhibits the activity of a T cell specific for the self-epitope.

The present disclosure provides a method of treating an infection in an individual, the method comprising administering to the individual an effective amount of a) a multimeric polypeptide as described above or elsewhere herein; or b) one or more recombinant expression vectors comprising nucleotide sequences encoding the multimeric polypeptide; or c) one or more mRNAs comprising nucleotide sequences encoding the multimeric polypeptide, wherein the epitope is a pathogen-associated epitope, wherein the immunomodulatory polypeptide is an activating polypeptide, and wherein said administering effective to selectively modulate the activity of a pathogen-associated epitope-specific T cell in an individual. In some cases, the pathogen is a virus, a bacterium, or a protozoan. In some cases, the administering is subcutaneous (i.e., the administering is carried out via subcutaneous administration). In some cases, the administering is intravenous (i.e., the administering is carried out via intravenous administration). In some cases, the administering is intramuscular (i.e., the administering is carried out via intramuscular administration). In some cases, the administering is systemic. In some cases, the administering is distal to a treatment site. (i.e., the administering is carried out via subcutaneous administration) the administering is local. (i.e., the administering is carried out via subcutaneous administration) the administering is at or near a treatment site.

The present disclosure provides a composition comprising: a) a multimeric polypeptide as described above or elsewhere herein; and b) a pharmaceutically acceptable excipient.

The present disclosure provides a composition comprising: a) a nucleic acid as described above or elsewhere herein, or a recombinant expression vector as described above or elsewhere herein; and b) a pharmaceutically acceptable excipient.

### BRIEF DESCRIPTION OF THE DRAWINGS

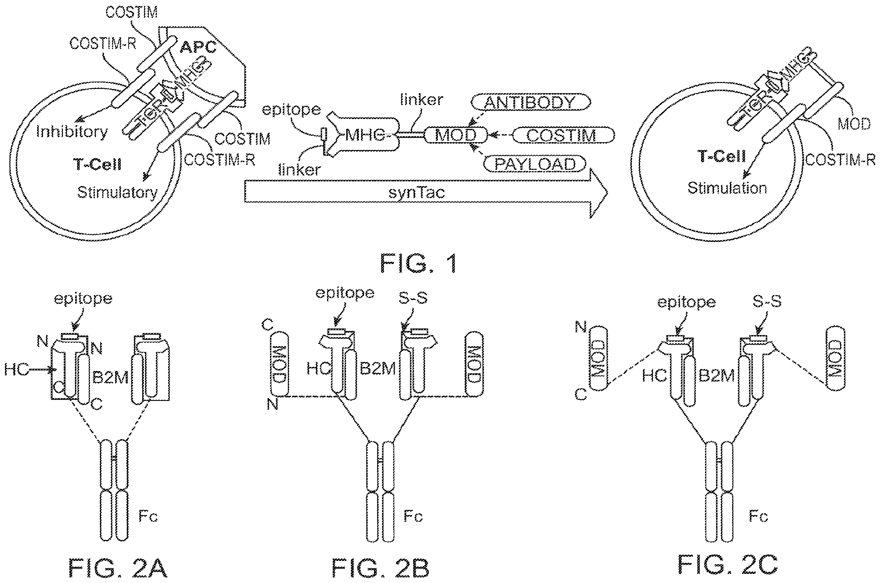

FIG. 1: SynTac: an artificial immunological synapse for T cell activation. The panel on the left depicts the traditional two-signal hypothesis for T cell activation. Namely, targeted T cell engagement through unique TCR:MHC-epitope interactions between the T cell and Antigen Presenting Cell (APC), followed by stimulation or inhibition through costimulatory molecule engagement. The middle panel is a schematic representation of the synTac molecule followed by a mode of action for synTac (Right). Analogous to the natural response (Left), the synTac fusion protein allows for highly specific cell targeting through the MHC-epitope. Following this is a T cell modulatory domain ("MOD") which acts via costimulatory molecule engagement, and can provide for either activation or inhibition. This elicits a clonal, not global, T cell response. Notably, the MOD can be any known or approved antibody, antibody fragment, costimulatory molecule, or other literature validated payload (cytokines, toxins, etc.) as well as new entrants.
FIG. 2A-2C: The synTac Fc-fusion construction. One strategy exploits an Fc-fusion construction to increase the valency, stability and therapeutic window of the associated products. Briefly, the Fc region is a native covalent homo-dimer, formed through interaction of two identical immunoglobulin CH2-CH3 domains (termed Fc) and stabilized through two disulfide bonds between the CH2 domains, illustrated as two thin lines. FIG. 2A shows a single chain peptide MHC protein linked at its carboxy terminus to an IgG Fc region. To introduce alternative protein linkages (such as an MOD), the construct was split into respective heavy and light chains and fuse both peptides and proteins to various ends. One construction, FIG. 2B, results in an amino-terminal association of the peptide to the light chain (beta 2 microglobulin, B2M) followed by a carboxy terminal extension of the light chain to the MOD effector molecule. In this scenario the heavy chain (HLA-molecule, HC) is fused to the Fc region. Constructs are held together covalently through disulfide bridges (labeled as S-S). An alternative orientation, FIG. 2C, places the MOD amino-terminal of the Fc fused heavy chain with the peptide still linked to the B2M light chain.

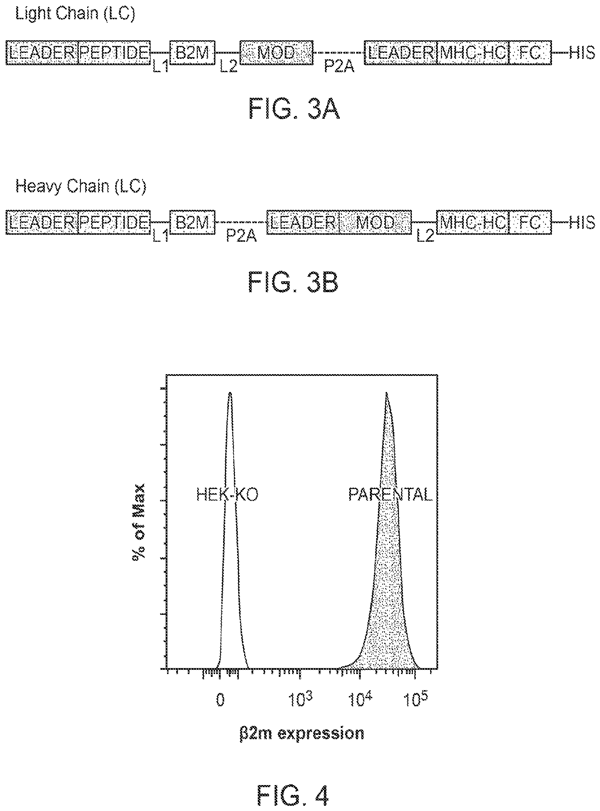

FIG. 3A-3B: The overall design for the two base synTac molecules. This construct utilizes a native human B2M leader sequence (LEADER) to allow for efficient secretion and ER processing immediately followed by a candidate epitope (labeled as PEPTIDE). For the light chain linkage (LC, FIG. 3A), this is coupled to the native B2M molecule through linker L1 and the MOD through linker L2. This entire cassette is linked to another B2M leader sequence, the MHC heavy chain and an IgG1 Fc domain by a viral porcine teschovirus-1 (P2A) "self-cleaving" peptide to allow for stoichiometric expression of each chain. The Heavy Chain (HC, FIG. 3B) linkage is similar however the viral P2A peptide now follows the B2M and the MOD follows the second leader peptide. Both constructs terminate in an 8x His tag.
FIG. 4: CRISPR/CAS mediated Knock-out of endogenous Beta-2-Microglobulin. Guide RNA was designed against endogenous B2M, transfected along with a plasmid encoding CRISPR/CAS and allowed to culture for three days. The cultured cells were surface stained for B2M and counter selected (sorted on loss of fluorescence) by fluorescence activated cell sorting (FACS). The sorted cells were allowed to recover and subjected to two more rounds of staining, counter-sorting and recovery (3 rounds in total) to ensure efficient knock-out. The final pool was quality checked by monitoring B2M surface expression via FACS, shown above.

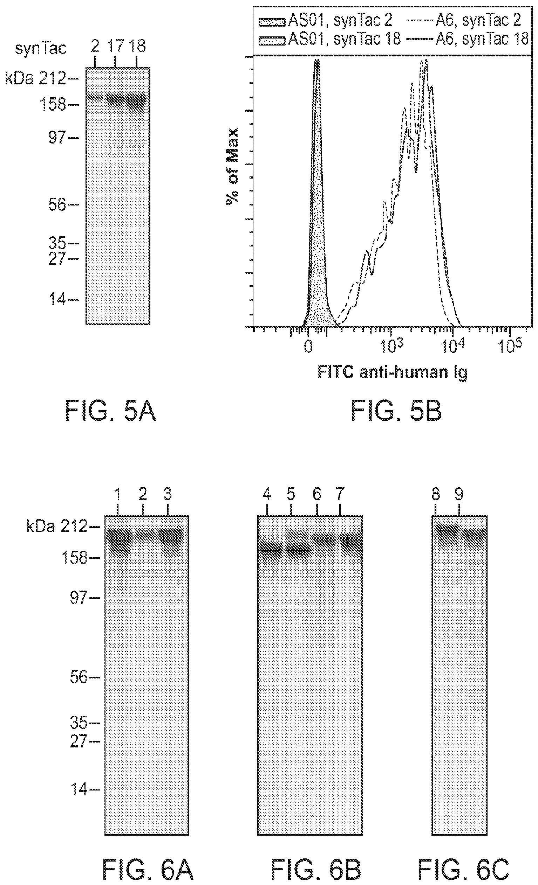

FIG. 5A-5B: Production and activity testing of synTac constructs with engineered disulfide bonds. High-level expression was demonstrated for one construct (H236-L12, labeled as synTac 18) with modest expression for a second (H237-L12, synTac 17). The dt-SCT disulfide schema is used a positive control (labeled as synTac 2). Non-reducing PAGE suggests that the high molecular weight, disulfide linked, moiety was formed as expected (FIG. 5A). All expressing constructs were scaled up to the 100 ml scale, purified and activity tested through binding of cognate TCR expressed on the surface of HEK cells (termed A6), as monitored by FACS fluorescence, suggesting proper folding and activity (FIG. 5B). Cells expressing non-cognate TCR (termed AS01) were used as a negative control.
FIG. 6A-6B: Expression of various synTac protein fusions. Successful expression of (FIG. 6A) light chain linked synTac fusions with various targeting peptides and HLA isotypes with a PD-L1 MOD domain, specifically 1) HTLV-human-HLA-A02, 2) IGRP-murine H2-Kd and 3) TUM-murine H2-Kd, (FIG. 6B) IGRP based synTac fusion bearing various MOD domains, 4) the Ig variable domain of PD-L1, 5) 4-1BBL, 6) anti-CD28 single chain Fv, and 7) B7-1W88A, (FIG. 6C) IGRP based synTac fusions expressed as a heavy chain linkage, bearing various MODS, 8) PD-L1 and 9) anti-CD28 single chain Fv.

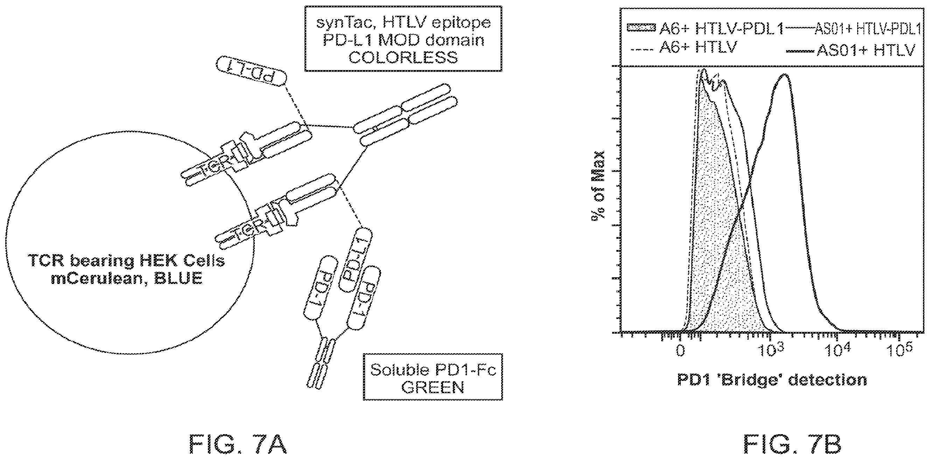

FIG. 7A-7B: TCR-synTac-PD1 Bridging: validating the integrity of the synTac protein components. HEK cells expressing a cognate TCR (A6) were used as a positive control and cells expressing a non-cognate TCR (AS01) were generated and used as a negative control along with untransduced parental cells. Cells were challenged with non-fluorescent purified HTLV-PD-L1 synTac variants and incubated with its cognate receptor PD1 fused to murine IgG2a. The PD1-Fc fusion was detected using a FITC labeled anti-mouse secondary antibody. A schematic of the reaction is illustrated in FIG. 7A, FACs results shown in FIG. 7B. As expected, co-localized fluorescence was only observed when HTLV presented synTac WITH a PD-L1 MOD was challenged against cognate (A6) HEK cell lines. Of note, this was not observed when challenged against non-cognate TCR bearing HEK cells or parental cells, when challenged against FITC-PD1-Fc only or when the MOD was absent.

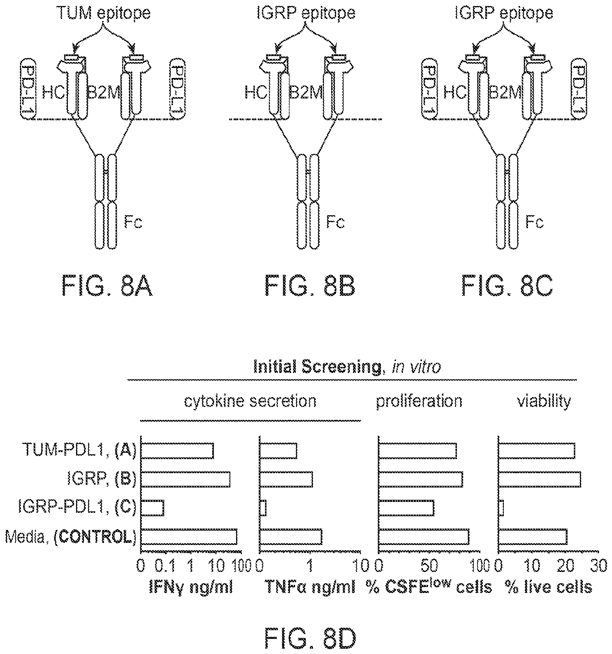

FIG. 8A-8D: SynTac in action: *in vitro* T cell assays. CD8+ T cells from 8.3 transgenic NOD mice were cultures in the presence of immobilized anti-CD3 antibody to stimulate polyclonal T cell activation. Stimulated cultures were treated with soluble versions of either synTac TUM-PD-L1 (FIG. 8A) or synTac IGRP-PD-L1 (FIG. 8C) to examine the antigen specificity of any suppressive effect. A version of synTac IGRP without PD-L1 (FIG. 8B) served as an effector control for the MOD domain. Before seeding, cells were labeled with carboxyfluorescein succinimidyl ester (CFSE) in order to monitor the extent of T cell activation-induced cellular proliferation. Cells were harvested at 5 days and examined using flow cytometry for viability and proliferation. Supernatants were also examined for the expression of the CD8+ T cell effector cytokines IFNγ and TNFα using a multiplexed flow cytometric bead assay. All CD8+ T cell activation parameters examined were suppressed in an antigen-specific and effector (i.e. MOD) domain-dependent manner (FIG. 8D).

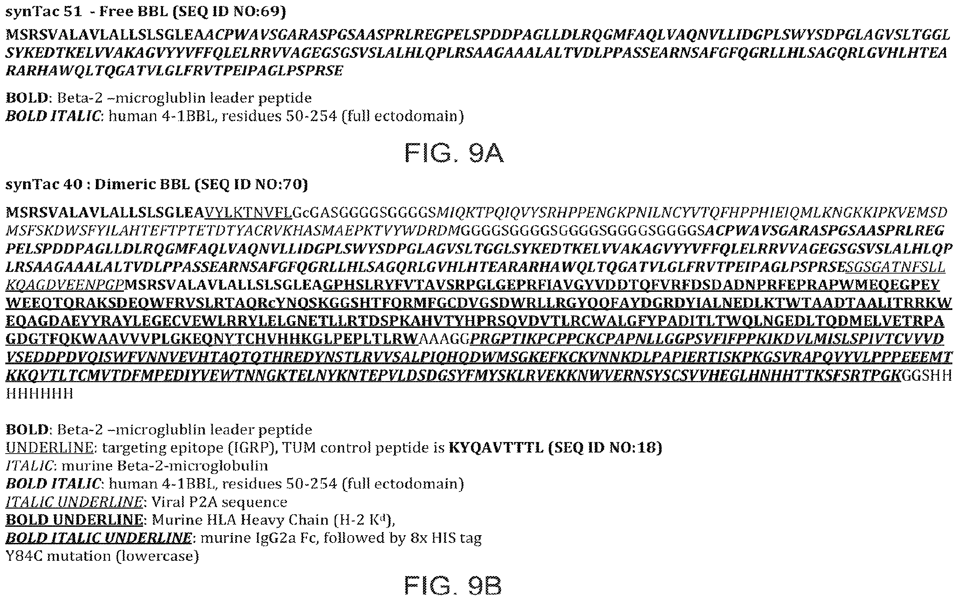

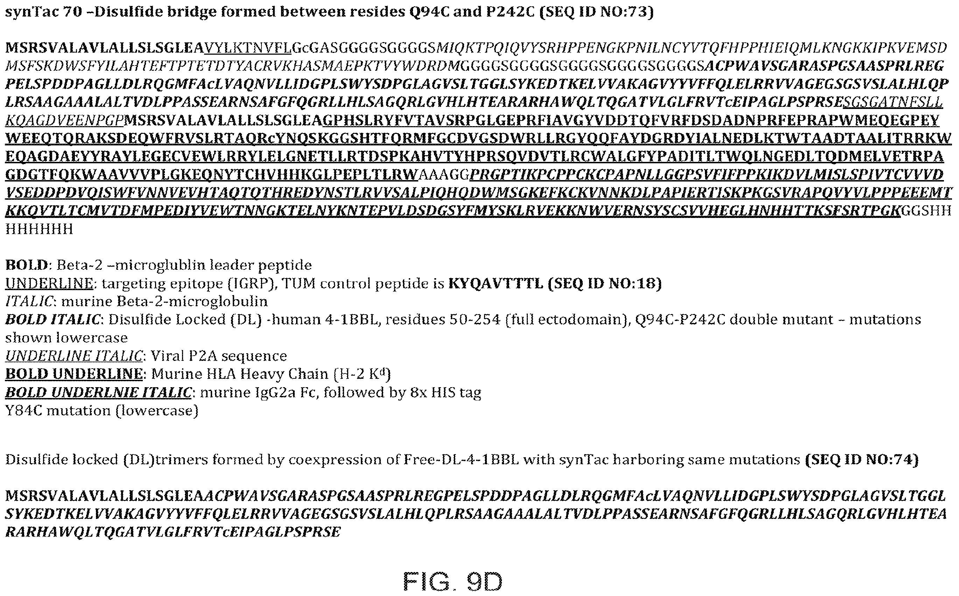

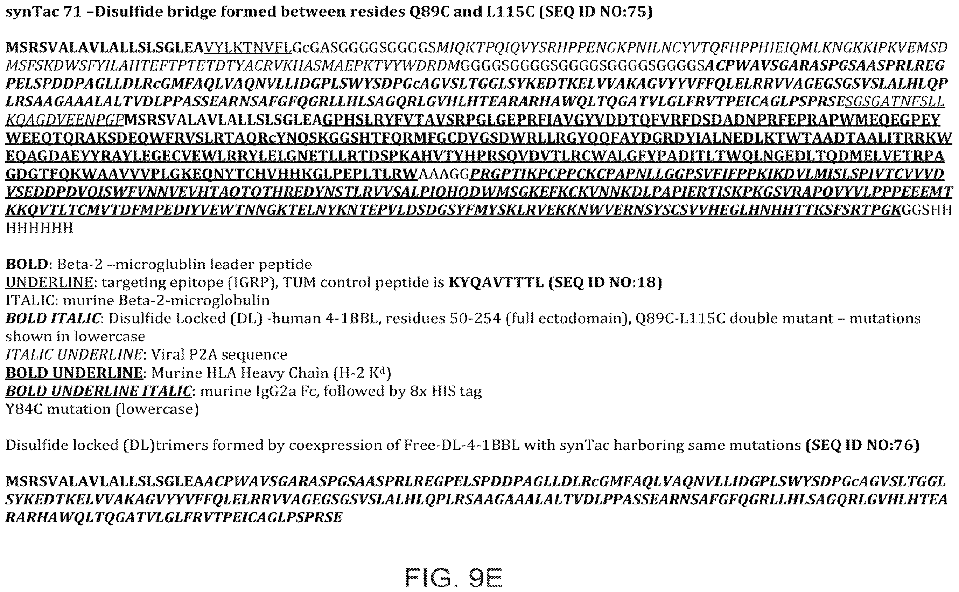

FIG. 9A-9F provide amino acid sequences and domain structure of synTac polypeptides.

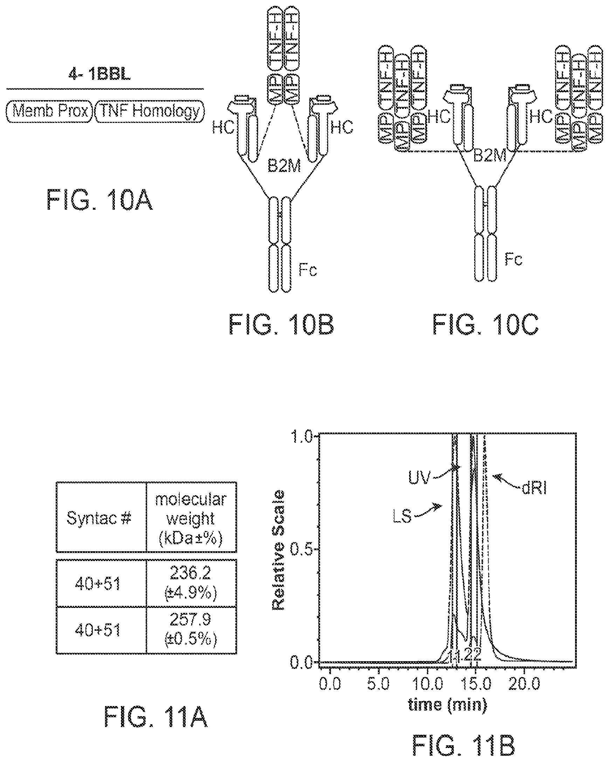

FIG. 10A-10C depict constructs for *4-1BBL trimeric expression.* Cartoon representation of (FIG. 10A) monomeric form of the native 4-1BBL ectodomain (residues 50-254), showing membrane proximal (Memb Prox, MP) and the TNF homology (TNF-H) domains, (FIG. 10B) 4-1BBL dimeric synTac, and (FIG. 10C) fully active dual trimeric form of 4-1BBL synTac generated through coexpression of traditional synTac constructs with a "free" from of 4-1BBL ecto-domain (residues 50-254, FIG. 10A) having no affinity tag. All constructs assemble when expressed together in mammalian cells. Purification proceeds through the Fc region (protein A/G) followed by size exclusion, allowing for separation of 4-1BBL trimeric synTac from free BBL.
FIG. 11A-11B. Multiangle light scattering (MALS) analysis of trimers 4-1BBL bearing synTac proteins. (FIG. 11A) Molecular weight of major species identified through MALS, showing examples of multiple independent measurements. (FIG. 11B) Representative traces from MALS of synTac 40+51, with relatively high levels of light scattering and low UV absorption, reflecting the presence of a small amount of protein with a high molecular weight. Low molecular weight buffer components result in large changes in refractive index (either positive or negative) without associated change in UV absorbance.

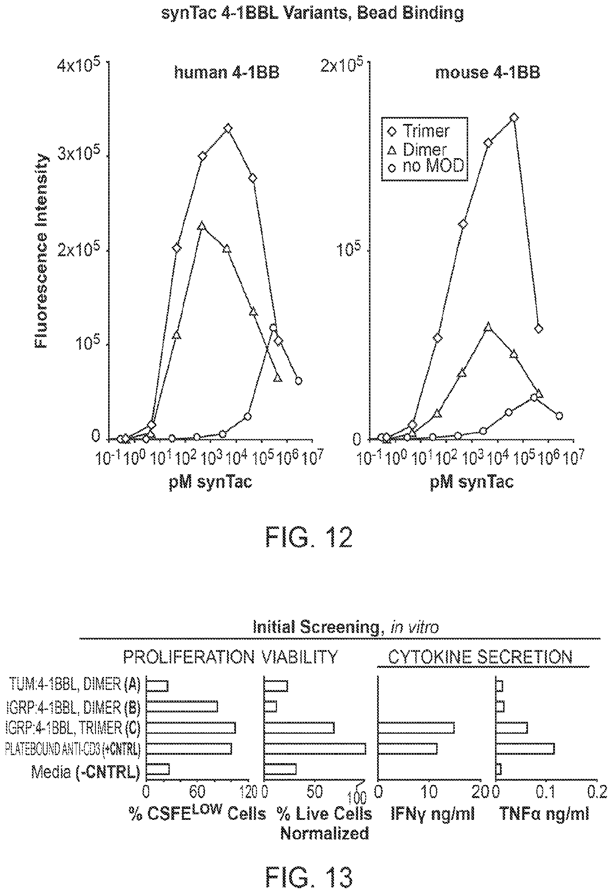

FIG. 12. SynTac 4-1BBL receptor binding. Protein A microbeads were coated to saturation with recombinant human or mouse 4-1BB-Fc fusion protein and used to bind synTac constructs bearing 4-1BB ligand (dimer and trimer) as the co-modulatory domain, followed by a fluorescent detection antibody specific for the synTac heavy chain isotype. The extent of specific binding of synTac 4-1BBL to bead-borne 4-1BB was then measured by high throughput flow cytometry. Using this system, the degree of cross reactivity and relative affinities of 4-1BBL for both human and murine 4-1BB was explored in the context of the synTac scaffold. 4-1BBL bearing synTacs were shown to bind cognate receptor, but not "receptor-less" (termed no MOD) Fc bound microbeads, suggesting a well-folded and active protein reagent. Notably, the trimer bound in an affinity range expected for dual trimeric engagement with the original dimer showing a 10 fold reduction in binding affinity and all constructs cross react between murine and human receptors.
FIG. 13. CD8⁺ T cells from 8.3 transgenic NOD mice were cultured in the presence of immobilized anti-CD3 antibody to stimulate polyclonal T cell activation. Stimulated cultures were treated with soluble versions of either synTac TUM-41BBL (A) or synTac IGRP-41BBL (B and C) to examine the antigen specificity of any stimulatory effect. Control treatments were media alone (- CNTRL) or immobilized anti-CD3 (+ CNTRL) to benchmark response magnitude. Cells were labeled with carboxyfluorescein succinimidyl ester (CFSE) in order to monitor the extent of T cell activation-induced cellular proliferation. After 4 days, the cells were harvested and examined using flow cytometry for viability and proliferation. Supernatants were also examined for the expression of the CD8⁺ T cell effector cytokines IFNγ and TNFα using a multiplexed flow cytometric bead assay. All CD8⁺ T cell activation parameters examined were activated in an antigen-specific and effector (i.e. MOD) domain-dependent manner.

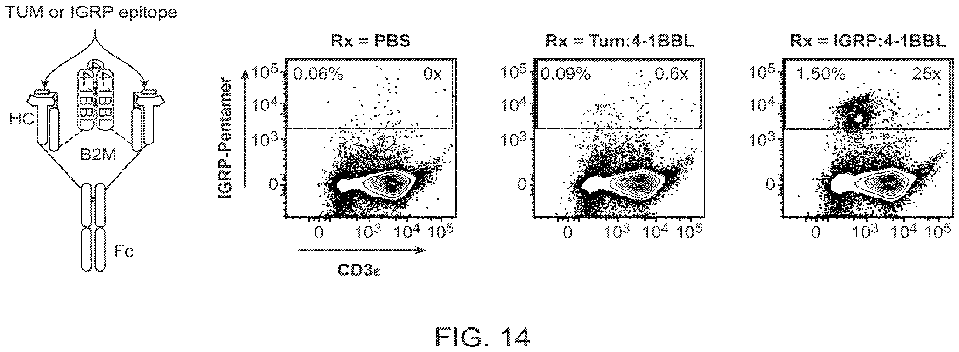

FIG. 14. Single Dose *in vivo* T cell stimulation assays. NOD mice were injected intraperitoneally with synTac IGRP-41BBL, synTac TUM-41BBL or PBS. Six days post injection, the mice were sacrificed and splenocytes were examined via flow cytometry for relative frequencies of IGRP-specific CD8 T cells using an appropriate peptide-MHC pentamer stain. IGRP-41BBL treatment was associated with a much higher frequency of IGRP-specific CD8 T cells versus controls, supporting a significant *in vivo* expansion from a single dose.

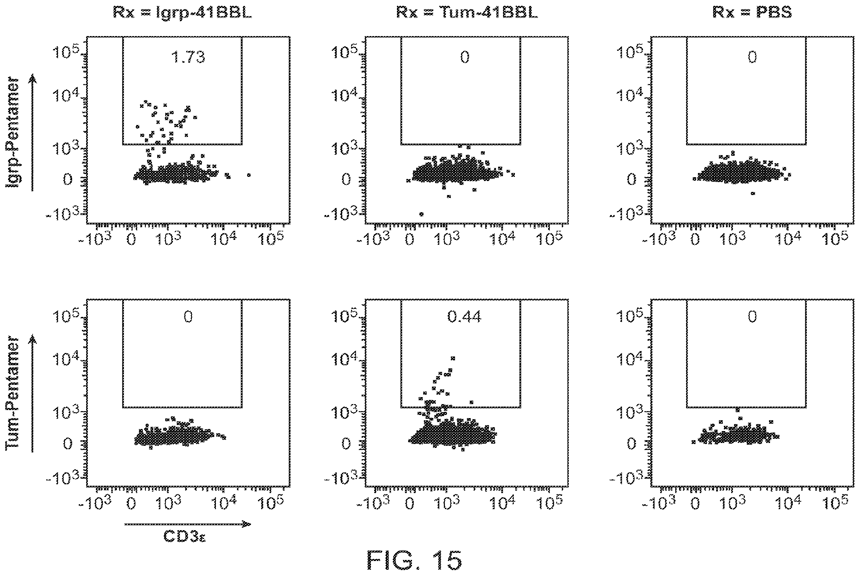

FIG. 15. Multi Dose *in vivo* T cell stimulation assays. NOD mice were injected intraperitoneally with synTac IGRP-41BBL, synTac TUM-41BBL or PBS for three doses over two weeks. Seven days post injection, the mice were sacrificed and PBMC's (from blood) were examined via flow cytometry for relative frequencies of IGRP-specific CD8 T cells using an appropriate peptide-MHC pentamer stain. IGRP-41BBL treatment was associated with a higher frequency of IGRP-specific CD8 T cells versus controls, supporting a significant *in vivo* expansion from a multiple doses, including rare-tumor specific T cells (TUM).

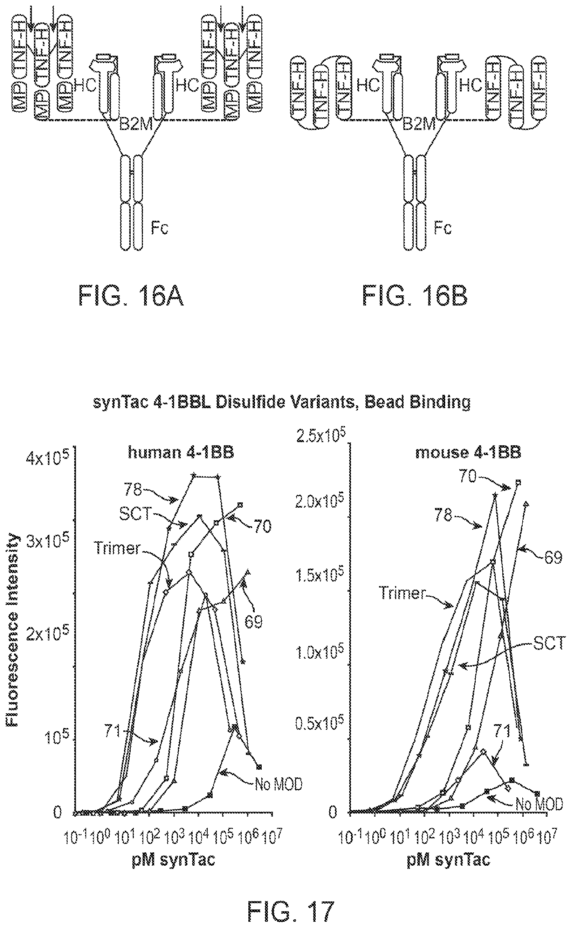

FIG. 16A-16B. Schematics of optimized constructs for 4-1BBL trimeric expression. Disulfide locking (FIG. 16A, DL) and single chain trimers (FIG. 16B, SCT).
FIG. 17. SynTac 4-1BBL receptor binding. Protein A microbeads were coated to saturation with recombinant human or mouse 4-1BB-Fc fusion protein and used to bind synTac constructs bearing 4-1BB ligand (Disulfide Locked trimers (69, 70 and 71) and Single Chain trimer (SCT) as the co-modulatory domain, followed by a fluorescent detection antibody specific for the synTac heavy chain isotype. The Native trimer shown is a binding control (Trimer). The extent of specific binding of synTac 4-1BBL to bead-borne 4-1BB was then measured by high throughput flow cytometry. 4-1BBL bearing synTacs were shown to bind cognate receptor, but not "receptor-less" (termed "no MOD") Fc bound microbeads, suggesting a well-folded and active protein reagent. All constructs cross react between murine and human receptors.

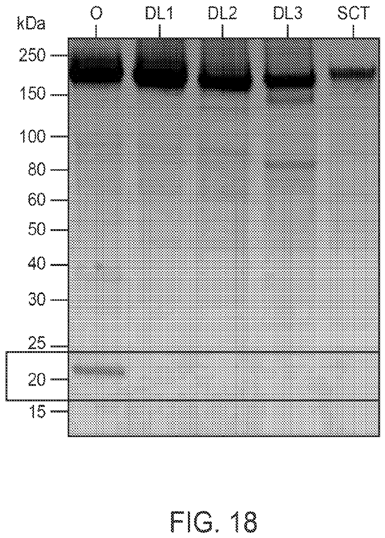

FIG. 18. Expression Validation of optimized 4-1BBL constructs. SynTac's produced by co-expression, with the original 4-1BBL modulator (synTac 40/51, with no disulfide lock, labeled as "O" (for original)) and three optimized constructs containing engineered disulfide locks restraining the trimer conformation. Two native residues in each construct were replaced for cysteine residues (Q94C:P245C (labeled as "DL1" in gel), Q94C:P242C "DL2", and Q89C:L115C "DL3", termed synTac 69, 70 and 71 respectively), co-expressed in human cells with a "free" non tagged version harboring the same mutations (termed 98,99,100 respectively) to allow for covalent locking in the cell. The degree of disulfide bonding was observed by amount of released (non-covalently bound) "free" 4-1BBL in non-reduced SDS PAGE analysis. Free-BBL would migrate at -20 kDa (BOX), confirming disulfide locking of engineered constructs. SynTac carrying a single-chain-trimer version (SCT) of 4-1BBL is also shown following affinity and gel filtration purification (labeled as "SCT"). Accurate mass confirmed by multi angle light scattering (MALS).

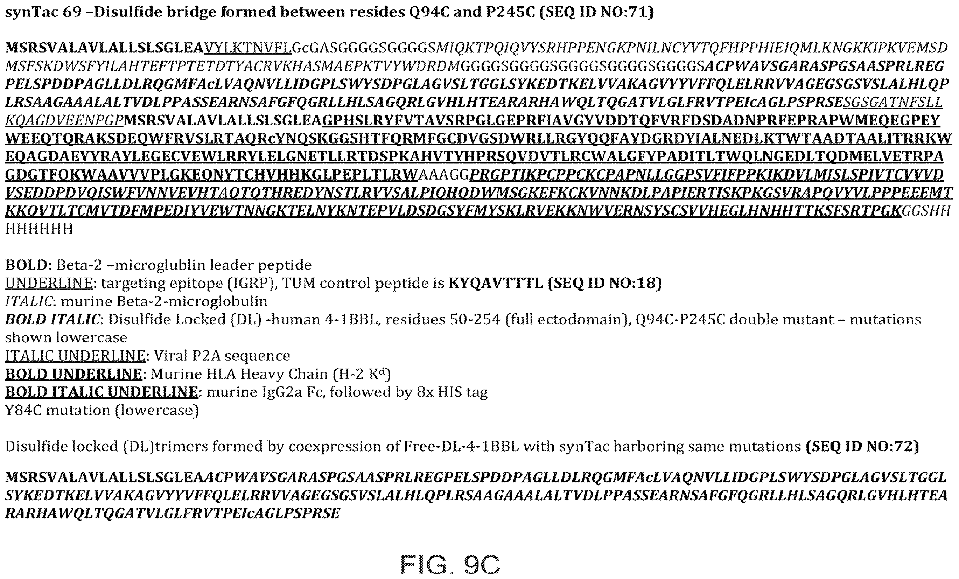

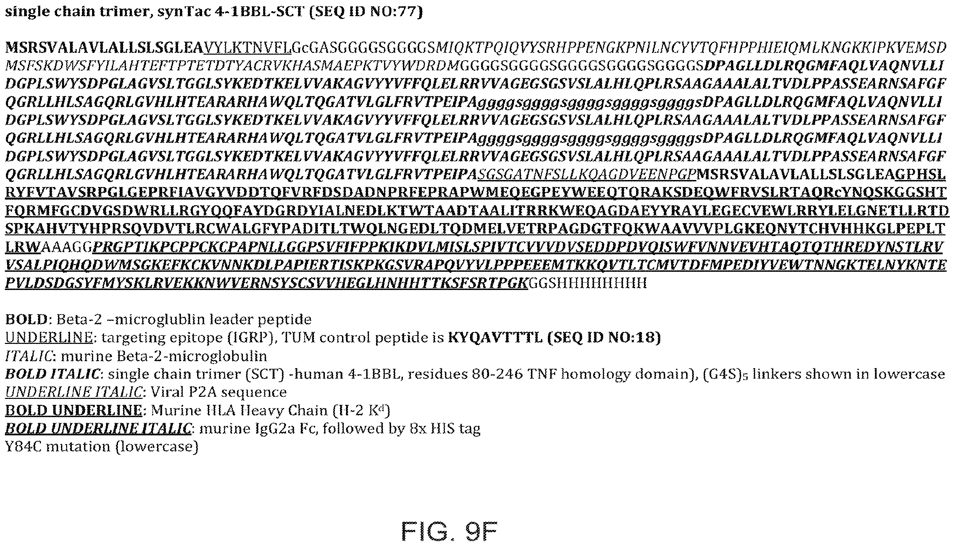

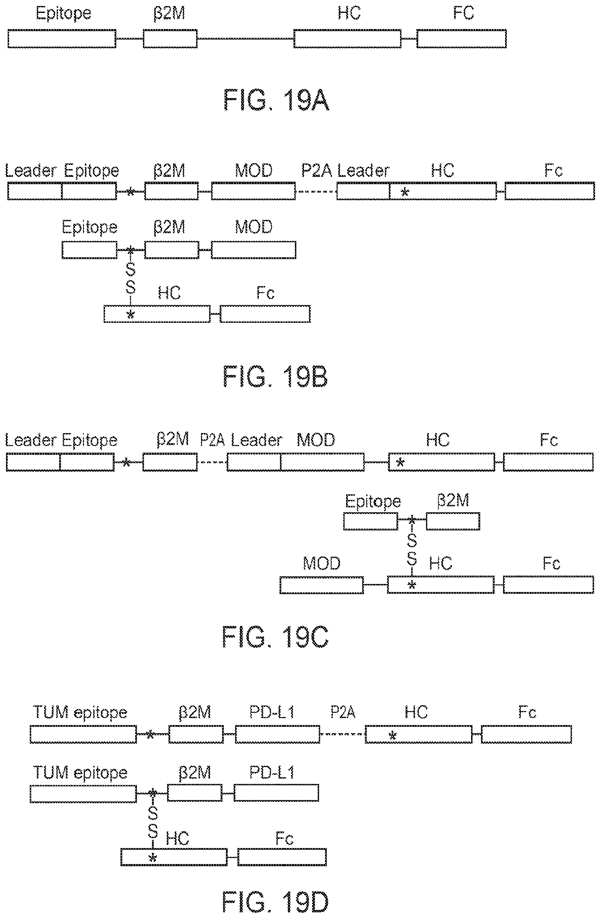

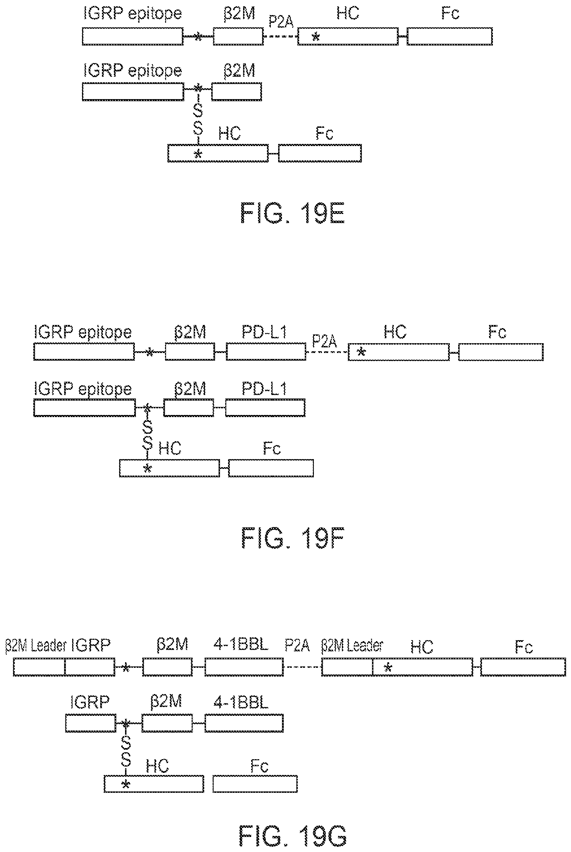

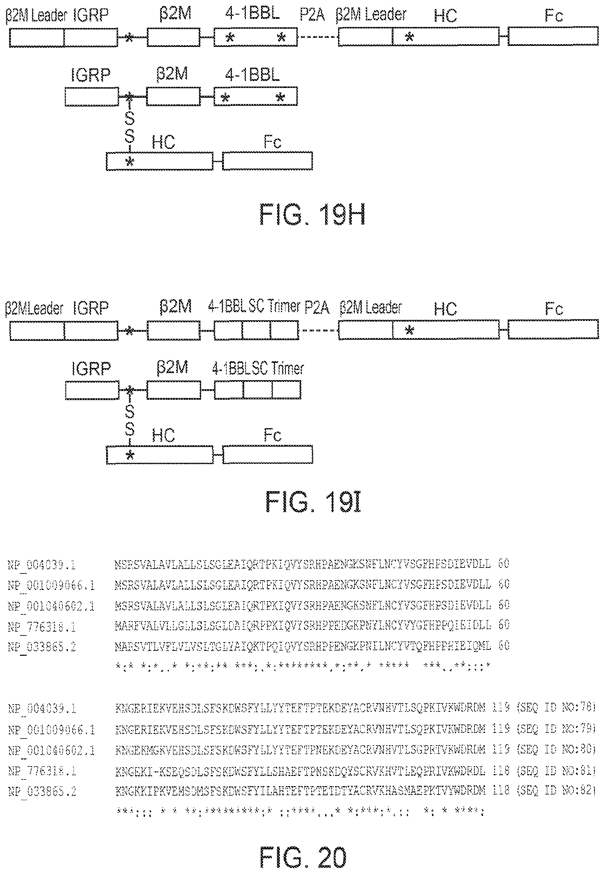

FIG. 19A-19I. Schematic depictions of embodiments of synTac constructs of the present disclosure. FIG. 19A-19C depict constructs described in relation to FIG. 2A-2C respectively; in FIG. 19B and 19C the P2A uncleaved polypeptide is depicted (top) and the cleaved polypeptide (through P2A-mediated self-cleavage) is depicted (bottom) with disulfide bonding (SS), mediated by cysteine substitution (*), illustrated. FIG. 19D-19F depict constructs described above in relation to FIG. 8A-8C respectively; in each of FIG. 19D-19F the P2A uncleaved form is depicted above (top) the P2A-mediated self-cleaved polypeptide (bottom) with disulfide bonding (SS), mediated by cysteine substitution (*), illustrated. FIG. 19G depicts a generalized version of the synTac40 construct in relationship to FIG. 9B with the uncleaved (top) and self-cleaved/disulfide bonded (bottom) polypeptides illustrated. FIG 19H depicts a generalized version of synTac69, synTac70 and synTac71 in relationship to FIG. 9C-9E, the uncleaved (top) and self-cleaved/disulfide bonded (bottom) polypeptides are illustrated and additional cysteine substitutions in the 4-1BBL domain are also indicated (*). FIG. 19I depicts a generalized version of the synTac 4-1BBL single chain trimer (SCT) in relationship to FIG. 9F, the uncleaved (top) and self-cleaved/disulfide bonded (bottom) polypeptides are illustrated.
FIG. 20 provides an multiple amino acid sequence alignment of beta-2 microglobulin (B2M) precursors (i.e., including the leader sequence) from *Homo sapiens* (NP_004039.1; SEQ ID NO:78), *Pan troglodytes* (NP_001009066.1; SEQ ID NO:79), *Macaca mulatta* (NP_001040602.1; SEQ ID NO:80), Bos Taurus (NP_776318.1; SEQ ID NO:81) and Mus musculus (NP_033865.2; SEQ ID NO:82).

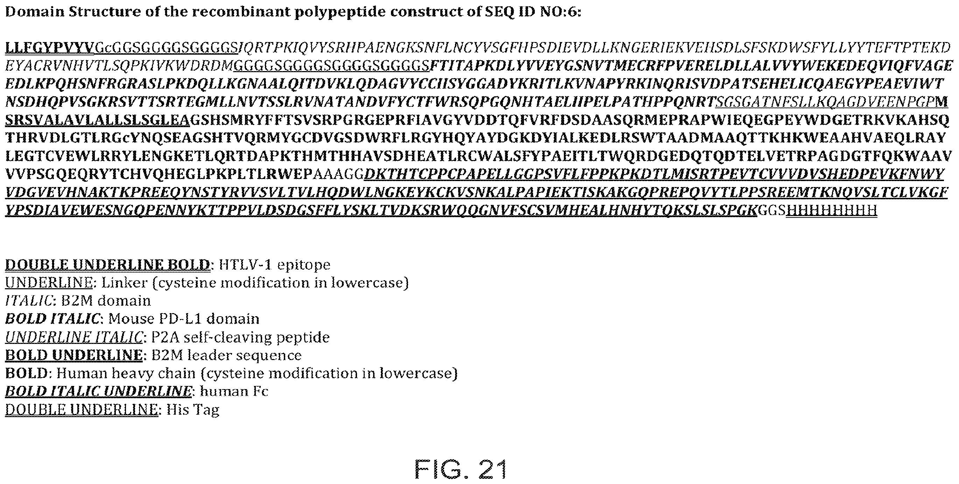

FIG. 21 provides the domain structure of the construct of SEQ ID NO:6.

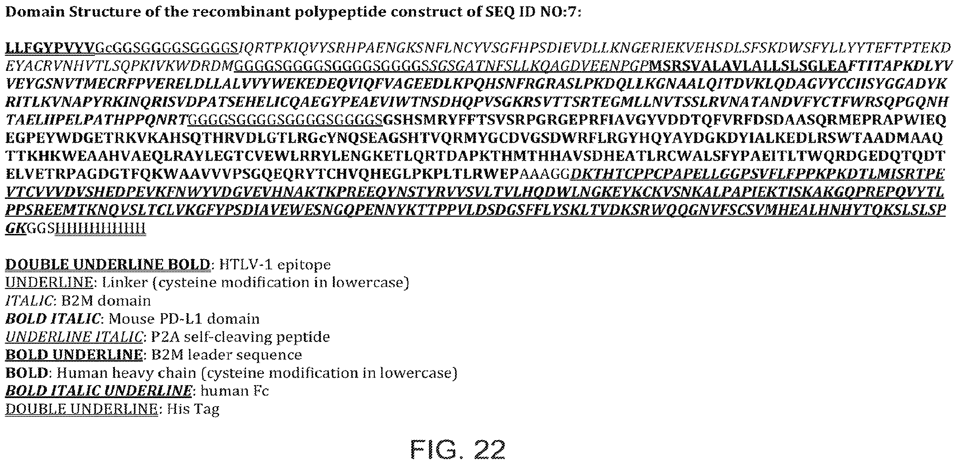

FIG. 22 provides the domain structure of the construct of SEQ ID NO:7.
FIG. 23 depicts the effect of *in vivo* administration of synTac IGRP-PDL1, synTac TUM-PDL1, or phosphate-buffered saline (PBS) on the frequency of IGRP-specific CD8⁺ T cells.
FIG. 24A-24C provide amino acid sequences of immunoglobulin Fc polypeptides.

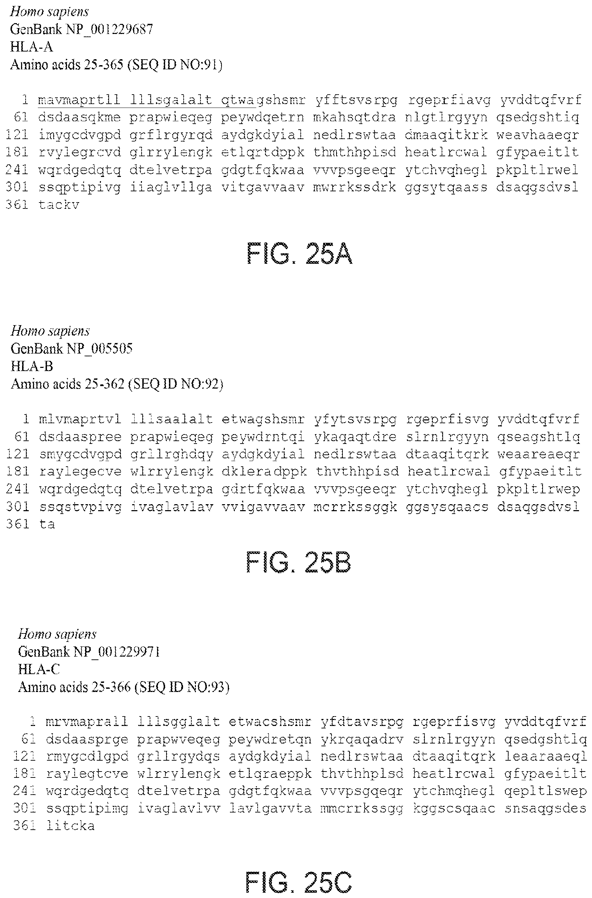

FIG. 25A-25C provide amino acid sequences of human leukocyte antigen (HLA) Class I heavy chain polypeptides.

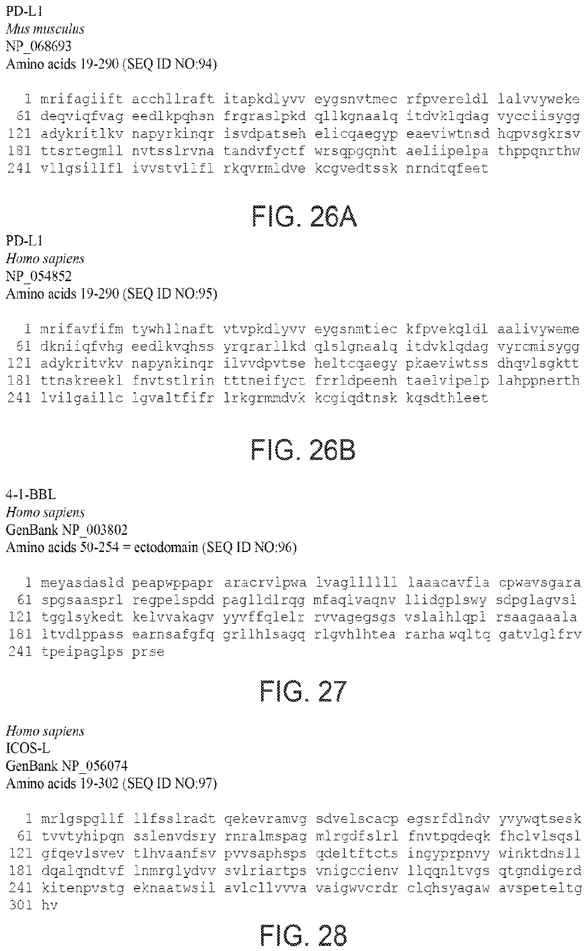

FIG. 26A-26B provide amino acid sequences of PD-L1 polypeptides.
FIG. 27 provides an amino acid sequence of a 4-1BBL polypeptide.
FIG. 28 provides an amino acid sequence of an ICOS-L polypeptide.

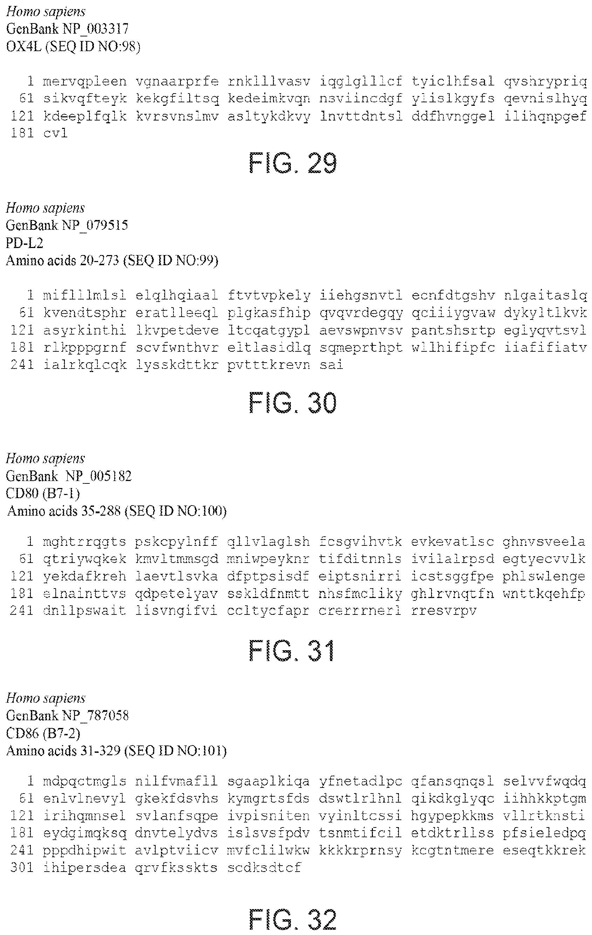

FIG. 29 provides an amino acid sequence of an OX40L polypeptide.
FIG. 30 provides an amino acid sequence of a PD-L2 polypeptide.
FIG. 31 provides an amino acid sequence of a CD80 (B7-1) polypeptide.
FIG. 32 provides an amino acid sequence of a CD86 (B7-2) polypeptide.

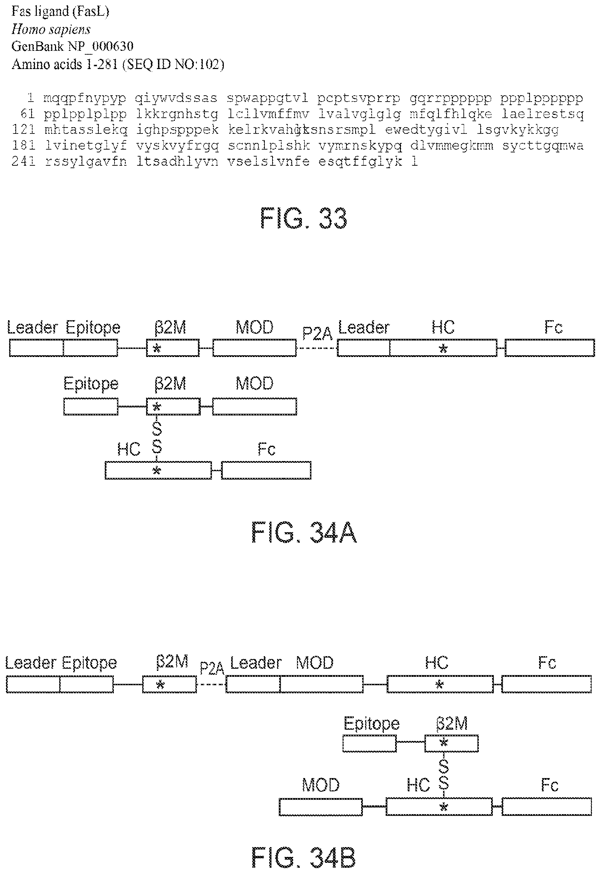

FIG. 33 provides an amino acid sequence of a Fas ligand (FAS-L) polypeptide.

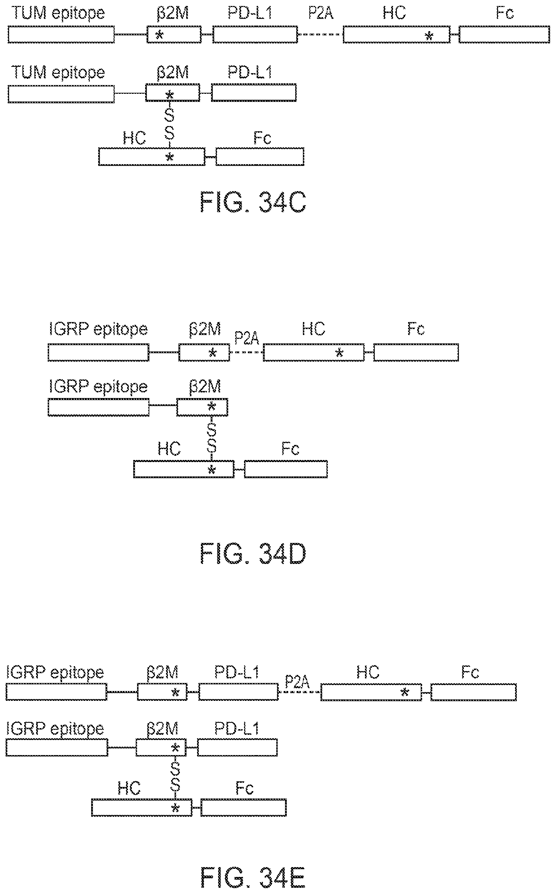

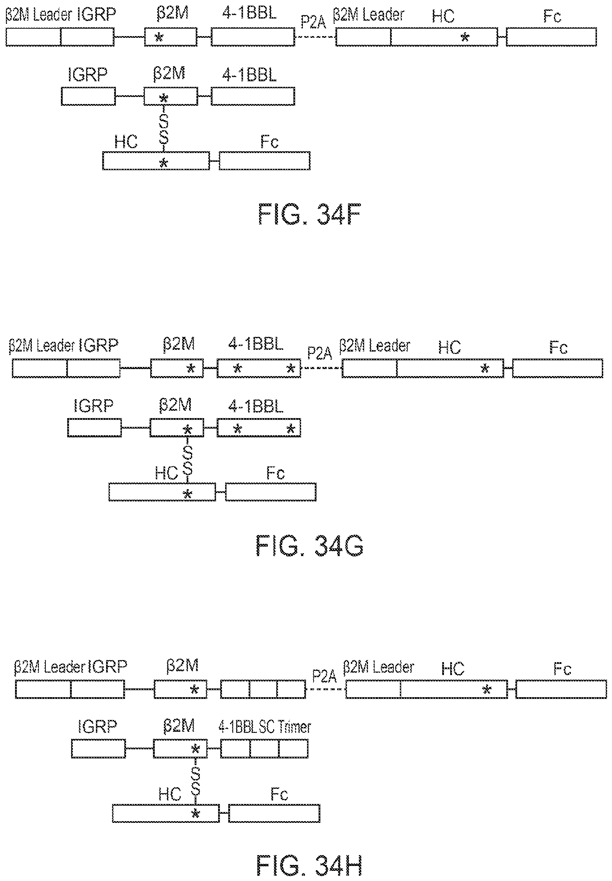

FIG. 34A-34H provide schematic depictions of embodiments of synTac constructs of the present disclosure, where disulfide bonding (SS), mediated by cysteine substitution (*), is illustrated. In these embodiments, disulfide bonds are formed between MHC (e.g., HLA) polypeptides present in separate polypeptides.

### DEFINITIONS

A "leader sequence" as used herein includes any signal peptide that can be processed by a mammalian cell, including the human B2M leader. Such sequences are well-known in the art.

As used herein, "contiguous with" with regard to, for example, element A and element B, means element A is adjacent to element B and bonded to element B, preferably, unless otherwise specified, via a covalent bond. For example, for a first sequence of amino acids contiguous with a second sequence of amino acids, the C-terminal of the first sequence of amino acids can be joined by a peptide bond to the N-terminal of the second sequence of amino acids.

The terms "peptide," "polypeptide," and "protein" are used interchangeably herein, and refer to a polymeric form of amino acids of any length, which can include coded and non-coded amino acids, chemically or biochemically modified or derivatized amino acids, and polypeptides having modified peptide backbones. The terms also include polypeptides that have co-translational (e.g., signal peptide cleavage) and post- translational modifications of the polypeptide, such as, for example, disulfide-bond formation, glycosylation, acetylation, phosphorylation, proteolytic cleavage, and the like. Furthermore, as used herein, a "polypeptide" refers to a protein that includes modifications, such as deletions, additions, and substitutions (generally conservative in nature as would be known to a person in the art) to the native sequence, as long as the protein maintains the desired activity. These modifications can be deliberate, as through site-directed mutagenesis, or can be accidental, such as through mutations of hosts that produce the proteins, or errors due to PCR amplification or other recombinant DNA methods.

The term "recombinant", as used herein to describe a nucleic acid molecule, means a polynucleotide of genomic, cDNA, viral, semisynthetic, and/or synthetic origin, which, by virtue of its origin or manipulation, is not associated with all or a portion of the polynucleotide sequences with which it is associated in nature. The term "recombinant," as used with respect to a protein or polypeptide, refers to a polypeptide produced by expression from a recombinant polynucleotide. The term "recombinant," as used with respect to a host cell or a virus, refers to a host cell or virus into which a recombinant polynucleotide has been introduced. Recombinant is also used herein to refer to, with reference to material (e.g., a cell, a nucleic acid, a protein, or a vector) that the material has been modified by the introduction of a heterologous material (e.g., a cell, a nucleic acid, a protein, or a vector).

The terms "polynucleotide," "oligonucleotide," "nucleic acid" and "nucleic acid molecule" are used interchangeably herein to include a polymeric form of nucleotides, either ribonucleotides or deoxyribonucleotides. This term refers only to the primary structure of the molecule. Thus, the terms include triple-, double- and single-stranded DNA, as well as triple-, double- and single-stranded RNA. The terms also include such molecules with modifications, such as by methylation and/or by capping, and unmodified forms of a polynucleotide. More particularly, the terms "polynucleotide," "oligonucleotide," "nucleic acid" and "nucleic acid molecule" include polydeoxyribonucleotides (containing 2-deoxy-D-ribose), polyribonucleotides (containing D-ribose), any other type of polynucleotide which is an N- or C-glycoside of a purine or pyrimidine base, and other polymers containing non-nucleotidic backbones, polymers, and other synthetic sequence-specific nucleic acid polymers providing that the polymers contain nucleobases in a configuration which allows for base pairing and base stacking, such as is found in DNA and RNA.

The term "vector" as used herein refers a vehicle capable of transferring nucleic acid sequences to target cells. For example, a vector may comprise a coding sequence capable of being expressed in a target cell. As used herein, "vector construct," "expression vector," and "gene transfer vector," generally refer to any nucleic acid construct capable of directing the expression of a gene of interest and which is useful in transferring the gene of interest into target cells. Thus, the term includes cloning and expression vehicles, as well as integrating vectors and non-integrating vectors. Vectors are thus capable of transferring nucleic acid sequences to target cells and, in some instances, are used to manipulate nucleic acid sequence, e.g., recombine nucleic acid sequences (i.e. to make recombinant nucleic acid sequences) and the like. For purposes of this disclsosure examples of vectors include, but are not limited to, plasmids, phage, transposons, cosmids, virus, and the like.

An "expression cassette", as used herein, comprises any nucleic acid construct capable of directing the expression of any RNA transcript including gene/coding sequence of interest as well as non-translated RNAs. Such cassettes can be constructed into a "vector," "vector construct," "expression vector," or "gene transfer vector," in order to transfer the expression cassette into target cells. Thus, the term includes cloning and expression vehicles, as well as viral vectors. A transcript of an expression cassette may be expressed stably or transiently and may be expressed from a cassette that integrates into the host genome (in a targeted or untargeted manner) or remain non-integrated as desired.

"Operably linked" refers to a juxtaposition wherein the components so described are in a relationship permitting them to function in their intended manner. For instance, a promoter is operably linked to a coding sequence if the promoter affects its transcription or expression. As used herein, the terms "heterologous promoter" and "heterologous control regions" refer to promoters and other control regions that are not normally associated with a particular nucleic acid in nature. For example, a "transcriptional control region heterologous to a coding region" is a transcriptional control region that is not normally associated with the coding region in nature.

The term "immunological synapse" or "immune synapse" as used herein generally refers to the natural interface between two interacting immune cells of an adaptive immune response including, e.g., the interface between an antigen-presenting cell (APC) or target cell and an effector cell, e.g., a lymphocyte, an effector T cell, a natural killer cell, and the like. An immunological synapse between an APC and a T cell is generally initiated by the interaction of a T cell antigen receptor and major histocompatibility complex molecules, e.g., as described in Bromley et al., Annu Rev Immunol. 2001;19:375-96.

As used herein, the term "heterologous" used in reference to nucleic acid sequences, proteins or polypeptides, means that these molecules are not naturally occurring in the cell from which the heterologous nucleic acid sequence, protein or polypeptide was derived. For example, the nucleic acid sequence coding for a human polypeptide that is inserted into a cell that is not a human cell is a heterologous nucleic acid sequence in that particular context. Whereas heterologous nucleic acids may be derived from different organism or animal species, such nucleic acid need not be derived from separate organism species to be heterologous. For example, in some instances, a synthetic nucleic acid sequence or a polypeptide encoded therefrom may be heterologous to a cell into which it is introduced in that the cell did not previously contain the synthetic nucleic acid. As such, a synthetic nucleic acid sequence or a polypeptide encoded therefrom may be considered heterologous to a human cell, e.g., even if one or more components of the synthetic nucleic acid sequence or a polypeptide encoded therefrom was originally derived from a human cell.

A "host cell," as used herein, denotes an *in vivo* or *in vitro* eukaryotic cell or a cell from a multicellular organism (e.g., a cell line) cultured as a unicellular entity, which eukaryotic cells can be, or have been, used as recipients for a nucleic acid (e.g., an expression vector that comprises a nucleotide sequence encoding a multimeric polypeptide of the present disclosure), and include the progeny of the original cell which has been genetically modified by the nucleic acid. It is understood that the progeny of a single cell may not necessarily be completely identical in morphology or in genomic or total DNA complement as the original parent, due to natural, accidental, or deliberate mutation. A "recombinant host cell" (also referred to as a "genetically modified host cell") is a host cell into which has been introduced a heterologous nucleic acid, e.g., an expression vector. For example, a genetically modified eukaryotic host cell is genetically modified by virtue of introduction into a suitable eukaryotic host cell a heterologous nucleic acid, e.g., an exogenous nucleic acid that is foreign to the eukaryotic host cell, or a recombinant nucleic acid that is not normally found in the eukaryotic host cell.

In some instances, nucleic acid or amino acid sequences, including polypeptides and nucleic acids encoding polypeptides, are referred to based on "sequence similarity" or "sequence identity", e.g., as compared to one or more reference sequences. In other instances, a mutant or variant sequence may be referred to based on comparison to one or more reference sequences. For sequence comparison, typically one sequence acts as a reference sequence, to which test sequences are compared. When using a sequence comparison algorithm, test and reference sequences are input into a computer, subsequence coordinates are designated, if necessary, and sequence algorithm program parameters are designated. The sequence comparison algorithm then calculates the percent sequence identity for the test sequence(s) relative to the reference sequence, based on the designated program parameters.

Where necessary or desired, optimal alignment of sequences for comparison can be conducted, for example, by the local homology algorithm of Smith and Waterman (Adv. Appl. Math. 2:482 (1981),, by the homology alignment algorithm of Needleman and Wunsch (J. Mol. Biol. 48:443-53 (1970),, by the search for similarity method of Pearson and Lipman (Proc. Natl. Acad. Sci. USA 85:2444-48 (1988),, by computerized implementations of these algorithms (e.g., GAP, BESTFIT, FASTA, and TFASTA in the Wisconsin Genetics Software Package, Genetics Computer Group, 575 Science Dr., Madison, Wis.), or by visual inspection. (See generally Ausubel et al. (eds.), Current Protocols in Molecular Biology, 4th ed., John Wiley and Sons, New York (1999)).

"T cell" includes all types of immune cells expressing CD3, including T-helper cells (CD4⁺ cells), cytotoxic T-cells (CD8⁺ cells), T-regulatory cells (Treg), and NK-T cells.

"Co-stimulatory ligand," as the term is used herein, includes a molecule on an antigen presenting cell (e.g., an APC, dendritic cell, B cell, and the like) that specifically binds a cognate co-stimulatory molecule on a T cell, thereby providing a signal which, in addition to the primary signal provided by, for instance, binding of a TCR/CD3 complex with an MHC molecule loaded with peptide, mediates a T cell response, including, but not limited to, proliferation, activation, differentiation, and the like. A co-stimulatory ligand can include, but is not limited to, CD7, B7-1 (CD80), B7-2 (CD86), PD-L1, PD-L2, 4-1BBL, OX40L, Fas ligand (FasL), inducible costimulatory ligand (ICOS-L), intercellular adhesion molecule (ICAM), CD30L, CD40, CD70, CD83, HLA-G, MICA, MICB, HVEM, lymphotoxin beta receptor, 3/TR6, ILT3, ILT4, HVEM, an agonist or antibody that binds Toll ligand receptor and a ligand that specifically binds with B7-H3. A co-stimulatory ligand also encompasses, inter alia, an antibody that specifically binds with a co-stimulatory molecule present on a T cell, such as, but not limited to, CD27, CD28, 4-1BB, OX40, CD30, CD40, PD-1, ICOS, lymphocyte function-associated antigen-1 (LFA-1), CD2, CD7, LIGHT, NKG2C, B7-H3, and a ligand that specifically binds to CD83.

The terms "purifying", "isolating", and the like, refer to the removal of a desired substance, e.g., a recombinant protein, from a solution containing undesired substances, e.g., contaminates, or the removal of undesired substances from a solution containing a desired substances, leaving behind essentially only the desired substance. In some instances, a purified substance may be essentially free of other substances, e.g., contaminates. Purifying, as used herein, may refer to a range of different resultant purities, e.g., wherein the purified substance makes up more than 80% of all the substance in the solution, including more than 85%, more than 90%, more than 91%, more than 92%, more than 93%, more than 94%, more than 95%, more than 96%, more than 97%, more than 98%, more than 99%, more than 99.5%, more than 99.9%, and the like. As will be understood by those of skill in the art, generally, components of the solution itself, e.g., water or buffer, or salts are not considered when determining the purity of a substance.

As used herein, the terms "treatment," "treating," and the like, refer to obtaining a desired pharmacologic and/or physiologic effect. The effect may be prophylactic in terms of completely or partially preventing a disease or symptom thereof and/or may be therapeutic in terms of a partial or complete cure for a disease and/or adverse effect attributable to the disease. "Treatment," as used herein, covers any treatment of a disease in a mammal, e.g., in a human, and includes: (a) preventing the disease from occurring in a subject which may be predisposed to the disease but has not yet been diagnosed as having it; (b) inhibiting the disease, i.e., arresting its development; and (c) relieving the disease, i.e., causing regression of the disease.

The terms "individual," "subject," "host," and "patient," used interchangeably herein, refer to a mammal, including, but not limited to, murines (e.g., rats, mice), lagomorphs (e.g., rabbits), non-human primates, humans, canines, felines, ungulates (e.g., equines, bovines, ovines, porcines, caprines), etc.

A "therapeutically effective amount" or "efficacious amount" refers to the amount of an agent, or combined amounts of two agents, that, when administered to a mammal or other subject for treating a disease, is sufficient to effect such treatment for the disease. The "therapeutically effective amount" will vary depending on the agent(s), the disease and its severity and the age, weight, etc., of the subject to be treated.

Before the present invention is further described, it is to be understood that this invention is not limited to particular embodiments described, but is defined by the appended claims. It is also to be understood that the terminology used herein is for the purpose of describing particular embodiments only, and is not intended to be limiting, since the scope of the present invention will be limited only by the appended claims.

Where a range of values is provided, it is understood that each intervening value, to the tenth of the unit of the lower limit unless the context clearly dictates otherwise, between the upper and lower limit of that range and any other stated or intervening value in that stated range, is encompassed within the invention. The upper and lower limits of these smaller ranges may independently be included in the smaller ranges, and are also encompassed within the invention, subject to any specifically excluded limit in the stated range. Where the stated range includes one or both of the limits, ranges excluding either or both of those included limits are also included in the invention.

Unless defined otherwise, all technical and scientific terms used herein have the same meaning as commonly understood by one of ordinary skill in the art to which this invention belongs. Although any methods and materials similar or equivalent to those described herein can also be used in the practice or testing of the present invention, the preferred methods and materials are now described.

It must be noted that as used herein and in the appended claims, the singular forms "a," "an," and "the" include plural referents unless the context clearly dictates otherwise. Thus, for example, reference to "a multimeric polypeptide" includes a plurality of such polypeptides and reference to "the immunomodulatory polypeptide" includes reference to one or more immunomodulatory polypeptides and equivalents thereof known to those skilled in the art, and so forth. It is further noted that the claims may be drafted to exclude any optional element. As such, this statement is intended to serve as antecedent basis for use of such exclusive terminology as "solely," "only" and the like in connection with the recitation of claim elements, or use of a "negative" limitation.

It is appreciated that certain features of the invention, which are, for clarity, described in the context of separate embodiments, may also be provided in combination in a single embodiment. Conversely, various features of the invention, which are, for brevity, described in the context of a single embodiment, may also be provided separately or in any suitable sub-combination. All combinations of the embodiments pertaining to the invention are specifically embraced by the present invention and are disclosed herein just as if each and every combination was individually and explicitly disclosed. In addition, all sub-combinations of the various embodiments and elements thereof are also specifically embraced by the present invention and are disclosed herein just as if each and every such sub-combination was individually and explicitly disclosed herein.

The publications discussed herein are provided solely for their disclosure prior to the filing date of the present application. Nothing herein is to be construed as an admission that the present invention is not entitled to antedate such publication by virtue of prior invention. Further, the dates of publication provided may be different from the actual publication dates which may need to be independently confirmed.

### DETAILED DESCRIPTION OF THE INVENTION

Herein is described a novel protein-based therapeutic platform that recapitulates a traditional immune response; an artificial immunological synapse for T cell activation (synTac). A novel fusion protein linking a costimulatory molecule to an MHC-epitope to allow for precise T cell engagement and clonal T cell activation, or inhibition, depending on the MOD molecule portion.

### MULTIMERIC POLYPEPTIDES

The present disclosure provides multimeric (e.g., heterodimeric, heterotrimeric) polypeptides. The present disclosure provides polyprotein precursors of a multimeric polypeptide of the present disclosure. The present disclosure provides precursor gene products, e.g., polyprotein precursors of a multimeric polypeptide of the present disclosure, and mRNA gene products encoding two or more polypeptide chains of a multimeric polypeptide of the present disclosure.

Also provided is a recombinant polypeptide construct comprising (i) a candidate epitope peptide bound by a first amino acid linker sequence contiguous with a sequence of amino acids comprising a sequence identical to a human native B2M peptide sequence contiguous with a second amino acid linker sequence contiguous with a T Cell modulatory domain peptide, wherein (i) is bound by one, or more than one, disulfide bond to (ii) a sequence of amino acids having the sequence of a MHC heavy chain contiguous with a third amino acid linker sequence contiguous with a sequence of amino acids identical to an immunoglobulin Fc domain. In an embodiment, the recombinant polypeptide construct comprises

Also provided is recombinant polypeptide construct comprising (i) a candidate epitope peptide bound by a first amino acid linker sequence contiguous with a sequence of amino acids comprising a sequence identical to a human native B2M peptide sequence, wherein (i) is bound by one, or more than one, disulfide bond to (ii) a T Cell modulatory domain peptide contiguous with a second amino acid linker sequence contiguous with a sequence of amino acids having the sequence of a MHC heavy chain contiguous a third amino acid linker sequence contiguous with a sequence of amino acids identical to an immunoglobulin Fc domain. In an embodiment, the recombinant polypeptide construct comprises

Also provided is a protein comprising two of the recombinant polypeptide constructs described herein joined by one or more disulfide bonds between the respective immunoglobulin Fc domains thereof.

Also provided is a protein comprising two of the recombinant polypeptide constructs described herein joined by one or more disulfide bonds between the respective immunoglobulin Fc domains thereof.

This invention provides a synTac platform: an artificial immunological synapse for targeted T cell activation as defined by the appended claims.

In an embodiment, the beta 2 microglobulin has the same sequence as a human beta 2 microglobulin. In an embodiment, the Histocompatibility Complex heavy chain sequence has the same sequence as a human HLA-A sequence. In an embodiment, the Histocompatibility Complex heavy chain transmembrane domain has the same sequence as a human Major Histocompatibility Complex I (MHC I) heavy chain transmembrane domain. In an embodiment, the Histocompatibility Complex heavy chain transmembrane domain has the same sequence as a human Major Histocompatibility Complex II (MHC II) heavy chain transmembrane domain.

Also provided is a composition comprising a plurality of the constructs.

In an embodiment, the candidate epitope peptide is an 8, 9, 10, 11 or 12 amino acid peptide. In an embodiment, the candidate epitope peptide is 13, 14, 15, 16, or 17 amino acid peptide. In an embodiment, the candidate epitope peptide is a nonamer (9 amino acids in length).

The present disclosure provides multimeric polypeptides that comprise two or more (e.g., 2, 3, 4, or more) polypeptide chains. In some cases, a multimeric polypeptide of the present disclosure comprises: a) a first polypeptide comprising, in order from N-terminus to C-terminus: i) an epitope; ii) a first major histocompatibility complex (MHC) polypeptide; and b) a second polypeptide comprising, in order from N-terminus to C-terminus: i) a second MHC polypeptide; and ii) optionally an immunoglobulin (Ig) Fc polypeptide or a non-Ig scaffold, wherein the multimeric polypeptide comprises one or more immunomodulatory domains, where the one or more immunomodulatory domains is(are): A) at the C-terminus of the first polypeptide; B) at the N-terminus of the second polypeptide; C) at the C-terminus of the second polypeptide; or D) at the C-terminus of the first polypeptide and at the N-terminus of the second polypeptide.

In some cases, a multimeric polypeptide of the present disclosure comprises a first polypeptide and a second polypeptide, where the first polypeptide comprises, in order from amino terminus (N-terminus) to carboxyl terminus (C-terminus): a) an epitope (e.g., a T-cell epitope); b) a first major histocompatibility complex (MHC) polypeptide and c) an immunomodulatory polypeptide; and where the second polypeptide comprises, in order from N-terminus to C-terminus: a) a second MHC polypeptide; and b) an immunoglobulin (Ig) Fc polypeptide. In other cases, a multimeric polypeptide of the present disclosure comprises a first polypeptide and a second polypeptide, where the first polypeptide comprises, in order from N-terminus to C-terminus: a) an epitope (e.g., a T-cell epitope); and b) a first MHC polypeptide; and where the second polypeptide comprises, in order from N-terminus to C-terminus: a) an immunomodulatory polypeptide; b) a second MHC polypeptide; and c) an Ig Fc polypeptide. In some instances, the first and the second MHC polypeptides are Class I MHC polypeptides; e.g., in some cases, the first MHC polypeptide is an MHC Class I β2-microglobulin (B2M) polypeptide, and the second MHC polypeptide is an MHC Class I heavy chain (H chain). In other cases, the first and the second MHC polypeptides are Class II MHC polypeptides; e.g., in some cases, the first MHC polypeptide is an MHC Class II α-chain polypeptide, and the second MHC polypeptide is an MHC Class II β-chain polypeptide. In other cases, the first polypeptide is an MHC Class II β-chain polypeptide, and the second MHC polypeptide is an MHC Class II α-chain polypeptide. In some cases, the multimeric polypeptide includes two or more immunomodulatory polypeptides. Where a multimeric polypeptide of the present disclosure includes two or more immunomodulatory polypeptides, in some cases, the two or more immunomodulatory polypeptides are present in the same polypeptide chain, and may be in tandem. Where a multimeric polypeptide of the present disclosure includes two or more immunomodulatory polypeptides, in some cases, the two or more immunomodulatory polypeptides are present in separate polypeptides. In some cases, a multimeric polypeptide of the present disclosure is a heterodimer. In some cases, a multimeric polypeptide of the present disclosure is a trimeric polypeptide.

In some cases, a multimeric polypeptide of the present disclosure comprises: a) a first polypeptide comprising, in order from N-terminus to C-terminus: i) an epitope; and ii) a first MHC polypeptide; and b) a second polypeptide comprising, in order from N-terminus to C-terminus: i) a second MHC polypeptide; and ii) an Ig Fc polypeptide; and iii) an immunomodulatory domain. In some cases, a multimeric polypeptide of the present disclosure comprises: a) a first polypeptide comprising, in order from N-terminus to C-terminus: i) an epitope; and ii) a first MHC polypeptide; and b) a second polypeptide comprising, in order from N-terminus to C-terminus: i) a second MHC polypeptide; and ii) an immunomodulatory domain. In some cases, a multimeric polypeptide of the present disclosure comprises: a) a first polypeptide comprising, in order from N-terminus to C-terminus: i) an epitope; and ii) a first MHC polypeptide; and b) a second polypeptide comprising, in order from N-terminus to C-terminus: i) an immunomodulatory domain; and ii) a second MHC polypeptide. In some cases, a multimeric polypeptide of the present disclosure comprises: a) a first polypeptide comprising, in order from N-terminus to C-terminus: i) an epitope; ii) a first MHC polypeptide; and iii) an immunomodulatory domain; and b) a second polypeptide comprising, in order from N-terminus to C-terminus: i) a second MHC polypeptide. In some cases, where a multimeric polypeptide of the present disclosure comprises a non-Ig scaffold, the non-Ig scaffold is an XTEN peptide, a transferrin polypeptide, an Fc receptor polypeptide, an elastin-like polypeptide, a silk-like polypeptide, or a silk-elastin-like polypeptide.

In some cases, a multimeric polypeptide of the present disclosure is monovalent. In some cases, a multimeric polypeptide of the present disclosure is multivalent. For example, depending on the Fc polypeptide present in a multimeric polypeptide of the present disclosure, the multimeric polypeptide can be a homodimer, where two molecules of the multimeric polypeptide are present in the homodimer, where the two molecules of the multimeric polypeptide can be disulfide linked to one another, e.g., via the Fc polypeptide present in the two molecules. As another example, a multimeric polypeptide of the present disclosure can comprise three, four, or five molecules of the multimeric polypeptide, where the molecules of the multimeric polypeptide can be disulfide linked to one another, e.g., via the Fc polypeptide present in the molecules.

### Linkers

A multimeric polypeptide of the present disclosure can include linker peptides interposed between, e.g., an epitope and an MHC polypeptide, between an MHC polypeptide and an immunomodulatory polypeptide, or between an MHC polypeptide and an Ig Fc polypeptide.

Suitable linkers (also referred to as "spacers") can be readily selected and can be of any of a number of suitable lengths, such as from 1 amino acid (e.g., Gly) to 20 amino acids, from 2 amino acids to 15 amino acids, from 3 amino acids to 12 amino acids, including 4 amino acids to 10 amino acids, 5 amino acids to 9 amino acids, 6 amino acids to 8 amino acids, or 7 amino acids to 8 amino acids, and can be 1, 2, 3, 4, 5, 6, or 7 amino acids.

Exemplary linkers include glycine polymers (G)ₙ, glycine-serine polymers (including, for example, (GS)ₙ, (GSGGS)ₙ (SEQ ID NO:8) and (GGGS)ₙ (SEQ ID NO:9), where n is an integer of at least one), glycine-alanine polymers, alanine-serine polymers, and other flexible linkers known in the art. Glycine and glycine-serine polymers can be used; both Gly and Ser are relatively unstructured, and therefore can serve as a neutral tether between components. Glycine polymers can be used; glycine accesses significantly more phi-psi space than even alanine, and is much less restricted than residues with longer side chains (see Scheraga, Rev. Computational Chem. 11173-142 (1992)). Exemplary linkers can comprise amino acid sequences including, but not limited to, GGSG (SEQ ID NO:10), GGSGG (SEQ ID NO:11), GSGSG (SEQ ID NO:12), GSGGG (SEQ ID NO:13), GGGSG (SEQ ID NO:14), GSSSG (SEQ ID NO:15), and the like.

In some cases, a linker polypeptide, present in a first polypeptide of a multimeric polypeptide of the present disclosure, includes a cysteine residue that can form a disulfide bond with a cysteine residue present in a second polypeptide of a multimeric polypeptide of the present disclosure. In some cases, for example, a suitable linker comprises the amino acid sequence G**C**GASGGGGSGGGGS (SEQ ID NO:16).

### Epitopes

An epitope present in a multimeric polypeptide of the present disclosure can have a length of from about 4 amino acids to about 25 amino acids, e.g., the epitope can have a length of from 4 amino acids (aa) to 10 aa, from 10 aa to 15 aa, from 15 aa to 20 aa, or from 20 aa to 25 aa. For example, an epitope present in a multimeric polypeptide of the present disclosure can have a length of 4 amino acids (aa), 5 aa, 6 aa, 7, aa, 8 aa, 9 aa, 10 aa, 11 aa, 12 aa, 13 aa, 14 aa, 15 aa, 16 aa, 17 aa, 18 aa, 19 aa, 20 aa, 21 aa, 22 aa, 23 aa, 24 aa, or 25 aa. In some cases, an epitope present in a multimeric polypeptide of the present disclosure has a length of from 5 amino acids to 10 amino acids, e.g., 5 aa, 6 aa, 7 aa, 8 aa, 9 aa, or 10 aa.

An epitope present in a multimeric polypeptide of the present disclosure is specifically bound by a T-cell, i.e., the epitope is specifically bound by an epitope-specific T cell. An epitope-specific T cell binds an epitope having a reference amino acid sequence, but does not substantially bind an epitope that differs from the reference amino acid sequence. For example, an epitope-specific T cell binds an epitope having a reference amino acid sequence, and binds an epitope that differs from the reference amino acid sequence, if at all, with an affinity that is less than 10⁻⁶ M, less than 10⁻⁵ M, or less than 10⁻⁴ M. An epitope-specific T cell can bind an epitope for which it is specific with an affinity of at least 10⁻⁷ M, at least 10⁻⁸ M, at least 10⁻⁹ M, or at least 10⁻¹⁰ M.

Non-limiting examples of epitopes include, e.g., the human T-lymphotrophic virus-1 epitope LLFGYPVYV (SEQ ID NO:17); the tumor epitope KYQAVTTTL (SEQ ID NO:18); and the islet-specific glucose-6-phosphatase catalytic subunit-related protein (IGRP) epitope VYLKTNVFL (SEQ ID NO: 19) or TYLKTNLFL (SEQ ID NO:20). Yang et al. (2006) J. Immunol. 176:2781.

### MHC polypeptides

As noted above, a multimeric polypeptide of the present disclosure includes MHC polypeptides. For the purposes of the instant disclosure, the term "major histocompatibility complex (MHC) polypeptides" is meant to include MHC polypeptides of various species, including human MHC (also referred to as human leukocyte antigen (HLA)) polypeptides, rodent (e.g., mouse, rat, etc.) MHC polypeptides, and MHC polypeptides of other mammalian species (e.g., lagomorphs, non-human primates, canines, felines, ungulates (e.g., equines, bovines, ovines, caprines, etc.), and the like. The term "MHC polypeptide" is meant to include Class I MHC polypeptides (e.g., β-2 microglobulin and MHC class I heavy chain) and MHC Class II polypeptides (e.g., MHC Class II α polypeptide and MHC Class II β polypeptide).

As noted above, in some embodiments of a multimeric polypeptide of the present disclosure, the first and the second MHC polypeptides are Class I MHC polypeptides; e.g., in some cases, the first MHC polypeptide is an MHC Class I β2-microglobulin (B2M) polypeptide, and the second MHC polypeptide is an MHC Class I heavy chain (H chain). In other cases, the first and the second MHC polypeptides are Class II MHC polypeptides; e.g., in some cases, the first MHC polypeptide is an MHC Class II α-chain polypeptide, and the second MHC polypeptide is an MHC Class II β-chain polypeptide. In other cases, the first polypeptide is an MHC Class II β-chain polypeptide, and the second MHC polypeptide is an MHC Class II α-chain polypeptide.

In some cases, an MHC polypeptide of a multimeric polypeptide of the present disclosure is a human MHC polypeptide, where human MHC polypeptides are also referred to as "human leukocyte antigen" ("HLA") polypeptides. In some cases, an MHC polypeptide of a multimeric polypeptide of the present disclosure is a Class I HLA polypeptide, e.g., a β2-microglobulin polypeptide, or a Class I HLA heavy chain polypeptide. Class I HLA heavy chain polypeptides include HLA-A heavy chain polypeptides, HLA-B heavy chain polypeptides, HLA-C heavy chain polypeptides, HLA-E heavy chain polypeptides, HLA-F heavy chain polypeptides, and HLA-G heavy chain polypeptides. In some cases, an MHC polypeptide of a multimeric polypeptide of the present disclosure is a Class II HLA polypeptide, e.g., a Class II HLA α chain or a Class II HLA β chain. MHC Class II polypeptides include MCH Class II DP α and β polypeptides, DM α and β polypeptides, DOA α and β polypeptides, DOB α and β polypeptides, DQ α and β polypeptides, and DR α and β polypeptides.

As an example, an MHC Class I heavy chain polypeptide of a multimeric polypeptide of the present disclosure can comprise an amino acid sequence having at least 75%, at least 80%, at least 85%, at least 90%, at least 95%, at least 98%, at least 99%, or 100%, amino acid sequence identity to amino acids 25-365 of the amino acid sequence of the human HLA-A heavy chain polypeptide depicted in Figure 25A.

As an example, an MHC Class I heavy chain polypeptide of a multimeric polypeptide of the present disclosure can comprise an amino acid sequence having at least 75%, at least 80%, at least 85%, at least 90%, at least 95%, at least 98%, at least 99%, or 100%, amino acid sequence identity to amino acids 25-365 of the amino acid sequence of the following human HLA-A heavy chain amino acid sequence:

As another example, an MHC Class I heavy chain polypeptide of a multimeric polypeptide of the present disclosure can comprise an amino acid sequence having at least 75%, at least 80%, at least 85%, at least 90%, at least 95%, at least 98%, at least 99%, or 100%, amino acid sequence identity to amino acids 25-362 of the amino acid sequence of the human HLA-B heavy chain polypeptide depicted in Figure 25B.

As another example, an MHC Class I heavy chain polypeptide of a multimeric polypeptide of the present disclosure can comprise an amino acid sequence having at least 75%, at least 80%, at least 85%, at least 90%, at least 95%, at least 98%, at least 99%, or 100%, amino acid sequence identity to amino acids 25-362 of the amino acid sequence of the human HLA-C heavy chain polypeptide depicted in Figure 25C.

As another example, an MHC Class I heavy chain polypeptide of a multimeric polypeptide of the present disclosure can comprise an amino acid sequence having at least 75%, at least 80%, at least 85%, at least 90%, at least 95%, at least 98%, at least 99%, or 100%, amino acid sequence identity to the following amino acid sequence:

A β2-microglobulin (B2M) polypeptide of a multimeric polypeptide of the present disclosure can be a human B2M polypeptide, a non-human primate B2M polypeptide, a murine B2M polypeptide, and the like. In some instances, a B2M polypeptide comprises an amino acid sequence having at least 75%, at least 80%, at least 85%, at least 90%, at least 95%, at least 98%, at least 99%, or 100%, amino acid sequence identity to a B2M amino acid sequence depicted in FIG. 20.

In some cases, an MHC polypeptide comprises a single amino acid substitution relative to a reference MHC polypeptide (where a reference MHC polypeptide can be a wild-type MHC polypeptide), where the single amino acid substitution substitutes an amino acid with a cysteine (Cys) residue. Such cysteine residues, when present in an MHC polypeptide of a first polypeptide of a multimeric polypeptide of the present disclosure, can form a disulfide bond with a cysteine residue present in a second polypeptide chain of a multimeric polypeptide of the present disclosure.

In some cases, a first MHC polypeptide in a first polypeptide of a multimeric polypeptide of the present disclosure, and/or the second MHC polypeptide in the second polypeptide of a multimeric polypeptide of the present disclosure, includes an amino acid substitution to substitute an amino acid with a cysteine, where the substituted cysteine in the first MHC polypeptide forms a disulfide bond with a cysteine in the second MHC polypeptide, where a cysteine in the first MHC polypeptide forms a disulfide bond with the substituted cysteine in the second MHC polypeptide, or where the substituted cysteine in the first MHC polypeptide forms a disulfide bond with the substituted cysteine in the second MHC polypeptide.

For example, in some cases, one of following pairs of residues in an HLA β2-microglobulin and an HLA Class I heavy chain is substituted with cysteines: 1) B2M residue 12, HLA Class I heavy chain residue 236; 2) B2M residue 12, HLA Class I heavy chain residue 237; 3) B2M residue 8, HLA Class I heavy chain residue 234; 4) B2M residue 10, HLA Class I heavy chain residue 235; 5) B2M residue 24, HLA Class I heavy chain residue 236; 6) B2M residue 28, HLA Class I heavy chain residue 232; 7) B2M residue 98, HLA Class I heavy chain residue 192; 8) B2M residue 99, HLA Class I heavy chain residue 234; 9) B2M residue 3, HLA Class I heavy chain residue 120; 10) B2M residue 31, HLA Class I heavy chain residue 96; 11) B2M residue 53, HLA Class I heavy chain residue 35; 12) B2M residue 60, HLA Class I heavy chain residue 96; 13) B2M residue 60, HLA Class I heavy chain residue 122; 14) B2M residue 63, HLA Class I heavy chain residue 27; 15) B2M residue Arg3, HLA Class I heavy chain residue Gly120; 16) B2M residue His31, HLA Class I heavy chain residue Gln96; 17) B2M residue Asp53, HLA Class I heavy chain residue Arg35; 18) B2M residue Trp60, HLA Class I heavy chain residue Gln96; 19) B2M residue Trp60, HLA Class I heavy chain residue Asp 122; 20) B2M residue Tyr63, HLA Class I heavy chain residue Tyr27; 21) B2M residue Lys6, HLA Class I heavy chain residue Glu232; 22) B2M residue Gln8, HLA Class I heavy chain residue Arg234; 23) B2M residue Tyr10, HLA Class I heavy chain residue Pro235; 24) B2M residue Ser11, HLA Class I heavy chain residue Gln242; 25) B2M residue Asn24, HLA Class I heavy chain residue Ala236; 26) B2M residue Ser28, HLA Class I heavy chain residue Glu232; 27) B2M residue Asp98, HLA Class I heavy chain residue His192; and 28) B2M residue Met99, HLA Class I heavy chain residue Arg234. The amino acid numbering of the MHC/HLA Class I heavy chain is in reference to the mature MHC/HLA Class I heavy chain, without a signal peptide. For example, in the amino acid sequence depicted in Figure 25A, which includes a signal peptide, Gly120 is Gly144; Gln96 is Gln120; etc.

### Immunomodulatory polypeptides

An immunomodulatory polypeptide of a multimeric polypeptide of the present disclosure can be an activating immunomodulatory polypeptide or an inhibitory immunomodulatory polypeptide. In some cases, a multimeric polypeptide of the present disclosure includes a single immunomodulatory polypeptide. In some cases, a multimeric polypeptide of the present disclosure includes two immunomodulatory polypeptides. In some cases, the two immunomodulatory polypeptides are in tandem in a polypeptide chain. In some cases, the two immunomodulatory polypeptides are in separate polypeptide chains. In some cases, the two immunomodulatory polypeptides are in separate polypeptide chains and are disulfide linked to one another.

An immunomodulatory polypeptide of a multimeric polypeptide of the present disclosure is in some cases a T-cell modulatory polypeptide. In some cases, the T-cell modulatory polypeptide is a stimulatory (activating) T-cell modulatory polypeptide. In some cases, the T-cell modulatory polypeptide is an inhibitory T-cell modulatory polypeptide. A T-cell modulatory polypeptide can be an antibody, a peptide ligand, a T-cell co-stimulatory polypeptide, a cytokine, or a toxin.

In some cases, an immunomodulatory polypeptide of a multimeric polypeptide of the present disclosure is an antibody-based or non-antibody-based recognition moiety that specifically binds a co-stimulatory polypeptide that is expressed on the surface of an epitope-specific T cell. Antibody-based recognition moieties include, e.g., antibodies; fragments of antibodies that retain specific binding to antigen, including, but not limited to, Fab, Fv, single-chain Fv (scFv), and Fd fragments; chimeric antibodies; humanized antibodies; single-chain antibodies (scAb), single domain antibodies (dAb); single domain heavy chain antibodies; single domain light chain antibodies; and the like. Suitable non-antibody-based recognition moieties include, e.g., affibodies; engineered Kunitz domains; monobodies (adnectins); anticalins; aptamers; designed ankyrin repeat domains (DARPins); a binding site of a cysteine-rich polypeptide (e.g., cysteine-rich knottin peptides); avimers; afflins; and the like. An antibody-based or non-antibody-based recognition moiety specifically binds co-stimulatory polypeptide that is expressed on the surface of an epitope-specific T cell, where such co-stimulatory polypeptides include, but are not limited to, CTLA4, PD1, ICOS, OX40, CD20, and 4-1BB. Co-stimulatory polypeptides that are expressed on the surface of an epitope-specific T cell are known in the art.

In some cases, an immunomodulatory polypeptide of a multimeric polypeptide of the present disclosure is a T-cell co-stimulatory polypeptide. In some cases, an immunomodulatory polypeptide of a multimeric polypeptide of the present disclosure is a T-cell co-stimulatory polypeptide and is a member of the tumor necrosis factor (TNF) superfamily; e.g., a FasL polypeptide, a 41BBL polypeptide, a CD40 polypeptide, an OX40L polypeptide, a CD30L polypeptide, a CD70 polypeptide, etc. In some cases, an immunomodulatory polypeptide of a multimeric polypeptide of the present disclosure is a T-cell co-stimulatory polypeptide and is a member of the immunoglobulin (Ig) superfamily; e.g., a CD7 polypeptide, a CD86 polypeptide, an ICAM polypeptide, etc.

Suitable immunomodulatory polypeptides of a multimeric polypeptide of the present disclosure include, but are not limited to, CD80 (B7-1), CD86 (B7-2), 4-1BBL, OX40L, ICOS-L, ICAM, PD-L1, FasL, and PD-L2. Suitable immunomodulatory polypeptides of a multimeric polypeptide of the present disclosure include, e.g., CD7, CD30L, CD40, CD70, CD83, HLA-G, MICA, MICB, HVEM, lymphotoxin beta receptor, 3/TR6, ILT3, ILT4, and HVEM.

In some cases, a T-cell modulatory polypeptide of a multimeric polypeptide of the present disclosure is a PD-L1 polypeptide. In some cases, a PD-L1 polypeptide of a multimeric polypeptide of the present disclosure comprises an amino acid sequence having at least 75%, at least 80%, at least 85%, at least 90%, at least 95%, at least 98%, at least 99%, or 100%, amino acid sequence identity to amino acids 19-290 of a PD-L1 amino acid sequence depicted in Figure 26A or 26B.

In some cases, a T-cell modulatory polypeptide of a multimeric polypeptide of the present disclosure is a 4-1BBL polypeptide. In some cases, a 4-1BBL polypeptide of a multimeric polypeptide of the present disclosure comprises an amino acid sequence having at least 75%, at least 80%, at least 85%, at least 90%, at least 95%, at least 98%, at least 99%, or 100%, amino acid sequence identity to amino acids 50-254 of the 4-1BBL amino acid sequence depicted in Figure 27.

In some cases, a T-cell modulatory polypeptide of a multimeric polypeptide of the present disclosure is an ICOS-L polypeptide. In some cases, an ICOS-L polypeptide of a multimeric polypeptide of the present disclosure comprises an amino acid sequence having at least 75%, at least 80%, at least 85%, at least 90%, at least 95%, at least 98%, at least 99%, or 100%, amino acid sequence identity to amino acids 19-302 of the ICOS-L amino acid sequence depicted in Figure 28.

In some cases, a T-cell modulatory polypeptide of a multimeric polypeptide of the present disclosure is an OX40L polypeptide. In some cases, an OX40L polypeptide of a multimeric polypeptide of the present disclosure comprises an amino acid sequence having at least 75%, at least 80%, at least 85%, at least 90%, at least 95%, at least 98%, at least 99%, or 100%, amino acid sequence identity to amino acids 1-183 of the OX40L amino acid sequence depicted in Figure 29.

In some cases, a T-cell modulatory polypeptide of a multimeric polypeptide of the present disclosure is a PD-L2 polypeptide. In some cases, a PD-L2 polypeptide of a multimeric polypeptide of the present disclosure comprises an amino acid sequence having at least 75%, at least 80%, at least 85%, at least 90%, at least 95%, at least 98%, at least 99%, or 100%, amino acid sequence identity to amino acids 20-273 of the PD-L2 amino acid sequence depicted in Figure 30.

In some cases, a T-cell modulatory polypeptide of a multimeric polypeptide of the present disclosure is a CD80 (B7-1) polypeptide. In some cases, a CD80 polypeptide of a multimeric polypeptide of the present disclosure comprises an amino acid sequence having at least 75%, at least 80%, at least 85%, at least 90%, at least 95%, at least 98%, at least 99%, or 100%, amino acid sequence identity to amino acids 35-288 of the CD80 amino acid sequence depicted in Figure 31.

In some cases, a T-cell modulatory polypeptide of a multimeric polypeptide of the present disclosure is a CD86 polypeptide. In some cases, a CD86 polypeptide of a multimeric polypeptide of the present disclosure comprises an amino acid sequence having at least 75%, at least 80%, at least 85%, at least 90%, at least 95%, at least 98%, at least 99%, or 100%, amino acid sequence identity to amino acids 31-329 of the CD86 amino acid sequence depicted in Figure 32.

In some cases, a T-cell modulatory polypeptide of a multimeric polypeptide of the present disclosure is a FasL polypeptide. In some cases, a FasL polypeptide of a multimeric polypeptide of the present disclosure comprises an amino acid sequence having at least 75%, at least 80%, at least 85%, at least 90%, at least 95%, at least 98%, at least 99%, or 100%, amino acid sequence identity to amino acids 1-281 of the FasL amino acid sequence depicted in Figure 33.

Further T cell modulatory domains (MODs) that can be employed in the invention include naturally occurring or synthetic human gene products (protein), affinity reagents (e.g., an antibody, antibody fragment, single chain Fvs, aptamers, nanobody) targeting a human gene product, including, but not limited to all secreted proteins arising from classical and non-classical (e.g., FGF2, IL1, S100A4) secretion mechanisms, and ecto-domains of all cell surface proteins anchored by naturally occurring genetically encoded protein segments (single or multiple membrane spans) or post-translational modifications such as GPI linkages). Any naturally occurring or synthetic affinity reagent (e.g., antibody, antibody fragment, single chain Fvs, aptamer, nanobody, lectin, etc) targeting a cell surface glycan or other post-translational modification (e.g., sulfation). Examples include, but are not limited to, members of the TNF/TNFR family (OX40L, ICOSL, FASL, LTA, LTB TRAIL, CD153, TNFSF9, RANKL, TWEAK, TNFSF13, TNFSF13b, TNFSF14, TNFSF15, TNFSF18, CD40LG, CD70) or affinity reagents directed at the TNF/TNFR family members; members of the Immunoglobulin superfamily (VISTA, PD1, PD-L1, PD-L2, B71, B72, CTLA4, CD28, TIM3, CD4, CD8, CD19, T cell receptor chains, ICOS, ICOS ligand, HHLA2, butyrophilins, BTLA, B7-H3, B7-H4, CD3, CD79a, CD79b, IgSF CAMS (including CD2, CD58, CD48, CD150, CD229, CD244, ICAM-1), Leukocyte immunoglobulin like receptors (LILR), killer cell immunoglobulin like receptors (KIR)), lectin superfamily members, selectins, cytokines/chemokine and cytokine/chemokine receptors, growth factors and growth factor receptors), adhesion molecules (integrins, fibronectins, cadherins), or ecto-domains of multi-span integral membrane protein, or affinity reagents directed at the Immunoglobulin superfamily and listed gene products. In addition, active homologs/orthologs of these gene products, including but not limited to, viral sequences (e.g., CMV, EBV), bacterial sequences, fungal sequences, eukaryotic pathogens (e.g., *Schistosoma, Plasmodium, Babesia, Eimeria, Theileria, Toxoplasma, Entamoeba, Leishmania*, *and Trypanosoma*), and mammalian -derived coding regions. In addition. a MOD may comprise a small molecules drug targeting a human gene product.

### Fc polypeptides

A multimeric polypeptide of the present disclosure comprises an Fc polypeptide, or another suitable scaffold polypeptide.

Suitable scaffold polypeptides include antibody-based scaffold polypeptides and non-antibody-based scaffolds. Non-antibody-based scaffolds include, e.g., albumin, an XTEN (extended recombinant) polypeptide, transferrin, an Fc receptor polypeptide, an elastin-like polypeptide (see, e.g., Hassouneh et al. (2012) Methods Enzymol. 502:215; e.g., a polypeptide comprising a pentapeptide repeat unit of (Val-Pro-Gly-X-Gly), where X iany amino acid other than proline), an albumin-binding polypeptide, a silk-like polypeptide (see, e.g., Valluzzi et al. (2002) Philos Trans R Soc Lond B Biol Sci. 357:165), a silk-elastin-like polypeptide (SELP; see, e.g., Megeed et al. (2002) Adv Drug Deliv Rev. 54:1075), and the like. Suitable XTEN polypeptides include, e.g., those disclosed in WO 2009/023270, WO 2010/091122, WO 2007/103515, US 2010/0189682, and US 2009/0092582; see also Schellenberger et al. (2009) Nat Biotechnol. 27:1186). Suitable albumin polypeptides include, e.g., human serum albumin.

Suitable scaffold polypeptides will in some cases be a half-life extending polypeptides. Thus, in some cases, a suitable scaffold polypeptide increases the *in vivo* half-life (e.g., the serum half-life) of the multimeric polypeptide, compared to a control multimeric polypeptide lacking the scaffold polypeptide. For example, in some cases, a scaffold polypeptide increases the *in vivo* half-life (e.g., the serum half-life) of the multimeric polypeptide, compared to a control multimeric polypeptide lacking the scaffold polypeptide, by at least about 10%, at least about 15%, at least about 20%, at least about 25%, at least about 50%, at least about 2-fold, at least about 2.5-fold, at least about 5-fold, at least about 10-fold, at least about 25-fold, at least about 50-fold, at least about 100-fold, or more than 100-fold. As an example, in some cases, an Fc polypeptide increases the *in vivo* half-life (e.g., the serum half-life) of the multimeric polypeptide, compared to a control multimeric polypeptide lacking the Fc polypeptide, by at least about 10%, at least about 15%, at least about 20%, at least about 25%, at least about 50%, at least about 2-fold, at least about 2.5-fold, at least about 5-fold, at least about 10-fold, at least about 25-fold, at least about 50-fold, at least about 100-fold, or more than 100-fold.

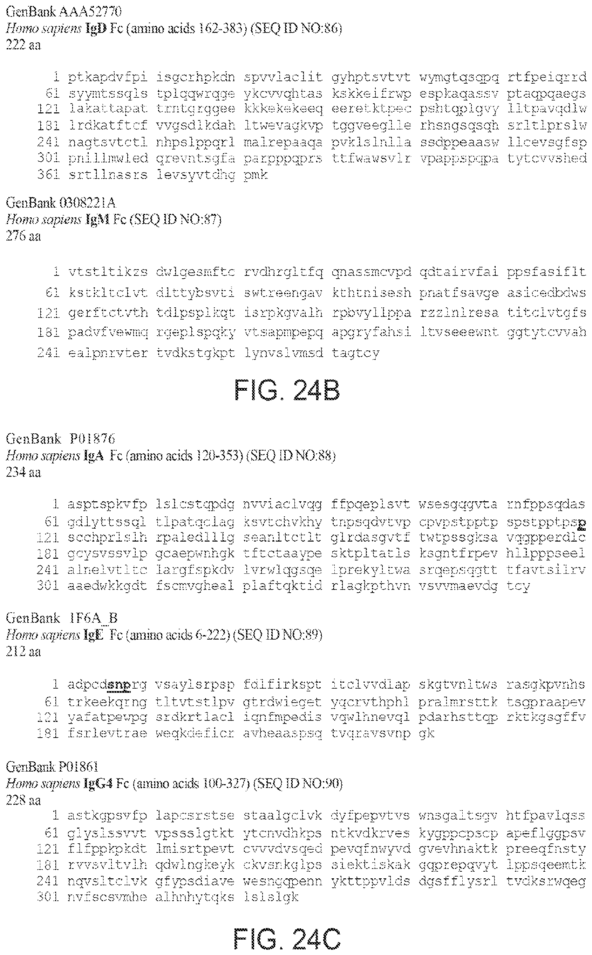

The Fc polypeptide of a multimeric polypeptide of the present disclosure can be a human IgG1 Fc, a human IgG2 Fc, a human IgG3 Fc, a human IgG4 Fc, etc. In some cases, the Fc polypeptide comprises an amino acid sequence having at least about 70%, at least about 75%, at least about 80%, at least about 85%, at least about 90%, at least about 95%, at least about 98%, at least about 99%, or 100%, amino acid sequence identity to an amino acid sequence of an Fc region depicted in Figures 24A-C. In some cases, the Fc region comprises an amino acid sequence having at least about 70%, at least about 75%, at least about 80%, at least about 85%, at least about 90%, at least about 95%, at least about 98%, at least about 99%, or 100%, amino acid sequence identity to the human IgG1 Fc polypeptide depicted in Figure 24A. In some cases, the Fc polypeptide comprises an amino acid sequence having at least about 70%, at least about 75%, at least about 80%, at least about 85%, at least about 90%, at least about 95%, at least about 98%, at least about 99%, or 100%, amino acid sequence identity to the human IgG2 Fc polypeptide depicted in Figure 24A; e.g., the Fc polypeptide comprises an amino acid sequence having at least about 70%, at least about 75%, at least about 80%, at least about 85%, at least about 90%, at least about 95%, at least about 98%, at least about 99%, or 100%, amino acid sequence identity to amino acids 99-325 of the human IgG2 Fc polypeptide depicted in Figure 24A. In some cases, the Fc polypeptide comprises an amino acid sequence having at least about 70%, at least about 75%, at least about 80%, at least about 85%, at least about 90%, at least about 95%, at least about 98%, at least about 99%, or 100%, amino acid sequence identity to the human IgG3 Fc polypeptide depicted in Figure 24A; e.g., the Fc polypeptide comprises an amino acid sequence having at least about 70%, at least about 75%, at least about 80%, at least about 85%, at least about 90%, at least about 95%, at least about 98%, at least about 99%, or 100%, amino acid sequence identity to amino acids 19-246 of the human IgG3 Fc polypeptide depicted in Figure 24A. In some cases, the Fc polypeptide comprises an amino acid sequence having at least about 70%, at least about 75%, at least about 80%, at least about 85%, at least about 90%, at least about 95%, at least about 98%, at least about 99%, or 100%, amino acid sequence identity to the human IgM Fc polypeptide depicted in FIG. 24B; e.g., the Fc polypeptide comprises an amino acid sequence having at least about 70%, at least about 75%, at least about 80%, at least about 85%, at least about 90%, at least about 95%, at least about 98%, at least about 99%, or 100%, amino acid sequence identity to amino acids 1-276 to the human IgM Fc polypeptide depicted in FIG. 24B. In some cases, the Fc polypeptide comprises an amino acid sequence having at least about 70%, at least about 75%, at least about 80%, at least about 85%, at least about 90%, at least about 95%, at least about 98%, at least about 99%, or 100%, amino acid sequence identity to the human IgA Fc polypeptide depicted in Figure 24C; e.g., the Fc polypeptide comprises an amino acid sequence having at least about 70%, at least about 75%, at least about 80%, at least about 85%, at least about 90%, at least about 95%, at least about 98%, at least about 99%, or 100%, amino acid sequence identity to amino acids 1-234 to the human IgA Fc polypeptide depicted in FIG. 24C.

### Additional polypeptides

A polypeptide chain of a multimeric polypeptide of the present disclosure can include one or more polypeptides in addition to those described above. Suitable additional polypeptides include epitope tags and affinity domains. The one or more additional polypeptide can be included at the N-terminus of a polypeptide chain of a multimeric polypeptide of the present disclosure, at the C-terminus of a polypeptide chain of a multimeric polypeptide of the present disclosure, or internally within a polypeptide chain of a multimeric polypeptide of the present disclosure.

### Epitope tag

Suitable epitope tags include, but are not limited to, hemagglutinin (HA; e.g., YPYDVPDYA (SEQ ID NO:23); FLAG (e.g., DYKDDDDK (SEQ ID NO:24); c-myc (e.g., EQKLISEEDL; SEQ ID NO:25), and the like.

### Affinity domain

Affinity domains include peptide sequences that can interact with a binding partner, e.g., such as one immobilized on a solid support, useful for identification or purification. DNA sequences encoding multiple consecutive single amino acids, such as histidine, when fused to the expressed protein, may be used for one-step purification of the recombinant protein by high affinity binding to a resin column, such as nickel sepharose. Exemplary affinity domains include His5 (HHHHH) (SEQ ID NO:26), HisX6 (HHHHHH) (SEQ ID NO:27), C-myc (EQKLISEEDL) (SEQ ID NO:25), Flag (DYKDDDDK) (SEQ ID NO:24), StrepTag (WSHPQFEK) (SEQ ID NO:28), hemagglutinin, e.g., HA Tag (YPYDVPDYA) (SEQ ID NO:23), glutathione-S-transferase (GST), thioredoxin, cellulose binding domain, RYIRS (SEQ ID NO:30), Phe-His-His-Thr (SEQ ID NO:31), chitin binding domain, S-peptide, T7 peptide, SH2 domain, C-end RNA tag, WEAAAREACCRECCARA (SEQ ID NO:32), metal binding domains, e.g., zinc binding domains or calcium binding domains such as those from calcium-binding proteins, e.g., calmodulin, troponin C, calcineurin B, myosin light chain, recoverin, S-modulin, visinin, VILIP, neurocalcin, hippocalcin, frequenin, caltractin, calpain large-subunit, S100 proteins, parvalbumin, calbindin D9K, calbindin D28K, and calretinin, inteins, biotin, streptavidin, MyoD, Id, leucine zipper sequences, and maltose binding protein.

### Modifications

A multimeric polypeptide of the present disclosure can include one or more non-polypeptide moieties covalently linked to the multimeric polypeptide. Suitable non-polypeptide moieties include, e.g., biocompatible fatty acids and derivatives thereof; Hydroxy Alkyl Starch (HAS) e.g. Hydroxy Ethyl Starch (HES); poly(ethylene glycol); hyaluronic acid (HA); heparosan polymers (HEP); phosphorylcholine-based polymers; dextran; poly-sialic acids (PSA); and the like. In some cases, the non-polypeptide moiety increases the *in vivo* half-life of the multimeric polypeptide, compared to a control multimeric polypeptide that does not comprise the non-polypeptide moiety.

In some cases, a multimeric polypeptide of the present disclosure includes a detectable label. Suitable detectable labels include radioisotopes such as ¹²³¹I (iodine), ¹⁸F (fluorine), ⁹⁹Tc (technetium), ¹¹¹In (indium), ⁶⁷Ga (gallium), radioactive Gd isotopes (¹⁵³Gd); contrast agents such as gadolinium (Gd), dysprosium, and iron; an enzyme which generates a detectable product (e.g., luciferase, β-galactosidase, horse radish peroxidase, alkaline phosphatase, and the like); a fluorescent protein; a chromogenic protein, dye (e.g., fluorescein isothiocyanate, rhodamine, phycoerythrin, and the like); fluorescence emitting metals, e.g., ¹⁵²Eu, or others of the lanthanide series; chemiluminescent compounds, e.g., luminol, isoluminol, acridinium salts, and the like; bioluminescent compounds; and the like.

### Activity

Depending on the nature of the immunomodulatory ("MOD") polypeptide present in a multimeric polypeptide of the present disclosure, the multimeric polypeptide can activate or inhibit a target T cell. A multimeric polypeptide of the present disclosure selectively activates or inhibits a target T cell that is specific for the epitope present in the multimeric polypeptide. "Target T cells" include epitope-specific CD4⁺ T cells, epitope-specific CD8⁺ T cells. In some cases, the target CD4⁺ T cell is a helper T cell (e.g., a Th1, Th2, or Th17 cell). In some cases, the target CD4⁺ T cell is a CD4⁺/CD25⁺/FoxP3⁺ regulatory T (Treg) cell. In some cases, the target T cell is a CD8⁺ T cell and is a cytotoxic T cell. In some cases, the target T cell is a memory T cell, which can be a CD4⁺ T cell or a CD8⁺ T cell, where memory T cells are generally CD45RO⁺. In some cases, the target T cell is an NK-T cell.

In some cases, a multimeric polypeptide of the present disclosure enhances T cell homing and trafficking. For example, in some cases, a multimeric polypeptide of the present disclosure, when contacted with a target T cell, increases extravasation of the target T cell to a treatment site. In some cases, a multimeric polypeptide of the present disclosure, when contacted with a target T cell, increases extravasation of the target T cell to a treatment site by at least 10%, at least 15%, at least 20%, at least 25%, at least 30%, at least 40%, at least 50%, at least 75%, at least 2-fold, at least 5-fold, at least 10-fold, at least 15-fold, at least 20-fold, at least 25-fold, at least 50-fold, at least 100-fold, or more than 100-fold, compared to the level of extravasation of the target T cell not contacted with the multimeric polypeptide. Increased extravasation can increase the number of T cells at a treatment site. In some cases, a multimeric polypeptide of the present disclosure, when contacted with a target T cell, increases the number of T cells at a treatment site.

In some cases, a multimeric polypeptide of the present disclosure increases the expression by a target T cell of one or more proteins that mediate or regulate lymphocyte trafficking by the target T cell. For example, in some cases, a multimeric polypeptide of the present disclosure, when contacted with a target T cell, increases the level of one or more adhesion molecules and/or chemokine receptor molecules in the target T cell. For example, in some cases, a multimeric polypeptide of the present disclosure, when contacted with a target T cell, increases the expression of one or more adhesion molecules and/or chemokine receptor molecules by the target T cell by at least 2-fold, at least 5-fold, at least 10-fold, at least 15-fold, at least 20-fold, at least 25-fold, at least 50-fold, at least 100-fold, or more than 100-fold, compared to the level of the adhesion molecule and/or chemokine receptor molecule produced by the target T cell not contacted with the multimeric polypeptide. Examples of adhesion molecules include adhesion molecules produced by CD8 T cells, where examples of such adhesion molecules include, but are not limited to, CD44, LFA-1, and VLA-4. Examples of chemokine receptors include chemokine receptors produced by CD8 T cells, where examples of such chemokine receptors include, but are not limited to, CCR5, CCR7 and CXCR3.

In some cases, a multimeric polypeptide of the present disclosure results in the generation of memory T cells capable of rapid cytotoxic responses against a previously experienced epitope. For example, in some cases, a multimeric polypeptide of the present disclosure, when contacted with a target T cell, results in the generation of memory T cells comprising 0.5% or more of the antigen-specific T cell pool. For example, in some cases, a multimeric polypeptide of the present disclosure, when contacted with a target T cell, results in the generation of memory T cells comprising 0.5% or more, 1% or more, 2% or more, 3% or more, 4% or more, 5% or more, 10% or more, 15% or more, or 20% or more, of the antigen-specific T cell pool. An example of a cell surface marker of T memory cells is CD45RO.

In some cases, a multimeric polypeptide of the present disclosure increases proliferation of a target T cell. For example, in some cases, a multimeric polypeptide of the present disclosure, when contacted with a target T cell, increases proliferation of the target T cell by at least 10%, at least 15%, at least 20%, at least 25%, at least 30%, at least 40%, at least 50%, at least 75%, at least 2-fold, at least 5-fold, at least 10-fold, at least 15-fold, at least 20-fold, at least 25-fold, at least 50-fold, at least 100-fold, or more than 100-fold, compared to the proliferation of the target T cell not contacted with the multimeric polypeptide.

In some cases, a multimeric polypeptide of the present disclosure increases cytotoxic activity of a T cell toward a target cell. For example, in some cases, a multimeric polypeptide of the present disclosure, when contacted with a target T cell, increases cytotoxic activity of the T cell toward a target cell by at least 10%, at least 15%, at least 20%, at least 25%, at least 30%, at least 40%, at least 50%, at least 75%, at least 2-fold, at least 5-fold, at least 10-fold, at least 15-fold, at least 20-fold, at least 25-fold, at least 50-fold, at least 100-fold, or more than 100-fold, compared to the cytotoxic activity of the T cell toward the target cell not contacted with the multimeric polypeptide. Targets of T cells include virus-infected cells, cancer cells, and the like.

In some cases, a multimeric polypeptide of the present disclosure increases cytokine production by a target T cell. For example, in some cases, a multimeric polypeptide of the present disclosure, when contacted with a target T cell, increases cytokine production by the T cell by at least 10%, at least 15%, at least 20%, at least 25%, at least 30%, at least 40%, at least 50%, at least 75%, at least 2-fold, at least 5-fold, at least 15-fold, at least 20-fold, at least 25-fold, at least 50-fold, at least 100-fold, or more than 100-fold, compared to the level of cytokine produced by the target T cell not contacted with the multimeric polypeptide. Examples of cytokines include cytokines produced by Th1 cells, e.g., IL-2, IFN-γ, and TNF-α; cytokines produced by Th17 cells, e.g., IL-17, IL-21, and IL-22; cytokines produced by Treg cells, e.g., TGF-β, IL-35, and IL-10.

In some cases, a multimeric polypeptide of the present disclosure inhibits cytokine production by a target T cell. For example, in some cases, a multimeric polypeptide of the present disclosure, when contacted with a target T cell, inhibits cytokine production by a target T cell by at least 10%, at least 15%, at least 20%, at least 25%, at least 30%, at least 40%, at least 50%, at least 60%, at least 70%, at least 80%, or at least 90%, or more than 90%, compared to the level of cytokine produced by the target T cell not contacted with the multimeric polypeptide. Examples of cytokines include cytokines produced by Th2 cells, e.g., IL-4, IL-5, IL-6, IL-10, and IL-13.

### Exemplary embodiments

Non-limiting examples of a multimeric polypeptide of the present disclosure include:
1) a multimeric polypeptide comprising: a) a first polypeptide comprising, in order from N-terminus to C-terminus: i) a T-cell epitope; ii) an MHC Class I β2-microglobulin polypeptide; and iii) a 4-BBL polypeptide; and b) a second polypeptide comprising, in order from N-terminus to C-terminus: i) an MHC Class I heavy chain polypeptide; and ii) an Ig Fc polypeptide. In some cases, the first polypeptide and the second polypeptide are disulfide linked to one another. In some cases, the first polypeptide comprises a linker polypeptide between the epitope and the β2-microglobulin polypeptide. In some cases, the first polypeptide and the second polypeptide are disulfide linked to one another via a cysteine residue present in the linker polypeptide, and a cysteine residue present in the MHC Class I heavy chain polypeptide. In some cases, the first polypeptide and the second polypeptide are disulfide linked to one another via a cysteine residue present in the MHC Class I β2-microglobulin polypeptide, and a cysteine residue present in the MHC Class I heavy chain polypeptide; in some of these embodiments, the MHC Class I β2-microglobulin polypeptide and/or the MHC Class I heavy chain polypeptide include an amino acid substitution to provide a cysteine that participates in the disulfide bond. In some cases, the Ig Fc polypeptide is an IgG1 Fc polypeptide. In some cases, the Ig Fc polypeptide is an IgG2 Fc polypeptide. In some cases, the Ig Fc polypeptide is an IgG3 Fc polypeptide. In some cases, the Ig Fc polypeptide is an IgA Fc polypeptide or an IgM Fc polypeptide. In some cases, MHC Class II polypeptides are used in place of the MHC Class I polypeptides. In some cases, the multimeric polypeptide includes an epitope tag and/or an affinity domain C-terminal to the Fc polypeptide;
2) a multimeric polypeptide comprising: a) a first polypeptide comprising, in order from N-terminus to C-terminus: i) a T-cell epitope; ii) an MHC Class I β2-microglobulin polypeptide; and iii) a PD-L1 polypeptide; and b) a second polypeptide comprising, in order from N-terminus to C-terminus: i) an MHC Class I heavy chain polypeptide; and ii) an Ig Fc polypeptide. In some cases, the first polypeptide and the second polypeptide are disulfide linked to one another. In some cases, the first polypeptide comprises a linker polypeptide between the epitope and the β2-microglobulin polypeptide. In some cases, the first polypeptide and the second polypeptide are disulfide linked to one another via a cysteine residue present in the linker polypeptide, and a cysteine residue present in the MHC Class I heavy chain polypeptide. In some cases, the first polypeptide and the second polypeptide are disulfide linked to one another via a cysteine residue present in the MHC Class I β2-microglobulin polypeptide, and a cysteine residue present in the MHC Class I heavy chain polypeptide; in some of these embodiments, the MHC Class I β2-microglobulin polypeptide and/or the MHC Class I heavy chain polypeptide include an amino acid substitution to provide a cysteine that participates in the disulfide bond. In some cases, the Ig Fc polypeptide is an IgG1 Fc polypeptide. In some cases, the Ig Fc polypeptide is an IgG2 Fc polypeptide. In some cases, the Ig Fc polypeptide is an IgG3 Fc polypeptide. In some cases, the Ig Fc polypeptide is an IgA Fc polypeptide or an IgM Fc polypeptide. In some cases, MHC Class II polypeptides are used in place of the MHC Class I polypeptides. In some cases, the multimeric polypeptide includes an epitope tag and/or an affinity domain C-terminal to the Fc polypeptide;
3) a multimeric polypeptide comprising: a) a first polypeptide comprising, in order from N-terminus to C-terminus: i) a T-cell epitope; ii) an MHC Class I β2-microglobulin polypeptide; and iii) ICOS-L polypeptide; and b) a second polypeptide comprising, in order from N-terminus to C-terminus: i) an MHC Class I heavy chain polypeptide; and ii) an Ig Fc polypeptide. In some cases, the first polypeptide and the second polypeptide are disulfide linked to one another. In some cases, the first polypeptide comprises a linker polypeptide between the epitope and the β2-microglobulin polypeptide. In some cases, the first polypeptide and the second polypeptide are disulfide linked to one another via a cysteine residue present in the linker polypeptide, and a cysteine residue present in the MHC Class I heavy chain polypeptide. In some cases, the first polypeptide and the second polypeptide are disulfide linked to one another via a cysteine residue present in the MHC Class I β2-microglobulin polypeptide, and a cysteine residue present in the MHC Class I heavy chain polypeptide; in some of these embodiments, the MHC Class I β2-microglobulin polypeptide and/or the MHC Class I heavy chain polypeptide include an amino acid substitution to provide a cysteine that participates in the disulfide bond. In some cases, the Ig Fc polypeptide is an IgG1 Fc polypeptide. In some cases, the Ig Fc polypeptide is an IgG2 Fc polypeptide. In some cases, the Ig Fc polypeptide is an IgG3 Fc polypeptide. In some cases, the Ig Fc polypeptide is an IgA Fc polypeptide or an IgM Fc polypeptide. In some cases, MHC Class II polypeptides are used in place of the MHC Class I polypeptides. In some cases, the multimeric polypeptide includes an epitope tag and/or an affinity domain C-terminal to the Fc polypeptide;
4) a multimeric polypeptide comprising: a) a first polypeptide comprising, in order from N-terminus to C-terminus: i) a T-cell epitope; ii) an MHC Class I β2-microglobulin polypeptide; and iii) an OX40L polypeptide; and b) a second polypeptide comprising, in order from N-terminus to C-terminus: i) an MHC Class I heavy chain polypeptide; and ii) an Ig Fc polypeptide. In some cases, the first polypeptide and the second polypeptide are disulfide linked to one another. In some cases, the first polypeptide comprises a linker polypeptide between the epitope and the β2-microglobulin polypeptide. In some cases, the first polypeptide and the second polypeptide are disulfide linked to one another via a cysteine residue present in the linker polypeptide, and a cysteine residue present in the MHC Class I heavy chain polypeptide. In some cases, the first polypeptide and the second polypeptide are disulfide linked to one another via a cysteine residue present in the MHC Class I β2-microglobulin polypeptide, and a cysteine residue present in the MHC Class I heavy chain polypeptide; in some of these embodiments, the MHC Class I β2-microglobulin polypeptide and/or the MHC Class I heavy chain polypeptide include an amino acid substitution to provide a cysteine that participates in the disulfide bond. In some cases, the Ig Fc polypeptide is an IgG1 Fc polypeptide. In some cases, the Ig Fc polypeptide is an IgG2 Fc polypeptide. In some cases, the Ig Fc polypeptide is an IgG3 Fc polypeptide. In some cases, the Ig Fc polypeptide is an IgA Fc polypeptide or an IgM Fc polypeptide. In some cases, MHC Class II polypeptides are used in place of the MHC Class I polypeptides. In some cases, the multimeric polypeptide includes an epitope tag and/or an affinity domain C-terminal to the Fc polypeptide;
5) a multimeric polypeptide comprising: a) a first polypeptide comprising, in order from N-terminus to C-terminus: i) a T-cell epitope; ii) an MHC Class I β2-microglobulin polypeptide; and iii) a CD80 polypeptide; and b) a second polypeptide comprising, in order from N-terminus to C-terminus: i) an MHC Class I heavy chain polypeptide; and ii) an Ig Fc polypeptide. In some cases, the first polypeptide and the second polypeptide are disulfide linked to one another. In some cases, the first polypeptide comprises a linker polypeptide between the epitope and the β2-microglobulin polypeptide. In some cases, the first polypeptide and the second polypeptide are disulfide linked to one another via a cysteine residue present in the linker polypeptide, and a cysteine residue present in the MHC Class I heavy chain polypeptide. In some cases, the first polypeptide and the second polypeptide are disulfide linked to one another via a cysteine residue present in the MHC Class I β2-microglobulin polypeptide, and a cysteine residue present in the MHC Class I heavy chain polypeptide; in some of these embodiments, the MHC Class I β2-microglobulin polypeptide and/or the MHC Class I heavy chain polypeptide include an amino acid substitution to provide a cysteine that participates in the disulfide bond. In some cases, the Ig Fc polypeptide is an IgG1 Fc polypeptide. In some cases, the Ig Fc polypeptide is an IgG2 Fc polypeptide. In some cases, the Ig Fc polypeptide is an IgG3 Fc polypeptide. In some cases, the Ig Fc polypeptide is an IgA Fc polypeptide or an IgM Fc polypeptide. In some cases, MHC Class II polypeptides are used in place of the MHC Class I polypeptides. In some cases, the multimeric polypeptide includes an epitope tag and/or an affinity domain C-terminal to the Fc polypeptide;
6) a multimeric polypeptide comprising: a) a first polypeptide comprising, in order from N-terminus to C-terminus: i) a T-cell epitope; ii) an MHC Class I β2-microglobulin polypeptide; and iii) a CD86 polypeptide; and b) a second polypeptide comprising, in order from N-terminus to C-terminus: i) an MHC Class I heavy chain polypeptide; and ii) an Ig Fc polypeptide. In some cases, the first polypeptide and the second polypeptide are disulfide linked to one another. In some cases, the first polypeptide comprises a linker polypeptide between the epitope and the β2-microglobulin polypeptide. In some cases, the first polypeptide and the second polypeptide are disulfide linked to one another via a cysteine residue present in the linker polypeptide, and a cysteine residue present in the MHC Class I heavy chain polypeptide. In some cases, the first polypeptide and the second polypeptide are disulfide linked to one another via a cysteine residue present in the MHC Class I β2-microglobulin polypeptide, and a cysteine residue present in the MHC Class I heavy chain polypeptide; in some of these embodiments, the MHC Class I β2-microglobulin polypeptide and/or the MHC Class I heavy chain polypeptide include an amino acid substitution to provide a cysteine that participates in the disulfide bond. In some cases, the Ig Fc polypeptide is an IgG1 Fc polypeptide. In some cases, the Ig Fc polypeptide is an IgG2 Fc polypeptide. In some cases, the Ig Fc polypeptide is an IgG3 Fc polypeptide. In some cases, the Ig Fc polypeptide is an IgA Fc polypeptide or an IgM Fc polypeptide. In some cases, MHC Class II polypeptides are used in place of the MHC Class I polypeptides. In some cases, the multimeric polypeptide includes an epitope tag and/or an affinity domain C-terminal to the Fc polypeptide;
7) a multimeric polypeptide comprising: a) a first polypeptide comprising, in order from N-terminus to C-terminus: i) a T-cell epitope; ii) an MHC Class I β2-microglobulin polypeptide; and iii) a PD-L2 polypeptide; and b) a second polypeptide comprising, in order from N-terminus to C-terminus: i) an MHC Class I heavy chain polypeptide; and ii) an Ig Fc polypeptide. In some cases, the first polypeptide and the second polypeptide are disulfide linked to one another. In some cases, the first polypeptide comprises a linker polypeptide between the epitope and the β2-microglobulin polypeptide. In some cases, the first polypeptide and the second polypeptide are disulfide linked to one another via a cysteine residue present in the linker polypeptide, and a cysteine residue present in the MHC Class I heavy chain polypeptide. In some cases, the first polypeptide and the second polypeptide are disulfide linked to one another via a cysteine residue present in the MHC Class I β2-microglobulin polypeptide, and a cysteine residue present in the MHC Class I heavy chain polypeptide; in some of these embodiments, the MHC Class I β2-microglobulin polypeptide and/or the MHC Class I heavy chain polypeptide include an amino acid substitution to provide a cysteine that participates in the disulfide bond. In some cases, the Ig Fc polypeptide is an IgG1 Fc polypeptide. In some cases, the Ig Fc polypeptide is an IgG2 Fc polypeptide. In some cases, the Ig Fc polypeptide is an IgG3 Fc polypeptide. In some cases, the Ig Fc polypeptide is an IgA Fc polypeptide or an IgM Fc polypeptide. In some cases, MHC Class II polypeptides are used in place of the MHC Class I polypeptides. In some cases, the multimeric polypeptide includes an epitope tag and/or an affinity domain C-terminal to the Fc polypeptide;
8) a multimeric polypeptide comprising: a) a first polypeptide comprising, in order from N-terminus to C-terminus: i) a T-cell epitope; and ii) an MHC Class I β2-microglobulin polypeptide; and b) a second polypeptide comprising, in order from N-terminus to C-terminus: i) i) a 4-BBL polypeptide; ii) an MHC Class I heavy chain polypeptide; and iii) an Ig Fc polypeptide. In some cases, the first polypeptide and the second polypeptide are disulfide linked to one another. In some cases, the first polypeptide comprises a linker polypeptide between the epitope and the β2-microglobulin polypeptide. In some cases, the first polypeptide and the second polypeptide are disulfide linked to one another via a cysteine residue present in the linker polypeptide, and a cysteine residue present in the MHC Class I heavy chain polypeptide. In some cases, the first polypeptide and the second polypeptide are disulfide linked to one another via a cysteine residue present in the MHC Class I β2-microglobulin polypeptide, and a cysteine residue present in the MHC Class I heavy chain polypeptide; in some of these embodiments, the MHC Class I β2-microglobulin polypeptide and/or the MHC Class I heavy chain polypeptide include an amino acid substitution to provide a cysteine that participates in the disulfide bond. In some cases, the Ig Fc polypeptide is an IgG1 Fc polypeptide. In some cases, the Ig Fc polypeptide is an IgG2 Fc polypeptide. In some cases, the Ig Fc polypeptide is an IgG3 Fc polypeptide. In some cases, the Ig Fc polypeptide is an IgA Fc polypeptide or an IgM Fc polypeptide. In some cases, MHC Class II polypeptides are used in place of the MHC Class I polypeptides. In some cases, the multimeric polypeptide includes an epitope tag and/or an affinity domain C-terminal to the Fc polypeptide;
9) a multimeric polypeptide comprising: a) a first polypeptide comprising, in order from N-terminus to C-terminus: i) a T-cell epitope; and ii) an MHC Class I β2-microglobulin polypeptide; and b) a second polypeptide comprising, in order from N-terminus to C-terminus: i) i) a PD-L1 polypeptide; ii) an MHC Class I heavy chain polypeptide; and iii) an Ig Fc polypeptide. In some cases, the first polypeptide and the second polypeptide are disulfide linked to one another. In some cases, the first polypeptide comprises a linker polypeptide between the epitope and the β2-microglobulin polypeptide. In some cases, the first polypeptide and the second polypeptide are disulfide linked to one another via a cysteine residue present in the linker polypeptide, and a cysteine residue present in the MHC Class I heavy chain polypeptide. In some cases, the first polypeptide and the second polypeptide are disulfide linked to one another via a cysteine residue present in the MHC Class I β2-microglobulin polypeptide, and a cysteine residue present in the MHC Class I heavy chain polypeptide; in some of these embodiments, the MHC Class I β2-microglobulin polypeptide and/or the MHC Class I heavy chain polypeptide include an amino acid substitution to provide a cysteine that participates in the disulfide bond. In some cases, the Ig Fc polypeptide is an IgG1 Fc polypeptide. In some cases, the Ig Fc polypeptide is an IgG2 Fc polypeptide. In some cases, the Ig Fc polypeptide is an IgG3 Fc polypeptide. In some cases, the Ig Fc polypeptide is an IgA Fc polypeptide or an IgM Fc polypeptide. In some cases, MHC Class II polypeptides are used in place of the MHC Class I polypeptides. In some cases, the multimeric polypeptide includes an epitope tag and/or an affinity domain C-terminal to the Fc polypeptide;
10) a multimeric polypeptide comprising: a) a first polypeptide comprising, in order from N-terminus to C-terminus: i) a T-cell epitope; and ii) an MHC Class I β2-microglobulin polypeptide; and b) a second polypeptide comprising, in order from N-terminus to C-terminus: i) i) an ICOS-L polypeptide; ii) an MHC Class I heavy chain polypeptide; and iii) an Ig Fc polypeptide. In some cases, the first polypeptide and the second polypeptide are disulfide linked to one another. In some cases, the first polypeptide comprises a linker polypeptide between the epitope and the β2-microglobulin polypeptide. In some cases, the first polypeptide and the second polypeptide are disulfide linked to one another via a cysteine residue present in the linker polypeptide, and a cysteine residue present in the MHC Class I heavy chain polypeptide. In some cases, the first polypeptide and the second polypeptide are disulfide linked to one another via a cysteine residue present in the MHC Class I β2-microglobulin polypeptide, and a cysteine residue present in the MHC Class I heavy chain polypeptide; in some of these embodiments, the MHC Class I β2-microglobulin polypeptide and/or the MHC Class I heavy chain polypeptide include an amino acid substitution to provide a cysteine that participates in the disulfide bond. In some cases, the Ig Fc polypeptide is an IgG1 Fc polypeptide. In some cases, the Ig Fc polypeptide is an IgG2 Fc polypeptide. In some cases, the Ig Fc polypeptide is an IgG3 Fc polypeptide. In some cases, the Ig Fc polypeptide is an IgA Fc polypeptide or an IgM Fc polypeptide. In some cases, MHC Class II polypeptides are used in place of the MHC Class I polypeptides. In some cases, the multimeric polypeptide includes an epitope tag and/or an affinity domain C-terminal to the Fc polypeptide;
11) a multimeric polypeptide comprising: a) a first polypeptide comprising, in order from N-terminus to C-terminus: i) a T-cell epitope; and ii) an MHC Class I β2-microglobulin polypeptide; and b) a second polypeptide comprising, in order from N-terminus to C-terminus: i) i) an OX40L polypeptide; ii) an MHC Class I heavy chain polypeptide; and iii) an Ig Fc polypeptide. In some cases, the first polypeptide and the second polypeptide are disulfide linked to one another. In some cases, the first polypeptide comprises a linker polypeptide between the epitope and the β2-microglobulin polypeptide. In some cases, the first polypeptide and the second polypeptide are disulfide linked to one another via a cysteine residue present in the linker polypeptide, and a cysteine residue present in the MHC Class I heavy chain polypeptide. In some cases, the first polypeptide and the second polypeptide are disulfide linked to one another via a cysteine residue present in the MHC Class I β2-microglobulin polypeptide, and a cysteine residue present in the MHC Class I heavy chain polypeptide; in some of these embodiments, the MHC Class I β2-microglobulin polypeptide and/or the MHC Class I heavy chain polypeptide include an amino acid substitution to provide a cysteine that participates in the disulfide bond. In some cases, the Ig Fc polypeptide is an IgG1 Fc polypeptide. In some cases, the Ig Fc polypeptide is an IgG2 Fc polypeptide. In some cases, the Ig Fc polypeptide is an IgG3 Fc polypeptide. In some cases, the Ig Fc polypeptide is an IgA Fc polypeptide or an IgM Fc polypeptide. In some cases, MHC Class II polypeptides are used in place of the MHC Class I polypeptides. In some cases, the multimeric polypeptide includes an epitope tag and/or an affinity domain C-terminal to the Fc polypeptide;
12) a multimeric polypeptide comprising: a) a first polypeptide comprising, in order from N-terminus to C-terminus: i) a T-cell epitope; and ii) an MHC Class I β2-microglobulin polypeptide; and b) a second polypeptide comprising, in order from N-terminus to C-terminus: i) i) a CD80 polypeptide; ii) an MHC Class I heavy chain polypeptide; and iii) an Ig Fc polypeptide. In some cases, the first polypeptide and the second polypeptide are disulfide linked to one another. In some cases, the first polypeptide comprises a linker polypeptide between the epitope and the β2-microglobulin polypeptide. In some cases, the first polypeptide and the second polypeptide are disulfide linked to one another via a cysteine residue present in the linker polypeptide, and a cysteine residue present in the MHC Class I heavy chain polypeptide. In some cases, the first polypeptide and the second polypeptide are disulfide linked to one another via a cysteine residue present in the MHC Class I β2-microglobulin polypeptide, and a cysteine residue present in the MHC Class I heavy chain polypeptide; in some of these embodiments, the MHC Class I β2-microglobulin polypeptide and/or the MHC Class I heavy chain polypeptide include an amino acid substitution to provide a cysteine that participates in the disulfide bond. In some cases, the Ig Fc polypeptide is an IgG1 Fc polypeptide. In some cases, the Ig Fc polypeptide is an IgG2 Fc polypeptide. In some cases, the Ig Fc polypeptide is an IgG3 Fc polypeptide. In some cases, the Ig Fc polypeptide is an IgA Fc polypeptide or an IgM Fc polypeptide. In some cases, MHC Class II polypeptides are used in place of the MHC Class I polypeptides. In some cases, the multimeric polypeptide includes an epitope tag and/or an affinity domain C-terminal to the Fc polypeptide;
13) a multimeric polypeptide comprising: a) a first polypeptide comprising, in order from N-terminus to C-terminus: i) a T-cell epitope; and ii) an MHC Class I β2-microglobulin polypeptide; and b) a second polypeptide comprising, in order from N-terminus to C-terminus: i) i) a CD86 polypeptide; ii) an MHC Class I heavy chain polypeptide; and iii) an Ig Fc polypeptide. In some cases, the first polypeptide and the second polypeptide are disulfide linked to one another. In some cases, the first polypeptide comprises a linker polypeptide between the epitope and the β2-microglobulin polypeptide. In some cases, the first polypeptide and the second polypeptide are disulfide linked to one another via a cysteine residue present in the linker polypeptide, and a cysteine residue present in the MHC Class I heavy chain polypeptide. In some cases, the first polypeptide and the second polypeptide are disulfide linked to one another via a cysteine residue present in the MHC Class I β2-microglobulin polypeptide, and a cysteine residue present in the MHC Class I heavy chain polypeptide; in some of these embodiments, the MHC Class I β2-microglobulin polypeptide and/or the MHC Class I heavy chain polypeptide include an amino acid substitution to provide a cysteine that participates in the disulfide bond. In some cases, the Ig Fc polypeptide is an IgG1 Fc polypeptide. In some cases, the Ig Fc polypeptide is an IgG2 Fc polypeptide. In some cases, the Ig Fc polypeptide is an IgG3 Fc polypeptide. In some cases, the Ig Fc polypeptide is an IgA Fc polypeptide or an IgM Fc polypeptide. In some cases, MHC Class II polypeptides are used in place of the MHC Class I polypeptides. In some cases, the multimeric polypeptide includes an epitope tag and/or an affinity domain C-terminal to the Fc polypeptide;
14) a multimeric polypeptide comprising: a) a first polypeptide comprising, in order from N-terminus to C-terminus: i) a T-cell epitope; and ii) an MHC Class I β2-microglobulin polypeptide; and b) a second polypeptide comprising, in order from N-terminus to C-terminus: i) i) a PD-L2 polypeptide; ii) an MHC Class I heavy chain polypeptide; and iii) an Ig Fc polypeptide. In some cases, the first polypeptide and the second polypeptide are disulfide linked to one another. In some cases, the first polypeptide comprises a linker polypeptide between the epitope and the β2-microglobulin polypeptide. In some cases, the first polypeptide and the second polypeptide are disulfide linked to one another via a cysteine residue present in the linker polypeptide, and a cysteine residue present in the MHC Class I heavy chain polypeptide. In some cases, the first polypeptide and the second polypeptide are disulfide linked to one another via a cysteine residue present in the MHC Class I β2-microglobulin polypeptide, and a cysteine residue present in the MHC Class I heavy chain polypeptide; in some of these embodiments, the MHC Class I β2-microglobulin polypeptide and/or the MHC Class I heavy chain polypeptide include an amino acid substitution to provide a cysteine that participates in the disulfide bond. In some cases, the Ig Fc polypeptide is an IgG1 Fc polypeptide. In some cases, the Ig Fc polypeptide is an IgG2 Fc polypeptide. In some cases, the Ig Fc polypeptide is an IgG3 Fc polypeptide. In some cases, the Ig Fc polypeptide is an IgA Fc polypeptide or an IgM Fc polypeptide. In some cases, MHC Class II polypeptides are used in place of the MHC Class I polypeptides. In some cases, the multimeric polypeptide includes an epitope tag and/or an affinity domain C-terminal to the Fc polypeptide;
15) a multimeric polypeptide comprising: a) a first polypeptide comprising, in order from N-terminus to C-terminus: i) a T-cell epitope; and ii) an MHC Class I β2-microglobulin polypeptide; and b) a second polypeptide comprising, in order from N-terminus to C-terminus: i) i) a FasL polypeptide; ii) an MHC Class I heavy chain polypeptide; and iii) an Ig Fc polypeptide. In some cases, the first polypeptide and the second polypeptide are disulfide linked to one another. In some cases, the first polypeptide comprises a linker polypeptide between the epitope and the β2-microglobulin polypeptide. In some cases, the first polypeptide and the second polypeptide are disulfide linked to one another via a cysteine residue present in the linker polypeptide, and a cysteine residue present in the MHC Class I heavy chain polypeptide. In some cases, the first polypeptide and the second polypeptide are disulfide linked to one another via a cysteine residue present in the MHC Class I β2-microglobulin polypeptide, and a cysteine residue present in the MHC Class I heavy chain polypeptide; in some of these embodiments, the MHC Class I β2-microglobulin polypeptide and/or the MHC Class I heavy chain polypeptide include an amino acid substitution to provide a cysteine that participates in the disulfide bond. In some cases, the Ig Fc polypeptide is an IgG1 Fc polypeptide. In some cases, the Ig Fc polypeptide is an IgG2 Fc polypeptide. In some cases, the Ig Fc polypeptide is an IgG3 Fc polypeptide. In some cases, the Ig Fc polypeptide is an IgA Fc polypeptide or an IgM Fc polypeptide. In some cases, MHC Class II polypeptides are used in place of the MHC Class I polypeptides. In some cases, the multimeric polypeptide includes an epitope tag and/or an affinity domain C-terminal to the Fc polypeptide;
16) a multimeric polypeptide comprising: a) a first polypeptide comprising, in order from N-terminus to C-terminus: i) a T-cell epitope; ii) an MHC Class I β2-microglobulin polypeptide; and iii) two 4-BBL polypeptides in tandem; and b) a second polypeptide comprising, in order from N-terminus to C-terminus: i) an MHC Class I heavy chain polypeptide; and ii) an Ig Fc polypeptide. In some cases, the first polypeptide and the second polypeptide are disulfide linked to one another. In some cases, the first polypeptide comprises a linker polypeptide between the epitope and the β2-microglobulin polypeptide. In some cases, the first polypeptide and the second polypeptide are disulfide linked to one another via a cysteine residue present in the linker polypeptide, and a cysteine residue present in the MHC Class I heavy chain polypeptide. In some cases, the first polypeptide and the second polypeptide are disulfide linked to one another via a cysteine residue present in the MHC Class I β2-microglobulin polypeptide, and a cysteine residue present in the MHC Class I heavy chain polypeptide; in some of these embodiments, the MHC Class I β2-microglobulin polypeptide and/or the MHC Class I heavy chain polypeptide include an amino acid substitution to provide a cysteine that participates in the disulfide bond. In some cases, the Ig Fc polypeptide is an IgG1 Fc polypeptide. In some cases, the Ig Fc polypeptide is an IgG2 Fc polypeptide. In some cases, the Ig Fc polypeptide is an IgG3 Fc polypeptide. In some cases, the Ig Fc polypeptide is an IgA Fc polypeptide or an IgM Fc polypeptide. In some cases, MHC Class II polypeptides are used in place of the MHC Class I polypeptides. In some cases, the multimeric polypeptide includes an epitope tag and/or an affinity domain C-terminal to the Fc polypeptide;
17) a multimeric polypeptide comprising: a) a first polypeptide comprising, in order from N-terminus to C-terminus: i) a T-cell epitope; ii) an MHC Class I β2-microglobulin polypeptide; and iii) two PD-L1 polypeptides in tandem; and b) a second polypeptide comprising, in order from N-terminus to C-terminus: i) an MHC Class I heavy chain polypeptide; and ii) an Ig Fc polypeptide. In some cases, the first polypeptide and the second polypeptide are disulfide linked to one another. In some cases, the first polypeptide comprises a linker polypeptide between the epitope and the β2-microglobulin polypeptide. In some cases, the first polypeptide and the second polypeptide are disulfide linked to one another via a cysteine residue present in the linker polypeptide, and a cysteine residue present in the MHC Class I heavy chain polypeptide. In some cases, the first polypeptide and the second polypeptide are disulfide linked to one another via a cysteine residue present in the MHC Class I β2-microglobulin polypeptide, and a cysteine residue present in the MHC Class I heavy chain polypeptide; in some of these embodiments, the MHC Class I β2-microglobulin polypeptide and/or the MHC Class I heavy chain polypeptide include an amino acid substitution to provide a cysteine that participates in the disulfide bond. In some cases, the Ig Fc polypeptide is an IgG1 Fc polypeptide. In some cases, the Ig Fc polypeptide is an IgG2 Fc polypeptide. In some cases, the Ig Fc polypeptide is an IgG3 Fc polypeptide. In some cases, the Ig Fc polypeptide is an IgA Fc polypeptide or an IgM Fc polypeptide. In some cases, MHC Class II polypeptides are used in place of the MHC Class I polypeptides. In some cases, the multimeric polypeptide includes an epitope tag and/or an affinity domain C-terminal to the Fc polypeptide;
18) a multimeric polypeptide comprising: a) a first polypeptide comprising, in order from N-terminus to C-terminus: i) a T-cell epitope; ii) an MHC Class I β2-microglobulin polypeptide; and iii) two ICOS-L polypeptides in tandem; and b) a second polypeptide comprising, in order from N-terminus to C-terminus: i) an MHC Class I heavy chain polypeptide; and ii) an Ig Fc polypeptide. In some cases, the first polypeptide and the second polypeptide are disulfide linked to one another. In some cases, the first polypeptide comprises a linker polypeptide between the epitope and the β2-microglobulin polypeptide. In some cases, the first polypeptide and the second polypeptide are disulfide linked to one another via a cysteine residue present in the linker polypeptide, and a cysteine residue present in the MHC Class I heavy chain polypeptide. In some cases, the first polypeptide and the second polypeptide are disulfide linked to one another via a cysteine residue present in the MHC Class I β2-microglobulin polypeptide, and a cysteine residue present in the MHC Class I heavy chain polypeptide; in some of these embodiments, the MHC Class I β2-microglobulin polypeptide and/or the MHC Class I heavy chain polypeptide include an amino acid substitution to provide a cysteine that participates in the disulfide bond. In some cases, the Ig Fc polypeptide is an IgG1 Fc polypeptide. In some cases, the Ig Fc polypeptide is an IgG2 Fc polypeptide. In some cases, the Ig Fc polypeptide is an IgG3 Fc polypeptide. In some cases, the Ig Fc polypeptide is an IgA Fc polypeptide or an IgM Fc polypeptide. In some cases, MHC Class II polypeptides are used in place of the MHC Class I polypeptides. In some cases, the multimeric polypeptide includes an epitope tag and/or an affinity domain C-terminal to the Fc polypeptide;
19) a multimeric polypeptide comprising: a) a first polypeptide comprising, in order from N-terminus to C-terminus: i) a T-cell epitope; ii) an MHC Class I β2-microglobulin polypeptide; and iii) two OX40L polypeptides in tandem; and b) a second polypeptide comprising, in order from N-terminus to C-terminus: i) an MHC Class I heavy chain polypeptide; and ii) an Ig Fc polypeptide. In some cases, the first polypeptide and the second polypeptide are disulfide linked to one another. In some cases, the first polypeptide comprises a linker polypeptide between the epitope and the β2-microglobulin polypeptide. In some cases, the first polypeptide and the second polypeptide are disulfide linked to one another via a cysteine residue present in the linker polypeptide, and a cysteine residue present in the MHC Class I heavy chain polypeptide. In some cases, the first polypeptide and the second polypeptide are disulfide linked to one another via a cysteine residue present in the MHC Class I β2-microglobulin polypeptide, and a cysteine residue present in the MHC Class I heavy chain polypeptide; in some of these embodiments, the MHC Class I β2-microglobulin polypeptide and/or the MHC Class I heavy chain polypeptide include an amino acid substitution to provide a cysteine that participates in the disulfide bond. In some cases, the Ig Fc polypeptide is an IgG1 Fc polypeptide. In some cases, the Ig Fc polypeptide is an IgG2 Fc polypeptide. In some cases, the Ig Fc polypeptide is an IgG3 Fc polypeptide. In some cases, the Ig Fc polypeptide is an IgA Fc polypeptide or an IgM Fc polypeptide. In some cases, MHC Class II polypeptides are used in place of the MHC Class I polypeptides. In some cases, the multimeric polypeptide includes an epitope tag and/or an affinity domain C-terminal to the Fc polypeptide;
20) a multimeric polypeptide comprising: a) a first polypeptide comprising, in order from N-terminus to C-terminus: i) a T-cell epitope; ii) an MHC Class I β2-microglobulin polypeptide; and iii) two CD80 polypeptides in tandem; and b) a second polypeptide comprising, in order from N-terminus to C-terminus: i) an MHC Class I heavy chain polypeptide; and ii) an Ig Fc polypeptide. In some cases, the first polypeptide and the second polypeptide are disulfide linked to one another. In some cases, the first polypeptide comprises a linker polypeptide between the epitope and the β2-microglobulin polypeptide. In some cases, the first polypeptide and the second polypeptide are disulfide linked to one another via a cysteine residue present in the linker polypeptide, and a cysteine residue present in the MHC Class I heavy chain polypeptide. In some cases, the first polypeptide and the second polypeptide are disulfide linked to one another via a cysteine residue present in the MHC Class I β2-microglobulin polypeptide, and a cysteine residue present in the MHC Class I heavy chain polypeptide; in some of these embodiments, the MHC Class I β2-microglobulin polypeptide and/or the MHC Class I heavy chain polypeptide include an amino acid substitution to provide a cysteine that participates in the disulfide bond. In some cases, the Ig Fc polypeptide is an IgG1 Fc polypeptide. In some cases, the Ig Fc polypeptide is an IgG2 Fc polypeptide. In some cases, the Ig Fc polypeptide is an IgG3 Fc polypeptide. In some cases, the Ig Fc polypeptide is an IgA Fc polypeptide or an IgM Fc polypeptide. In some cases, MHC Class II polypeptides are used in place of the MHC Class I polypeptides. In some cases, the multimeric polypeptide includes an epitope tag and/or an affinity domain C-terminal to the Fc polypeptide;
21) a multimeric polypeptide comprising: a) a first polypeptide comprising, in order from N-terminus to C-terminus: i) a T-cell epitope; ii) an MHC Class I β2-microglobulin polypeptide; and iii) two CD86 polypeptides in tandem; and b) a second polypeptide comprising, in order from N-terminus to C-terminus: i) an MHC Class I heavy chain polypeptide; and ii) an Ig Fc polypeptide. In some cases, the first polypeptide and the second polypeptide are disulfide linked to one another. In some cases, the first polypeptide comprises a linker polypeptide between the epitope and the β2-microglobulin polypeptide. In some cases, the first polypeptide and the second polypeptide are disulfide linked to one another via a cysteine residue present in the linker polypeptide, and a cysteine residue present in the MHC Class I heavy chain polypeptide. In some cases, the first polypeptide and the second polypeptide are disulfide linked to one another via a cysteine residue present in the MHC Class I β2-microglobulin polypeptide, and a cysteine residue present in the MHC Class I heavy chain polypeptide; in some of these embodiments, the MHC Class I β2-microglobulin polypeptide and/or the MHC Class I heavy chain polypeptide include an amino acid substitution to provide a cysteine that participates in the disulfide bond. In some cases, the Ig Fc polypeptide is an IgG1 Fc polypeptide. In some cases, the Ig Fc polypeptide is an IgG2 Fc polypeptide. In some cases, the Ig Fc polypeptide is an IgG3 Fc polypeptide. In some cases, the Ig Fc polypeptide is an IgA Fc polypeptide or an IgM Fc polypeptide. In some cases, MHC Class II polypeptides are used in place of the MHC Class I polypeptides. In some cases, the multimeric polypeptide includes an epitope tag and/or an affinity domain C-terminal to the Fc polypeptide;
22) a multimeric polypeptide comprising: a) a first polypeptide comprising, in order from N-terminus to C-terminus: i) a T-cell epitope; ii) an MHC Class I β2-microglobulin polypeptide; and iii) two PD-L2 polypeptides in tandem; and b) a second polypeptide comprising, in order from N-terminus to C-terminus: i) an MHC Class I heavy chain polypeptide; and ii) an Ig Fc polypeptide. In some cases, the first polypeptide and the second polypeptide are disulfide linked to one another. In some cases, the first polypeptide comprises a linker polypeptide between the epitope and the β2-microglobulin polypeptide. In some cases, the first polypeptide and the second polypeptide are disulfide linked to one another via a cysteine residue present in the linker polypeptide, and a cysteine residue present in the MHC Class I heavy chain polypeptide. In some cases, the first polypeptide and the second polypeptide are disulfide linked to one another via a cysteine residue present in the MHC Class I β2-microglobulin polypeptide, and a cysteine residue present in the MHC Class I heavy chain polypeptide; in some of these embodiments, the MHC Class I β2-microglobulin polypeptide and/or the MHC Class I heavy chain polypeptide include an amino acid substitution to provide a cysteine that participates in the disulfide bond. In some cases, the Ig Fc polypeptide is an IgG1 Fc polypeptide. In some cases, the Ig Fc polypeptide is an IgG2 Fc polypeptide. In some cases, the Ig Fc polypeptide is an IgG3 Fc polypeptide. In some cases, the Ig Fc polypeptide is an IgA Fc polypeptide or an IgM Fc polypeptide. In some cases, MHC Class II polypeptides are used in place of the MHC Class I polypeptides. In some cases, the multimeric polypeptide includes an epitope tag and/or an affinity domain C-terminal to the Fc polypeptide;
23) a multimeric polypeptide comprising: a) a first polypeptide comprising, in order from N-terminus to C-terminus: i) a T-cell epitope; ii) an MHC Class I β2-microglobulin polypeptide; and iii) two FasL polypeptides in tandem; and b) a second polypeptide comprising, in order from N-terminus to C-terminus: i) an MHC Class I heavy chain polypeptide; and ii) an Ig Fc polypeptide. In some cases, the first polypeptide and the second polypeptide are disulfide linked to one another. In some cases, the first polypeptide comprises a linker polypeptide between the epitope and the β2-microglobulin polypeptide. In some cases, the first polypeptide and the second polypeptide are disulfide linked to one another via a cysteine residue present in the linker polypeptide, and a cysteine residue present in the MHC Class I heavy chain polypeptide. In some cases, the first polypeptide and the second polypeptide are disulfide linked to one another via a cysteine residue present in the MHC Class I β2-microglobulin polypeptide, and a cysteine residue present in the MHC Class I heavy chain polypeptide; in some of these embodiments, the MHC Class I β2-microglobulin polypeptide and/or the MHC Class I heavy chain polypeptide include an amino acid substitution to provide a cysteine that participates in the disulfide bond. In some cases, the Ig Fc polypeptide is an IgG1 Fc polypeptide. In some cases, the Ig Fc polypeptide is an IgG2 Fc polypeptide. In some cases, the Ig Fc polypeptide is an IgG3 Fc polypeptide. In some cases, the Ig Fc polypeptide is an IgA Fc polypeptide or an IgM Fc polypeptide. In some cases, MHC Class II polypeptides are used in place of the MHC Class I polypeptides. In some cases, the multimeric polypeptide includes an epitope tag and/or an affinity domain C-terminal to the Fc polypeptide;
24) a multimeric polypeptide comprising: a) a first polypeptide comprising, in order from N-terminus to C-terminus: i) a T-cell epitope; ii) an MHC Class I β2-microglobulin polypeptide; and iii) a first 4-1BBL polypeptide; b) a second polypeptide comprising, in order from N-terminus to C-terminus: i) an MHC Class I heavy chain polypeptide; and ii) an Ig Fc polypeptide; and c) a third polypeptide comprising a second 4-1BBL polypeptide. In some cases, the first polypeptide and the second polypeptide are disulfide linked to one another. In some cases, the first polypeptide and the second polypeptide are disulfide linked to one another; and the first and the third polypeptides are disulfide linked to one another. In some cases, the first polypeptide comprises a linker polypeptide between the epitope and the β2-microglobulin polypeptide. In some cases, the first polypeptide and the second polypeptide are disulfide linked to one another via a cysteine residue present in the linker polypeptide, and a cysteine residue present in the MHC Class I heavy chain polypeptide. In some cases, the first polypeptide and the second polypeptide are disulfide linked to one another via a cysteine residue present in the MHC Class I β2-microglobulin polypeptide, and a cysteine residue present in the MHC Class I heavy chain polypeptide; in some of these embodiments, the MHC Class I β2-microglobulin polypeptide and/or the MHC Class I heavy chain polypeptide include an amino acid substitution to provide a cysteine that participates in the disulfide bond. In some cases, the first polypeptide and the third polypeptide are disulfide linked to one another via a cysteine residue present in (or substituted into) the first and the second 4-1BBL polypeptides. In some cases, the Ig Fc polypeptide is an IgG1 Fc polypeptide. In some cases, the Ig Fc polypeptide is an IgG2 Fc polypeptide. In some cases, the Ig Fc polypeptide is an IgG3 Fc polypeptide. In some cases, the Ig Fc polypeptide is an IgA Fc polypeptide or an IgM Fc polypeptide. In some cases, MHC Class II polypeptides are used in place of the MHC Class I polypeptides. In some cases, the multimeric polypeptide includes an epitope tag and/or an affinity domain C-terminal to the Fc polypeptide;
25) a multimeric polypeptide comprising: a) a first polypeptide comprising, in order from N-terminus to C-terminus: i) a T-cell epitope; ii) an MHC Class I β2-microglobulin polypeptide; and iii) a first PD-L1 polypeptide; b) a second polypeptide comprising, in order from N-terminus to C-terminus: i) an MHC Class I heavy chain polypeptide; and ii) an Ig Fc polypeptide; and c) a third polypeptide comprising a second PD-L1 polypeptide. In some cases, the first polypeptide and the second polypeptide are disulfide linked to one another. In some cases, the first polypeptide and the second polypeptide are disulfide linked to one another; and the first and the third polypeptides are disulfide linked to one another. In some cases, the first polypeptide comprises a linker polypeptide between the epitope and the β2-microglobulin polypeptide. In some cases, the first polypeptide and the second polypeptide are disulfide linked to one another via a cysteine residue present in the linker polypeptide, and a cysteine residue present in the MHC Class I heavy chain polypeptide. In some cases, the first polypeptide and the second polypeptide are disulfide linked to one another via a cysteine residue present in the MHC Class I β2-microglobulin polypeptide, and a cysteine residue present in the MHC Class I heavy chain polypeptide; in some of these embodiments, the MHC Class I β2-microglobulin polypeptide and/or the MHC Class I heavy chain polypeptide include an amino acid substitution to provide a cysteine that participates in the disulfide bond. In some cases, the first polypeptide and the third polypeptide are disulfide linked to one another via a cysteine residue present in (or substituted into) the first and the second PD-L1 polypeptides. In some cases, the Ig Fc polypeptide is an IgG1 Fc polypeptide. In some cases, the Ig Fc polypeptide is an IgG2 Fc polypeptide. In some cases, the Ig Fc polypeptide is an IgG3 Fc polypeptide. In some cases, the Ig Fc polypeptide is an IgA Fc polypeptide or an IgM Fc polypeptide. In some cases, MHC Class II polypeptides are used in place of the MHC Class I polypeptides. In some cases, the multimeric polypeptide includes an epitope tag and/or an affinity domain C-terminal to the Fc polypeptide;
26) a multimeric polypeptide comprising: a) a first polypeptide comprising, in order from N-terminus to C-terminus: i) a T-cell epitope; ii) an MHC Class I β2-microglobulin polypeptide; and iii) a first ICOS-L polypeptide; b) a second polypeptide comprising, in order from N-terminus to C-terminus: i) an MHC Class I heavy chain polypeptide; and ii) an Ig Fc polypeptide; and c) a third polypeptide comprising a second ICOS-L polypeptide. In some cases, the first polypeptide and the second polypeptide are disulfide linked to one another. In some cases, the first polypeptide and the second polypeptide are disulfide linked to one another; and the first and the third polypeptides are disulfide linked to one another. In some cases, the first polypeptide comprises a linker polypeptide between the epitope and the β2-microglobulin polypeptide. In some cases, the first polypeptide and the second polypeptide are disulfide linked to one another via a cysteine residue present in the linker polypeptide, and a cysteine residue present in the MHC Class I heavy chain polypeptide. In some cases, the first polypeptide and the second polypeptide are disulfide linked to one another via a cysteine residue present in the MHC Class I β2-microglobulin polypeptide, and a cysteine residue present in the MHC Class I heavy chain polypeptide; in some of these embodiments, the MHC Class I β2-microglobulin polypeptide and/or the MHC Class I heavy chain polypeptide include an amino acid substitution to provide a cysteine that participates in the disulfide bond. In some cases, the first polypeptide and the third polypeptide are disulfide linked to one another via a cysteine residue present in (or substituted into) the first and the second ICOS-L polypeptides. In some cases, the Ig Fc polypeptide is an IgG1 Fc polypeptide. In some cases, the Ig Fc polypeptide is an IgG2 Fc polypeptide. In some cases, the Ig Fc polypeptide is an IgG3 Fc polypeptide. In some cases, the Ig Fc polypeptide is an IgA Fc polypeptide or an IgM Fc polypeptide. In some cases, MHC Class II polypeptides are used in place of the MHC Class I polypeptides. In some cases, the multimeric polypeptide includes an epitope tag and/or an affinity domain C-terminal to the Fc polypeptide;
27) a multimeric polypeptide comprising: a) a first polypeptide comprising, in order from N-terminus to C-terminus: i) a T-cell epitope; ii) an MHC Class I β2-microglobulin polypeptide; and iii) a first OX40L polypeptide; b) a second polypeptide comprising, in order from N-terminus to C-terminus: i) an MHC Class I heavy chain polypeptide; and ii) an Ig Fc polypeptide; and c) a third polypeptide comprising a second OX40L polypeptide. In some cases, the first polypeptide and the second polypeptide are disulfide linked to one another. In some cases, the first polypeptide and the second polypeptide are disulfide linked to one another; and the first and the third polypeptides are disulfide linked to one another. In some cases, the first polypeptide comprises a linker polypeptide between the epitope and the β2-microglobulin polypeptide. In some cases, the first polypeptide and the second polypeptide are disulfide linked to one another via a cysteine residue present in the linker polypeptide, and a cysteine residue present in the MHC Class I heavy chain polypeptide. In some cases, the first polypeptide and the second polypeptide are disulfide linked to one another via a cysteine residue present in the MHC Class I β2-microglobulin polypeptide, and a cysteine residue present in the MHC Class I heavy chain polypeptide; in some of these embodiments, the MHC Class I β2-microglobulin polypeptide and/or the MHC Class I heavy chain polypeptide include an amino acid substitution to provide a cysteine that participates in the disulfide bond. In some cases, the first polypeptide and the third polypeptide are disulfide linked to one another via a cysteine residue present in (or substituted into) the first and the second OX40L polypeptides. In some cases, the Ig Fc polypeptide is an IgG1 Fc polypeptide. In some cases, the Ig Fc polypeptide is an IgG2 Fc polypeptide. In some cases, the Ig Fc polypeptide is an IgG3 Fc polypeptide. In some cases, the Ig Fc polypeptide is an IgA Fc polypeptide or an IgM Fc polypeptide. In some cases, MHC Class II polypeptides are used in place of the MHC Class I polypeptides. In some cases, the multimeric polypeptide includes an epitope tag and/or an affinity domain C-terminal to the Fc polypeptide;
28) a multimeric polypeptide comprising: a) a first polypeptide comprising, in order from N-terminus to C-terminus: i) a T-cell epitope; ii) an MHC Class I β2-microglobulin polypeptide; and iii) a first CD80 polypeptide; b) a second polypeptide comprising, in order from N-terminus to C-terminus: i) an MHC Class I heavy chain polypeptide; and ii) an Ig Fc polypeptide; and c) a third polypeptide comprising a second CD80 polypeptide. In some cases, the first polypeptide and the second polypeptide are disulfide linked to one another. In some cases, the first polypeptide and the second polypeptide are disulfide linked to one another; and the first and the third polypeptides are disulfide linked to one another. In some cases, the first polypeptide comprises a linker polypeptide between the epitope and the β2-microglobulin polypeptide. In some cases, the first polypeptide and the second polypeptide are disulfide linked to one another via a cysteine residue present in the linker polypeptide, and a cysteine residue present in the MHC Class I heavy chain polypeptide. In some cases, the first polypeptide and the second polypeptide are disulfide linked to one another via a cysteine residue present in the MHC Class I β2-microglobulin polypeptide, and a cysteine residue present in the MHC Class I heavy chain polypeptide; in some of these embodiments, the MHC Class I β2-microglobulin polypeptide and/or the MHC Class I heavy chain polypeptide include an amino acid substitution to provide a cysteine that participates in the disulfide bond. In some cases, the first polypeptide and the third polypeptide are disulfide linked to one another via a cysteine residue present in (or substituted into) the first and the second CD80 polypeptides. In some cases, the Ig Fc polypeptide is an IgG1 Fc polypeptide. In some cases, the Ig Fc polypeptide is an IgG2 Fc polypeptide. In some cases, the Ig Fc polypeptide is an IgG3 Fc polypeptide. In some cases, the Ig Fc polypeptide is an IgA Fc polypeptide or an IgM Fc polypeptide. In some cases, MHC Class II polypeptides are used in place of the MHC Class I polypeptides. In some cases, the multimeric polypeptide includes an epitope tag and/or an affinity domain C-terminal to the Fc polypeptide;
29) a multimeric polypeptide comprising: a) a first polypeptide comprising, in order from N-terminus to C-terminus: i) a T-cell epitope; ii) an MHC Class I β2-microglobulin polypeptide; and iii) a first CD86 polypeptide; b) a second polypeptide comprising, in order from N-terminus to C-terminus: i) an MHC Class I heavy chain polypeptide; and ii) an Ig Fc polypeptide; and c) a third polypeptide comprising a second CD86 polypeptide. In some cases, the first polypeptide and the second polypeptide are disulfide linked to one another. In some cases, the first polypeptide and the second polypeptide are disulfide linked to one another; and the first and the third polypeptides are disulfide linked to one another. In some cases, the first polypeptide comprises a linker polypeptide between the epitope and the β2-microglobulin polypeptide. In some cases, the first polypeptide and the second polypeptide are disulfide linked to one another via a cysteine residue present in the linker polypeptide, and a cysteine residue present in the MHC Class I heavy chain polypeptide. In some cases, the first polypeptide and the second polypeptide are disulfide linked to one another via a cysteine residue present in the MHC Class I β2-microglobulin polypeptide, and a cysteine residue present in the MHC Class I heavy chain polypeptide; in some of these embodiments, the MHC Class I β2-microglobulin polypeptide and/or the MHC Class I heavy chain polypeptide include an amino acid substitution to provide a cysteine that participates in the disulfide bond. In some cases, the first polypeptide and the third polypeptide are disulfide linked to one another via a cysteine residue present in (or substituted into) the first and the second CD86 polypeptides. In some cases, the Ig Fc polypeptide is an IgG1 Fc polypeptide. In some cases, the Ig Fc polypeptide is an IgG2 Fc polypeptide. In some cases, the Ig Fc polypeptide is an IgG3 Fc polypeptide. In some cases, the Ig Fc polypeptide is an IgA Fc polypeptide or an IgM Fc polypeptide. In some cases, MHC Class II polypeptides are used in place of the MHC Class I polypeptides. In some cases, the multimeric polypeptide includes an epitope tag and/or an affinity domain C-terminal to the Fc polypeptide;
30) a multimeric polypeptide comprising: a) a first polypeptide comprising, in order from N-terminus to C-terminus: i) a T-cell epitope; ii) an MHC Class I β2-microglobulin polypeptide; and iii) a CD80 polypeptide; b) a second polypeptide comprising, in order from N-terminus to C-terminus: i) an MHC Class I heavy chain polypeptide; and ii) an Ig Fc polypeptide; and c) a third polypeptide comprising a CD86 polypeptide. In some cases, the first polypeptide and the second polypeptide are disulfide linked to one another. In some cases, the first polypeptide and the second polypeptide are disulfide linked to one another; and the first and the third polypeptides are disulfide linked to one another. In some cases, the first polypeptide comprises a linker polypeptide between the epitope and the β2-microglobulin polypeptide. In some cases, the first polypeptide and the second polypeptide are disulfide linked to one another via a cysteine residue present in the linker polypeptide, and a cysteine residue present in the MHC Class I heavy chain polypeptide. In some cases, the first polypeptide and the second polypeptide are disulfide linked to one another via a cysteine residue present in the MHC Class I β2-microglobulin polypeptide, and a cysteine residue present in the MHC Class I heavy chain polypeptide; in some of these embodiments, the MHC Class I β2-microglobulin polypeptide and/or the MHC Class I heavy chain polypeptide include an amino acid substitution to provide a cysteine that participates in the disulfide bond. In some cases, the first polypeptide and the third polypeptide are disulfide linked to one another via a cysteine residue present in (or substituted into) the CD80 polypeptide and the CD86 polypeptides. In some cases, the Ig Fc polypeptide is an IgG1 Fc polypeptide. In some cases, the Ig Fc polypeptide is an IgG2 Fc polypeptide. In some cases, the Ig Fc polypeptide is an IgG3 Fc polypeptide. In some cases, the Ig Fc polypeptide is an IgA Fc polypeptide or an IgM Fc polypeptide. In some cases, MHC Class II polypeptides are used in place of the MHC Class I polypeptides. In some cases, the multimeric polypeptide includes an epitope tag and/or an affinity domain C-terminal to the Fc polypeptide; and
31) a multimeric polypeptide comprising: a) a first polypeptide comprising, in order from N-terminus to C-terminus: i) a T-cell epitope; ii) an MHC Class I β2-microglobulin polypeptide; and iii) a first PD-L2 polypeptide; b) a second polypeptide comprising, in order from N-terminus to C-terminus: i) an MHC Class I heavy chain polypeptide; and ii) an Ig Fc polypeptide; and c) a third polypeptide comprising a second PD-L2 polypeptide. In some cases, the first polypeptide and the second polypeptide are disulfide linked to one another. In some cases, the first polypeptide and the second polypeptide are disulfide linked to one another; and the first and the third polypeptides are disulfide linked to one another. In some cases, the first polypeptide comprises a linker polypeptide between the epitope and the β2-microglobulin polypeptide. In some cases, the first polypeptide and the second polypeptide are disulfide linked to one another via a cysteine residue present in the linker polypeptide, and a cysteine residue present in the MHC Class I heavy chain polypeptide. In some cases, the first polypeptide and the second polypeptide are disulfide linked to one another via a cysteine residue present in the MHC Class I β2-microglobulin polypeptide, and a cysteine residue present in the MHC Class I heavy chain polypeptide; in some of these embodiments, the MHC Class I β2-microglobulin polypeptide and/or the MHC Class I heavy chain polypeptide include an amino acid substitution to provide a cysteine that participates in the disulfide bond. In some cases, the first polypeptide and the third polypeptide are disulfide linked to one another via a cysteine residue present in (or substituted into) the first and the second PD-L2 polypeptides. In some cases, the Ig Fc polypeptide is an IgG1 Fc polypeptide. In some cases, the Ig Fc polypeptide is an IgG2 Fc polypeptide. In some cases, the Ig Fc polypeptide is an IgG3 Fc polypeptide. In some cases, the Ig Fc polypeptide is an IgA Fc polypeptide or an IgM Fc polypeptide. In some cases, MHC Class II polypeptides are used in place of the MHC Class I polypeptides. In some cases, the multimeric polypeptide includes an epitope tag and/or an affinity domain C-terminal to the Fc polypeptide; and
32) a multimeric polypeptide comprising: a) a first polypeptide comprising, in order from N-terminus to C-terminus: i) a T-cell epitope; ii) an MHC Class I β2-microglobulin polypeptide; and iii) a first FasL polypeptide; b) a second polypeptide comprising, in order from N-terminus to C-terminus: i) an MHC Class I heavy chain polypeptide; and ii) an Ig Fc polypeptide; and c) a third polypeptide comprising a second FasL polypeptide. In some cases, the first polypeptide and the second polypeptide are disulfide linked to one another. In some cases, the first polypeptide and the second polypeptide are disulfide linked to one another; and the first and the third polypeptides are disulfide linked to one another. In some cases, the first polypeptide comprises a linker polypeptide between the epitope and the β2-microglobulin polypeptide. In some cases, the first polypeptide and the second polypeptide are disulfide linked to one another via a cysteine residue present in the linker polypeptide, and a cysteine residue present in the MHC Class I heavy chain polypeptide. In some cases, the first polypeptide and the second polypeptide are disulfide linked to one another via a cysteine residue present in the MHC Class I β2-microglobulin polypeptide, and a cysteine residue present in the MHC Class I heavy chain polypeptide; in some of these embodiments, the MHC Class I β2-microglobulin polypeptide and/or the MHC Class I heavy chain polypeptide include an amino acid substitution to provide a cysteine that participates in the disulfide bond. In some cases, the first polypeptide and the third polypeptide are disulfide linked to one another via a cysteine residue present in (or substituted into) the first and the second FasL polypeptides. In some cases, the Ig Fc polypeptide is an IgG1 Fc polypeptide. In some cases, the Ig Fc polypeptide is an IgG2 Fc polypeptide. In some cases, the Ig Fc polypeptide is an IgG3 Fc polypeptide. In some cases, the Ig Fc polypeptide is an IgA Fc polypeptide or an IgM Fc polypeptide. In some cases, MHC Class II polypeptides are used in place of the MHC Class I polypeptides. In some cases, the multimeric polypeptide includes an epitope tag and/or an affinity domain C-terminal to the Fc polypeptide.

### POLYPROTEIN PRECURSORS

This disclosure provides a recombinant polypeptide comprising a sequence of amino acids identical to a first B2M leader sequence contiguous with a candidate epitope peptide contiguous with a first amino acid linker sequence contiguous with a sequence of amino acids identical to a human native B2M peptide sequence contiguous with a second amino acid linker sequence contiguous with a T cell modulatory domain peptide sequence contiguous with a third amino acid linker contiguous with a second B2M leader sequence contiguous with a sequence of amino acids identical to a MHC heavy chain contiguous with a sequence of amino acids identical to an immunoglobulin Fc domain.

The first amino acid may be any sequence of amino acids 50 amino acids or less to a minimum of 5 amino acids. The second amino acid linker may be any sequence of amino acids 70 amino acids or less to a minimum of 5 amino acids. The third amino acid linker may be a viral 2A peptide, or a peptide with known protease cleavage ability (e.g., a furin cleavage site, Tobacco Etch Virus [TEV] sequence, Precission protease site, or thrombin protease). For example, the first amino acid comprises GGGGSGGGGSGGGGS (SEQ ID NO: 1). In another example, the second amino acid linker comprises GGGGSGGGGSGGGGSGGGGS (SEQ ID NO:2). In another example, the third amino acid linker comprises SGSGATNFSLLKQAGDVEENPGP (SEQ ID NO:3).

This disclosure also provides recombinant polypeptide comprising a sequence of amino acids identical to a first B2M leader sequence contiguous with a candidate epitope peptide contiguous with a first amino acid linker sequence contiguous with a sequence of amino acids identical to a human native B2M peptide sequence contiguous with a second amino acid linker sequence contiguous with a second B2M leader sequence contiguous with a T cell modulatory domain peptide sequence contiguous with a third amino acid linker contiguous with a sequence of amino acids identical to a MHC heavy chain contiguous with a sequence of amino acids identical to an immunoglobulin Fc domain.

### Linkers

The first amino acid may be any sequence of amino acids 50 amino acids or less to a minimum of 5 amino acids. The second amino acid linker may be any sequence of amino acids 70 amino acids or less to a minimum of 5 amino acids. The third amino acid linker may be a viral 2A peptide, or a peptide with known protease cleavage ability (e.g., a furin cleavage site, Tobacco Etch Virus [TEV] sequence, Precission protease site, or thrombin protease). For example, the first amino acid comprises GGGGSGGGGSGGGGS (SEQ ID NO: 1). In another example, the second amino acid linker comprises GGGGSGGGGSGGGGSGGGGS (SEQ ID NO:2). In a further example, the third amino acid linker comprises SGSGATNFSLLKQAGDVEENPGP (SEQ ID NO:3).

In an example of the recombinant polypeptides, the third amino acid linker is self-cleaving. In an example of the recombinant polypeptides, the second amino acid linker is self-cleaving. In an example of the recombinant polypeptides, the self-cleaving peptide is a viral 2A peptide or has the sequence thereof. In an embodiment, the viral 2A peptide is a porcine teschovirus-1 (P2A), foot- and-mouth disease virus (F2A), *Thosea asigna* virus (T2A), equine rhinitis A virus (E2A) or viral porcine teschovirus-1 (P2A) peptide, or has the sequence of one thereof. Alternatively, this can also be delivered as two separate plasmids (or viruses) removing the 2A sequence entirely.

The proteolytically cleavable linker can include a protease recognition sequence recognized by a protease selected from the group consisting of alanine carboxypeptidase, Armillaria mellea astacin, bacterial leucyl aminopeptidase, cancer procoagulant, cathepsin B, clostripain, cytosol alanyl aminopeptidase, elastase, endoproteinase Arg-C, enterokinase, gastricsin, gelatinase, Gly-X carboxypeptidase, glycyl endopeptidase, human rhinovirus 3C protease, hypodermin C, IgA-specific serine endopeptidase, leucyl aminopeptidase, leucyl endopeptidase, lysC, lysosomal pro-X carboxypeptidase, lysyl aminopeptidase, methionyl aminopeptidase, myxobacter, nardilysin, pancreatic endopeptidase E, picornain 2A, picornain 3C, proendopeptidase, prolyl aminopeptidase, proprotein convertase I, proprotein convertase II, russellysin, saccharopepsin, semenogelase, T-plasminogen activator, thrombin, tissue kallikrein, tobacco etch virus (TEV), togavirin, tryptophanyl aminopeptidase, U-plasminogen activator, V8, venombin A, venombin AB, and Xaa-pro aminopeptidase. In some cases, the proteolytically cleavable linker can include a protease recognition sequence recognized by a host enzyme, e.g., an enzyme naturally produced by the host cell.

For example, the proteolytically cleavable linker can comprise a matrix metalloproteinase cleavage site, e.g., a cleavage site for a MMP selected from collagenase-1, -2, and -3 (MMP-1, -8, and -13), gelatinase A and B (MMP-2 and -9), stromelysin 1, 2, and 3 (MMP-3, -10, and -11), matrilysin (MMP-7), and membrane metalloproteinases (MT1-MMP and MT2-MMP). For example, the cleavage sequence of MMP-9 is Pro-X-X-Hy (wherein, X represents an arbitrary residue; Hy, a hydrophobic residue), e.g., Pro-X-X-Hy-(Ser/Thr), e.g., Pro-Leu/Gln-Gly-Met-Thr-Ser (SEQ ID NO:33) or Pro-Leu/Gln-Gly-Met-Thr (SEQ ID NO:21). Another example of a protease cleavage site is a plasminogen activator cleavage site, e.g., a uPA or a tissue plasminogen activator (tPA) cleavage site. Specific examples of cleavage sequences of uPA and tPA include sequences comprising Val-Gly-Arg. Another example of a protease cleavage site that can be included in a proteolytically cleavable linker is a tobacco etch virus (TEV) protease cleavage site, e.g., ENLYTQS (SEQ ID NO:34), where the protease cleaves between the glutamine and the serine. Another example of a protease cleavage site that can be included in a proteolytically cleavable linker is an enterokinase cleavage site, e.g., DDDDK (SEQ ID NO:35), where cleavage occurs after the lysine residue. Another example of a protease cleavage site that can be included in a proteolytically cleavable linker is a thrombin cleavage site, e.g., LVPR (SEQ ID NO:36). Another example of a protease cleavage site that can be included in a proteolytically cleavable linker is a furin cleavage site, e.g., Arg-X-(Arg/Lys)-Arg, where X is any amino acid. Additional suitable linkers comprising protease cleavage sites include linkers comprising one or more of the following amino acid sequences: LEVLFQGP (SEQ ID NO:37), cleaved by PreScission protease (a fusion protein comprising human rhinovirus 3C protease and glutathione-S-transferase; Walker et al. (1994) Biotechnol. 12:601); a thrombin cleavage site, e.g., CGLVPAGSGP (SEQ ID NO:38); SLLKSRMVPNFN (SEQ ID NO:39) or SLLIARRMPNFN (SEQ ID NO:40), cleaved by cathepsin B; SKLVQASASGVN (SEQ ID NO:41) or SSYLKASDAPDN (SEQ ID NO:42), cleaved by an Epstein-Barr virus protease; RPKPQQFFGLMN (SEQ ID NO:43) cleaved by MMP-3 (stromelysin); SLRPLALWRSFN (SEQ ID NO:44) cleaved by MMP-7 (matrilysin); SPQGIAGQRNFN (SEQ ID NO:45) cleaved by MMP-9; DVDERDVRGFASFL SEQ ID NO:46) cleaved by a thermolysin-like MMP; SLPLGLWAPNFN (SEQ ID NO:47) cleaved by matrix metalloproteinase 2(MMP-2); SLLIFRSWANFN (SEQ ID NO:48) cleaved by cathespin L; SGVVIATVIVIT (SEQ ID NO:49) cleaved by cathepsin D; SLGPQGIWGQFN (SEQ ID NO:50) cleaved by matrix metalloproteinase 1(MMP-1); KKSPGRVVGGSV (SEQ ID NO:51) cleaved by urokinase-type plasminogen activator; PQGLLGAPGILG (SEQ ID NO:52) cleaved by membrane type 1 matrixmetalloproteinase (MT-MMP); HGPEGLRVGFYESDVMGRGHARLVHVEEPHT (SEQ ID NO:53) cleaved by stromelysin 3 (or MMP-11), thermolysin, fibroblast collagenase and stromelysin-1; GPQGLAGQRGIV (SEQ ID NO:54) cleaved by matrix metalloproteinase 13 (collagenase-3); GGSGQRGRKALE (SEQ ID NO:55) cleaved by tissue-type plasminogen activator(tPA); SLSALLSSDIFN (SEQ ID NO:56) cleaved by human prostate-specific antigen; SLPRFKIIGGFN (SEQ ID NO:57) cleaved by kallikrein (hK3); SLLGIAVPGNFN (SEQ ID NO:58) cleaved by neutrophil elastase; and FFKNIVTPRTPP (SEQ ID NO:59) cleaved by calpain (calcium activated neutral protease). Additional examples suitable proteolytically cleavable linkers include: 1) ATNFSLLKQAGDVEENPGP (SEQ ID NO:60); 2) EGRGSLLTCGDVEENPGP (SEQ ID NO:61); 3) QCTNYALLKLAGDVESNPGP (SEQ ID NO:62); and 4) VKQTLNFDLLKLAGDVESNPGP (SEQ ID NO:63). Additional examples suitable proteolytically cleavable linkers include: 1) GSGATNFSLLKQAGDVEENPGP (SEQ ID NO:64); 2) GSGEGRGSLLTCGDVEENPGP (SEQ ID NO:65); 3) GSGQCTNYALLKLAGDVESNPGP (SEQ ID NO:66); and 4) GSGVKQTLNFDLLKLAGDVESNPGP (SEQ ID NO:67).

Examples of suitable linkers include 2A linkers (for example T2A), 2A-like linkers or functional equivalents thereof and combinations thereof. In some embodiments, the linkers include the picornaviral 2A-like linker, CHYSEL sequences of porcine teschovirus (P2A), Thosea asigna virus (T2A), and combinations, variants, and functional equivalents thereof. In other embodiments, the linker sequences may comprise Asp-Val/Ile-Glu-X-Asn-Pro-Gly^{2A}-Pro^{2B} motif, which results in cleavage between the 2A glycine and the 2B proline. For the purposes of the present disclosure, P2A (GSGATNFSLLKQAGDVEENPGP (SEQ ID NO:64)), T2A (GSGEGRGSLLTCGDVEENPGP (SEQ ID NO:65)), E2A (GSGQCTNYALLKLAGDVESNPGP (SEQ ID NO:66)), and F2A (GSGVKQTLNFDLLKLAGDVESNPGP (SEQ ID NO:67)) can be considered as either "proteolytic cleavage sites" or "ribosome skipping signals" (CHYSEL). See, e.g., Kim et al. (2011) PLoS ONE 6:e18556. The mechanism by which the encoded polypeptides are generated as two polypeptide chains may be by self cleaving of the linker, by ribosome skipping, or translational shunting. Regardless of the mechanism, the at least two polypeptide chains of a multimeric polypeptide of the present disclosure can be produced using a P2A, T2A, E2A, or F2A sequence. Suitable linkers include polypeptides comprising an amino acid sequence such as GSGATNFSLLKQAGDVEENPGP (SEQ ID NO:64), GSGEGRGSLLTCGDVEENPGP (SEQ ID NO:65), GSGQCTNYALLKLAGDVESNPGP (SEQ ID NO:66), GSGVKQTLNFDLLKLAGDVESNPGP (SEQ ID NO:67), or an amino acid sequence having from 1 to 5 amino acid substitutions relative to an amino acid sequence set forth in SEQ ID NOs:64-67 (e.g., an amino acid sequence having from 1 to 5 conservative amino acid substitutions relative to an amino acid sequence set forth in SEQ ID NOs:64-67). Suitable linkers include polypeptides comprising an amino acid sequence such as GSGATNFSLLKQAGDVEENPGP (SEQ ID NO:64), GSGEGRGSLLTCGDVEENPGP (SEQ ID NO:65), GSGQCTNYALLKLAGDVESNPGP (SEQ ID NO:66), GSGVKQTLNFDLLKLAGDVESNPGP (SEQ ID NO:67), or an amino acid sequence having from 1 to 10 amino acid substitutions relative to an amino acid sequence set forth in SEQ ID NOs:64-67 (e.g., an amino acid sequence having from 1 to 10 conservative amino acid substitutions relative to an amino acid sequence set forth in SEQ ID NOs:64-67).

### Epitopes

In an embodiment of the recombinant polypeptides, the candidate epitope comprises 7-20 amino acids. In an embodiment of the recombinant polypeptides, the epitope peptide is 5-20 amino acids for MHC class I. In an embodiment, the epitope peptide is 8-11 amino acids for MHC class I. In an embodiment, the epitope peptide is 5-40 amino acids for MHC class II. In an embodiment, the epitope peptide is 13-17 amino acids for MHC class II. In an embodiment, the epitope peptide is any naturally occurring or mutant human sequence, or any pathogen-derived sequence.

### MHC polypeptides

In an embodiment of the recombinant polypeptides, the first and/or second B2M leader sequence has the sequence of human B2M leader sequence.

In some cases, a leader peptide comprises an amino acid sequence having at least 75%, at least 80%, at least 85%, at least 90%, at least 95%, at least 98%, at least 99%, or 100%, amino acid sequence identity to the following human B2M leader sequence: MSRSVALAVLALLSLSGLEA (SEQ ID NO:68).

In some instances, a B2M leader sequence as described herein may be a mammalian B2M leader sequence including but not limited to, e.g., a human B2M leader sequence, a primate B2M leader sequence, a rodent B2M leader sequence, and the like. In some instances, a B2M leader as described herein comprises an amino acid sequence having at least 75%, at least 80%, at least 85%, at least 90%, at least 95%, at least 98%, at least 99%, or 100%, amino acid sequence identity with one of the B2M leader sequences depicted in FIG. 20.

In an embodiment, the B2M comprises the sequence:

In some instances, a B2M polypeptide comprises an amino acid sequence having at least 75%, at least 80%, at least 85%, at least 90%, at least 95%, at least 98%, at least 99%, or 100%, amino acid sequence identity to a B2M amino acid sequence depicted in FIG. 20.

In an embodiment of the recombinant polypeptides, the MHC heavy chain is a human MHC heavy chain. In an embodiment of the recombinant polypeptides, the MHC heavy chain is an MHC I molecule. Exemplary MHC I heavy chains include the alpha chain of HLA-A, HLA-B, HLA-C, HLA-E, HLA-F, HLA-G, HLA-K and HLA-L. In an embodiment of the recombinant polypeptides, the MHC heavy chain is an HLA-A02:01. In an embodiment, the HLA is HLA-A02. In an embodiment, the HLA-A02 comprises the sequence:

In an embodiment of the recombinant polypeptides, the MHC heavy chain is an MHC II molecule. Exemplary MHC II heavy chains include those of HLA-D.

In an embodiment of the recombinant polypeptides, the recombinant polypeptide further comprises a mutation in a human native B2M peptide sequence thereof and in the Heavy Chain sequence thereof so as to effect a disulfide bond between the B2M peptide sequence and Heavy Chain sequence.

In an embodiment of the recombinant polypeptides, the recombinant polypeptide the Heavy Chain sequence is an HLA and the disulfide bond links one of the following pairs of residues:
B2M residue 12, HLA residue 236;
B2M residue 12, HLA residue 237;
B2M residue 8, HLA residue 234;
B2M residue 10, HLA residue 235;
B2M residue 24, HLA residue 236;
B2M residue 28, HLA residue 232;
B2M residue 98, HLA residue 192;
B2M residue 99, HLA residue 234;
B2M residue 3, HLA residue 120;
B2M residue 31, HLA residue 96;
B2M residue 53, HLA residue 35;
B2M residue 60, HLA residue 96;
B2M residue 60, HLA residue 122;
B2M residue 63, HLA residue 27;
B2M residue Arg3, HLA residue Gly120;
B2M residue His31, HLA residue Gln96;
B2M residue Asp53, HLA residue Arg35;
B2M residue Trp60, HLA residue Gln96;
B2M residue Trp60, HLA residue Asp122;
B2M residue Tyr63, HLA residue Tyr27;
B2M residue Lys6, HLA residue Glu232;
B2M residue Gln8, HLA residue Arg234;
B2M residue Tyr10, HLA residue Pro235;
B2M residue Ser11, HLA residue Gln242;
B2M residue Asn24, HLA residue Ala236;
B2M residue Ser28, HLA residue Glu232;
B2M residue Asp98, HLA residue His 192; and
B2M residue Met99, HLA residue Arg234
   (See SEQ ID NO:s 4 and 5 for B2M and HLA sequences).

In an embodiment of the recombinant polypeptides, the Heavy Chain sequence is an HLA and wherein the disulfide bond links one of the following pairs of residues:
first linker position Gly 2, Heavy Chain (HLA) position Tyr 84;
Light Chain (B2M) position Arg 12, HLA Ala236; and/or
B2M residue Arg12, HLA residue Gly237.

### Fc polypeptides

In an embodiment of the recombinant polypeptides, the immunoglobulin Fc domain is an IgG Fc domain. In an embodiment of the recombinant polypeptides, the immunoglobulin Fc domain is an IgA Fc domain. In an embodiment of the recombinant polypeptides, the immunoglobulin Fc domain is an IgM Fc domain. In an embodiment of the recombinant polypeptides, the immunoglobulin Fc domain is a human immunoglobulin Fc domain. In an embodiment of the recombinant polypeptides, the immunoglobulin Fc domain is an IgG1 Fc domain.

### Immunomodulatory polypeptides

In an embodiment of the recombinant polypeptides, the T cell modulatory domain is an inhibitory domain.

In an embodiment of the recombinant polypeptides, the T cell modulatory domain is a stimulating domain.

In an embodiment of the recombinant polypeptides, the T cell modulatory domain is an antibody, and antibody fragment, a peptide ligand, a T cell costimulatory peptide, a cytokine or a toxin.

In an embodiment of the recombinant polypeptides, the T cell modulatory domain comprises a PD-L1 peptide, the Ig variable domain of a PD-L1 peptide, the T cell modulatory domain comprises 4-1BBL, the T cell modulatory domain comprises B7-1W88A, or the T cell modulatory domain comprises anti-CD28 single chain Fv.

Further T cell modulatory domains (MODs) that can be employed in the invention include naturally occurring or synthetic human gene products (protein), affinity reagents (e.g., an antibody, antibody fragment, single chain Fvs, aptamers, nanobody) targeting a human gene product, including, but not limited to all secreted proteins arising from classical and non-classical (e.g., FGF2, IL1, S100A4) secretion mechanisms, and ecto-domains of all cell surface proteins anchored by naturally occurring genetically encoded protein segments (single or multiple membrane spans) or post-translational modifications such as GPI linkages). Any naturally occurring or synthetic affinity reagent (e.g., antibody, antibody fragment, single chain Fvs, aptamer, nanobody, lectin, etc) targeting a cell surface glycan or other post-translational modification (e.g., sulfation). Examples include, but are not limited to, members of the TNF/TNFR family (OX40L, ICOSL, FASL, LTA, LTB TRAIL, CD153, TNFSF9, RANKL, TWEAK, TNFSF13, TNFSF13b, TNFSF14, TNFSF15, TNFSF18, CD40LG, CD70) or affinity reagents directed at the TNF/TNFR family members; members of the Immunoglobulin superfamily (VISTA, PD1, PD-L1, PD-L2, B71, B72, CTLA4, CD28, TIM3, CD4, CD8, CD19, T cell receptor chains, ICOS, ICOS ligand, HHLA2, butyrophilins, BTLA, B7-H3, B7-H4, CD3, CD79a, CD79b, IgSF CAMS (including CD2, CD58, CD48, CD150, CD229, CD244, ICAM-1), Leukocyte immunoglobulin like receptors (LILR), killer cell immunoglobulin like receptors (KIR)), lectin superfamily members, selectins, cytokines/chemokine and cytokine/chemokine receptors, growth factors and growth factor receptors), adhesion molecules (integrins, fibronectins, cadherins), or ecto-domains of multi-span intergral membrane protein, or affinity reagents directed at the Immunoglobulin superfamily and listed gene products. In addition, active homologs/orthologs of these gene products, including but not limited to, viral sequences (e.g., CMV, EBV), bacterial sequences, fungal sequences, eukaryotic pathogens (e.g., *Schistosoma, Plasmodium, Babesia, Eimeria, Theileria, Toxoplasma, Entamoeba, Leishmania, and Trypanosoma*), and mammalian -derived coding regions. In addition, a MOD may comprise a small molecules drug targeting a human gene product.

### Additional polypeptides

In an embodiment of the recombinant polypeptides, they further comprise a His-8 tag contiguous with the C-terminal thereof.

### NUCLEIC ACIDS

A nucleic acid is provided encoding any of the recombinant polypeptides described herein. In an embodiment, the nucleic acid is a DNA. In an embodiment, the nucleic acid is a cDNA. In an embodiment, the nucleic acid is an RNA. In an embodiment, the nucleic acid is an mRNA.

In an embodiment, the recombinant nucleic acid is a vector. In an embodiment, the vector is a viral vector. In an embodiment, the viral vector is a lentiviral vector.

The present disclosure provides nucleic acids comprising nucleotide sequences encoding a multimeric polypeptide of the present disclosure. In some cases, the individual polypeptide chains of a multimeric polypeptide of the present disclosure are encoded in separate nucleic acids. In some cases, all polypeptide chains of a multimeric polypeptide of the present disclosure are encoded in a single nucleic acid. In some cases, a first nucleic acid comprises a nucleotide sequence encoding a first polypeptide of a multimeric polypeptide of the present disclosure; and a second nucleic acid comprises a nucleotide sequence encoding a second polypeptide of a multimeric polypeptide of the present disclosure. In some cases, single nucleic acid comprises a nucleotide sequence encoding a first polypeptide of a multimeric polypeptide of the present disclosure and a second polypeptide of a multimeric polypeptide of the present disclosure. In some cases, a nucleic acid comprises a nucleotide sequence encoding a polyprotein precursor, as described above.

### Separate nucleic acids encoding individual polypeptide chains of a multimeric polypeptide

The present disclosure provides nucleic acids comprising nucleotide sequences encoding a multimeric polypeptide of the present disclosure. As noted above, in some cases, the individual polypeptide chains of a multimeric polypeptide of the present disclosure are encoded in separate nucleic acids. In some cases, nucleotide sequences encoding the separate polypeptide chains of a multimeric polypeptide of the present disclosure are operably linked to transcriptional control elements, e.g., promoters, such as promoters that are functional in a eukaryotic cell, where the promoter can be a constitutive promoter or an inducible promoter.

The present disclosure provides a first nucleic acid and a second nucleic acid, where the first nucleic acid comprises a nucleotide sequence encoding a first polypeptide of a multimeric polypeptide of the present disclosure, where the first polypeptide comprises, in order from N-terminus to C-terminus: a) an epitope (e.g., a T-cell epitope); b) a first MHC polypeptide; and c) an immunomodulatory polypeptide; and where the second nucleic acid comprises a nucleotide sequence encoding a second polypeptide of a multimeric polypeptide of the present disclosure, where the second polypeptide comprises, in order from N-terminus to C-terminus: a) a second MHC polypeptide; and b) an Ig Fc polypeptide. Suitable T-cell epitopes, MHC polypeptides, immunomodulatory polypeptides, and Ig Fc polypeptides, are described above. In some cases, the nucleotide sequences encoding the first and the second polypeptides are operably linked to transcriptional control elements. In some cases, the transcriptional control element is a promoter that is functional in a eukaryotic cell. In some cases, the nucleic acids are present in separate expression vectors.

The present disclosure provides a first nucleic acid and a second nucleic acid, where the first nucleic acid comprises a nucleotide sequence encoding a first polypeptide of a multimeric polypeptide of the present disclosure, where the first polypeptide comprises, in order from N-terminus to C-terminus: a) an epitope (e.g., a T-cell epitope); and b) a first MHC polypeptide; and where the second nucleic acid comprises a nucleotide sequence encoding a second polypeptide of a multimeric polypeptide of the present disclosure, where the second polypeptide comprises, in order from N-terminus to C-terminus: a) an immunomodulatory polypeptide; b) a second MHC polypeptide; and c) an Ig Fc polypeptide. Suitable T-cell epitopes, MHC polypeptides, immunomodulatory polypeptides, and Ig Fc polypeptides, are described above. In some cases, the nucleotide sequences encoding the first and the second polypeptides are operably linked to transcriptional control elements. In some cases, the transcriptional control element is a promoter that is functional in a eukaryotic cell. In some cases, the nucleic acids are present in separate expression vectors.

### Nucleic acid encoding two or more polypeptides present in a multimeric polypeptide

The present disclosure provides a nucleic acid comprising nucleotide sequences encoding at least the first polypeptide and the second polypeptide of a multimeric polypeptide of the present disclosure. In some cases, where a multimeric polypeptide of the present disclosure includes a first, second, and third polypeptide, the nucleic acid includes a nucleotide sequence encoding the first, second, and third polypeptides. In some cases, the nucleotide sequences encoding the first polypeptide and the second polypeptide of a multimeric polypeptide of the present disclosure includes a proteolytically cleavable linker interposed between the nucleotide sequence encoding the first polypeptide and the nucleotide sequence encoding the second polypeptide. In some cases, the nucleotide sequences encoding the first polypeptide and the second polypeptide of a multimeric polypeptide of the present disclosure includes an internal ribosome entry site (IRES) interposed between the nucleotide sequence encoding the first polypeptide and the nucleotide sequence encoding the second polypeptide. In some cases, the nucleotide sequences encoding the first polypeptide and the second polypeptide of a multimeric polypeptide of the present disclosure includes a ribosome skipping signal (or *cis*-acting hydrolase element, CHYSEL) interposed between the nucleotide sequence encoding the first polypeptide and the nucleotide sequence encoding the second polypeptide. Examples of nucleic acids are described below, where a proteolytically cleavable linker is provided between nucleotide sequences encoding the first polypeptide and the second polypeptide of a multimeric polypeptide of the present disclosure; in any of these embodiments, an IRES or a ribosome skipping signal can be used in place of the nucleotide sequence encoding the proteolytically cleavable linker.

In some cases, a first nucleic acid (e.g., a recombinant expression vector, an mRNA, a viral RNA, etc.) comprises a nucleotide sequence encoding a first polypeptide chain of a multimeric polypeptide of the present disclosure; and a second nucleic acid (e.g., a recombinant expression vector, an mRNA, a viral RNA, etc.) comprises a nucleotide sequence encoding a second polypeptide chain of a multimeric polypeptide of the present disclosure. In some cases, the nucleotide sequence encoding the first polypeptide, and the second nucleotide sequence encoding the second polypeptide, are each operably linked to transcriptional control elements, e.g., promoters, such as promoters that are functional in a eukaryotic cell, where the promoter can be a constitutive promoter or an inducible promoter.

The present disclosure provides a nucleic acid comprising a nucleotide sequence encoding a recombinant polypeptide, where the recombinant polypeptide comprises, in order from N-terminus to C-terminus: a) an epitope (e.g., a T-cell epitope); b) a first MHC polypeptide; c) an immunomodulatory polypeptide; d) a proteolytically cleavable linker; e) a second MHC polypeptide; and f) an immunoglobulin (Ig) Fc polypeptide. The present disclosure provides a nucleic acid comprising a nucleotide sequence encoding a recombinant polypeptide, where the recombinant polypeptide comprises, in order from N-terminus to C-terminus: a) a first leader peptide; b) the epitope; c) the first MHC polypeptide; d) the immunomodulatory polypeptide; e) the proteolytically cleavable linker; f) a second leader peptide; g) the second MHC polypeptide; and h) the Ig Fc polypeptide. The present disclosure provides a nucleic acid comprising a nucleotide sequence encoding a recombinant polypeptide, where the recombinant polypeptide comprises, in order from N-terminus to C-terminus: a) an epitope; b) a first MHC polypeptide; c) a proteolytically cleavable linker; d) an immunomodulatory polypeptide; e) a second MHC polypeptide; and f) an Ig Fc polypeptide. In some cases, the first leader peptide and the second leader peptide is a β2-M leader peptide. In some cases, the nucleotide sequence is operably linked to a transcriptional control element. In some cases, the transcriptional control element is a promoter that is functional in a eukaryotic cell.

Suitable MHC polypeptides are described above. In some cases, the first MHC polypeptide is a β2-microglobulin polypeptide; and wherein the second MHC polypeptide is an MHC class I heavy chain polypeptide. In some cases, the β2-microglobulin polypeptide comprises an amino acid sequence having at least 85% amino acid sequence identity to the amino acid sequence set forth in SEQ ID NO:4. In some cases, the MHC class I heavy chain polypeptide is an HLA-A, HLA-B, HLA-C, HLA-E, HLA-F, HLA-G, HLA-K, or HLA-L heavy chain. In some cases, the MHC class I heavy chain polypeptide comprises an amino acid sequence having at least 85% amino acid sequence identity to the amino acid sequence set forth in SEQ ID NO:5. In some cases, the first MHC polypeptide is an MHC Class II alpha chain polypeptide; and wherein the second MHC polypeptide is an MHC class II beta chain polypeptide.

Suitable Fc polypeptides are described above. In some cases, the Ig Fc polypeptide is an IgG1 Fc polypeptide, an IgG2 Fc polypeptide, an IgG3 Fc polypeptide, an IgG4 Fc polypeptide, an IgA Fc polypeptide, or an IgM Fc polypeptide. In some cases, the Ig Fc polypeptide comprises an amino acid sequence having at least 85% amino acid sequence identity to an amino acid sequence depicted in Figures 24A-24C.

Suitable immunomodulatory polypeptides are described above. In some cases, the immunomodulatory polypeptide is selected from a 4-1BBL polypeptide, a B7-1 polypeptide; a B7-2 polypeptide, an ICOS-L polypeptide, an OX-40L polypeptide, a CD80 polypeptide, a CD86 polypeptide, a PD-L1 polypeptide, a FasL polypeptide, and a PD-L2 polypeptide. In some cases, the immunomodulatory polypeptide is selected from a CD7, CD30L, CD40, CD70, CD83, HLA-G, MICA, MICB, HVEM, lymphotoxin beta receptor, 3/TR6, ILT3, ILT4, and HVEM.

Suitable proteolytically cleavable linkers are described above. In some cases, the proteolytically cleavable linker comprises an amino acid sequence selected from: a) LEVLFQGP (SEQ ID NO:37); b) ENLYTQS (SEQ ID NO:34); c) DDDDK (SEQ ID NO:35); d) LVPR (SEQ ID NO:36); and e) GSGATNFSLLKQAGDVEENPGP (SEQ ID NO:64).

In some cases, a linker between the epitope and the first MHC polypeptide comprises a first Cys residue, and the second MHC polypeptide comprises an amino acid substitution to provide a second Cys residue, such that the first and the second Cys residues provide for a disulfide linkage between the linker and the second MHC polypeptide. In some cases, first MHC polypeptide comprises an amino acid substitution to provide a first Cys residue, and the second MHC polypeptide comprises an amino acid substitution to provide a second Cys residue, such that the first Cys residue and the second Cys residue provide for a disulfide linkage between the first MHC polypeptide and the second MHC polypeptide.

### Recombinant expression vectors

The present disclosure provides recombinant expression vectors comprising nucleic acids of the present disclosure. In some cases, the recombinant expression vector is a non-viral vector. In some embodiments, the recombinant expression vector is a viral construct, e.g., a recombinant adeno-associated virus construct (see, e.g., U.S. Patent No. 7,078,387), a recombinant adenoviral construct, a recombinant lentiviral construct, a recombinant retroviral construct, a non-integrating viral vector, etc.

Suitable expression vectors include, but are not limited to, viral vectors (e.g. viral vectors based on vaccinia virus; poliovirus; adenovirus (see, e.g., Li et al., Invest Opthalmol Vis Sci 35:2543 2549, 1994; Borras et al., Gene Ther 6:515 524, 1999; Li and Davidson, PNAS 92:7700 7704, 1995; Sakamoto et al., H Gene Ther 5:1088 1097, 1999; WO 94/12649, WO 93/03769; WO 93/19191; WO 94/28938; WO 95/11984 and WO 95/00655); adeno-associated virus (see, e.g., Ali et al., Hum Gene Ther 9:81 86, 1998, Flannery et al., PNAS 94:6916 6921, 1997; Bennett et al., Invest Opthalmol Vis Sci 38:2857 2863, 1997; Jomary et al., Gene Ther 4:683 690, 1997, Rolling et al., Hum Gene Ther 10:641 648, 1999; Ali et al., Hum Mol Genet 5:591 594, 1996; Srivastava in WO 93/09239, Samulski et al., J. Vir. (1989) 63:3822-3828; Mendelson et al., Virol. (1988) 166:154-165; and Flotte et al., PNAS (1993) 90:10613-10617); SV40; herpes simplex virus; human immunodeficiency virus (see, e.g., Miyoshi et al., PNAS 94:10319 23, 1997; Takahashi et al., J Virol 73:7812 7816, 1999); a retroviral vector (e.g., Murine Leukemia Virus, spleen necrosis virus, and vectors derived from retroviruses such as Rous Sarcoma Virus, Harvey Sarcoma Virus, avian leukosis virus, a lentivirus, human immunodeficiency virus, myeloproliferative sarcoma virus, and mammary tumor virus); and the like.

Numerous suitable expression vectors are known to those of skill in the art, and many are commercially available. The following vectors are provided by way of example; for eukaryotic host cells: pXT1, pSG5 (Stratagene), pSVK3, pBPV, pMSG, and pSVLSV40 (Pharmacia). However, any other vector may be used so long as it is compatible with the host cell.

Depending on the host/vector system utilized, any of a number of suitable transcription and translation control elements, including constitutive and inducible promoters, transcription enhancer elements, transcription terminators, etc. may be used in the expression vector (see e.g., Bitter et al. (1987) Methods in Enzymology, 153:516-544).

In some embodiments, a nucleotide sequence encoding a DNA-targeting RNA and/or a site-directed modifying polypeptide is operably linked to a control element, e.g., a transcriptional control element, such as a promoter. The transcriptional control element may be functional in either a eukaryotic cell, e.g., a mammalian cell; or a prokaryotic cell (e.g., bacterial or archaeal cell). In some embodiments, a nucleotide sequence encoding a DNA-targeting RNA and/or a site-directed modifying polypeptide is operably linked to multiple control elements that allow expression of the nucleotide sequence encoding a DNA-targeting RNA and/or a site-directed modifying polypeptide in both prokaryotic and eukaryotic cells.

Non-limiting examples of suitable eukaryotic promoters (promoters functional in a eukaryotic cell) include those from cytomegalovirus (CMV) immediate early, herpes simplex virus (HSV) thymidine kinase, early and late SV40, long terminal repeats (LTRs) from retrovirus, and mouse metallothionein-I. Selection of the appropriate vector and promoter is well within the level of ordinary skill in the art. The expression vector may also contain a ribosome binding site for translation initiation and a transcription terminator. The expression vector may also include appropriate sequences for amplifying expression.

### GENETICALLY MODIFIED HOST CELLS

A cell is provided transformed with a nucleic acid encoding any of the recombinant polypeptides described herein. Examples of cells that can be transformed with a nucleic acid encoding any of the recombinant polypeptides include isolated mammalian cells, including but not limited to Human Embryonic Kidney (HEK), Chinese Hamster Ovary (CHO), NS0 (murine myeloma) cells, human amniocytic cells (CAP, CAP-T), yeast cells(including, but not limited to, *S. cerevisiae, Pichia pastoris*), plant cells (including, but not limited to, Tobacco NT1, BY-2), insect cells (including but not limited to SF9, S2, SF21, Tni (e.g. High 5)) or bacterial cells (including, but not limited to, *E. coli*).

The present disclosure provides a genetically modified host cell, where the host cell is genetically modified with a nucleic acid of the present disclosure.

Suitable host cells include eukaryotic cells, such as yeast cells, insect cells, and mammalian cells. In some cases, the host cell is a cell of a mammalian cell line. Suitable mammalian cell lines include human cell lines, non-human primate cell lines, rodent (e.g., mouse, rat) cell lines, and the like. Suitable mammalian cell lines include, but are not limited to, HeLa cells (e.g., American Type Culture Collection (ATCC) No. CCL-2), CHO cells (e.g., ATCC Nos. CRL9618, CCL61, CRL9096), 293 cells (e.g., ATCC No. CRL-1573), Vero cells, NIH 3T3 cells (e.g., ATCC No. CRL-1658), Huh-7 cells, BHK cells (e.g., ATCC No. CCL10), PC12 cells (ATCC No. CRL1721), COS cells, COS-7 cells (ATCC No. CRL1651), RAT1 cells, mouse L cells (ATCC No. CCLI.3), human embryonic kidney (HEK) cells (ATCC No. CRL1573), HLHepG2 cells, and the like.

In some cases, the host cell is a mammalian cell that has been genetically modified such that it does not synthesize endogenous MHC β2-M.

### METHODS OF PRODUCING A MULTIMERIC POLYPEPTIDE

The present disclosure provides methods of producing a multimeric polypeptide of the present disclosure. The methods generally involve culturing, in a culture medium, a host cell that is genetically modified with a recombinant expression vector comprising a nucleotide sequence encoding the multimeric polypeptide; and isolating the multimeric polypeptide from the genetically modified host cell and/or the culture medium. A host cell that is genetically modified with a recombinant expression vector comprising a nucleotide sequence encoding the multimeric polypeptide is also referred to as an "expression host." As noted above, in some cases, the individual polypeptide chains of a multimeric polypeptide of the present disclosure are encoded in separate recombinant expression vectors. In some cases, all polypeptide chains of a multimeric polypeptide of the present disclosure are encoded in a single recombinant expression vector.

Isolation of the multimeric polypeptide from the expression host cell (e.g., from a lysate of the expression host cell) and/or the culture medium in which the host cell is cultured, can be carried out using standard methods of protein purification.

For example, a lysate may be prepared of the expression host and the lysate purified using high performance liquid chromatography (HPLC), exclusion chromatography, gel electrophoresis, affinity chromatography, or other purification technique. Alternatively, where the multimeric polypeptide is secreted from the expression host cell into the culture medium, the multimeric polypeptide can be purified from the culture medium using HPLC, exclusion chromatography, gel electrophoresis, affinity chromatography, or other purification technique. In some cases, the compositions which are used will comprise at least 80% by weight of the desired product, at least about 85% by weight, at least about 95% by weight, or at least about 99.5% by weight, in relation to contaminants related to the method of preparation of the product and its purification. The percentages can be based upon total protein.

In some cases, e.g., where the multimeric polypeptide comprises an affinity tag, the multimeric polypeptide can be purified using an immobilized binding partner of the affinity tag.

### COMPOSITIONS

The present disclosure provides compositions, including pharmaceutical compositions, comprising a multimeric polypeptide of the present disclosure. The present disclosure provides compositions, including pharmaceutical compositions, comprising a nucleic acid or a recombinant expression vector of the present disclosure.

### Compositions comprising a multimeric polypeptide

A composition of the present disclosure can comprise, in addition to a multimeric polypeptide of the present disclosure, one or more of: a salt, e.g., NaCl, MgCl, KCl, MgSO₄, etc.; a buffering agent, e.g., a Tris buffer, N-(2-Hydroxyethyl)piperazine-N'-(2-ethanesulfonic acid) (HEPES), 2-(N-Morpholino)ethanesulfonic acid (MES), 2-(N-Morpholino)ethanesulfonic acid sodium salt (MES), 3-(N-Morpholino)propanesulfonic acid (MOPS), N-tris[Hydroxymethyl]methyl-3-aminopropanesulfonic acid (TAPS), etc.; a solubilizing agent; a detergent, e.g., a non-ionic detergent such as Tween-20, etc.; a protease inhibitor; glycerol; and the like.

The composition may comprise a pharmaceutically acceptable excipient, a variety of which are known in the art and need not be discussed in detail herein. Pharmaceutically acceptable excipients have been amply described in a variety of publications, including, for example, "Remington: The Science and Practice of Pharmacy", 19th Ed. (1995), or latest edition, Mack Publishing Co; A. Gennaro (2000) "Remington: The Science and Practice of Pharmacy", 20th edition, Lippincott, Williams, & Wilkins; Pharmaceutical Dosage Forms and Drug Delivery Systems (1999) H.C. Ansel et al., eds 7th ed., Lippincott, Williams, & Wilkins; and Handbook of Pharmaceutical Excipients (2000) A.H. Kibbe et al., eds., 3rd ed. Amer. Pharmaceutical Assoc.

A pharmaceutical composition can comprise a multimeric polypeptide of the present disclosure, and a pharmaceutically acceptable excipient. In some cases, a subject pharmaceutical composition will be suitable for administration to a subject, e.g., will be sterile. For example, in some embodiments, a subject pharmaceutical composition will be suitable for administration to a human subject, e.g., where the composition is sterile and is free of detectable pyrogens and/or other toxins.

The protein compositions may comprise other components, such as pharmaceutical grades of mannitol, lactose, starch, magnesium stearate, sodium saccharin, talcum, cellulose, glucose, sucrose, magnesium, carbonate, and the like. The compositions may contain pharmaceutically acceptable auxiliary substances as required to approximate physiological conditions such as pH adjusting and buffering agents, toxicity adjusting agents and the like, for example, sodium acetate, sodium chloride, potassium chloride, calcium chloride, sodium lactate, hydrochloride, sulfate salts, solvates (e.g., mixed ionic salts, water, organics), hydrates (e.g., water), and the like.

For example, compositions may include aqueous solution, powder form, granules, tablets, pills, suppositories, capsules, suspensions, sprays, and the like. The composition may be formulated according to the various routes of administration described below.

Where a multimeric polypeptide of the present disclosure is administered as an injectable (e.g. subcutaneously, intraperitoneally, and/or intravenous) directly into a tissue, a formulation can be provided as a ready-to-use dosage form, or as non-aqueous form (e.g. a reconstitutable storage-stable powder) or aqueous form, such as liquid composed of pharmaceutically acceptable carriers and excipients. The protein-containing formulations may also be provided so as to enhance serum half-life of the subject protein following administration. For example, the protein may be provided in a liposome formulation, prepared as a colloid, or other conventional techniques for extending serum half-life. A variety of methods are available for preparing liposomes, as described in, e.g., Szoka et al. 1980 Ann. Rev. Biophys. Bioeng. 9:467, U.S. Pat. Nos. 4,235,871, 4,501,728 and 4,837,028. The preparations may also be provided in controlled release or slow-release forms.

Other examples of formulations suitable for parenteral administration include isotonic sterile injection solutions, anti-oxidants, bacteriostats, and solutes that render the formulation isotonic with the blood of the intended recipient, suspending agents, solubilizers, thickening agents, stabilizers, and preservatives. For example, a subject pharmaceutical composition can be present in a container, e.g., a sterile container, such as a syringe. The formulations can be presented in unit-dose or multi-dose sealed containers, such as ampules and vials, and can be stored in a freeze-dried (lyophilized) condition requiring only the addition of the sterile liquid excipient, for example, water, for injections, immediately prior to use. Extemporaneous injection solutions and suspensions can be prepared from sterile powders, granules, and tablets.

The concentration of a multimeric polypeptide of the present disclosure in a formulation can vary widely (e.g., from less than about 0.1%, usually at or at least about 2% to as much as 20% to 50% or more by weight) and will usually be selected primarily based on fluid volumes, viscosities, and patient-based factors in accordance with the particular mode of administration selected and the patient's needs.

The present disclosure provides a container comprising a composition of the present disclosure, e.g., a liquid composition. The container can be, e.g., a syringe, an ampoule, and the like. In some cases, the container is sterile. In some cases, both the container and the composition are sterile.

### Compositions comprising a nucleic acid or a recombinant expression vector

The present disclosure provides compositions, e.g., pharmaceutical compositions, comprising a nucleic acid or a recombinant expression vector of the present disclosure. A wide variety of pharmaceutically acceptable excipients is known in the art and need not be discussed in detail herein. Pharmaceutically acceptable excipients have been amply described in a variety of publications, including, for example, A. Gennaro (2000) "Remington: The Science and Practice of Pharmacy", 20th edition, Lippincott, Williams, & Wilkins; Pharmaceutical Dosage Forms and Drug Delivery Systems (1999) H. C. Ansel et al., eds 7th ed., Lippincott, Williams, & Wilkins; and Handbook of Pharmaceutical Excipients (2000) A. H. Kibbe et al., eds., 3rd ed. Amer. Pharmaceutical Assoc.

A composition of the present disclosure can include: a) a subject nucleic acid or recombinant expression vector; and b) one or more of: a buffer, a surfactant, an antioxidant, a hydrophilic polymer, a dextrin, a chelating agent, a suspending agent, a solubilizer, a thickening agent, a stabilizer, a bacteriostatic agent, a wetting agent, and a preservative. Suitable buffers include, but are not limited to, (such as N,N-bis(2-hydroxyethyl)-2-aminoethanesulfonic acid (BES), bis(2-hydroxyethyl)amino-tris(hydroxymethyl)methane (BIS-Tris), N-(2-hydroxyethyl)piperazine-N'3-propanesulfonic acid (EPPS or HEPPS), glycylglycine, N-2-hydroxyehtylpiperazine-N'-2-ethanesulfonic acid (HEPES), 3-(N-morpholino)propane sulfonic acid (MOPS), piperazine-N,N'-bis(2-ethane-sulfonic acid) (PIPES), sodium bicarbonate, 3-(N-tris(hydroxymethyl)-methyl-amino)-2-hydroxy-propanesulfonic acid) TAPSO, (N-tris(hydroxymethyl)methyl-2-aminoethanesulfonic acid (TES), N-tris(hydroxymethyl)methylglycine (Tricine), tris(hydroxymethyl)-aminomethane (Tris), etc.). Suitable salts include, e.g., NaCl, MgCl₂, KCl, MgSO₄, etc.

A pharmaceutical formulation of the present disclosure can include a nucleic acid or recombinant expression vector of the present disclosure in an amount of from about 0.001% to about 90% (w/w). In the description of formulations, below, "subject nucleic acid or recombinant expression vector" will be understood to include a nucleic acid or recombinant expression vector of the present disclosure. For example, in some embodiments, a subject formulation comprises a nucleic acid or recombinant expression vector of the present disclosure.

A subject nucleic acid or recombinant expression vector can be admixed, encapsulated, conjugated or otherwise associated with other compounds or mixtures of compounds; such compounds can include, e.g., liposomes or receptor-targeted molecules. A subject nucleic acid or recombinant expression vector can be combined in a formulation with one or more components that assist in uptake, distribution and/or absorption.

A subject nucleic acid or recombinant expression vector composition can be formulated into any of many possible dosage forms such as, but not limited to, tablets, capsules, gel capsules, liquid syrups, soft gels, suppositories, and enemas. A subject nucleic acid or recombinant expression vector composition can also be formulated as suspensions in aqueous, non-aqueous or mixed media. Aqueous suspensions may further contain substances which increase the viscosity of the suspension including, for example, sodium carboxymethylcellulose, sorbitol and/or dextran. The suspension may also contain stabilizers.

A formulation comprising a subject nucleic acid or recombinant expression vector can be a liposomal formulation. As used herein, the term "liposome" means a vesicle composed of amphiphilic lipids arranged in a spherical bilayer or bilayers. Liposomes are unilamellar or multilamellar vesicles which have a membrane formed from a lipophilic material and an aqueous interior that contains the composition to be delivered. Cationic liposomes are positively charged liposomes that can interact with negatively charged DNA molecules to form a stable complex. Liposomes that are pH sensitive or negatively charged are believed to entrap DNA rather than complex with it. Both cationic and noncationic liposomes can be used to deliver a subject nucleic acid or recombinant expression vector.

Liposomes also include "sterically stabilized" liposomes, a term which, as used herein, refers to liposomes comprising one or more specialized lipids that, when incorporated into liposomes, result in enhanced circulation lifetimes relative to liposomes lacking such specialized lipids. Examples of sterically stabilized liposomes are those in which part of the vesicle-forming lipid portion of the liposome comprises one or more glycolipids or is derivatized with one or more hydrophilic polymers, such as a polyethylene glycol (PEG) moiety. Liposomes and their uses are further described in U.S. Pat. No. 6,287,860.

The formulations and compositions of the present disclosure may also include surfactants. The use of surfactants in drug products, formulations and in emulsions is well known in the art. Surfactants and their uses are further described in U.S. Pat. No. 6,287,860.

In one embodiment, various penetration enhancers are included, to effect the efficient delivery of nucleic acids. In addition to aiding the diffusion of non-lipophilic drugs across cell membranes, penetration enhancers also enhance the permeability of lipophilic drugs. Penetration enhancers may be classified as belonging to one of five broad categories, i.e., surfactants, fatty acids, bile salts, chelating agents, and non-chelating non-surfactants. Penetration enhancers and their uses are further described in U.S. Pat. No. 6,287,860.

Compositions and formulations for oral administration include powders or granules, microparticulates, nanoparticulates, suspensions or solutions in water or non-aqueous media, capsules, gel capsules, sachets, tablets, or minitablets. Thickeners, flavoring agents, diluents, emulsifiers, dispersing aids or binders may be desirable. Suitable oral formulations include those in which a subject antisense nucleic acid is administered in conjunction with one or more penetration enhancers surfactants and chelators. Suitable surfactants include, but are not limited to, fatty acids and/or esters or salts thereof, bile acids and/or salts thereof. Suitable bile acids/salts and fatty acids and their uses are further described in U.S. Pat. No. 6,287,860. Also suitable are combinations of penetration enhancers, for example, fatty acids/salts in combination with bile acids/salts. An exemplary suitable combination is the sodium salt of lauric acid, capric acid, and UDCA. Further penetration enhancers include, but are not limited to, polyoxyethylene-9-lauryl ether, and polyoxyethylene-20-cetyl ether. Suitable penetration enhancers also include propylene glycol, dimethylsulfoxide, triethanoiamine, N,N-dimethylacetamide, N,N-dimethylformamide, 2-pyrrolidone and derivatives thereof, tetrahydrofurfuryl alcohol, and AZONE™.

### METHODS OF MODULATING T CELL ACTIVITY

Also provided is a method of inhibiting a T cell clone which recognizes an epitope peptide comprising contacting a T cell of the clone with a recombinant peptide as described herein, wherein the recombinant peptide comprises the epitope peptide and comprises a T cell modulatory domain which is an inhibitory domain, in an amount effective to inhibit a T cell clone.

Also provided is a method of stimulating a T cell clone which recognizes an epitope peptide comprising contacting a T cell of the clone with a recombinant peptide as described herein, wherein the recombinant peptide comprises the epitope peptide and comprises a T cell modulatory domain which is an stimulatory domain, in an amount effective to stimulate a T cell clone.

The present disclosure provides a method of selectively modulating the activity of an epitope-specific T cell, the method comprising contacting the T cell with a multimeric polypeptide of the present disclosure, where contacting the T cell with a multimeric polypeptide of the present disclosure selectively modulates the activity of the epitope-specific T cell. In some cases, the contacting occurs *in vitro.* In some cases, the contacting occurs *in vivo.* In some cases, the contacting occurs *ex vivo.*

In some cases, e.g., where the target T cell is a CD8⁺ T cell, the multimeric polypeptide comprises Class I MHC polypeptides (e.g., β2-microglobulin and Class I MHC heavy chain). In some cases, e.g., where the target T cell is a CD4⁺ T cell, the multimeric polypeptide comprises Class II MHC polypeptides (e.g., Class II MHC α chain; Class II MHC β chain).

Where a multimeric polypeptide of the present disclosure includes an immunomodulatory polypeptide that is an activating polypeptide, contacting the T cell with the multimeric polypeptide activates the epitope-specific T cell. In some instances, the epitope-specific T cell is a T cell that is specific for an epitope present on a cancer cell, and contacting the epitope-specific T cell with the multimeric polypeptide increases cytotoxic activity of the T cell toward the cancer cell. In some instances, the epitope-specific T cell is a T cell that is specific for an epitope present on a cancer cell, and contacting the epitope-specific T cell with the multimeric polypeptide increases the number of the epitope-specific T cells.

In some instances, the epitope-specific T cell is a T cell that is specific for an epitope present on a virus-infected cell, and contacting the epitope-specific T cell with the multimeric polypeptide increases cytotoxic activity of the T cell toward the virus-infected cell. In some instances, the epitope-specific T cell is a T cell that is specific for an epitope present on a virus-infected cell, and contacting the epitope-specific T cell with the multimeric polypeptide increases the number of the epitope-specific T cells.

Where a multimeric polypeptide of the present disclosure includes an immunomodulatory polypeptide that is an inhibiting polypeptide, contacting the T cell with the multimeric inhibits the epitope-specific T cell. In some instances, the epitope-specific T cell is a self-reactive T cell that is specific for an epitope present in a self antigen, and the contacting reduces the number of the self-reactive T cells.

### TREATMENT METHODS

Also provided is a method of treating an autoimmune disorder by inhibiting a self-reactive T cell clone which recognizes an epitope peptide comprising contacting a T cell of the clone with a recombinant peptide as described herein, wherein the recombinant peptide comprises the epitope peptide and comprises a T cell modulatory domain which is an inhibitory domain, in an amount effective to treat an autoimmune disorder.

Also provided is a method of treating a cancer by stimulating a T cell clone which recognizes an epitope peptide on a cancer comprising contacting a T cell of the clone with a recombinant peptide as described herein, wherein the recombinant peptide comprises the epitope peptide and comprises a T cell modulatory domain which is an stimulatory domain, in an amount effective to treat the cancer.

In an embodiment, the cells transformed to express a recombinant polypeptide of the invention are isolated suspension-adapted cells. In an embodiment of the plurality of said isolated suspension-adapted cells, or of the recombinant nucleic acid, the nucleic acid comprises DNA.

In an embodiment, the T-cells comprise peripheral T-cells obtained from a subject. In an embodiment, the T-cells comprise T-cells in a subject. In an embodiment, the T-cells comprise peripheral T-cells in a subject. In an embodiment of the methods herein, the subject is human.

The present disclosure provides a method of selectively modulating the activity of an epitope-specific T cell in an individual, the method comprising administering to the individual an amount of the multimeric polypeptide of the present disclosure, or one or more nucleic acids encoding the multimeric polypeptide, effective to selectively modulate the activity of an epitope-specific T cell in an individual. In some cases, a treatment method of the present disclosure comprises administering to an individual in need thereof one or more recombinant expression vectors comprising nucleotide sequences encoding a multimeric polypeptide of the present disclosure. In some cases, a treatment method of the present disclosure comprises administering to an individual in need thereof one or more mRNA molecules comprising nucleotide sequences encoding a multimeric polypeptide of the present disclosure. In some cases, a treatment method of the present disclosure comprises administering to an individual in need thereof a multimeric polypeptide of the present disclosure.

The present disclosure provides a method of selectively modulating the activity of an epitope-specific T cell in an individual, the method comprising administering to the individual an effective amount of a multimeric polypeptide of the present disclosure, or one or more nucleic acids (e.g., expression vectors; mRNA; etc.) comprising nucleotide sequences encoding the multimeric polypeptide, where the multimeric polypeptide selectively modulates the activity of the epitope-specific T cell in the individual. Selectively modulating the activity of an epitope-specific T cell can treat a disease or disorder in the individual. Thus, the present disclosure provides a treatment method comprising administering to an individual in need thereof an effective amount of a multimeric polypeptide of the present disclosure.

In some cases, the immunomodulatory polypeptide is an activating polypeptide, and the multimeric polypeptide activates the epitope-specific T cell. In some cases, the epitope is a cancer-associated epitope, and the multimeric polypeptide increases the activity of a T cell specific for the cancer-associate epitope.

The present disclosure provides a method of treating cancer in an individual, the method comprising administering to the individual an effective amount of a multimeric polypeptide of the present disclosure, or one or more nucleic acids (e.g., expression vectors; mRNA; etc.) comprising nucleotide sequences encoding the multimeric polypeptide, where the multimeric polypeptide comprises a T-cell epitope that is a cancer epitope, and where the multimeric polypeptide comprises a stimulatory immunomodulatory polypeptide. In some cases, an "effective amount" of a multimeric polypeptide is an amount that, when administered in one or more doses to an individual in need thereof, reduces the number of cancer cells in the individual. For example, in some cases, an "effective amount" of a multimeric polypeptide of the present disclosure is an amount that, when administered in one or more doses to an individual in need thereof, reduces the number of cancer cells in the individual by at least 10%, at least 15%, at least 20%, at least 25%, at least 30%, at least 40%, at least 50%, at least 60%, at least 70%, at least 80%, at least 90%, or at least 95%, compared to the number of cancer cells in the individual before administration of the multimeric polypeptide, or in the absence of administration with the multimeric polypeptide. In some cases, an "effective amount" of a multimeric polypeptide of the present disclosure is an amount that, when administered in one or more doses to an individual in need thereof, reduces the number of cancer cells in the individual to undetectable levels. In some cases, an "effective amount" of a multimeric polypeptide of the present disclosure is an amount that, when administered in one or more doses to an individual in need thereof, reduces the tumor mass in the individual. For example, in some cases, an "effective amount" of a multimeric polypeptide of the present disclosure is an amount that, when administered in one or more doses to an individual in need thereof, reduces the tumor mass in the individual by at least 10%, at least 15%, at least 20%, at least 25%, at least 30%, at least 40%, at least 50%, at least 60%, at least 70%, at least 80%, at least 90%, or at least 95%, compared to the tumor mass in the individual before administration of the multimeric polypeptide, or in the absence of administration with the multimeric polypeptide. In some cases, an "effective amount" of a multimeric polypeptide of the present disclosure is an amount that, when administered in one or more doses to an individual in need thereof, increases survival time of the individual. For example, in some cases, an "effective amount" of a multimeric polypeptide of the present disclosure is an amount that, when administered in one or more doses to an individual in need thereof, increases survival time of the individual by at least 1 month, at least 2 months, at least 3 months, from 3 months to 6 months, from 6 months to 1 year, from 1 year to 2 years, from 2 years to 5 years, from 5 years to 10 years, or more than 10 years, compared to the expected survival time of the individual in the absence of administration with the multimeric polypeptide.

In some instances, the epitope-specific T cell is a T cell that is specific for an epitope present on a virus-infected cell, and contacting the epitope-specific T cell with the multimeric polypeptide increases cytotoxic activity of the T cell toward the virus-infected cell. In some instances, the epitope-specific T cell is a T cell that is specific for an epitope present on a virus-infected cell, and contacting the epitope-specific T cell with the multimeric polypeptide increases the number of the epitope-specific T cells.

Thus, the present disclosure provides a method of treating a virus infection in an individual, the method comprising administering to the individual an effective amount of a multimeric polypeptide of the present disclosure, or one or more nucleic acids comprising nucleotide sequences encoding the multimeric polypeptide, where the multimeric polypeptide comprises a T-cell epitope that is a viral epitope, and where the multimeric polypeptide comprises a stimulatory immunomodulatory polypeptide. In some cases, an "effective amount" of a multimeric polypeptide is an amount that, when administered in one or more doses to an individual in need thereof, reduces the number of virus-infected cells in the individual. For example, in some cases, an "effective amount" of a multimeric polypeptide of the present disclosure is an amount that, when administered in one or more doses to an individual in need thereof, reduces the number of virus-infected cells in the individual by at least 10%, at least 15%, at least 20%, at least 25%, at least 30%, at least 40%, at least 50%, at least 60%, at least 70%, at least 80%, at least 90%, or at least 95%, compared to the number of virus-infected cells in the individual before administration of the multimeric polypeptide, or in the absence of administration with the multimeric polypeptide. In some cases, an "effective amount" of a multimeric polypeptide of the present disclosure is an amount that, when administered in one or more doses to an individual in need thereof, reduces the number of virus-infected cells in the individual to undetectable levels.

Thus, the present disclosure provides a method of treating an infection in an individual, the method comprising administering to the individual an effective amount of a multimeric polypeptide of the present disclosure, or one or more nucleic acids comprising nucleotide sequences encoding the multimeric polypeptide, where the multimeric polypeptide comprises a T-cell epitope that is a pathogen-associated epitope, and where the multimeric polypeptide comprises a stimulatory immunomodulatory polypeptide. In some cases, an "effective amount" of a multimeric polypeptide is an amount that, when administered in one or more doses to an individual in need thereof, reduces the number of pathogens in the individual. For example, in some cases, an "effective amount" of a multimeric polypeptide of the present disclosure is an amount that, when administered in one or more doses to an individual in need thereof, reduces the number of pathogens in the individual by at least 10%, at least 15%, at least 20%, at least 25%, at least 30%, at least 40%, at least 50%, at least 60%, at least 70%, at least 80%, at least 90%, or at least 95%, compared to the number of pathogens in the individual before administration of the multimeric polypeptide, or in the absence of administration with the multimeric polypeptide. In some cases, an "effective amount" of a multimeric polypeptide of the present disclosure is an amount that, when administered in one or more doses to an individual in need thereof, reduces the number of pathogens in the individual to undetectable levels. Pathogens include viruses, bacteria, protozoans, and the like.

In some cases, the immunomodulatory polypeptide is an inhibitory polypeptide, and the multimeric polypeptide inhibits activity of the epitope-specific T cell. In some cases, the epitope is a self-epitope, and the multimeric polypeptide selectively inhibits the activity of a T cell specific for the self-epitope.

The present disclosure provides a method of treating an autoimmune disorder in an individual, the method comprising administering to the individual an effective amount of a multimeric polypeptide of the present disclosure, or one or more nucleic acids comprising nucleotide sequences encoding the multimeric polypeptide, where the multimeric polypeptide comprises a T-cell epitope that is a self epitope, and where the multimeric polypeptide comprises an inhibitory immunomodulatory polypeptide. In some cases, an "effective amount" of a multimeric polypeptide is an amount that, when administered in one or more doses to an individual in need thereof, reduces the number self-reactive T cells by at least 10%, at least 15%, at least 20%, at least 25%, at least 30%, at least 40%, at least 50%, at least 60%, at least 70%, at least 80%, at least 90%, or at least 95%, compared to number of self-reactive T cells in the individual before administration of the multimeric polypeptide, or in the absence of administration with the multimeric polypeptide. In some cases, an "effective amount" of a multimeric polypeptide is an amount that, when administered in one or more doses to an individual in need thereof, reduces production of Th2 cytokines in the individual. In some cases, an "effective amount" of a multimeric polypeptide is an amount that, when administered in one or more doses to an individual in need thereof, ameliorates one or more symptoms associated with an autoimmune disease in the individual.

As noted above, in some cases, in carrying out a subject treatment method, a multimeric polypeptide of the present disclosure is administered to an individual in need thereof, as the polypeptide *per se.* In other instances, in carrying out a subject treatment method, one or more nucleic acids comprising nucleotide sequences encoding a multimeric polypeptide of the present disclosure is/are administering to an individual in need thereof. Thus, in other instances, one or more nucleic acids of the present disclosure, e.g., one or more recombinant expression vectors of the present disclosure, is/are administered to an individual in need thereof.

### Formulations

Suitable formulations are described above, where suitable formulations include a pharmaceutically acceptable excipient. In some cases, a suitable formulation comprises: a) a multimeric polypeptide of the present disclosure; and b) a pharmaceutically acceptable excipient. In some cases, a suitable formulation comprises: a) a nucleic acid comprising a nucleotide sequence encoding a multimeric polypeptide of the present disclosure; and b) a pharmaceutically acceptable excipient; in some instances, the nucleic acid is an mRNA. In some cases, a suitable formulation comprises: a) a first nucleic acid comprising a nucleotide sequence encoding the first polypeptide of a multimeric polypeptide of the present disclosure; b) a second nucleic acid comprising a nucleotide sequence encoding the second polypeptide of a multimeric polypeptide of the present disclosure; and c) a pharmaceutically acceptable excipient. In some cases, a suitable formulation comprises: a) a recombinant expression vector comprising a nucleotide sequence encoding a multimeric polypeptide of the present disclosure; and b) a pharmaceutically acceptable excipient. In some cases, a suitable formulation comprises: a) a first recombinant expression vector comprising a nucleotide sequence encoding the first polypeptide of a multimeric polypeptide of the present disclosure; b) a second recombinant expression vector comprising a nucleotide sequence encoding the second polypeptide of a multimeric polypeptide of the present disclosure; and c) a pharmaceutically acceptable excipient.

Suitable pharmaceutically acceptable excipients are described above.

### Dosages

A suitable dosage can be determined by an attending physician or other qualified medical personnel, based on various clinical factors. As is well known in the medical arts, dosages for any one patient depend upon many factors, including the patient's size, body surface area, age, the particular polypeptide or nucleic acid to be administered, sex of the patient, time, and route of administration, general health, and other drugs being administered concurrently. A multimeric polypeptide of the present disclosure may be administered in amounts between 1 ng/kg body weight and 20 mg/kg body weight per dose, e.g. between 0.1 mg/kg body weight to 10 mg/kg body weight, e.g. between 0.5 mg/kg body weight to 5 mg/kg body weight; however, doses below or above this exemplary range are envisioned, especially considering the aforementioned factors. If the regimen is a continuous infusion, it can also be in the range of 1 µg to 10 mg per kilogram of body weight per minute.

In some cases, a suitable dose of a multimeric polypeptide of the present disclosure is from 0.01 µg to 100 g per kg of body weight, from 0.1 µg to 10 g per kg of body weight, from 1 µg to 1 g per kg of body weight, from 10 µg to 100 mg per kg of body weight, from 100 µg to 10 mg per kg of body weight, or from 100 µg to 1 mg per kg of body weight. Persons of ordinary skill in the art can easily estimate repetition rates for dosing based on measured residence times and concentrations of the administered agent in bodily fluids or tissues. Following successful treatment, it may be desirable to have the patient undergo maintenance therapy to prevent the recurrence of the disease state, wherein a multimeric polypeptide of the present disclosure is administered in maintenance doses, ranging from 0.01 µg to 100 g per kg of body weight, from 0.1 µg to 10 g per kg of body weight, from 1 µg to 1 g per kg of body weight, from 10 µg to 100 mg per kg of body weight, from 100 µg to 10 mg per kg of body weight, or from 100 µg to 1 mg per kg of body weight.

Those of skill will readily appreciate that dose levels can vary as a function of the specific multimeric polypeptide, the severity of the symptoms and the susceptibility of the subject to side effects. Preferred dosages for a given compound are readily determinable by those of skill in the art by a variety of means.

In some embodiments, multiple doses of a multimeric polypeptide of the present disclosure, a nucleic acid of the present disclosure, or a recombinant expression vector of the present disclosure are administered. The frequency of administration of a multimeric polypeptide of the present disclosure, a nucleic acid of the present disclosure, or a recombinant expression vector of the present disclosure can vary depending on any of a variety of factors, e.g., severity of the symptoms, etc. For example, in some embodiments, a multimeric polypeptide of the present disclosure, a nucleic acid of the present disclosure, or a recombinant expression vector of the present disclosure is administered once per month, twice per month, three times per month, every other week (qow), once per week (qw), twice per week (biw), three times per week (tiw), four times per week, five times per week, six times per week, every other day (qod), daily (qd), twice a day (qid), or three times a day (tid).

The duration of administration of a multimeric polypeptide of the present disclosure, a nucleic acid of the present disclosure, or a recombinant expression vector of the present disclosure, e.g., the period of time over which a multimeric polypeptide of the present disclosure, a nucleic acid of the present disclosure, or a recombinant expression vector of the present disclosure is administered, can vary, depending on any of a variety of factors, e.g., patient response, etc. For example, a multimeric polypeptide of the present disclosure, a nucleic acid of the present disclosure, or a recombinant expression vector of the present disclosure can be administered over a period of time ranging from about one day to about one week, from about two weeks to about four weeks, from about one month to about two months, from about two months to about four months, from about four months to about six months, from about six months to about eight months, from about eight months to about 1 year, from about 1 year to about 2 years, or from about 2 years to about 4 years, or more.

### Routes of administration

An active agent (a multimeric polypeptide of the present disclosure, a nucleic acid of the present disclosure, or a recombinant expression vector of the present disclosure) is administered to an individual using any available method and route suitable for drug delivery, including *in vivo* and *ex vivo* methods, as well as systemic and localized routes of administration.

Conventional and pharmaceutically acceptable routes of administration include intratumoral, peritumoral, intramuscular, intratracheal, intracranial, subcutaneous, intradermal, topical application, intravenous, intraarterial, rectal, nasal, oral, and other enteral and parenteral routes of administration. Routes of administration may be combined, if desired, or adjusted depending upon the multimeric polypeptide and/or the desired effect. A multimeric polypeptide of the present disclosure, or a nucleic acid or recombinant expression vector of the present disclosure, can be administered in a single dose or in multiple doses.

In some embodiments, a multimeric polypeptide of the present disclosure, a nucleic acid of the present disclosure, or a recombinant expression vector of the present disclosure is administered intravenously. In some embodiments, a multimeric polypeptide of the present disclosure, a nucleic acid of the present disclosure, or a recombinant expression vector of the present disclosure is administered intramuscularly. In some embodiments, a multimeric polypeptide of the present disclosure, a nucleic acid of the present disclosure, or a recombinant expression vector of the present disclosure is administered locally. In some embodiments, a multimeric polypeptide of the present disclosure, a nucleic acid of the present disclosure, or a recombinant expression vector of the present disclosure is administered intratumorally. In some embodiments, a multimeric polypeptide of the present disclosure, a nucleic acid of the present disclosure, or a recombinant expression vector of the present disclosure is administered peritumorally. In some embodiments, a multimeric polypeptide of the present disclosure, a nucleic acid of the present disclosure, or a recombinant expression vector of the present disclosure is administered intracranially. In some embodiments, a multimeric polypeptide of the present disclosure, a nucleic acid of the present disclosure, or a recombinant expression vector of the present disclosure is administered subcutaneously.

In some embodiments, a multimeric polypeptide of the present disclosure is administered intravenously. In some embodiments, a multimeric polypeptide of the present disclosure is administered intramuscularly. In some embodiments, a multimeric polypeptide of the present disclosure is administered locally. In some embodiments, a multimeric polypeptide of the present disclosure is administered intratumorally. In some embodiments, a multimeric polypeptide of the present disclosure is administered peritumorally. In some embodiments, a multimeric polypeptide of the present disclosure is administered intracranially. In some embodiments, a multimeric polypeptide is administered subcutaneously.

A multimeric polypeptide of the present disclosure, a nucleic acid of the present disclosure, or a recombinant expression vector of the present disclosure can be administered to a host using any available conventional methods and routes suitable for delivery of conventional drugs, including systemic or localized routes. In general, routes of administration contemplated by the disclosure include, but are not necessarily limited to, enteral, parenteral, or inhalational routes.

Parenteral routes of administration other than inhalation administration include, but are not necessarily limited to, topical, transdermal, subcutaneous, intramuscular, intraorbital, intracapsular, intraspinal, intrasternal, intratumoral, peritumoral, and intravenous routes, i.e., any route of administration other than through the alimentary canal. Parenteral administration can be carried to effect systemic or local delivery of a multimeric polypeptide of the present disclosure, a nucleic acid of the present disclosure, or a recombinant expression vector of the present disclosure. Where systemic delivery is desired, administration typically involves invasive or systemically absorbed topical or mucosal administration of pharmaceutical preparations.

### Subjects suitable for treatment

Subjects suitable for treatment with a method of the present disclosure include individuals who have cancer, including individuals who have been diagnosed as having cancer, individuals who have been treated for cancer but who failed to respond to the treatment, and individuals who have been treated for cancer and who initially responded but subsequently became refractory to the treatment. Subjects suitable for treatment with a method of the present disclosure include individuals who have an infection (e.g., an infection with a pathogen such as a bacterium, a virus, a protozoan, etc.), including individuals who have been diagnosed as having an infection, and individuals who have been treated for an infection but who failed to respond to the treatment. Subjects suitable for treatment with a method of the present disclosure include individuals who have bacterial infection, including individuals who have been diagnosed as having a bacterial infection, and individuals who have been treated for a bacterial infection but who failed to respond to the treatment. Subjects suitable for treatment with a method of the present disclosure include individuals who have a viral infection, including individuals who have been diagnosed as having a viral infection, and individuals who have been treated for a viral infection but who failed to respond to the treatment. Subjects suitable for treatment with a method of the present disclosure include individuals who have an autoimmune disease, including individuals who have been diagnosed as having an autoimmune disease, and individuals who have been treated for a autoimmune disease but who failed to respond to the treatment.

All combinations of the various elements described herein are within the scope of the invention as defined by the appended claims unless otherwise indicated herein or otherwise clearly contradicted by context.

This invention will be better understood from the Experimental Details, which follow. However, one skilled in the art will readily appreciate that the specific methods and results discussed are merely illustrative of the invention as defined by the appended claims.

### EXAMPLES

The following examples are put forth so as to provide those of ordinary skill in the art with a complete disclosure and description of how to make and use the present invention, and are not intended to limit the scope of what the inventors regard as their invention nor are they intended to represent that the experiments below are all or the only experiments performed. Efforts have been made to ensure accuracy with respect to numbers used (e.g. amounts, temperature, etc.) but some experimental errors and deviations should be accounted for. Unless indicated otherwise, parts are parts by weight, molecular weight is weight average molecular weight, temperature is in degrees Celsius, and pressure is at or near atmospheric. Standard abbreviations may be used, e.g., bp, base pair(s); kb, kilobase(s); pl, picoliter(s); s or sec, second(s); min, minute(s); h or hr, hour(s); aa, amino acid(s); kb, kilobase(s); bp, base pair(s); nt, nucleotide(s); i.m., intramuscular(ly); i.p., intraperitoneal(ly); s.c., subcutaneous(ly); and the like.

### EXAMPLE 1: GENERATION OF SYNTAC HETERODIMERS

Aspects of the instant disclosure pertain to a novel protein based therapeutic platform, "synTac," which mimics the interaction specificity and regulatory signals of the immunological synapse. SynTac is a fusion protein linking a costimulatory molecule to an MHC-epitope allowing for precise T cell engagement and clonal T cell activation or inhibition (FIG. 1), a soluble version of the body's natural response. In this way, synTac combines the best of epitopes, bispecific antibodies, soluble costimulatory molecules and ADCs. SynTac allows for highly specific cell targeting through the MHC-epitope, with a "single chain fusion" design disallowing cross presentation of the free epitope (FIG. 2A-2C). A T cell modulatory domain (alternatively described herein as "MOD") is also covalently attached, which elicits either activation or inhibition depending on the nature of the costimulatory engagement. This elicits an antigen-specific, not global, T cell response. Notably, the MOD can include any known antibody, antibody fragment, costimulatory molecule, or other literature-validated payload (cytokines, toxins, etc.), and does not need to be internalized to exert an effect on the T cell. Moreover, both targets are present on the surface of the same cell eliminating the "spacing problem" of traditional bispecific antibodies.

In one example, the strategy exploits an Fc-fusion construction, (a non-limiting example is set forth in FIG. 2A-2C), to increase the valency, stability and therapeutic window of the associated products. Briefly, the Fc region is a native covalent homo-dimer and stabilized through two disulfide bonds illustrated as two thin lines in FIG. 2A-2C. The presence of the Fc domain is known to prolong therapeutic activity by increasing plasma half-life, owing to its interaction with the neonatal Fc-receptor as well as to the slower renal clearance for larger sized bivalent molecules [23, 24]. From a biophysical perspective, the Fc domain folds independently and can improve the solubility and stability of the partner molecule both *in vitro and in vivo* [25], and the Fc region allows for easy cost-effective purification by protein-A/G affinity chromatography during production [26]. FIG. 2A shows a single chain peptide MHC protein (single chain trimer [27]) linked at its carboxy terminus to an IgG Fc region. As depicted, these single chain constructions are limited with respect to ones ability to extend the system through alternative protein linkages (such as the MOD). Specifically, linkages are preferably restricted to a region C-terminal of the MHC, depicted by dashed lines in FIG. 2A (which herein is termed direct linkage). MHC I or MHC II molecules can be used. Expression of constructs using a direct linkage approach is highly dependent on the MOD being used. A solution to this, disclosed herein, is to split the construct into respective heavy and light chains and fuse both peptides and proteins to various ends (FIG. 2B and FIG. 2C). One construction results in an amino-terminal association of the peptide to the light chain (beta 2 microglobulin) followed by a carboxy terminal extension of the light chain to the MOD effector molecule (FIG. 2B). In this scenario the heavy chain (HLA-molecule) is fused to the Fc region. All components associate during production within eukaryotic cells (e.g., HEK, CHO) and self assemble. Constructs are held together covalently through disulfide bridges. An alternative orientation (FIG. 2C) places the MOD amino-terminal of the Fc fused heavy chain with the peptide still linked to the B2M light chain. Again all components self assemble and form stable covalent interactions through disulfide bonds. Traditional bispecific antibodies often attempt to bridge two cells by dimerizing one amino terminal Fc payload with one carboxy terminal Fc payload. In contrast, a construction disclosed herein orients two different protein payloads, an MHC-epitope targeting mechanism and a MOD effector, to the surface of the same cell, similar to a CH1 - light chain interaction found within traditional antibodies. Further, the use of Fc fusions allows tailored engagement of associated effector functions, such as antibody-dependent cell-mediated cytotoxicity (ADCC), complement-dependent cytotoxicity (CDC) or phagocytosis, by modulation of the binding affinities to Fc receptors through mutations [28].

A design for two base synTac molecules is presented in FIG. 3A-3B. Briefly, this construct utilizes a native human B2M leader sequence to allow for efficient secretion and ER processing immediately followed by a candidate epitope (labeled as peptide). Once in the ER the leader sequence is fully removed and allows for the presentation of the peptide in the MHC binding pocket. For a "light chain" linkage (LC, FIG. 3A), this is coupled to the native B2M molecule through linker L1 and the MOD through linker L2. This entire cassette is linked to another B2M leader sequence, the MHC heavy chain (e.g. human HLA-A02:01 or murine H-2Kd in the examples), and an Fc domain (either human IgG1 or murine IgG2a) by a viral porcine teschovirus-1 (P2A) "self-cleaving" peptide to allow for stoichiometric expression of each chain. The P2A peptide was chosen as this has the highest reported "cleavage" efficiency of all viral 2A peptides expressed in mammalian cells [29]. The "heavy chain" (HC, FIG. 3B) linkage is similar however the viral P2A peptide now follows the B2M and the MOD follows the second leader peptide, leading to the protein construct shown in FIG. 2C. Both constructs can terminate in an 8x His tag for ease of purification.

*Specialized Expression Cells*: Although both chains are expressed and co-localize to the ER, owing to the P2A linkage, there was some concern that endogenous B2M from the expression host (suspension adapted HEK293 cells) could out-compete the recombinant version as HEK293 cells natively express HLA and B2M molecules. This would result in either deceased stability (e.g., manifesting in decreased overall yields) or a highly undesirable heterogeneous protein sample. To avoid this complication, the CRISPR/CAS system was leveraged to knock out native B2M from the HEK cell pool [30]. Briefly, guide RNA was designed against endogenous B2M, transfected along with a plasmid encoding CRISPR/CAS and allowed to culture for three days. The cultured cells were surface stained against anti-B2M and counter selected (sorted on loss of fluorescence) by fluorescence activated cell sorting (FACS). The sorted cells were allowed to recover and subjected to two more rounds of staining, counter-sorting and recovery (3 rounds in total) to ensure efficient (∼100%) knock-out. As illustrated in FIG. 4, the final pool was quality checked by monitoring surface expression of B2M via FACS, suggesting complete ablation of the endogenous B2M protein. Experiments leveraging next generation sequencing to quantify the knock-out percentages at a genomic level are then performed. The resulting HEK-293-B2M-KO line (termed HEK-KO) was used for all subsequent experiments.

*Engineered Disulfide Bonds*: To increase protein stability and circumvent the complications associated with potential peptide transfer to cellular MHC molecules (cross-presentation) and B2M release, single chain constructs are generally employed [27, 31]. However these single chain constructions (shown in FIG. 2A) are limited with respect to an ability to extend the system through alternative protein linkages (such as the MOD). A solution is to split the construct into respective heavy and light chains analogous to previous efforts [32] but now fuse both peptides and proteins to various ends as described (FIG. 2B and 2C). However, in the final construct, to retain the stability afforded by traditional single chain systems, the option of engineering disulfide bridges between the heavy and light chains was investigated (illustrated as S-S in FIG. 2), as seen in disulfide trapped single chain trimers [dt-SCT] [33]. Notably, as initial synTac production attempts utilizing the dt-SCT disulfide schema resulted in low levels of expression, and this being further dependent on the peptide being presented, the dt-SCT disulfide configuration was deemed not ideal for use in split protein systems. Thus, it was sought to identify alternative positions to engineer disulfide bridges better suited for split protein systems, such as synTac. Two positions were chosen from the light chain (2, 12) each with a disulfide bond potential for two positions in the heavy chain (119, 120 and 236, 237 respectively, from analysis of PDB 2X4R). Notably, these positions are highly conserved residues not known to interact with the peptide binding groove [34], TCR complex [35] or CD8 coreceptor [36]. High-level expression was demonstrated for one construct (H236-L12, with H referring to the heavy chain position and L referring to the light, labeled as synTac 18 in FIG. 5A-5B) with modest expression for a second (H237-L12, synTac 17 FIG. 5A-5B). The dt-SCT disulfide schema was used as a positive control (labeled as synTac 2). A high molecular weight moiety was formed as seen by non-reducing PAGE gels suggesting stable disulfide bond formation (FIG. 5A). All expressing constructs were scaled up to the 100 ml scale, purified and activity tested through binding of cognate TCR expressed on the surface of HEK cells (termed HEK-A6), as monitored by FACS fluorescence, suggesting proper folding and activity (FIG. 5B). Cells expressing non-cognate TCR (termed HEK-AS01) were used as a negative control. Additional constructs have been generated bearing only a C-terminal 8X His tag (monovalent).

*SynTac controls*: Previous work has focused on autoimmune diabetes [37], and a disease-relevant model system, specifically autoreactive CD8+ 8.3 T cells isolated from the pancreatic islets of a nonobese diabetic (NOD) mouse, has been used. Building on this work, synTac constructs were generated bearing a peptide composed of residues 206 to 214 of islet-specific glucose-6-phosphatase catalytic subunit-related protein (IGRP206-214) presented by the murine class-I H-2Kd allele (termed IGRP) known to interact with 8.3 T cells. A control synTac presenting the tumor-derived peptide (KYQAVTTTL, SEQ ID NO:18), which is not recognized by 8.3 T cells, was prepared in an identical fashion (e.g., murine H-2Kd presentation) and designated TUM. To determine the degree to which the system can tolerate multiple HLA alleles (e.g., murine H2-Kd, human HLA-A02, etc.), a third synTac variant was constructed bearing a previously validated human HLA-A02 restricted epitope (Human T-lymphotropic virus, Tax 11-19) and termed HTLV. To allow for targeted T cell depletion, initial synTac constructs used a light chain linkage format and carried a PD-L1 MOD domain (schematically illustrated in FIG. 2B). Each synTac variant (IGRP, TUM and HTLV) showed positive expression profiles in HEK-KO cells, non-reducing SDS page results shown in FIG. 6A. To examine the generality of the expression system, IGRP based synTac constructs with variant MOD domains were explored, including two MODs for T cell stimulation (i.e., humanized anti-CD28 single chain Fv and the extracellular domain of TNF ligand 4-1BBL), and another two MODs allowing for T cell inhibition (a single point mutant of B7-1 [W88A], known to bind only to CTLA4 [38] and a truncated variant of PD-L1 [Ig variable domain only]). All constructs expressed well in HEK-KO cells, FIG. 6B. The ability to express synTac proteins leveraging a heavy chain linkage format was further explored (schematically illustrated in FIG. 2C). For these an IGRP epitope was used as the targeting peptide and PD-L1 or humanized anti CD28 scFv as the MOD, again showing positive expression profiles in HEK-KO cells (FIG. 6C). These were subsequently produced at a scale of 1L or more and purified to homogeneity through both Ni2+ IMAC and size exclusion in an endotoxin free environment. All IGRP and TUM constructs were utilized in T cell proliferation assays and HTLV constructs for the TCR-synTac-PD1 bridging experiments below.

*TCR-synTac-PD1 Bridging*: While the solution profile following size exclusion is indicative of a well-folded protein, it is desirable to validate the integrity of each synTac component (both the MHC-epitope targeting mechanism and MOD) prior to employing these reagents in activity assays. The previously described HEK-A6 cells were used as a positive control and cells expressing a non-cognate TCR (AS01, responsive to an HLA-A0201-restricted Epstein-Bar virus epitope) were generated and used as a negative control along with untransduced parental cells, termed HEK-AS01 and PARENTAL respectively. TCR expression was confirmed by mCerulean fluorescence (TCR fusion reporter) and surface staining for the TCR signaling complex (CD3ε expression proxy). HEK-A6 cells were challenged with non-fluorescent purified HTLV-PD-L1 synTac variants and incubated with its cognate receptor PD1 fused to murine IgG2a. The PD-1-Fc fusion was detected using a FITC labeled anti-mouse secondary antibody. FITC fluorescence (i.e. 'bridging') was dependent on cognate TCR surface expression as shown in FIG. 7A-7B. In particular, FITC fluorescence was not observed when challenged against non-cognate TCR bearing HEK cells or parental cells (HEK-AS01, PARENTAL), when challenged against FITC-PD1-Fc only or when the MOD was absent.

*SynTac in action*: T cell Assays. As proof of concept for the targeting power of the synTac platform, an inhibitory synTac construct was tested in a T cell suppression assay. It was hypothesized that a light chain version of synTac IGRP fused to PD-L1 would specifically suppress IGRP206-214-specific T cells. CD8+ splenocytes were purified from a nonobese diabetic mouse transgenic for the 8.3 T cell receptor. This splenocyte subset contains primarily CD8+ T cells which are specific for the IGRP206-214 peptide in the context of H-2Kd. These CD8+ T cells were then cultured in the presence of immobilized anti-CD3 antibody, a treatment known to stimulate polyclonal T cell activation, and treated stimulated cultures with soluble versions of either synTac IGRP-PD-L1 or synTac TUM-PD-L1 to examine the antigen specificity of any suppressive effect. A version of synTac IGRP without PD-L1 served as an effector control for the MOD domain. Before seeding, cells were labeled with carboxyfluorescein succinimidyl ester (CFSE), a fluorescent cytosolic dye whose intensity halves with each cell division, in order to monitor the extent of T cell activation-induced cellular proliferation. After a 5 day culture period, cells were harvested and examined using flow cytometry for viability and proliferation. Supernatants were also examined for the expression of the CD8+ T cell effector cytokines IFNγ and TNFα using a multiplexed flow cytometric bead assay. All CD8+ T cell activation parameters examined were suppressed in an antigen-specific and effector (i.e. MOD) domain-dependent manner, shown in FIG. 8A-8D. That is, IGRP-PD-L1 synTac was highly suppressive relative to either TUM-PD-L1 synTac or IGRP-(without PD-L1) indicating that the activity of synTac was dependent on both the peptide-MHC and MOD domains (FIG. 8D). SynTac was able to suppress IFNγ secretion by approximately 100 fold and resulted in the death of the vast majority of cells, suggesting that synTac bearing PDL1 as a MOD domain is capable of functionally suppressing as well as eliminating targeted specificities.

*Affinity Attenuation*: A possible issue with the use of the PD-1/PDL-1 system as a modulating domain is that PD-L1 has the potential to bind more than one receptor, with concomitant differences in downstream signaling. PD-L1 has been shown to bind to both B7-1 and PD-1. To avoid the complication of off-target binding, single point mutants may be used that bind only the desired target, PD-1 (e.g., specifically G119D and G119R, and others as discussed herein) while retaining their T cell inhibitory potential when tested as independent Fc fusions. Notably, the mutant PD-L1 Fc fusions alone can be useful reagents for immunomodulation. In the context of synTac, these mutants offer a range of PD-1 binding affinities. IGRP based synTac fusion proteins bearing the G119D and G119R mutants have been produced.

*Modular Design*: Soluble monovalent MHC molecules have an intrinsically low affinity for their cognate T cell receptors and thus have not been useful reagents for diagnostic or therapeutic purposes. While dimeric MHC complexes have been used in various systems to visualize antigen specific T cells [39], higher avidity MHC tetramers and higher order multimers are more commonly used [40]. It is clear from the present work that the current dimeric synTac construction provides for high level expression of well folded protein and elicits targeted T cell responses, however in select cases it may be desirable to extend the synTac technology by increasing valency to enhance T cell targeting potential. To that end, synTac variants were designed again bearing an IGRP targeting mechanism, with the PD-L1 MOD as a light chain linkage in the context of an IgA and IgM Fc region. Through covalent association with the J-chain through disulfide bridges, the IgA and IgM backbone allows for tetramer and decamer based presentation respectively. Lentivirus was generated, HEK-KO cells transduced and expression tested, supporting an initial ability to express these reagents. If desired, one can link the MOD directly to the J-chain, as an N-terminal, C-terminal or dual fusion to change the valency of MOD to targeting molecule. Further, owing to the flexibility of the synTac configuration, one can present multiple peptide epitopes or MODs simultaneously (e.g., tri-specificity) by using a dual heavy chain/light chain linkage. In addition, other MODs include but are not limited to 4-1BBL and anti-CD28 for activation and B7W for inhibition. Select constructs can leverage additional targeting epitopes. Moreover, synTac variants with higher levels of valency (IgA and IgM) can be used as well as non-stoichiometrically linked MODs (e.g., J-chain linkages) as described.

### EXAMPLE 2: GENERATION OF TRIMERIC SYNTAC POLYPEPTIDES

*Stimulatory MOD (4-1BBL) receptor trimeric expression:* Initial efforts to generate active 4-1BBL bearing synTacs leveraged the light chain linkage variant (FIG. 3A). This was expressed as a single transfection (all pieces encoded on a single plasmid), split by a viral P2A sequence and resulted in highly expressed well-folded protein (FIG. 6B, lane 5). Gel filtration profiles coupled with multi-angle light scattering (MALS) data, suggested the initial version to be a well-folded dimer (as illustrated in FIG. 10B, FIG. 9B). It has been observed that 4-1BBL, a TNF family ligand, requires trimerization (e.g., three copies of the same protein, homo-trimer) for full activity. To achieve trimerization, the 4-1BBL bearing synTac construct along with "free" 4-1BBL (4-1BBL alone having no affinity tag [residues 50-254, including the membrane proximal and TNF homology domains, FIG. 10A; FIG. 9A]) were both expressed in the same cell (e.g., co-expression) to allow for native assembly and trimerization, as illustrated in FIG. 10C (original synTac construct in BLACK, Free BBL in GRAY) (co-expression of FIG. 9A and FIG. 9B constructs). Gel filtration chromatography coupled with multi-angle light scattering (MALS) data supports that the new version is the desired trimer (FIG. 11A-11B, labeled as synTac number 40 + 51). As described below (*MOD optimization*) the 4-1BBL constructs can be further optimized to further improve expression and purification profiles and increase stability and reproducibility.

*Stimulatory MOD receptor binding and human*/*mouse cross reactivity*: While the solution profile following size exclusion is indicative of a well-folded protein, it is desirable to validate the integrity of each synTac component (both the MHC-epitope targeting mechanism and MOD) prior to employing these reagents in activity assays. This particular targeting mechanism (IGRP peptide in the context of murine Kd) has been thoroughly validated (FIG. 7A-7B), thus the extent of 4-1BBL receptor binding was further investigated. To that end, Protein A microbeads were coated to saturation with recombinant human or mouse 4-1BB-Fc fusion protein (from commercial sources). 4-1BB coated microbeads were then used to bind synTac constructs bearing 4-1BB ligand (dimeric and trimeric versions) as the comodulatory domain, followed by a fluorescent detection antibody specific for the synTac heavy chain isotype. The extent of specific binding of synTac 4-1BBL to bead-borne 4-1BB was then measured by high throughput flow cytometry. Using this system, the degree of cross reactivity and relative affinities of 4-1BBL for both human AND murine 4-1BB was explored in the context of the synTac scaffold. 4-1BBL bearing synTacs (termed Trimer, Dimer) were shown to bind cognate receptor, but not "receptor-less" (termed no MOD) Fc bound microbeads, suggesting a well-folded and active protein reagent (FIG. 12). Further, the timer bound in an affinity range expected for dual trimeric engagement with the original dimer, showing a 10 fold reduction in binding affinity, again supporting dimeric presentation. MOD-less synTac (labeled as no MOD) was used as a negative control, showing no binding for 4-1BBL receptors. Notably, all constructs bind to both murine and human receptors (cross-react) and will thus allow for direct extension to *in vivo* murine trials.

*In vitro T cell stimulation assays*: In order to test the activity of the 4-1BBL synTacs, CD8 splenocytes were first purified from 8.3 TCR transgenic NOD mice and fluorescently labeled with CFSE to track proliferation before being treated *in vitro* with either soluble IGRP-41BBL synTac (dimer and trimer) or soluble TUM-41BBL synTac (FIG. 13). Control treatments were media alone or immobilized anti-CD3. After 4 days in culture, cells were examined by FACS for viability (DAPI exclusion) and proliferation (CFSE dilution). Supernatants were examined for IFNγ and TNFα levels by a flow cytometric ELISA. As in the case of syntac-PDL1 (FIG. 8A-8D), the *in vitro* activity of syntac-41BBL was highly antigen-specific, resulting in much greater viability, proliferation, and cytokine release in the case of syntac IGRP-41BBL versus TUM-41BBL. As expected, trimeric 4-1BBL was necessary for full activity (e.g., proliferation, viability and cytokine release). In addition, responses to IGRP-41BBL compared favorably to the immobilized anti-CD3 benchmark, suggesting that soluble syntac-41BBL can mediate high levels of T cell activation. All further related experiments described herein utilized trimeric syntac-41BBL.

*In vivo T cell stimulation* - *single dose*: Whether synTac-41BBL could exert similar effects on T cell activation *in vivo* was further examined. NOD mice were treated with synTac IGRP-41BBL versus synTac TUM-41BBL and the frequencies of IGRP specific CD8+ T cells in the spleen were determined. Unlike TCR transgenic NOD mice, in which most T cells are of a defined clonotype, standard NOD mice have highly diverse TCR repertoires and are a better approximation of a 'natural' immune repertoire. NOD mice were injected intraperitoneally with synTac IGRP-41BBL, synTac TUM-41BBL or PBS and sacrificed 6 days post injection. Splenocytes were then examined via flow cytometry for relative frequencies of IGRP-specific CD8 T cells using an appropriate peptide-MHC pentamer stain. IGRP-41BBL treatment was associated with a much higher frequency of IGRP-specific CD8 T cells versus controls. In addition, *in-vivo* expanded IGRP-specific cells were capable of producing IFNγ when re-stimulated *in vitro.* These results support the ability of syntac-41BBL to expand functional CD8 effector T cells in an antigen-specific manner (FIG. 14).

*In vivo T cell stimulation* - *multi dose*: The effect of altered treatment regimen on in vivo T cell activation was examined, with particular attention to an established tumor antigen, the "TUM" nonamer peptide. NOD mice were treated with synTac IGRP-41BBL versus synTac TUM-41BBL using three doses (as compared to the previous single dose) over a period of two weeks. The frequencies of IGRP- or TUM-specific CD8 T cells were determined. NOD mice were injected intraperitoneally with synTac IGRP-41BBL, synTac TUM-41BBL or PBS and sacrificed 7 days post injection. Blood (PBMC's) and splenocytes were then examined via flow cytometry for relative frequencies of IGRP- or TUM-specific CD8 T cells using an appropriate peptide-MHC pentamer stain. Again IGRP-41BBL treatment was associated with a much higher frequency of IGRP-specific CD8 T cells, while TUM-41BBL treatment was associated with a much higher frequency of TUM-specific CD8 T cells, versus irrelevant antigen and PBS controls (FIG. 15). A similar pattern was observed in the spleen. These results support the ability of a multidose syntac-41BBL regimen to expand functional CD8 effector T cells in an antigen-specific manner, including the antigen-specific expansion of rare-tumor specific T cells.

*In vivo T cell inhibition*: Non-obese diabetic (NOD) mice were injected intraperitoneally with synTac IGRP-PDL1, synTac TUM-PDL1, or PBS. Six days post injection, pancreata were dissociated and pancreatic cells were examined via flow cytometry for relative frequencies of IGRP-specific CD8⁺ T cells, using an appropriate peptide-MHC pentamer stain. As shown in FIG. 23, IGRP-PDL1 treatment was associated with a much lower frequency of IGRP-specific CD8⁺ T cells, compared to the control synTac TUM-PDL1- and PBS-treated mice. These data demonstrate antigen-specific *in vivo* depletion following a single dose of synTac.

*MOD Optimization*: Over the course of experimentation, it was observed that most of the target protein (4-1BBL trimeric synTac) displayed characteristics of higher order multimers in size exclusion chromatography and would degrade with time, likely through release/exchange of "free" BBL. Thus a 4-1BBL backbone with increased stability and ease of production was sought with an emphasis on covalent assembly of 4-1BBL. Toward that end the use of engineered disulfide bonds within the TNF homology domain of 4-1BBL (FIG. 16A; Disulfides indicated with arrows; FIG. 9C-9E) were explored. From analysis of the X-ray structure (PDB 2X29), three potential pairs of residues were chosen which have likely disulfide bond potential and are unlikely to interfere with receptor binding. Two native residues in each construct were replaced with cysteine residues (Q94C:P245C), Q94C:P242C, and Q89C:L115C, termed synTac 69, 70 and 71 respectively), expressed in human cells with a "free" nontagged version harboring the same mutations (termed 98, 99, 100 respectively) to allow for covalent locking and the degree of disulfide bonding was observed by non-reduced SDS PAGE analysis (FIG. 18; the following co-expression constructs are termed DL1 (disulfide lock-1, synTac 69/98), DL2 (70/99) and DL3 (71/100)). All three constructs expressed well, allowed for disulfide locking (FIG. 18) and bound to receptor (FIG. 17). While these covalent "disulfide-locked" variants of synTac-4-1BBL address the stability issues discussed, co-expression of "free" BBL (co-expression) is still required to allow for trimerization which can complicate the production and biomanufacture of stimulatory synTacs. One solution to this obstacle was found to be expression of the 4-1BBL TNF homology domain as a single contiguous construct, termed single chain trimer (4-1BBL-SCT, FIG. 16B; FIG. 9F). Specifically, three copies of 4-1BBL residues 80-246 (TNF homology domain only) were held covalently by two (G4S)s linker sequences (FIG. 16B, linkers illustrated as curved lines; FIG. 9F). Expression and gel filtration coupled with multi-angle light scattering (MALS) data supports that the new version is the desired covalent single chain trimer (FIG. 18) and bound well to 4-1BBL receptor (FIG. 17).

While the present invention has been described with reference to the specific embodiments thereof, it should be understood by those skilled in the art that various changes may be made and equivalents may be substituted without departing from the invention as defined by the appended claims. In addition, many modifications may be made to adapt a particular situation, material, composition of matter, process, process step or steps, to the objective of the present invention within the scope as defined by the appended claim..

### References

1. Kling, (2009) Nature Biotechnology, 2009. 27:11-12; 2. Wollheim (2002) Expert Opinion in Investigational Drugs, 11(7): 947-953.; 3. Mansh (2011) Yale J Biol Med, 84(4):381-389; 4. Scarpati et al (2014) OncoTargets and Therapy 7:203-209; 5. Rhodes (2007) Alimentary Pharmacology & Therapeutics. 27(1):19-30; 6. Amos et al. (2011) Blood. 118(3):499-509; 7. Hodi et al. (2010) New England Journal of Medicine. 363: p. 711-723; 8. Goldberg (2011) Cancer Immunology and Immunotherapy 344:269-278; 9. Kwon (2012) Molecular Cancer Therapeutics, 11:1062-1070; 10. Vitale et al. (2012) Clinical Cancer Research 18(14):3812-3812; 11. Ngiow et al. (2011) Cancer Research, 71(10):1- 12; 12. Robins et al. (2010) Sci Transl Med, 2(47):47ra64; 13. Reichert (2011) MAbs, 2011. 3(5):415-416; 14. Weiner (2010) Nature Reviews Immunology 10:317-327; 15. Senter (2013) Annual Review of Medicine, 64:15-29; 16. Chames (2009) MAbs 1: 539-547; 17. Jakobsen (2013) Oncolmmunology, 2(2):e22891; 18. Sharma, et al. (2014) Immunologic Research, 58(1):132-138; 19. Wu et al. (2007) Nature Biotechnology, 25: 1290-1297; 20. Dimasi et al. (2009) Journal of Molecular Biology, 393(3):672-692; 21. Grupp, (2011) Cancer Immunology and Immunotherapy, 244:149-172; 22. Xu (2014) Cancer Letters, 343(2):172- 178; 23. Suzuki et al. (2010) Journal of Immunology, 184(4):1968-1976; 24. Sun (2014) Journal of Pharmaceutical Sciences 103(1):53-64; 25. Lo et al. (1998) Protein Engineering 11(6):495-500; 26. Flanagan et al. (2007) Methods in Molecular Biology 378:33-52; 27. Yu et al. (2002) Journal of Immunology 168(7):3145-4149; 28. Hezareh (2001) Journal of Virology 75(24):12161-12168; 29. Kim et al. (2011) PlosOne 6(4):e18556; 30. Yan et al (2014) Methods in Molecular Biology 1114:245-267; 31. Kim et al. (2010) Journal of Immunology 184:4423-4430; 32. Kozono et al. (1994) Nature 369:151-154; 33. Truscott et al. (2007) Journal of Immunology 178:6280-6289; 34. Kellenberger (2005) Journal of Immunology 175:3819-3825; 35. Marrack et al. (2008) Annual Reviews of Immunology 26:171-203; 36. Wang and Margulies (2009) Journal of Immunology 183(4):2554-2564; 37. Samanta et al. (2011). Proc Natl Acad Sci U S A, 108(33):13682-13687; 38. Zheng et al. (1998) Proc Natl Acad Sci U S A, 95:6284-6289; 39. Schneck, J.P., Slansky, J., O'Herrin, S., Greten, T.F., Use of MHC - Ig dimers for visualizing antigen specific T cells. Current Protocol in Immunology, 1999: p. 173; 40. Altman, et al. (1996) Science, 274:94-96.

## Claims

1. A multimeric polypeptide comprising:
a heterodimeric polypeptide comprising:
a) a first polypeptide comprising, in order from N-terminus to C-terminus:
i) a peptide epitope; and
ii) a first major class II histocompatibility complex (MHC) polypeptide;
b) a second polypeptide comprising a second class II MHC polypeptide, and
c) at least one immunomodulatory polypeptide,
wherein the first and/or the second polypeptide comprises the at least one immunomodulatory polypeptide,
wherein the peptide epitope is an epitope of a self antigen, and
optionally, wherein the multimeric polypeptide comprises an immunoglobulin (Ig) Fc polypeptide or a non-Ig scaffold.

2. The multimeric polypeptide of claim 1, wherein the multimeric polypeptide comprises:
a1) a first polypeptide comprising, in order from N-terminus to C-terminus:
i) an epitope; and
ii) a first class II MHC polypeptide; and
b1) a second polypeptide comprising, in order from N-terminus to C-terminus:
i) at least one immunomodulatory polypeptide;
iii) a second class II MHC polypeptide; and
ii) an immunoglobulin (Ig) Fc polypeptide; or
a2) a first polypeptide comprising, in order from N-terminus to C-terminus:
i) an epitope;
ii) a first class II MHC polypeptide; and
iii) at least one immunomodulatory polypeptide; and
b2) a second polypeptide comprising, in order from N-terminus to C-terminus:
i) a second class II MHC polypeptide; and
ii) an Ig Fc polypeptide; or
a3) a first polypeptide comprising, in order from N-terminus to C-terminus:
i) an epitope; and
ii) a first class II MHC polypeptide; and
b3) a second polypeptide comprising, in order from N-terminus to C-terminus:
i) a second class II MHC polypeptide; and
ii) an Ig Fc polypeptide; and
iii) at least one immunomodulatory polypeptide; or
a4) a first polypeptide comprising, in order from N-terminus to C-terminus:
i) an epitope; and
ii) a first class II MHC polypeptide; and
b4) a second polypeptide comprising, in order from N-terminus to C-terminus:
i) a second class II MHC polypeptide; and
ii) at least one immunomodulatory polypeptide; or
a5) a first polypeptide comprising, in order from N-terminus to C-terminus:
i) an epitope; and
ii) a first class II MHC polypeptide; and
b5) a second polypeptide comprising, in order from N-terminus to C-terminus:
i) at least one immunomodulatory polypeptide; and
ii) a second class II MHC polypeptide; or
a6) a first polypeptide comprising, in order from N-terminus to C-terminus:
i) an epitope;
ii) a first class II MHC polypeptide; and
iii) at least one immunomodulatory polypeptide; and
b6) a second polypeptide comprising, in order from N-terminus to C-terminus:
i) a second class II MHC polypeptide.

3. The multimeric polypeptide of claim 1, wherein the non-Ig scaffold is an XTEN polypeptide, a transferrin polypeptide, an Fc receptor polypeptide, an elastin-like polypeptide, a silk-like polypeptide, or a silk-elastin-like polypeptide.

4. The multimeric polypeptide of any one of claims 1-3, wherein the first class II MHC polypeptide is an MHC Class II beta chain polypeptide; and wherein the second class II MHC polypeptide is an MHC class II alpha chain polypeptide.

5. The multimeric polypeptide of any one of claims 1-3, wherein the first class II MHC polypeptide is an MHC Class II alpha chain polypeptide; and wherein the second class II MHC polypeptide is an MHC class II beta chain polypeptide.

6. The multimeric polypeptide of any one of claims 1-5, wherein the at least one immunomodulatory polypeptide is selected from a 4-1BBL polypeptide, a B7-1 polypeptide; a B7-2 polypeptide, an ICOS-L polypeptide, an OX-40L polypeptide, a CD80 polypeptide, a CD86 polypeptide, a PD-L1 polypeptide, a FasL polypeptide, a cytokine and a PD-L2 polypeptide.

7. The multimeric polypeptide of claim 6, wherein the at least one immunomodulatory polypeptide is selected from a PD-L1 polypeptide, a FasL polypeptide and a cytokine.

8. The multimeric polypeptide of any one of claims 1-7, comprising 2 or more immunomodulatory polypeptides.

9. The multimeric polypeptide of claim 8, wherein the 2 or more immunomodulatory polypeptides are in tandem.

10. A protein comprising two of the multimeric polypeptides of any one of claims 1 to 9, wherein each of the two multimeric polypeptides comprises an immunoglobulin (Ig) Fc polypeptide, and wherein the two multimeric polypeptides are joined by one or more disulfide bonds between the respective Ig Fc polypeptides.

11. A nucleic acid comprising a nucleotide sequence encoding a first and/or second polypeptide according to any one of claims 1-9.

12. An expression vector comprising the nucleic acid of claim 11.

13. A host cell genetically modified with the expression vector of claim 12.

14. A method of selectively inhibiting the activity and/or reducing the number of a self-reactive T cell, the method comprising contacting the T cell *in vitro* with the multimeric polypeptide of any one of claims 1-9, or the protein of claim 10, wherein said contacting selectively inhibits the activity and/or reduces the number of the self-reactive T cells.

15. The multimeric polypeptide of any one of claims 1-9, or the protein of claim 10, for use in a method of selectively inhibiting the activity and/or reducing the number of self-reactive T cells in an individual, wherein said multimeric polypeptide or the protein is administered to selectively inhibit the activity and/or reduce the number of self-reactive T cells in the individual.

16. The multimeric polypeptide of any one of claims 1-9, or protein of claim 10, or nucleic acid of claim 11, or expression vector according to claim 12, or one or more mRNAs comprising nucleotide sequences encoding the multimeric polypeptide of any one of claims 1-9, or the protein of claim 10, for use in a method of treating an autoimmune disorder in an individual.

17. A composition comprising:
a) the multimeric polypeptide of any one of claims 1-9, or the protein of claim 10; and
b) a pharmaceutically acceptable excipient.

18. A multimeric polypeptide of any one of claims 1-9, or a protein of claim 10, for use in a method of selectively activating a target T cell that is specific for the peptide epitope present in the multimeric polypeptide, wherein the target T cells is an epitope-specific CD4⁺ T cell.

19. A multimeric polypeptide of any one of claims 1-9, or a protein of claim 10, for use in a method of selectively inhibiting a target T cell that is specific for the peptide epitope present in the multimeric polypeptide, wherein the target T cells is an epitope-specific CD4⁺ T cell.

20. A multimeric polypeptide or protein for use in claim 18, wherein the epitope-specific CD4⁺ T cell is a CD4⁺/CD25⁺/FoxP3⁺ regulatory T cell.

## Patentansprüche

1. Multimeres Polypeptid, das Folgendes umfasst:
ein heterodimeres Polypeptid, umfassend:
a) ein erstes Polypeptid, das in der Reihenfolge vom N-Terminus zum C-Terminus Folgendes umfasst:
i) ein Peptid-Epitop; und
ii) ein erstes Hauptklasse-II-Histokompatibilitätskomplex- (MHC-) Polypeptid;
b) ein zweites Polypeptid, das ein zweites Klasse-II-MHC-Polypeptid umfasst, und
c) zumindest ein immunmodulierendes Polypeptid,
wobei das erste und/oder das zweite Polypeptid das zumindest eine immunmodulierende Polypeptid umfasst,
wobei das Peptid-Epitop ein Epitop eines Selbstantigens ist, und
gegebenenfalls wobei das multimere Polypeptid ein Immunglobulin- (Ig-) Fc-Polypeptid oder ein Nicht-Ig-Gerüst umfasst.

2. Multimeres Polypeptid nach Anspruch 1, wobei das multimere Polypeptid Folgendes umfasst:
a1) ein erstes Polypeptid, das in der Reihenfolge vom N-Terminus zum C-Terminus Folgendes umfasst:
i) ein Epitop; und
ii) ein erstes Klasse-II-MHC-Polypeptid; und
b1) ein zweites Polypeptid, das in der Reihenfolge vom N-Terminus zum C-Terminus Folgendes umfasst:
i) zumindest ein immunmodulierendes Polypeptid;
iii) ein zweites Klasse-II-MHC-Polypeptid; und
ii) ein Immunglobulin- (Ig-) Fc-Polypeptid; oder
a2) ein erstes Polypeptid, das in der Reihenfolge vom N-Terminus zum C-Terminus Folgendes umfasst:
i) ein Epitop;
ii) ein erstes Klasse-II-MHC-Polypeptid; und
iii) zumindest ein immunmodulierendes Polypeptid; und
b2) ein zweites Polypeptid, das in der Reihenfolge vom N-Terminus zum C-Terminus Folgendes umfasst:
i) ein zweites Klasse-II-MHC-Polypeptid; und
ii) ein Ig-Fc-Polypeptid; oder
a3) ein erstes Polypeptid, das in der Reihenfolge vom N-Terminus zum C-Terminus Folgendes umfasst:
i) ein Epitop; und
ii) ein erstes Klasse-II-MHC-Polypeptid; und
b3) ein zweites Polypeptid, das in der Reihenfolge vom N-Terminus zum C-Terminus Folgendes umfasst:
i) ein zweites Klasse-II-MHC-Polypeptid; und
ii) ein Ig-Fc-Polypeptid; und
iii) zumindest ein immunmodulierendes Polypeptid; oder
a4) ein erstes Polypeptid, das in der Reihenfolge vom N-Terminus zum C-Terminus Folgendes umfasst:
i) ein Epitop; und
ii) ein erstes Klasse-II-MHC-Polypeptid; und
b4) ein zweites Polypeptid, das in der Reihenfolge vom N-Terminus zum C-Terminus Folgendes umfasst:
i) ein zweites Klasse-II-MHC-Polypeptid; und
ii) zumindest ein immunmodulierendes Polypeptid; oder
a5) ein erstes Polypeptid, das in der Reihenfolge vom N-Terminus zum C-Terminus Folgendes umfasst:
i) ein Epitop; und
ii) ein erstes Klasse-II-MHC-Polypeptid; und
b5) ein zweites Polypeptid, das in der Reihenfolge vom N-Terminus zum C-Terminus Folgendes umfasst:
i) zumindest ein immunmodulierendes Polypeptid; und
ii) ein zweites Klasse-II-MHC-Polypeptid; oder
a6) ein erstes Polypeptid, das in der Reihenfolge vom N-Terminus zum C-Terminus Folgendes umfasst:
i) ein Epitop;
ii) ein erstes Klasse-II-MHC-Polypeptid; und
iii) zumindest ein immunmodulierendes Polypeptid; und
b6) ein zweites Polypeptid, das in der Reihenfolge vom N-Terminus zum C-Terminus Folgendes umfasst:
i) ein zweites Klasse-II-MHC-Polypeptid.

3. Multimeres Polypeptid nach Anspruch 1, wobei das Nicht-Ig-Gerüst ein XTEN-Poly-peptid, ein Transferrin-Polypeptid, ein Fc-Rezeptor-Polypeptid, ein Elastin-ähnliches Polypeptid, ein Seiden-ähnliches Polypeptid oder ein Seide-Elastin-ähnliches Polypeptid ist.

4. Multimeres Polypeptid nach einem der Ansprüche 1 bis 3, wobei das erste Klasse-II-MHC-Polypeptid ein MHC-Klasse-II-beta-Kette-Polypeptid ist; und wobei das zweite Klasse-II-MHC-Polypeptid ein MHC-Klasse-II-alpha-Kette-Polypeptid ist.

5. Multimeres Polypeptid nach einem der Ansprüche 1 bis 3, wobei das erste Klasse-II-MHC-Polypeptid ein MHC-Klasse-II-alpha-Kette-Polypeptid ist; und wobei das zweite Klasse-II-MHC-Polypeptid ein MHC-Klasse-II-beta-Kette-Polypeptid ist.

6. Multimeres Polypeptid nach einem der Ansprüche 1 bis 5, wobei das zumindest eine immunmodulierende Polypeptid aus einem 4-1BBL-Polypeptid, einem B7-1-Polypeptid; einem B7-2-Polypeptid, einem ICOS-L-Polypeptid, einem OX-40L-Polypeptid, einem CD80-Polypeptid, einem CD86-Polypeptid, einem PD-L1-Polypeptid, einem FasL-Polypeptid, einem Zytokin und einem PD-L2-Polypeptid ausgewählt ist.

7. Multimeres Polypeptid nach Anspruch 6, wobei das zumindest eine immunmodulierende Polypeptid aus einem PD-L1-Polypeptid, einem FasL-Polypeptid und einem Zytokin ausgewählt ist.

8. Multimeres Polypeptid nach einem der Ansprüche 1 bis 7, umfassend 2 oder mehr immunmodulierende Polypeptide.

9. Multimeres Polypeptid nach Anspruch 8, wobei die 2 oder mehr immunmodulierenden Polypeptide im Tandem vorliegen.

10. Protein, umfassend zwei multimere Polypeptide nach einem der Ansprüche 1 bis 9, wobei jedes der zwei multimeren Polypeptide ein Immunglobulin- (Ig-) Fc-Polypeptid umfasst und wobei die zwei multimeren Polypeptide über eine oder mehrere Disulfidbindungen zwischen den jeweiligen Ig-Fc-Polypeptiden verbunden sind.

11. Nucleinsäure, umfassend eine Nucleotidsequenz, die für ein erstes und/oder zweites Polypeptid nach einem der Ansprüche 1 bis 9 kodiert.

12. Expressionsvektor, umfassend eine Nucleinsäure nach Anspruch 11.

13. Wirtszelle, die mit einem Expressionsvektor nach Anspruch 12 genetisch modifiziert wurde.

14. Verfahren zum selektiven Inhibieren der Aktivität und/oder Reduzieren der Anzahl von selbstreaktiven T-Zellen, wobei das Verfahren das Kontaktieren der T-Zellen in vitro mit einem multimeren Polypeptid nach einem der Ansprüche 1 bis 9 oder einem Protein nach Anspruch 10 umfasst, wobei das Kontaktieren die Aktivität von selbstreaktiven T-Zellen selektiv inhibiert und/oder die Anzahl derselben reduziert.

15. Multimeres Polypeptid nach einem der Ansprüche 1 bis 9 oder Protein nach Anspruch 10 zur Verwendung in einem Verfahren zum selektiven Inhibieren der Aktivität von selbstreaktiven T-Zellen und/oder Reduzieren der Anzahl derselben in einem Individuum, wobei das multimere Polypeptid oder das Protein verabreicht wird, um die Aktivität von selbstreaktiven T-Zellen in dem Individuum selektiv zu inhibieren und/oder die Anzahl derselben zu reduzieren.

16. Multimeres Polypeptid nach einem der Ansprüche 1-9 oder Protein nach Anspruch 10 oder Nucleinsäure nach Anspruch 11 oder Expressionsvektor nach Anspruch 12 oder eine oder mehrere mRNAs, umfassend für ein multimeres Polypeptid nach einem der Ansprüche 1 bis 9 oder für Protein nach Anspruch 10 kodierende Nucleotidsequenzen, zur Verwendung in einem Verfahren zum Behandeln einer Autoimmunerkrankung in einem Individuum.

17. Zusammensetzung, die Folgendes umfasst:
a) ein multimeres Polypeptid nach einem der Ansprüche 1 bis 9 oder Protein nach Anspruch 10; und
b) einen pharmazeutisch annehmbaren Exzipienten.

18. Multimeres Polypeptid nach einem der Ansprüche 1 bis 9 oder Protein nach Anspruch 10 zur Verwendung in einem Verfahren zum selektiven Aktivieren einer Ziel-T-Zelle, die für das in dem multimeren Polypeptid vorhandene Peptid-Epitop spezifisch ist, wobei die Ziel-T-Zellen epitopspezifische CD4⁺-T-Zellen sind.

19. Multimeres Polypeptid nach einem der Ansprüche 1 bis 9 oder Protein nach Anspruch 10 zur Verwendung in einem Verfahren zum selektiven Inhibieren einer Ziel-T-Zelle, die für das in dem multimeren Polypeptid vorhandene Peptid-Epitop spezifisch ist, wobei die Ziel-T-Zellen epitopspezifische CD4⁺-T-Zellen sind.

20. Multimeres Polypeptid oder Protein zur Verwendung nach Anspruch 18, wobei die epitopspezifische CD4⁺-T-Zelle eine regulierende CD4⁺/CD25⁺/FoxP3⁺-T-Zelle ist.

## Revendications

1. Polypeptide multimérique comprenant :
un polypeptide hétérodimérique comprenant :
a) un premier polypeptide comprenant, dans l'ordre de l'extrémité N-terminale vers l'extrémité C-terminale :
i) un épitope peptidique ; et
ii) un premier polypeptide du complexe majeur d'histocompatibilité (CMH) de classe II ;
b) un deuxième polypeptide comprenant un deuxième polypeptide du CMH de classe II, et
c) au moins un polypeptide immunomodulateur,
dans lequel le premier et/ou le deuxième polypeptide comprend le au moins un polypeptide immunomodulateur,
dans lequel l'épitope peptidique est un épitope d'un auto-antigène, et
facultativement, dans lequel le polypeptide multimérique comprend un polypeptide Fc d'immunoglobuline (Ig) ou un échafaudage non-Ig.

2. Polypeptide multimérique selon la revendication 1, où le polypeptide multimérique comprend :
a1) un premier polypeptide comprenant, dans l'ordre de l'extrémité N-terminale vers l'extrémité C-terminale :
i) un épitope ; et
ii) un premier polypeptide du CMH de classe II ; et
b1) un deuxième polypeptide comprenant, dans l'ordre de l'extrémité N-terminale vers l'extrémité C-terminale :
i) au moins un polypeptide immunomodulateur ;
iii) un deuxième polypeptide du CMH de classe II ; et
ii) un polypeptide Fc d'immunoglobuline (Ig) ; ou
a2) un premier polypeptide comprenant, dans l'ordre de l'extrémité N-terminale vers l'extrémité C-terminale :
i) un épitope ; et
ii) un premier polypeptide du CMH de classe II ; et
iii) au moins un polypeptide immunomodulateur ; et
b2) un deuxième polypeptide comprenant, dans l'ordre de l'extrémité N-terminale vers l'extrémité C-terminale :
i) un deuxième polypeptide du CMH de classe II ; et
ii) un polypeptide Fc d'Ig ; ou
a3) un premier polypeptide comprenant, dans l'ordre de l'extrémité N-terminale vers l'extrémité C-terminale :
i) un épitope ; et
ii) un premier polypeptide du CMH de classe II ; et
b3) un deuxième polypeptide comprenant, dans l'ordre de l'extrémité N-terminale vers l'extrémité C-terminale :
i) un deuxième polypeptide du CMH de classe II ; et
ii) un polypeptide Fc d'Ig ; et
iii) au moins un polypeptide immunomodulateur ; ou
a4) un premier polypeptide comprenant, dans l'ordre de l'extrémité N-terminale vers l'extrémité C-terminale :
i) un épitope ; et
ii) un premier polypeptide du CMH de classe II ; et
b4) un deuxième polypeptide comprenant, dans l'ordre de l'extrémité N-terminale vers l'extrémité C-terminale :
i) un deuxième polypeptide du CMH de classe II ; et
ii) au moins un polypeptide immunomodulateur ; ou
a5) un premier polypeptide comprenant, dans l'ordre de l'extrémité N-terminale vers l'extrémité C-terminale :
i) un épitope ; et
ii) un premier polypeptide du CMH de classe II ; et
b5) un deuxième polypeptide comprenant, dans l'ordre de l'extrémité N-terminale vers l'extrémité C-terminale :
i) au moins un polypeptide immunomodulateur ; et
ii) un deuxième polypeptide du CMH de classe II ; ou
a6) un premier polypeptide comprenant, dans l'ordre de l'extrémité N-terminale vers l'extrémité C-terminale :
i) un épitope ;
ii) un premier polypeptide du CMH de classe II ; et
iii) au moins un polypeptide immunomodulateur ; et
b6) un deuxième polypeptide comprenant, dans l'ordre de l'extrémité N-terminale vers l'extrémité C-terminale :
i) un deuxième polypeptide du CMH de classe II.

3. Polypeptide multimérique selon la revendication 1, dans lequel l'échafaudage non-Ig est un polypeptide XTEN, un polypeptide de transferrine, un polypeptide de récepteur Fc, un polypeptide de type élastine, un polypeptide de type soie, ou un polypeptide de type soie-élastine.

4. Polypeptide multimérique selon l'une quelconque des revendications 1 à 3, dans lequel le premier polypeptide du CMH de classe II est un polypeptide de chaîne bêta du CMH de classe II ; et dans lequel le deuxième polypeptide du CMH de classe II est un polypeptide de chaîne alpha du CMH de classe II.

5. Polypeptide multimérique selon l'une quelconque des revendications 1 à 3, dans lequel le premier polypeptide du CMH de classe II est un polypeptide de chaîne alpha du CMH de classe II ; et dans lequel le deuxième polypeptide du CMH de classe II est un polypeptide de chaîne bêta du CMH de classe II.

6. Polypeptide multimérique selon l'une quelconque des revendications 1 à 5, dans lequel le au moins un polypeptide immunomodulateur est sélectionné parmi un polypeptide 4-1BBL, un polypeptide B7-1 ; un polypeptide B7-2, un polypeptide ICOS-L, un polypeptide OX-40L, un polypeptide CD80, un polypeptide CD86, un polypeptide PD-L1, un polypeptide FasL, une cytokine et un polypeptide PD-L2.

7. Polypeptide multimérique selon la revendication 6, dans lequel le au moins un polypeptide immunomodulateur est sélectionné parmi un polypeptide PD-L1, un polypeptide FasL et une cytokine.

8. Polypeptide multimérique selon l'une quelconque des revendications 1 à 7, comprenant 2 polypeptides immunomodulateurs ou plus.

9. Polypeptide multimérique selon la revendication 8, dans lequel les 2 polypeptides immunomodulateurs ou plus sont en tandem.

10. Protéine comprenant deux des polypeptides multimériques selon l'une quelconque des revendications 1 à 9, dans laquelle chacun des deux polypeptides multimériques comprend un polypeptide Fc d'immunoglobuline (Ig), et dans laquelle les deux polypeptides multimériques sont joints par une ou plusieurs liaisons disulfure entre les polypeptides Fc d'Ig respectifs.

11. Acide nucléique comprenant une séquence de nucléotides codant pour un premier et/ou deuxième polypeptide selon l'une quelconque des revendications 1 à 9.

12. Vecteur d'expression comprenant l'acide nucléique selon la revendication 11.

13. Cellule hôte génétiquement modifiée avec le vecteur d'expression selon la revendication 12.

14. Procédé pour sélectivement inhiber l'activité et/ou réduire le nombre d'une cellule T auto-réactive, le procédé comprenant la mise en contact de la cellule T *in vitro* avec le polypeptide multimérique selon l'une quelconque des revendications 1-9, ou la protéine selon la revendication 10, dans lequel ladite mise en contact inhibe sélectivement l'activité et/ou réduit le nombre des cellules T auto-réactives.

15. Polypeptide multimérique selon l'une quelconque des revendications 1 à 9, ou protéine selon la revendication 10, destiné(e) à être utilisé(e) dans un procédé pour sélectivement inhiber l'activité et/ou réduire le nombre de cellules T auto-réactives chez un individu, où ledit polypeptide multimérique ou la protéine est administré(e) pour sélectivement inhiber l'activité et/ou réduire le nombre de cellules T auto-réactives chez l'individu.

16. Polypeptide multimérique selon l'une quelconque des revendications 1 à 9, ou protéine selon la revendication 10, ou acide nucléique selon la revendication 11, ou vecteur d'expression selon la revendication 12, ou un ou plusieurs ARNm comprenant des séquences de nucléotides codant pour le polypeptide multimérique selon l'une quelconque des revendications 1 à 9, ou la protéine selon la revendication 10, destiné(e) à être utilisé(e) dans un procédé de traitement d'un trouble auto-immun chez un individu.

17. Composition comprenant :
a) le polypeptide multimérique selon l'une quelconque des revendications 1 à 9, ou la protéine selon la revendication 10 ; et
b) un excipient pharmaceutiquement acceptable.

18. Polypeptide multimérique selon l'une quelconque des revendications 1 à 9, ou protéine selon la revendication 10, destiné(e) à être utilisé(e) dans un procédé pour sélectivement activer une cellule T cible qui est spécifique pour l'épitope peptidique présent dans le polypeptide multimérique, où la cellule T cible est une cellule T CD4⁺ spécifique d'un épitope.

19. Polypeptide multimérique selon l'une quelconque des revendications 1 à 9, ou protéine selon la revendication 10, destiné(e) à être utilisé(e) dans un procédé pour sélectivement inhiber une cellule T cible qui est spécifique pour l'épitope peptidique présent dans le polypeptide multimérique, où la cellule T cible est une cellule T CD4⁺ spécifique d'un épitope.

20. Polypeptide multimérique ou protéine destiné(e) à être utilisé (e) selon la revendication 18, où la cellule T CD4⁺ spécifique d'un épitope est une cellule T régulatrice CD4⁺/CD25⁺/FoxP3⁺.


### SEQUENCE LISTING

<110> Albert Einstein College of Medicine, Inc.
<120> Syntac Polypeptides and Uses Thereof
<130> JEC/FP7434640
<140> EP
   <141> 2015-06-15
<150> EP 15809277.5
   <151> 2015-06-15
<150> PCT/US2015/035777
   <151> 2015-06-15
<150> US 62/013715
   <151> 2014-06-18
<160> 102
<170> PatentIn version 3.5
<210> 1
   <211> 15
   <212> PRT
   <213> Artificial Sequence
<220>
   <223> Synthetic peptide
<400> 1
   Gly Gly Gly Gly Ser Gly Gly Gly Gly Ser Gly Gly Gly Gly Ser
   1               5                   10                  15
<210> 2
   <211> 20
   <212> PRT
   <213> Artificial Sequence
<220>
   <223> Synthetic peptide
<400> 2
   Gly Gly Gly Gly Ser Gly Gly Gly Gly Ser Gly Gly Gly Gly Ser Gly
   1               5                   10                  15
   Gly Gly Gly Ser
               20
<210> 3
   <211> 23
   <212> PRT
   <213> Artificial Sequence
<220>
   <223> Synthetic peptide
<400> 3
   Ser Gly Ser Gly Ala Thr Asn Phe Ser Leu Leu Lys Gln Ala Gly Asp
   1               5                   10                  15
   Val Glu Glu Asn Pro Gly Pro
               20
<210> 4
   <211> 99
   <212> PRT
   <213> Artificial Sequence
<220>
   <223> Synthetic peptide
<400> 4
<210> 5
   <211> 276
   <212> PRT
   <213> Artificial Sequence
<220>
   <223> Synthetic peptide
<400> 5
<210> 6
   <211> 925
   <212> PRT
   <213> Artificial Sequence
<220>
   <223> Synthetic peptide
<400> 6
   Leu Leu Phe Gly Tyr Pro Val Tyr Val Gly Cys Gly Gly Ser Gly Gly
   1               5                   10                  15
   Gly Gly Ser Gly Gly Gly Gly Ser Ile Gln Arg Thr Pro Lys Ile Gln
               20                  25                  30
   Val Tyr Ser Arg His Pro Ala Glu Asn Gly Lys Ser Asn Phe Leu Asn
           35                  40                  45
   Cys Tyr Val Ser Gly Phe His Pro Ser Asp Ile Glu Val Asp Leu Leu
       50                  55                  60
   Lys Asn Gly Glu Arg Ile Glu Lys Val Glu His Ser Asp Leu Ser Phe
   65                  70                  75                  80
   Ser Lys Asp Trp Ser Phe Tyr Leu Leu Tyr Tyr Thr Glu Phe Thr Pro
                   85                  90                  95
   Thr Glu Lys Asp Glu Tyr Ala Cys Arg Val Asn His Val Thr Leu Ser
               100                 105                 110
   Gln Pro Lys Ile Val Lys Trp Asp Arg Asp Met Gly Gly Gly Gly Ser
           115                 120                 125
   Gly Gly Gly Gly Ser Gly Gly Gly Gly Ser Gly Gly Gly Gly Ser Phe
       130                 135                 140
   Thr Ile Thr Ala Pro Lys Asp Leu Tyr Val Val Glu Tyr Gly Ser Asn
   145                 150                 155                 160
   Val Thr Met Glu Cys Arg Phe Pro Val Glu Arg Glu Leu Asp Leu Leu
                   165                 170                 175
   Ala Leu Val Val Tyr Trp Glu Lys Glu Asp Glu Gln Val Ile Gln Phe
               180                 185                 190
   Val Ala Gly Glu Glu Asp Leu Lys Pro Gln His Ser Asn Phe Arg Gly
           195                 200                 205
   Arg Ala Ser Leu Pro Lys Asp Gln Leu Leu Lys Gly Asn Ala Ala Leu
       210                 215                 220
   Gln Ile Thr Asp Val Lys Leu Gln Asp Ala Gly Val Tyr Cys Cys Ile
   225                 230                 235                 240
   Ile Ser Tyr Gly Gly Ala Asp Tyr Lys Arg Ile Thr Leu Lys Val Asn
                   245                 250                 255
   Ala Pro Tyr Arg Lys Ile Asn Gln Arg Ile Ser Val Asp Pro Ala Thr
               260                 265                 270
   Ser Glu His Glu Leu Ile Cys Gln Ala Glu Gly Tyr Pro Glu Ala Glu
           275                 280                 285
   Val Ile Trp Thr Asn Ser Asp His Gln Pro Val Ser Gly Lys Arg Ser
       290                 295                 300
   Val Thr Thr Ser Arg Thr Glu Gly Met Leu Leu Asn Val Thr Ser Ser
   305                 310                 315                 320
   Leu Arg Val Asn Ala Thr Ala Asn Asp Val Phe Tyr Cys Thr Phe Trp
                   325                 330                 335
   Arg Ser Gln Pro Gly Gln Asn His Thr Ala Glu Leu Ile Ile Pro Glu
               340                 345                 350
   Leu Pro Ala Thr His Pro Pro Gln Asn Arg Thr Ser Gly Ser Gly Ala
           355                 360                 365
   Thr Asn Phe Ser Leu Leu Lys Gln Ala Gly Asp Val Glu Glu Asn Pro
       370                 375                 380
   Gly Pro Met Ser Arg Ser Val Ala Leu Ala Val Leu Ala Leu Leu Ser
   385                 390                 395                 400
   Leu Ser Gly Leu Glu Ala Gly Ser His Ser Met Arg Tyr Phe Phe Thr
                   405                 410                 415
   Ser Val Ser Arg Pro Gly Arg Gly Glu Pro Arg Phe Ile Ala Val Gly
               420                 425                 430
   Tyr Val Asp Asp Thr Gln Phe Val Arg Phe Asp Ser Asp Ala Ala Ser
           435                 440                 445
   Gln Arg Met Glu Pro Arg Ala Pro Trp Ile Glu Gln Glu Gly Pro Glu
   450 455 460
   Tyr Trp Asp Gly Glu Thr Arg Lys Val Lys Ala His Ser Gln Thr His
   465 470 475 480
   Arg Val Asp Leu Gly Thr Leu Arg Gly Cys Tyr Asn Gln Ser Glu Ala
   485 490 495
   Gly Ser His Thr Val Gln Arg Met Tyr Gly Cys Asp Val Gly Ser Asp
   500 505 510
   Trp Arg Phe Leu Arg Gly Tyr His Gln Tyr Ala Tyr Asp Gly Lys Asp
   515 520 525
   Tyr Ile Ala Leu Lys Glu Asp Leu Arg Ser Trp Thr Ala Ala Asp Met
   530 535 540
   Ala Ala Gln Thr Thr Lys His Lys Trp Glu Ala Ala His Val Ala Glu
   545 550 555 560
   Gln Leu Arg Ala Tyr Leu Glu Gly Thr Cys Val Glu Trp Leu Arg Arg
   565 570 575
   Tyr Leu Glu Asn Gly Lys Glu Thr Leu Gln Arg Thr Asp Ala Pro Lys
   580 585 590
   Thr His Met Thr His His Ala Val Ser Asp His Glu Ala Thr Leu Arg
   595 600 605
   Cys Trp Ala Leu Ser Phe Tyr Pro Ala Glu Ile Thr Leu Thr Trp Gln
   610 615 620
   Arg Asp Gly Glu Asp Gln Thr Gln Asp Thr Glu Leu Val Glu Thr Arg
   625 630 635 640
   Pro Ala Gly Asp Gly Thr Phe Gln Lys Trp Ala Ala Val Val Val Pro
   645 650 655
   Ser Gly Gln Glu Gln Arg Tyr Thr Cys His Val Gln His Glu Gly Leu
   660 665 670
   Pro Lys Pro Leu Thr Leu Arg Trp Glu Pro Ala Ala Ala Gly Gly Asp
   675 680 685
   Lys Thr His Thr Cys Pro Pro Cys Pro Ala Pro Glu Leu Leu Gly Gly
   690 695 700
   Pro Ser Val Phe Leu Phe Pro Pro Lys Pro Lys Asp Thr Leu Met Ile
   705 710 715 720
   Ser Arg Thr Pro Glu Val Thr Cys Val Val Val Asp Val Ser His Glu
   725 730 735
   Asp Pro Glu Val Lys Phe Asn Trp Tyr Val Asp Gly Val Glu Val His
   740 745 750
   Asn Ala Lys Thr Lys Pro Arg Glu Glu Gln Tyr Asn Ser Thr Tyr Arg
   755 760 765
   Val Val Ser Val Leu Thr Val Leu His Gln Asp Trp Leu Asn Gly Lys
   770 775 780
   Glu Tyr Lys Cys Lys Val Ser Asn Lys Ala Leu Pro Ala Pro Ile Glu
   785 790 795 800
   Lys Thr Ile Ser Lys Ala Lys Gly Gln Pro Arg Glu Pro Gln Val Tyr
   805 810 815
   Thr Leu Pro Pro Ser Arg Glu Glu Met Thr Lys Asn Gln Val Ser Leu
   820 825 830
   Thr Cys Leu Val Lys Gly Phe Tyr Pro Ser Asp Ile Ala Val Glu Trp
   835 840 845
   Glu Ser Asn Gly Gln Pro Glu Asn Asn Tyr Lys Thr Thr Pro Pro Val
   850 855 860
   Leu Asp Ser Asp Gly Ser Phe Phe Leu Tyr Ser Lys Leu Thr Val Asp
   865 870 875 880
   Lys Ser Arg Trp Gln Gln Gly Asn Val Phe Ser Cys Ser Val Met His
   885 890 895
   Glu Ala Leu His Asn His Tyr Thr Gln Lys Ser Leu Ser Leu Ser Pro
   900 905 910
   Gly Lys Gly Gly Ser His His His His His His His His
   915 920 925
<210> 7
   <211> 945
   <212> PRT
   <213> Artificial Sequence
<220>
   <223> Synthetic peptide
<400> 7
   Leu Leu Phe Gly Tyr Pro Val Tyr Val Gly Cys Gly Gly Ser Gly Gly
   1               5                   10                  15
   Gly Gly Ser Gly Gly Gly Gly Ser Ile Gln Arg Thr Pro Lys Ile Gln
               20                  25                  30
   Val Tyr Ser Arg His Pro Ala Glu Asn Gly Lys Ser Asn Phe Leu Asn
           35                  40                  45
   Cys Tyr Val Ser Gly Phe His Pro Ser Asp Ile Glu Val Asp Leu Leu
       50                  55                  60
   Lys Asn Gly Glu Arg Ile Glu Lys Val Glu His Ser Asp Leu Ser Phe
   65                  70                  75                  80
   Ser Lys Asp Trp Ser Phe Tyr Leu Leu Tyr Tyr Thr Glu Phe Thr Pro
                   85                  90                  95
   Thr Glu Lys Asp Glu Tyr Ala Cys Arg Val Asn His Val Thr Leu Ser
               100                 105                 110
   Gln Pro Lys Ile Val Lys Trp Asp Arg Asp Met Gly Gly Gly Gly Ser
           115                 120                 125
   Gly Gly Gly Gly Ser Gly Gly Gly Gly Ser Gly Gly Gly Gly Ser Ser
       130                 135                 140
   Gly Ser Gly Ala Thr Asn Phe Ser Leu Leu Lys Gln Ala Gly Asp Val
   145                 150                 155                 160
   Glu Glu Asn Pro Gly Pro Met Ser Arg Ser Val Ala Leu Ala Val Leu
                   165                 170                 175
   Ala Leu Leu Ser Leu Ser Gly Leu Glu Ala Phe Thr Ile Thr Ala Pro
               180                 185                 190
   Lys Asp Leu Tyr Val Val Glu Tyr Gly Ser Asn Val Thr Met Glu Cys
           195                 200                 205
   Arg Phe Pro Val Glu Arg Glu Leu Asp Leu Leu Ala Leu Val Val Tyr
       210                 215                 220
   Trp Glu Lys Glu Asp Glu Gln Val Ile Gln Phe Val Ala Gly Glu Glu
   225                 230                 235                 240
   Asp Leu Lys Pro Gln His Ser Asn Phe Arg Gly Arg Ala Ser Leu Pro
   245 250 255
   Lys Asp Gln Leu Leu Lys Gly Asn Ala Ala Leu Gln Ile Thr Asp Val
   260 265 270
   Lys Leu Gln Asp Ala Gly Val Tyr Cys Cys Ile Ile Ser Tyr Gly Gly
   275 280 285
   Ala Asp Tyr Lys Arg Ile Thr Leu Lys Val Asn Ala Pro Tyr Arg Lys
   290 295 300
   Ile Asn Gln Arg Ile Ser Val Asp Pro Ala Thr Ser Glu His Glu Leu
   305 310 315 320
   Ile Cys Gln Ala Glu Gly Tyr Pro Glu Ala Glu Val Ile Trp Thr Asn
   325 330 335
   Ser Asp His Gln Pro Val Ser Gly Lys Arg Ser Val Thr Thr Ser Arg
   340 345 350
   Thr Glu Gly Met Leu Leu Asn Val Thr Ser Ser Leu Arg Val Asn Ala
   355 360 365
   Thr Ala Asn Asp Val Phe Tyr Cys Thr Phe Trp Arg Ser Gln Pro Gly
   370 375 380
   Gln Asn His Thr Ala Glu Leu Ile Ile Pro Glu Leu Pro Ala Thr His
   385 390 395 400
   Pro Pro Gln Asn Arg Thr Gly Gly Gly Gly Ser Gly Gly Gly Gly Ser
   405 410 415
   Gly Gly Gly Gly Ser Gly Gly Gly Gly Ser Gly Ser His Ser Met Arg
   420 425 430
   Tyr Phe Phe Thr Ser Val Ser Arg Pro Gly Arg Gly Glu Pro Arg Phe
   435 440 445
   Ile Ala Val Gly Tyr Val Asp Asp Thr Gln Phe Val Arg Phe Asp Ser
   450 455 460
   Asp Ala Ala Ser Gln Arg Met Glu Pro Arg Ala Pro Trp Ile Glu Gln
   465 470 475 480
   Glu Gly Pro Glu Tyr Trp Asp Gly Glu Thr Arg Lys Val Lys Ala His
   485 490 495
   Ser Gln Thr His Arg Val Asp Leu Gly Thr Leu Arg Gly Cys Tyr Asn
   500 505 510
   Gln Ser Glu Ala Gly Ser His Thr Val Gln Arg Met Tyr Gly Cys Asp
   515 520 525
   Val Gly Ser Asp Trp Arg Phe Leu Arg Gly Tyr His Gln Tyr Ala Tyr
   530 535 540
   Asp Gly Lys Asp Tyr Ile Ala Leu Lys Glu Asp Leu Arg Ser Trp Thr
   545 550 555 560
   Ala Ala Asp Met Ala Ala Gln Thr Thr Lys His Lys Trp Glu Ala Ala
   565 570 575
   His Val Ala Glu Gln Leu Arg Ala Tyr Leu Glu Gly Thr Cys Val Glu
   580 585 590
   Trp Leu Arg Arg Tyr Leu Glu Asn Gly Lys Glu Thr Leu Gln Arg Thr
   595 600 605
   Asp Ala Pro Lys Thr His Met Thr His His Ala Val Ser Asp His Glu
   610 615 620
   Ala Thr Leu Arg Cys Trp Ala Leu Ser Phe Tyr Pro Ala Glu Ile Thr
   625 630 635 640
   Leu Thr Trp Gln Arg Asp Gly Glu Asp Gln Thr Gln Asp Thr Glu Leu
   645 650 655
   Val Glu Thr Arg Pro Ala Gly Asp Gly Thr Phe Gln Lys Trp Ala Ala
   660 665 670
   Val Val Val Pro Ser Gly Gln Glu Gln Arg Tyr Thr Cys His Val Gln
   675 680 685
   His Glu Gly Leu Pro Lys Pro Leu Thr Leu Arg Trp Glu Pro Ala Ala
   690 695 700
   Ala Gly Gly Asp Lys Thr His Thr Cys Pro Pro Cys Pro Ala Pro Glu
   705 710 715 720
   Leu Leu Gly Gly Pro Ser Val Phe Leu Phe Pro Pro Lys Pro Lys Asp
   725 730 735
   Thr Leu Met Ile Ser Arg Thr Pro Glu Val Thr Cys Val Val Val Asp
               740                 745                 750
   Val Ser His Glu Asp Pro Glu Val Lys Phe Asn Trp Tyr Val Asp Gly
           755                 760                 765
   Val Glu Val His Asn Ala Lys Thr Lys Pro Arg Glu Glu Gln Tyr Asn
       770                 775                 780
   Ser Thr Tyr Arg Val Val Ser Val Leu Thr Val Leu His Gln Asp Trp
   785                 790                 795                 800
   Leu Asn Gly Lys Glu Tyr Lys Cys Lys Val Ser Asn Lys Ala Leu Pro
                   805                 810                 815
   Ala Pro Ile Glu Lys Thr Ile Ser Lys Ala Lys Gly Gln Pro Arg Glu
               820                 825                 830
   Pro Gln Val Tyr Thr Leu Pro Pro Ser Arg Glu Glu Met Thr Lys Asn
           835                 840                 845
   Gln Val Ser Leu Thr Cys Leu Val Lys Gly Phe Tyr Pro Ser Asp Ile
       850                 855                 860
   Ala Val Glu Trp Glu Ser Asn Gly Gln Pro Glu Asn Asn Tyr Lys Thr
   865                 870                 875                 880
   Thr Pro Pro Val Leu Asp Ser Asp Gly Ser Phe Phe Leu Tyr Ser Lys
                   885                 890                 895
   Leu Thr Val Asp Lys Ser Arg Trp Gln Gln Gly Asn Val Phe Ser Cys
               900                 905                 910
   Ser Val Met His Glu Ala Leu His Asn His Tyr Thr Gln Lys Ser Leu
           915                 920                 925
   Ser Leu Ser Pro Gly Lys Gly Gly Ser His His His His His His His
       930                 935                 940
   His
   945
<210> 8
   <211> 5
   <212> PRT
   <213> Artificial Sequence
<220>
   <223> Synthetic peptide
<220>
   <221> REPEAT
   <222> (1)..(5)
   <223> Repeat n times where n is an integer of at least 1
<400> 8
   Gly Ser Gly Gly Ser
   1               5
<210> 9
   <211> 4
   <212> PRT
   <213> Artificial Sequence
<220>
   <223> Synthetic peptide
<220>
   <221> REPEAT
   <222> (1)..(4)
   <223> Repeat n times where n is an integer of at least 1
<400> 9
   Gly Gly Gly Ser
   1
<210> 10
   <211> 4
   <212> PRT
   <213> Artificial Sequence
<220>
   <223> Synthetic peptide
<400> 10
   Gly Gly Ser Gly
   1
<210> 11
   <211> 5
   <212> PRT
   <213> Artificial Sequence
<220>
   <223> Synthetic peptide
<400> 11
   Gly Gly Ser Gly Gly
   1               5
<210> 12
   <211> 5
   <212> PRT
   <213> Artificial Sequence
<220>
   <223> Synthetic peptide
<400> 12
   Gly Ser Gly Ser Gly
   1               5
<210> 13
   <211> 5
   <212> PRT
   <213> Artificial Sequence
<220>
   <223> Synthetic peptide
<400> 13
   Gly Ser Gly Gly Gly
   1               5
<210> 14
   <211> 5
   <212> PRT
   <213> Artificial Sequence
<220>
   <223> Synthetic peptide
<400> 14
   Gly Gly Gly Ser Gly
   1               5
<210> 15
   <211> 5
   <212> PRT
   <213> Artificial Sequence
<220>
   <223> Synthetic peptide
<400> 15
   Gly Ser Ser Ser Gly
   1               5
<210> 16
   <211> 15
   <212> PRT
   <213> Artificial Sequence
<220>
   <223> Synthetic peptide
<400> 16
   Gly Cys Gly Ala Ser Gly Gly Gly Gly Ser Gly Gly Gly Gly Ser
   1 5 10 15
<210> 17
   <211> 9
   <212> PRT
   <213> Artificial Sequence
<220>
   <223> Synthetic peptide
<400> 17
   Leu Leu Phe Gly Tyr Pro Val Tyr Val
   1 5
<210> 18
   <211> 9
   <212> PRT
   <213> Artificial Sequence
<220>
   <223> Synthetic peptide
<400> 18
   Lys Tyr Gln Ala Val Thr Thr Thr Leu
   1 5
<210> 19
   <211> 9
   <212> PRT
   <213> Artificial Sequence
<220>
   <223> Synthetic peptide
<400> 19
   Val Tyr Leu Lys Thr Asn Val Phe Leu
   1 5
<210> 20
   <211> 9
   <212> PRT
   <213> Artificial Sequence
<220>
   <223> Synthetic peptide
<400> 20
   Thr Tyr Leu Lys Thr Asn Leu Phe Leu
   1 5
<210> 21
   <211> 5
   <212> PRT
   <213> Artificial Sequence
<220>
   <223> Synthetic peptide
<220>
   <221> MISC_FEATURE
   <222> (2)..(2)
   <223> X can be L or Q
<400> 21
   Pro Xaa Gly Met Thr
   1               5
<210> 22
   <211> 274
   <212> PRT
   <213> Artificial Sequence
<220>
   <223> Synthetic peptide
<400> 22
   Gly Pro His Ser Leu Arg Tyr Phe Val Thr Ala Val Ser Arg Pro Gly
   1               5                   10                  15
   Leu Gly Glu Pro Arg Phe Ile Ala Val Gly Tyr Val Asp Asp Thr Gln
               20                  25                  30
   Phe Val Arg Phe Asp Ser Asp Ala Asp Asn Pro Arg Phe Glu Pro Arg
           35                  40                  45
   Ala Pro Trp Met Glu Gln Glu Gly Pro Glu Tyr Trp Glu Glu Gln Thr
       50                  55                  60
   Gln Arg Ala Lys Ser Asp Glu Gln Trp Phe Arg Val Ser Leu Arg Thr
   65                  70                  75                  80
   Ala Gln Arg Tyr Tyr Asn Gln Ser Lys Gly Gly Ser His Thr Phe Gln
                   85                  90                  95
   Arg Met Phe Gly Cys Asp Val Gly Ser Asp Trp Arg Leu Leu Arg Gly
               100                 105                 110
   Tyr Gln Gln Phe Ala Tyr Asp Gly Arg Asp Tyr Ile Ala Leu Asn Glu
           115                 120                 125
   Asp Leu Lys Thr Trp Thr Ala Ala Asp Thr Ala Ala Leu Ile Thr Arg
       130                 135                 140
   Arg Lys Trp Glu Gln Ala Gly Asp Ala Glu Tyr Tyr Arg Ala Tyr Leu
   145 150 155 160
   Glu Gly Glu Cys Val Glu Trp Leu Arg Arg Tyr Leu Glu Leu Gly Asn
   165 170 175
   Glu Thr Leu Leu Arg Thr Asp Ser Pro Lys Ala His Val Thr Tyr His
   180 185 190
   Pro Arg Ser Gln Val Asp Val Thr Leu Arg Cys Trp Ala Leu Gly Phe
   195 200 205
   Tyr Pro Ala Asp Ile Thr Leu Thr Trp Gln Leu Asn Gly Glu Asp Leu
   210 215 220
   Thr Gln Asp Met Glu Leu Val Glu Thr Arg Pro Ala Gly Asp Gly Thr
   225 230 235 240
   Phe Gln Lys Trp Ala Ala Val Val Val Pro Leu Gly Lys Glu Gln Asn
   245 250 255
   Tyr Thr Cys His Val His His Lys Gly Leu Pro Glu Pro Leu Thr Leu
   260 265 270
   Arg Trp
<210> 23
   <211> 9
   <212> PRT
   <213> Artificial Sequence
<220>
   <223> Synthetic peptide
<400> 23
   Tyr Pro Tyr Asp Val Pro Asp Tyr Ala
   1 5
<210> 24
   <211> 8
   <212> PRT
   <213> Artificial Sequence
<220>
   <223> Synthetic peptide
<400> 24
   Asp Tyr Lys Asp Asp Asp Asp Lys
   1 5
<210> 25
   <211> 10
   <212> PRT
   <213> Artificial Sequence
<220>
   <223> Synthetic peptide
<400> 25
   Glu Gln Lys Leu Ile Ser Glu Glu Asp Leu
   1               5                   10
<210> 26
   <211> 5
   <212> PRT
   <213> Artificial Sequence
<220>
   <223> Synthetic peptide
<400> 26
   His His His His His
   1               5
<210> 27
   <211> 6
   <212> PRT
   <213> Artificial Sequence
<220>
   <223> Synthetic peptide
<400> 27
   His His His His His His
   1               5
<210> 28
   <211> 8
   <212> PRT
   <213> Artificial Sequence
<220>
   <223> Synthetic peptide
<400> 28
   Trp Ser His Pro Gln Phe Glu Lys
   1               5
<210> 29
   <211> 9
   <212> PRT
   <213> Artificial Sequence
<220>
   <223> Synthetic peptide
<400> 29
   Tyr Pro Tyr Asp Val Pro Asp Tyr Ala
   1               5
<210> 30
   <211> 5
   <212> PRT
   <213> Artificial Sequence
<220>
   <223> Synthetic peptide
<400> 30
   Arg Tyr Ile Arg Ser
   1               5
<210> 31
   <211> 4
   <212> PRT
   <213> Artificial Sequence
<220>
   <223> Synthetic peptide
<400> 31
   Phe His His Thr
   1
<210> 32
   <211> 17
   <212> PRT
   <213> Artificial Sequence
<220>
   <223> Synthetic peptide
<400> 32
   Trp Glu Ala Ala Ala Arg Glu Ala Cys Cys Arg Glu Cys Cys Ala Arg
   1               5                   10                  15
   Ala
<210> 33
   <211> 6
   <212> PRT
   <213> Artificial Sequence
<220>
   <223> Synthetic peptide
<220>
   <221> SITE
   <222> (2)..(2)
   <223> X can be L or Q
<400> 33
   Pro Xaa Gly Met Thr Ser
   1               5
<210> 34
   <211> 7
   <212> PRT
   <213> Artificial Sequence
<220>
   <223> Synthetic peptide
<400> 34
   Glu Asn Leu Tyr Thr Gln Ser
   1               5
<210> 35
   <211> 5
   <212> PRT
   <213> Artificial Sequence
<220>
   <223> Synthetic peptide
<400> 35
   Asp Asp Asp Asp Lys
   1               5
<210> 36
   <211> 4
   <212> PRT
   <213> Artificial Sequence
<220>
   <223> Synthetic peptide
<400> 36
   Leu Val Pro Arg
   1
<210> 37
   <211> 8
   <212> PRT
   <213> Artificial Sequence
<220>
   <223> Synthetic peptide
<400> 37
   Leu Glu Val Leu Phe Gln Gly Pro
   1               5
<210> 38
   <211> 10
   <212> PRT
   <213> Artificial Sequence
<220>
   <223> Synthetic peptide
<400> 38
   Cys Gly Leu Val Pro Ala Gly Ser Gly Pro
   1               5                   10
<210> 39
   <211> 12
   <212> PRT
   <213> Artificial Sequence
<220>
   <223> Synthetic peptide
<400> 39
   Ser Leu Leu Lys Ser Arg Met Val Pro Asn Phe Asn
   1               5                   10
<210> 40
   <211> 12
   <212> PRT
   <213> Artificial Sequence
<220>
   <223> Synthetic peptide
<400> 40
   Ser Leu Leu Ile Ala Arg Arg Met Pro Asn Phe Asn
   1               5                   10
<210> 41
   <211> 12
   <212> PRT
   <213> Artificial Sequence
<220>
   <223> Synthetic peptide
<400> 41
   Ser Lys Leu Val Gln Ala Ser Ala Ser Gly Val Asn
   1               5                   10
<210> 42
   <211> 12
   <212> PRT
   <213> Artificial Sequence
<220>
   <223> Synthetic peptide
<400> 42
   Ser Ser Tyr Leu Lys Ala Ser Asp Ala Pro Asp Asn
   1               5                   10
<210> 43
   <211> 12
   <212> PRT
   <213> Artificial Sequence
<220>
   <223> Synthetic peptide
<400> 43
   Arg Pro Lys Pro Gln Gln Phe Phe Gly Leu Met Asn
   1               5                   10
<210> 44
   <211> 12
   <212> **PRT**
   <213> Artificial Sequence
<220>
   <223> Synthetic peptide
<400> 44
   Ser Leu Arg Pro Leu Ala Leu Trp Arg Ser Phe Asn
   1               5                   10
<210> 45
   <211> 12
   <212> PRT
   <213> Artificial Sequence
<220>
   <223> Synthetic peptide
<400> 45
   Ser Pro Gln Gly Ile Ala Gly Gln Arg Asn Phe Asn
   1               5                   10
<210> 46
   <211> 14
   <212> PRT
   <213> Artificial Sequence
<220>
   <223> Synthetic peptide
<400> 46
   Asp Val Asp Glu Arg Asp Val Arg Gly Phe Ala Ser Phe Leu
   1 5 10
<210> 47
   <211> 12
   <212> PRT
   <213> Artificial Sequence
<220>
   <223> Synthetic peptide
<400> 47
   Ser Leu Pro Leu Gly Leu Trp Ala Pro Asn Phe Asn
   1 5 10
<210> 48
   <211> 12
   <212> PRT
   <213> Artificial Sequence
<220>
   <223> Synthetic peptide
<400> 48
   Ser Leu Leu Ile Phe Arg Ser Trp Ala Asn Phe Asn
   1 5 10
<210> 49
   <211> 12
   <212> PRT
   <213> Artificial Sequence
<220>
   <223> Synthetic peptide
<400> 49
   Ser Gly Val Val Ile Ala Thr Val Ile Val Ile Thr
   1 5 10
<210> 50
   <211> 12
   <212> PRT
   <213> Artificial Sequence
<220>
   <223> Synthetic peptide
<400> 50
   Ser Leu Gly Pro Gln Gly Ile Trp Gly Gln Phe Asn
   1 5 10
<210> 51
   <211> 12
   <212> PRT
   <213> Artificial Sequence
<220>
   <223> Synthetic peptide
<400> 51
   Lys Lys Ser Pro Gly Arg Val Val Gly Gly Ser Val
   1               5                   10
<210> 52
   <211> 12
   <212> PRT
   <213> Artificial Sequence
<220>
   <223> Synthetic peptide
<400> 52
   Pro Gln Gly Leu Leu Gly Ala Pro Gly Ile Leu Gly
   1               5                   10
<210> 53
   <211> 31
   <212> PRT
   <213> Artificial Sequence
<220>
   <223> Synthetic peptide
<400> 53
   His Gly Pro Glu Gly Leu Arg Val Gly Phe Tyr Glu Ser Asp Val Met
   1               5                   10                  15
   Gly Arg Gly His Ala Arg Leu Val His Val Glu Glu Pro His Thr
               20                  25                  30
<210> 54
   <211> 12
   <212> PRT
   <213> Artificial Sequence
<220>
   <223> Synthetic peptide
<400> 54
   Gly Pro Gln Gly Leu Ala Gly Gln Arg Gly Ile Val
   1               5                   10
<210> 55
   <211> 12
   <212> PRT
   <213> Artificial Sequence
<220>
   <223> Synthetic peptide
<400> 55
   Gly Gly Ser Gly Gln Arg Gly Arg Lys Ala Leu Glu
   1 5 10
<210> 56
   <211> 12
   <212> PRT
   <213> Artificial Sequence
<220>
   <223> Synthetic peptide
<400> 56
   Ser Leu Ser Ala Leu Leu Ser Ser Asp Ile Phe Asn
   1 5 10
<210> 57
   <211> 12
   <212> PRT
   <213> Artificial Sequence
<220>
   <223> Synthetic peptide
<400> 57
   Ser Leu Pro Arg Phe Lys Ile Ile Gly Gly Phe Asn
   1 5 10
<210> 58
   <211> 12
   <212> PRT
   <213> Artificial Sequence
<220>
   <223> Synthetic peptide
<400> 58
   Ser Leu Leu Gly Ile Ala Val Pro Gly Asn Phe Asn
   1 5 10
<210> 59
   <211> 12
   <212> PRT
   <213> Artificial Sequence
<220>
   <223> Synthetic peptide
<400> 59
   Phe Phe Lys Asn Ile Val Thr Pro Arg Thr Pro Pro
   1               5                   10
<210> 60
   <211> 19
   <212> PRT
   <213> Artificial Sequence
<220>
   <223> Synthetic peptide
<400> 60
   Ala Thr Asn Phe Ser Leu Leu Lys Gln Ala Gly Asp Val Glu Glu Asn
   1               5                   10                  15
   Pro Gly Pro
<210> 61
   <211> 18
   <212> PRT
   <213> Artificial Sequence
<220>
   <223> Synthetic peptide
<400> 61
   Glu Gly Arg Gly Ser Leu Leu Thr Cys Gly Asp Val Glu Glu Asn Pro
   1               5                   10                  15
   Gly Pro
<210> 62
   <211> 20
   <212> PRT
   <213> Artificial Sequence
<220>
   <223> Synthetic peptide
<400> 62
   Gln Cys Thr Asn Tyr Ala Leu Leu Lys Leu Ala Gly Asp Val Glu Ser
   1               5                   10                  15
   Asn Pro Gly Pro
               20
<210> 63
   <211> 22
   <212> PRT
   <213> Artificial Sequence
<220>
   <223> Synthetic peptide
<400> 63
   Val Lys Gln Thr Leu Asn Phe Asp Leu Leu Lys Leu Ala Gly Asp Val
   1               5                   10                  15
   Glu Ser Asn Pro Gly Pro
               20
<210> 64
   <211> 22
   <212> PRT
   <213> Artificial Sequence
<220>
   <223> Synthetic peptide
<400> 64
   Gly Ser Gly Ala Thr Asn Phe Ser Leu Leu Lys Gln Ala Gly Asp Val
   1               5                   10                  15
   Glu Glu Asn Pro Gly Pro
               20
<210> 65
   <211> 21
   <212> PRT
   <213> Artificial Sequence
<220>
   <223> Synthetic peptide
<400> 65
   Gly Ser Gly Glu Gly Arg Gly Ser Leu Leu Thr Cys Gly Asp Val Glu
   1               5                   10                  15
   Glu Asn Pro Gly Pro
               20
<210> 66
   <211> 23
   <212> PRT
   <213> Artificial Sequence
<220>
   <223> Synthetic peptide
<400> 66
   Gly Ser Gly Gln Cys Thr Asn Tyr Ala Leu Leu Lys Leu Ala Gly Asp
   1 5 10 15
   Val Glu Ser Asn Pro Gly Pro
   20
<210> 67
   <211> 25
   <212> PRT
   <213> Artificial Sequence
<220>
   <223> Synthetic peptide
<400> 67
   Gly Ser Gly Val Lys Gln Thr Leu Asn Phe Asp Leu Leu Lys Leu Ala
   1 5 10 15
   Gly Asp Val Glu Ser Asn Pro Gly Pro
   20 25
<210> 68
   <211> 20
   <212> PRT
   <213> Homo sapiens
<400> 68
   Met Ser Arg Ser Val Ala Leu Ala Val Leu Ala Leu Leu Ser Leu Ser
   1 5 10 15
   Gly Leu Glu Ala
   20
<210> 69
   <211> 225
   <212> PRT
   <213> Artificial Sequence
<220>
   <223> Synthetic polypeptide
<400> 69
   Met Ser Arg Ser Val Ala Leu Ala Val Leu Ala Leu Leu Ser Leu Ser
   1               5                   10                  15
   Gly Leu Glu Ala Ala Cys Pro Trp Ala Val Ser Gly Ala Arg Ala Ser
               20                  25                  30
   Pro Gly Ser Ala Ala Ser Pro Arg Leu Arg Glu Gly Pro Glu Leu Ser
           35                  40                  45
   Pro Asp Asp Pro Ala Gly Leu Leu Asp Leu Arg Gln Gly Met Phe Ala
       50                  55                  60
   Gln Leu Val Ala Gln Asn Val Leu Leu Ile Asp Gly Pro Leu Ser Trp
   65                  70                  75                  80
   Tyr Ser Asp Pro Gly Leu Ala Gly Val Ser Leu Thr Gly Gly Leu Ser
                   85                  90                  95
   Tyr Lys Glu Asp Thr Lys Glu Leu Val Val Ala Lys Ala Gly Val Tyr
               100                 105                 110
   Tyr Val Phe Phe Gln Leu Glu Leu Arg Arg Val Val Ala Gly Glu Gly
           115                 120                 125
   Ser Gly Ser Val Ser Leu Ala Leu His Leu Gln Pro Leu Arg Ser Ala
       130                 135                 140
   Ala Gly Ala Ala Ala Leu Ala Leu Thr Val Asp Leu Pro Pro Ala Ser
   145                 150                 155                 160
   Ser Glu Ala Arg Asn Ser Ala Phe Gly Phe Gln Gly Arg Leu Leu His
                   165                 170                 175
   Leu Ser Ala Gly Gln Arg Leu Gly Val His Leu His Thr Glu Ala Arg
               180                 185                 190
   Ala Arg His Ala Trp Gln Leu Thr Gln Gly Ala Thr Val Leu Gly Leu
           195                 200                 205
   Phe Arg Val Thr Pro Glu Ile Pro Ala Gly Leu Pro Ser Pro Arg Ser
       210                 215                 220
   Glu
   225
<210> 70
   <211> 939
   <212> PRT
   <213> Artificial Sequence
<220>
   <223> Synthetic polypeptide
<400> 70
   Met Ser Arg Ser Val Ala Leu Ala Val Leu Ala Leu Leu Ser Leu Ser
   1               5                   10                  15
   Gly Leu Glu Ala Val Tyr Leu Lys Thr Asn Val Phe Leu Gly Cys Gly
               20                  25                  30
   Ala Ser Gly Gly Gly Gly Ser Gly Gly Gly Gly Ser Met Ile Gln Lys
           35                  40                  45
   Thr Pro Gln Ile Gln Val Tyr Ser Arg His Pro Pro Glu Asn Gly Lys
       50                  55                  60
   Pro Asn Ile Leu Asn Cys Tyr Val Thr Gln Phe His Pro Pro His Ile
   65                  70                  75                  80
   Glu Ile Gln Met Leu Lys Asn Gly Lys Lys Ile Pro Lys Val Glu Met
                   85                  90                  95
   Ser Asp Met Ser Phe Ser Lys Asp Trp Ser Phe Tyr Ile Leu Ala His
               100                 105                 110
   Thr Glu Phe Thr Pro Thr Glu Thr Asp Thr Tyr Ala Cys Arg Val Lys
           115                 120                 125
   His Ala Ser Met Ala Glu Pro Lys Thr Val Tyr Trp Asp Arg Asp Met
       130                 135                 140
   Gly Gly Gly Gly Ser Gly Gly Gly Gly Ser Gly Gly Gly Gly Ser Gly
   145                 150                 155                 160
   Gly Gly Gly Ser Gly Gly Gly Gly Ser Ala Cys Pro Trp Ala Val Ser
                   165                 170                 175
   Gly Ala Arg Ala Ser Pro Gly Ser Ala Ala Ser Pro Arg Leu Arg Glu
               180                 185                 190
   Gly Pro Glu Leu Ser Pro Asp Asp Pro Ala Gly Leu Leu Asp Leu Arg
           195                 200                 205
   Gln Gly Met Phe Ala Gln Leu Val Ala Gln Asn Val Leu Leu Ile Asp
       210                 215                 220
   Gly Pro Leu Ser Trp Tyr Ser Asp Pro Gly Leu Ala Gly Val Ser Leu
   225                 230                 235                 240
   Thr Gly Gly Leu Ser Tyr Lys Glu Asp Thr Lys Glu Leu Val Val Ala
                   245                 250                 255
   Lys Ala Gly Val Tyr Tyr Val Phe Phe Gln Leu Glu Leu Arg Arg Val
               260                 265                 270
   Val Ala Gly Glu Gly Ser Gly Ser Val Ser Leu Ala Leu His Leu Gln
           275                 280                 285
   Pro Leu Arg Ser Ala Ala Gly Ala Ala Ala Leu Ala Leu Thr Val Asp
       290                 295                 300
   Leu Pro Pro Ala Ser Ser Glu Ala Arg Asn Ser Ala Phe Gly Phe Gln
   305                 310                 315                 320
   Gly Arg Leu Leu His Leu Ser Ala Gly Gln Arg Leu Gly Val His Leu
                   325                 330                 335
   His Thr Glu Ala Arg Ala Arg His Ala Trp Gln Leu Thr Gln Gly Ala
               340                 345                 350
   Thr Val Leu Gly Leu Phe Arg Val Thr Pro Glu Ile Pro Ala Gly Leu
           355                 360                 365
   Pro Ser Pro Arg Ser Glu Ser Gly Ser Gly Ala Thr Asn Phe Ser Leu
       370                 375                 380
   Leu Lys Gln Ala Gly Asp Val Glu Glu Asn Pro Gly Pro Met Ser Arg
   385                 390                 395                 400
   Ser Val Ala Leu Ala Val Leu Ala Leu Leu Ser Leu Ser Gly Leu Glu
                   405                 410                 415
   Ala Gly Pro His Ser Leu Arg Tyr Phe Val Thr Ala Val Ser Arg Pro
               420                 425                 430
   Gly Leu Gly Glu Pro Arg Phe Ile Ala Val Gly Tyr Val Asp Asp Thr
           435                 440                 445
   Gln Phe Val Arg Phe Asp Ser Asp Ala Asp Asn Pro Arg Phe Glu Pro
       450                 455                 460
   Arg Ala Pro Trp Met Glu Gln Glu Gly Pro Glu Tyr Trp Glu Glu Gln
   465                 470                 475                 480
   Thr Gln Arg Ala Lys Ser Asp Glu Gln Trp Phe Arg Val Ser Leu Arg
                   485                 490                 495
   Thr Ala Gln Arg Cys Tyr Asn Gln Ser Lys Gly Gly Ser His Thr Phe
               500                 505                 510
   Gln Arg Met Phe Gly Cys Asp Val Gly Ser Asp Trp Arg Leu Leu Arg
           515                 520                 525
   Gly Tyr Gln Gln Phe Ala Tyr Asp Gly Arg Asp Tyr Ile Ala Leu Asn
       530                 535                 540
   Glu Asp Leu Lys Thr Trp Thr Ala Ala Asp Thr Ala Ala Leu Ile Thr
   545 550 555 560
   Arg Arg Lys Trp Glu Gln Ala Gly Asp Ala Glu Tyr Tyr Arg Ala Tyr
   565 570 575
   Leu Glu Gly Glu Cys Val Glu Trp Leu Arg Arg Tyr Leu Glu Leu Gly
   580 585 590
   Asn Glu Thr Leu Leu Arg Thr Asp Ser Pro Lys Ala His Val Thr Tyr
   595 600 605
   His Pro Arg Ser Gln Val Asp Val Thr Leu Arg Cys Trp Ala Leu Gly
   610 615 620
   Phe Tyr Pro Ala Asp Ile Thr Leu Thr Trp Gln Leu Asn Gly Glu Asp
   625 630 635 640
   Leu Thr Gln Asp Met Glu Leu Val Glu Thr Arg Pro Ala Gly Asp Gly
   645 650 655
   Thr Phe Gln Lys Trp Ala Ala Val Val Val Pro Leu Gly Lys Glu Gln
   660 665 670
   Asn Tyr Thr Cys His Val His His Lys Gly Leu Pro Glu Pro Leu Thr
   675 680 685
   Leu Arg Trp Ala Ala Ala Gly Gly Pro Arg Gly Pro Thr Ile Lys Pro
   690 695 700
   Cys Pro Pro Cys Lys Cys Pro Ala Pro Asn Leu Leu Gly Gly Pro Ser
   705 710 715 720
   Val Phe Ile Phe Pro Pro Lys Ile Lys Asp Val Leu Met Ile Ser Leu
   725 730 735
   Ser Pro Ile Val Thr Cys Val Val Val Asp Val Ser Glu Asp Asp Pro
   740 745 750
   Asp Val Gln Ile Ser Trp Phe Val Asn Asn Val Glu Val His Thr Ala
   755 760 765
   Gln Thr Gln Thr His Arg Glu Asp Tyr Asn Ser Thr Leu Arg Val Val
   770 775 780
   Ser Ala Leu Pro Ile Gln His Gln Asp Trp Met Ser Gly Lys Glu Phe
   785 790 795 800
   Lys Cys Lys Val Asn Asn Lys Asp Leu Pro Ala Pro Ile Glu Arg Thr
                   805                 810                 815
   Ile Ser Lys Pro Lys Gly Ser Val Arg Ala Pro Gln Val Tyr Val Leu
               820                 825                 830
   Pro Pro Pro Glu Glu Glu Met Thr Lys Lys Gln Val Thr Leu Thr Cys
           835                 840                 845
   Met Val Thr Asp Phe Met Pro Glu Asp Ile Tyr Val Glu Trp Thr Asn
       850                 855                 860
   Asn Gly Lys Thr Glu Leu Asn Tyr Lys Asn Thr Glu Pro Val Leu Asp
   865                 870                 875                 880
   Ser Asp Gly Ser Tyr Phe Met Tyr Ser Lys Leu Arg Val Glu Lys Lys
                   885                 890                 895
   Asn Trp Val Glu Arg Asn Ser Tyr Ser Cys Ser Val Val His Glu Gly
               900                 905                 910
   Leu His Asn His His Thr Thr Lys Ser Phe Ser Arg Thr Pro Gly Lys
           915                 920                 925
   Gly Gly Ser His His His His His His His His
       930                 935
<210> 71
   <211> 939
   <212> PRT
   <213> Artificial Sequence
<220>
   <223> Synthetic polypeptide
<400> 71
   Met Ser Arg Ser Val Ala Leu Ala Val Leu Ala Leu Leu Ser Leu Ser
   1               5                   10                  15
   Gly Leu Glu Ala Val Tyr Leu Lys Thr Asn Val Phe Leu Gly Cys Gly
               20                  25                  30
   Ala Ser Gly Gly Gly Gly Ser Gly Gly Gly Gly Ser Met Ile Gln Lys
           35                  40                  45
   Thr Pro Gln Ile Gln Val Tyr Ser Arg His Pro Pro Glu Asn Gly Lys
       50                  55                  60
   Pro Asn Ile Leu Asn Cys Tyr Val Thr Gln Phe His Pro Pro His Ile
   65 70 75 80
   Glu Ile Gln Met Leu Lys Asn Gly Lys Lys Ile Pro Lys Val Glu Met
   85 90 95
   Ser Asp Met Ser Phe Ser Lys Asp Trp Ser Phe Tyr Ile Leu Ala His
   100 105 110
   Thr Glu Phe Thr Pro Thr Glu Thr Asp Thr Tyr Ala Cys Arg Val Lys
   115 120 125
   His Ala Ser Met Ala Glu Pro Lys Thr Val Tyr Trp Asp Arg Asp Met
   130 135 140
   Gly Gly Gly Gly Ser Gly Gly Gly Gly Ser Gly Gly Gly Gly Ser Gly
   145 150 155 160
   Gly Gly Gly Ser Gly Gly Gly Gly Ser Ala Cys Pro Trp Ala Val Ser
   165 170 175
   Gly Ala Arg Ala Ser Pro Gly Ser Ala Ala Ser Pro Arg Leu Arg Glu
   180 185 190
   Gly Pro Glu Leu Ser Pro Asp Asp Pro Ala Gly Leu Leu Asp Leu Arg
   195 200 205
   Gln Gly Met Phe Ala Cys Leu Val Ala Gln Asn Val Leu Leu Ile Asp
   210 215 220
   Gly Pro Leu Ser Trp Tyr Ser Asp Pro Gly Leu Ala Gly Val Ser Leu
   225 230 235 240
   Thr Gly Gly Leu Ser Tyr Lys Glu Asp Thr Lys Glu Leu Val Val Ala
   245 250 255
   Lys Ala Gly Val Tyr Tyr Val Phe Phe Gln Leu Glu Leu Arg Arg Val
   260 265 270
   Val Ala Gly Glu Gly Ser Gly Ser Val Ser Leu Ala Leu His Leu Gln
   275 280 285
   Pro Leu Arg Ser Ala Ala Gly Ala Ala Ala Leu Ala Leu Thr Val Asp
   290 295 300
   Leu Pro Pro Ala Ser Ser Glu Ala Arg Asn Ser Ala Phe Gly Phe Gln
   305 310 315 320
   Gly Arg Leu Leu His Leu Ser Ala Gly Gln Arg Leu Gly Val His Leu
   325 330 335
   His Thr Glu Ala Arg Ala Arg His Ala Trp Gln Leu Thr Gln Gly Ala
   340 345 350
   Thr Val Leu Gly Leu Phe Arg Val Thr Pro Glu Ile Cys Ala Gly Leu
   355 360 365
   Pro Ser Pro Arg Ser Glu Ser Gly Ser Gly Ala Thr Asn Phe Ser Leu
   370 375 380
   Leu Lys Gln Ala Gly Asp Val Glu Glu Asn Pro Gly Pro Met Ser Arg
   385 390 395 400
   Ser Val Ala Leu Ala Val Leu Ala Leu Leu Ser Leu Ser Gly Leu Glu
   405 410 415
   Ala Gly Pro His Ser Leu Arg Tyr Phe Val Thr Ala Val Ser Arg Pro
   420 425 430
   Gly Leu Gly Glu Pro Arg Phe Ile Ala Val Gly Tyr Val Asp Asp Thr
   435 440 445
   Gln Phe Val Arg Phe Asp Ser Asp Ala Asp Asn Pro Arg Phe Glu Pro
   450 455 460
   Arg Ala Pro Trp Met Glu Gln Glu Gly Pro Glu Tyr Trp Glu Glu Gln
   465 470 475 480
   Thr Gln Arg Ala Lys Ser Asp Glu Gln Trp Phe Arg Val Ser Leu Arg
   485 490 495
   Thr Ala Gln Arg Cys Tyr Asn Gln Ser Lys Gly Gly Ser His Thr Phe
   500 505 510
   Gln Arg Met Phe Gly Cys Asp Val Gly Ser Asp Trp Arg Leu Leu Arg
   515 520 525
   Gly Tyr Gln Gln Phe Ala Tyr Asp Gly Arg Asp Tyr Ile Ala Leu Asn
   530 535 540
   Glu Asp Leu Lys Thr Trp Thr Ala Ala Asp Thr Ala Ala Leu Ile Thr
   545 550 555 560
   Arg Arg Lys Trp Glu Gln Ala Gly Asp Ala Glu Tyr Tyr Arg Ala Tyr
   565 570 575
   Leu Glu Gly Glu Cys Val Glu Trp Leu Arg Arg Tyr Leu Glu Leu Gly
               580                 585                 590
   Asn Glu Thr Leu Leu Arg Thr Asp Ser Pro Lys Ala His Val Thr Tyr
           595                 600                 605
   His Pro Arg Ser Gln Val Asp Val Thr Leu Arg Cys Trp Ala Leu Gly
       610                 615                 620
   Phe Tyr Pro Ala Asp Ile Thr Leu Thr Trp Gln Leu Asn Gly Glu Asp
   625                 630                 635                 640
   Leu Thr Gln Asp Met Glu Leu Val Glu Thr Arg Pro Ala Gly Asp Gly
                   645                 650                 655
   Thr Phe Gln Lys Trp Ala Ala Val Val Val Pro Leu Gly Lys Glu Gln
               660                 665                 670
   Asn Tyr Thr Cys His Val His His Lys Gly Leu Pro Glu Pro Leu Thr
           675                 680                 685
   Leu Arg Trp Ala Ala Ala Gly Gly Pro Arg Gly Pro Thr Ile Lys Pro
       690                 695                 700
   Cys Pro Pro Cys Lys Cys Pro Ala Pro Asn Leu Leu Gly Gly Pro Ser
   705                 710                 715                 720
   Val Phe Ile Phe Pro Pro Lys Ile Lys Asp Val Leu Met Ile Ser Leu
                   725                 730                 735
   Ser Pro Ile Val Thr Cys Val Val Val Asp Val Ser Glu Asp Asp Pro
               740                 745                 750
   Asp Val Gln Ile Ser Trp Phe Val Asn Asn Val Glu Val His Thr Ala
           755                 760                 765
   Gln Thr Gln Thr His Arg Glu Asp Tyr Asn Ser Thr Leu Arg Val Val
       770                 775                 780
   Ser Ala Leu Pro Ile Gln His Gln Asp Trp Met Ser Gly Lys Glu Phe
   785                 790                 795                 800
   Lys Cys Lys Val Asn Asn Lys Asp Leu Pro Ala Pro Ile Glu Arg Thr
                   805                 810                 815
   Ile Ser Lys Pro Lys Gly Ser Val Arg Ala Pro Gln Val Tyr Val Leu
               820                 825                 830
   Pro Pro Pro Glu Glu Glu Met Thr Lys Lys Gln Val Thr Leu Thr Cys
   835 840 845
   Met Val Thr Asp Phe Met Pro Glu Asp Ile Tyr Val Glu Trp Thr Asn
   850 855 860
   Asn Gly Lys Thr Glu Leu Asn Tyr Lys Asn Thr Glu Pro Val Leu Asp
   865 870 875 880
   Ser Asp Gly Ser Tyr Phe Met Tyr Ser Lys Leu Arg Val Glu Lys Lys
   885 890 895
   Asn Trp Val Glu Arg Asn Ser Tyr Ser Cys Ser Val Val His Glu Gly
   900 905 910
   Leu His Asn His His Thr Thr Lys Ser Phe Ser Arg Thr Pro Gly Lys
   915 920 925
   Gly Gly Ser His His His His His His His His
   930 935
<210> 72
   <211> 225
   <212> PRT
   <213> Artificial Sequence
<220>
   <223> Synthetic polypeptide
<400> 72
   Met Ser Arg Ser Val Ala Leu Ala Val Leu Ala Leu Leu Ser Leu Ser
   1 5 10 15
   Gly Leu Glu Ala Ala Cys Pro Trp Ala Val Ser Gly Ala Arg Ala Ser
   20 25 30
   Pro Gly Ser Ala Ala Ser Pro Arg Leu Arg Glu Gly Pro Glu Leu Ser
   35 40 45
   Pro Asp Asp Pro Ala Gly Leu Leu Asp Leu Arg Gln Gly Met Phe Ala
   50 55 60
   Cys Leu Val Ala Gln Asn Val Leu Leu Ile Asp Gly Pro Leu Ser Trp
   65 70 75 80
   Tyr Ser Asp Pro Gly Leu Ala Gly Val Ser Leu Thr Gly Gly Leu Ser
   85 90 95
   Tyr Lys Glu Asp Thr Lys Glu Leu Val Val Ala Lys Ala Gly Val Tyr
               100                 105                 110
   Tyr Val Phe Phe Gln Leu Glu Leu Arg Arg Val Val Ala Gly Glu Gly
           115                 120                 125
   Ser Gly Ser Val Ser Leu Ala Leu His Leu Gln Pro Leu Arg Ser Ala
       130                 135                 140
   Ala Gly Ala Ala Ala Leu Ala Leu Thr Val Asp Leu Pro Pro Ala Ser
   145                 150                 155                 160
   Ser Glu Ala Arg Asn Ser Ala Phe Gly Phe Gln Gly Arg Leu Leu His
                   165                 170                 175
   Leu Ser Ala Gly Gln Arg Leu Gly Val His Leu His Thr Glu Ala Arg
               180                 185                 190
   Ala Arg His Ala Trp Gln Leu Thr Gln Gly Ala Thr Val Leu Gly Leu
           195                 200                 205
   Phe Arg Val Thr Pro Glu Ile Cys Ala Gly Leu Pro Ser Pro Arg Ser
       210                 215                 220
   Glu
   225
<210> 73
   <211> 939
   <212> PRT
   <213> Artificial Sequence
<220>
   <223> Synthetic polypeptide
<400> 73
   Met Ser Arg Ser Val Ala Leu Ala Val Leu Ala Leu Leu Ser Leu Ser
   1               5                   10                  15
   Gly Leu Glu Ala Val Tyr Leu Lys Thr Asn Val Phe Leu Gly Cys Gly
               20                  25                  30
   Ala Ser Gly Gly Gly Gly Ser Gly Gly Gly Gly Ser Met Ile Gln Lys
           35                  40                  45
   Thr Pro Gln Ile Gln Val Tyr Ser Arg His Pro Pro Glu Asn Gly Lys
       50                  55                  60
   Pro Asn Ile Leu Asn Cys Tyr Val Thr Gln Phe His Pro Pro His Ile
   65 70 75 80
   Glu Ile Gln Met Leu Lys Asn Gly Lys Lys Ile Pro Lys Val Glu Met
   85 90 95
   Ser Asp Met Ser Phe Ser Lys Asp Trp Ser Phe Tyr Ile Leu Ala His
   100 105 110
   Thr Glu Phe Thr Pro Thr Glu Thr Asp Thr Tyr Ala Cys Arg Val Lys
   115 120 125
   His Ala Ser Met Ala Glu Pro Lys Thr Val Tyr Trp Asp Arg Asp Met
   130 135 140
   Gly Gly Gly Gly Ser Gly Gly Gly Gly Ser Gly Gly Gly Gly Ser Gly
   145 150 155 160
   Gly Gly Gly Ser Gly Gly Gly Gly Ser Ala Cys Pro Trp Ala Val Ser
   165 170 175
   Gly Ala Arg Ala Ser Pro Gly Ser Ala Ala Ser Pro Arg Leu Arg Glu
   180 185 190
   Gly Pro Glu Leu Ser Pro Asp Asp Pro Ala Gly Leu Leu Asp Leu Arg
   195 200 205
   Gln Gly Met Phe Ala Cys Leu Val Ala Gln Asn Val Leu Leu Ile Asp
   210 215 220
   Gly Pro Leu Ser Trp Tyr Ser Asp Pro Gly Leu Ala Gly Val Ser Leu
   225 230 235 240
   Thr Gly Gly Leu Ser Tyr Lys Glu Asp Thr Lys Glu Leu Val Val Ala
   245 250 255
   Lys Ala Gly Val Tyr Tyr Val Phe Phe Gln Leu Glu Leu Arg Arg Val
   260 265 270
   Val Ala Gly Glu Gly Ser Gly Ser Val Ser Leu Ala Leu His Leu Gln
   275 280 285
   Pro Leu Arg Ser Ala Ala Gly Ala Ala Ala Leu Ala Leu Thr Val Asp
   290 295 300
   Leu Pro Pro Ala Ser Ser Glu Ala Arg Asn Ser Ala Phe Gly Phe Gln
   305 310 315 320
   Gly Arg Leu Leu His Leu Ser Ala Gly Gln Arg Leu Gly Val His Leu
                   325                 330                 335
   His Thr Glu Ala Arg Ala Arg His Ala Trp Gln Leu Thr Gln Gly Ala
               340                 345                 350
   Thr Val Leu Gly Leu Phe Arg Val Thr Cys Glu Ile Pro Ala Gly Leu
           355                 360                 365
   Pro Ser Pro Arg Ser Glu Ser Gly Ser Gly Ala Thr Asn Phe Ser Leu
       370                 375                 380
   Leu Lys Gln Ala Gly Asp Val Glu Glu Asn Pro Gly Pro Met Ser Arg
   385                 390                 395                 400
   Ser Val Ala Leu Ala Val Leu Ala Leu Leu Ser Leu Ser Gly Leu Glu
                   405                 410                 415
   Ala Gly Pro His Ser Leu Arg Tyr Phe Val Thr Ala Val Ser Arg Pro
               420                 425                 430
   Gly Leu Gly Glu Pro Arg Phe Ile Ala Val Gly Tyr Val Asp Asp Thr
           435                 440                 445
   Gln Phe Val Arg Phe Asp Ser Asp Ala Asp Asn Pro Arg Phe Glu Pro
       450                 455                 460
   Arg Ala Pro Trp Met Glu Gln Glu Gly Pro Glu Tyr Trp Glu Glu Gln
   465                 470                 475                 480
   Thr Gln Arg Ala Lys Ser Asp Glu Gln Trp Phe Arg Val Ser Leu Arg
                   485                 490                 495
   Thr Ala Gln Arg Cys Tyr Asn Gln Ser Lys Gly Gly Ser His Thr Phe
               500                 505                 510
   Gln Arg Met Phe Gly Cys Asp Val Gly Ser Asp Trp Arg Leu Leu Arg
           515                 520                 525
   Gly Tyr Gln Gln Phe Ala Tyr Asp Gly Arg Asp Tyr Ile Ala Leu Asn
       530                 535                 540
   Glu Asp Leu Lys Thr Trp Thr Ala Ala Asp Thr Ala Ala Leu Ile Thr
   545                 550                 555                 560
   Arg Arg Lys Trp Glu Gln Ala Gly Asp Ala Glu Tyr Tyr Arg Ala Tyr
                   565                 570                 575
   Leu Glu Gly Glu Cys Val Glu Trp Leu Arg Arg Tyr Leu Glu Leu Gly
   580 585 590
   Asn Glu Thr Leu Leu Arg Thr Asp Ser Pro Lys Ala His Val Thr Tyr
   595 600 605
   His Pro Arg Ser Gln Val Asp Val Thr Leu Arg Cys Trp Ala Leu Gly
   610 615 620
   Phe Tyr Pro Ala Asp Ile Thr Leu Thr Trp Gln Leu Asn Gly Glu Asp
   625 630 635 640
   Leu Thr Gln Asp Met Glu Leu Val Glu Thr Arg Pro Ala Gly Asp Gly
   645 650 655
   Thr Phe Gln Lys Trp Ala Ala Val Val Val Pro Leu Gly Lys Glu Gln
   660 665 670
   Asn Tyr Thr Cys His Val His His Lys Gly Leu Pro Glu Pro Leu Thr
   675 680 685
   Leu Arg Trp Ala Ala Ala Gly Gly Pro Arg Gly Pro Thr Ile Lys Pro
   690 695 700
   Cys Pro Pro Cys Lys Cys Pro Ala Pro Asn Leu Leu Gly Gly Pro Ser
   705 710 715 720
   Val Phe Ile Phe Pro Pro Lys Ile Lys Asp Val Leu Met Ile Ser Leu
   725 730 735
   Ser Pro Ile Val Thr Cys Val Val Val Asp Val Ser Glu Asp Asp Pro
   740 745 750
   Asp Val Gln Ile Ser Trp Phe Val Asn Asn Val Glu Val His Thr Ala
   755 760 765
   Gln Thr Gln Thr His Arg Glu Asp Tyr Asn Ser Thr Leu Arg Val Val
   770 775 780
   Ser Ala Leu Pro Ile Gln His Gln Asp Trp Met Ser Gly Lys Glu Phe
   785 790 795 800
   Lys Cys Lys Val Asn Asn Lys Asp Leu Pro Ala Pro Ile Glu Arg Thr
   805 810 815
   Ile Ser Lys Pro Lys Gly Ser Val Arg Ala Pro Gln Val Tyr Val Leu
                   820                 825                 830
   Pro Pro Pro Glu Glu Glu Met Thr Lys Lys Gln Val Thr Leu Thr Cys
           835                 840                 845
   Met Val Thr Asp Phe Met Pro Glu Asp Ile Tyr Val Glu Trp Thr Asn
       850                 855                 860
   Asn Gly Lys Thr Glu Leu Asn Tyr Lys Asn Thr Glu Pro Val Leu Asp
   865                 870                 875                 880
   Ser Asp Gly Ser Tyr Phe Met Tyr Ser Lys Leu Arg Val Glu Lys Lys
                   885                 890                 895
   Asn Trp Val Glu Arg Asn Ser Tyr Ser Cys Ser Val Val His Glu Gly
               900                 905                 910
   Leu His Asn His His Thr Thr Lys Ser Phe Ser Arg Thr Pro Gly Lys
           915                 920                 925
   Gly Gly Ser His His His His His His His His
       930                 935
<210> 74
   <211> 225
   <212> PRT
   <213> Artificial Sequence
<220>
   <223> Synthetic polypeptide
<400> 74
   Met Ser Arg Ser Val Ala Leu Ala Val Leu Ala Leu Leu Ser Leu Ser
   1               5                   10                  15
   Gly Leu Glu Ala Ala Cys Pro Trp Ala Val Ser Gly Ala Arg Ala Ser
               20                  25                  30
   Pro Gly Ser Ala Ala Ser Pro Arg Leu Arg Glu Gly Pro Glu Leu Ser
           35                  40                  45
   Pro Asp Asp Pro Ala Gly Leu Leu Asp Leu Arg Gln Gly Met Phe Ala
       50                  55                  60
   Cys Leu Val Ala Gln Asn Val Leu Leu Ile Asp Gly Pro Leu Ser Trp
   65                  70                  75                  80
   Tyr Ser Asp Pro Gly Leu Ala Gly Val Ser Leu Thr Gly Gly Leu Ser
                   85                  90                  95
   Tyr Lys Glu Asp Thr Lys Glu Leu Val Val Ala Lys Ala Gly Val Tyr
               100                 105                 110
   Tyr Val Phe Phe Gln Leu Glu Leu Arg Arg Val Val Ala Gly Glu Gly
           115                 120                 125
   Ser Gly Ser Val Ser Leu Ala Leu His Leu Gln Pro Leu Arg Ser Ala
       130                 135                 140
   Ala Gly Ala Ala Ala Leu Ala Leu Thr Val Asp Leu Pro Pro Ala Ser
   145                 150                 155                 160
   Ser Glu Ala Arg Asn Ser Ala Phe Gly Phe Gln Gly Arg Leu Leu His
                   165                 170                 175
   Leu Ser Ala Gly Gln Arg Leu Gly Val His Leu His Thr Glu Ala Arg
               180                 185                 190
   Ala Arg His Ala Trp Gln Leu Thr Gln Gly Ala Thr Val Leu Gly Leu
           195                 200                 205
   Phe Arg Val Thr Cys Glu Ile Pro Ala Gly Leu Pro Ser Pro Arg Ser
       210                 215                 220
   Glu
   225
<210> 75
   <211> 939
   <212> PRT
   <213> Artificial Sequence
<220>
   <223> Synthetic polypeptide
<400> 75
   Met Ser Arg Ser Val Ala Leu Ala Val Leu Ala Leu Leu Ser Leu Ser
   1               5                   10                  15
   Gly Leu Glu Ala Val Tyr Leu Lys Thr Asn Val Phe Leu Gly Cys Gly
               20                  25                  30
   Ala Ser Gly Gly Gly Gly Ser Gly Gly Gly Gly Ser Met Ile Gln Lys
           35                  40                  45
   Thr Pro Gln Ile Gln Val Tyr Ser Arg His Pro Pro Glu Asn Gly Lys
       50                  55                  60
   Pro Asn Ile Leu Asn Cys Tyr Val Thr Gln Phe His Pro Pro His Ile
   65 70 75 80
   Glu Ile Gln Met Leu Lys Asn Gly Lys Lys Ile Pro Lys Val Glu Met
   85 90 95
   Ser Asp Met Ser Phe Ser Lys Asp Trp Ser Phe Tyr Ile Leu Ala His
   100 105 110
   Thr Glu Phe Thr Pro Thr Glu Thr Asp Thr Tyr Ala Cys Arg Val Lys
   115 120 125
   His Ala Ser Met Ala Glu Pro Lys Thr Val Tyr Trp Asp Arg Asp Met
   130 135 140
   Gly Gly Gly Gly Ser Gly Gly Gly Gly Ser Gly Gly Gly Gly Ser Gly
   145 150 155 160
   Gly Gly Gly Ser Gly Gly Gly Gly Ser Ala Cys Pro Trp Ala Val Ser
   165 170 175
   Gly Ala Arg Ala Ser Pro Gly Ser Ala Ala Ser Pro Arg Leu Arg Glu
   180 185 190
   Gly Pro Glu Leu Ser Pro Asp Asp Pro Ala Gly Leu Leu Asp Leu Arg
   195 200 205
   Cys Gly Met Phe Ala Gln Leu Val Ala Gln Asn Val Leu Leu Ile Asp
   210 215 220
   Gly Pro Leu Ser Trp Tyr Ser Asp Pro Gly Cys Ala Gly Val Ser Leu
   225 230 235 240
   Thr Gly Gly Leu Ser Tyr Lys Glu Asp Thr Lys Glu Leu Val Val Ala
   245 250 255
   Lys Ala Gly Val Tyr Tyr Val Phe Phe Gln Leu Glu Leu Arg Arg Val
   260 265 270
   Val Ala Gly Glu Gly Ser Gly Ser Val Ser Leu Ala Leu His Leu Gln
   275 280 285
   Pro Leu Arg Ser Ala Ala Gly Ala Ala Ala Leu Ala Leu Thr Val Asp
   290 295 300
   Leu Pro Pro Ala Ser Ser Glu Ala Arg Asn Ser Ala Phe Gly Phe Gln
   305 310 315 320
   Gly Arg Leu Leu His Leu Ser Ala Gly Gln Arg Leu Gly Val His Leu
   325 330 335
   His Thr Glu Ala Arg Ala Arg His Ala Trp Gln Leu Thr Gln Gly Ala
   340 345 350
   Thr Val Leu Gly Leu Phe Arg Val Thr Pro Glu Ile Cys Ala Gly Leu
   355 360 365
   Pro Ser Pro Arg Ser Glu Ser Gly Ser Gly Ala Thr Asn Phe Ser Leu
   370 375 380
   Leu Lys Gln Ala Gly Asp Val Glu Glu Asn Pro Gly Pro Met Ser Arg
   385 390 395 400
   Ser Val Ala Leu Ala Val Leu Ala Leu Leu Ser Leu Ser Gly Leu Glu
   405 410 415
   Ala Gly Pro His Ser Leu Arg Tyr Phe Val Thr Ala Val Ser Arg Pro
   420 425 430
   Gly Leu Gly Glu Pro Arg Phe Ile Ala Val Gly Tyr Val Asp Asp Thr
   435 440 445
   Gln Phe Val Arg Phe Asp Ser Asp Ala Asp Asn Pro Arg Phe Glu Pro
   450 455 460
   Arg Ala Pro Trp Met Glu Gln Glu Gly Pro Glu Tyr Trp Glu Glu Gln
   465 470 475 480
   Thr Gln Arg Ala Lys Ser Asp Glu Gln Trp Phe Arg Val Ser Leu Arg
   485 490 495
   Thr Ala Gln Arg Cys Tyr Asn Gln Ser Lys Gly Gly Ser His Thr Phe
   500 505 510
   Gln Arg Met Phe Gly Cys Asp Val Gly Ser Asp Trp Arg Leu Leu Arg
   515 520 525
   Gly Tyr Gln Gln Phe Ala Tyr Asp Gly Arg Asp Tyr Ile Ala Leu Asn
   530 535 540
   Glu Asp Leu Lys Thr Trp Thr Ala Ala Asp Thr Ala Ala Leu Ile Thr
   545 550 555 560
   Arg Arg Lys Trp Glu Gln Ala Gly Asp Ala Glu Tyr Tyr Arg Ala Tyr
   565 570 575
   Leu Glu Gly Glu Cys Val Glu Trp Leu Arg Arg Tyr Leu Glu Leu Gly
   580 585 590
   Asn Glu Thr Leu Leu Arg Thr Asp Ser Pro Lys Ala His Val Thr Tyr
   595 600 605
   His Pro Arg Ser Gln Val Asp Val Thr Leu Arg Cys Trp Ala Leu Gly
   610 615 620
   Phe Tyr Pro Ala Asp Ile Thr Leu Thr Trp Gln Leu Asn Gly Glu Asp
   625 630 635 640
   Leu Thr Gln Asp Met Glu Leu Val Glu Thr Arg Pro Ala Gly Asp Gly
   645 650 655
   Thr Phe Gln Lys Trp Ala Ala Val Val Val Pro Leu Gly Lys Glu Gln
   660 665 670
   Asn Tyr Thr Cys His Val His His Lys Gly Leu Pro Glu Pro Leu Thr
   675 680 685
   Leu Arg Trp Ala Ala Ala Gly Gly Pro Arg Gly Pro Thr Ile Lys Pro
   690 695 700
   Cys Pro Pro Cys Lys Cys Pro Ala Pro Asn Leu Leu Gly Gly Pro Ser
   705 710 715 720
   Val Phe Ile Phe Pro Pro Lys Ile Lys Asp Val Leu Met Ile Ser Leu
   725 730 735
   Ser Pro Ile Val Thr Cys Val Val Val Asp Val Ser Glu Asp Asp Pro
   740 745 750
   Asp Val Gln Ile Ser Trp Phe Val Asn Asn Val Glu Val His Thr Ala
   755 760 765
   Gln Thr Gln Thr His Arg Glu Asp Tyr Asn Ser Thr Leu Arg Val Val
   770 775 780
   Ser Ala Leu Pro Ile Gln His Gln Asp Trp Met Ser Gly Lys Glu Phe
   785 790 795 800
   Lys Cys Lys Val Asn Asn Lys Asp Leu Pro Ala Pro Ile Glu Arg Thr
   805 810 815
   Ile Ser Lys Pro Lys Gly Ser Val Arg Ala Pro Gln Val Tyr Val Leu
               820                 825                 830
   Pro Pro Pro Glu Glu Glu Met Thr Lys Lys Gln Val Thr Leu Thr Cys
           835                 840                 845
   Met Val Thr Asp Phe Met Pro Glu Asp Ile Tyr Val Glu Trp Thr Asn
       850                 855                 860
   Asn Gly Lys Thr Glu Leu Asn Tyr Lys Asn Thr Glu Pro Val Leu Asp
   865                 870                 875                 880
   Ser Asp Gly Ser Tyr Phe Met Tyr Ser Lys Leu Arg Val Glu Lys Lys
                   885                 890                 895
   Asn Trp Val Glu Arg Asn Ser Tyr Ser Cys Ser Val Val His Glu Gly
               900                 905                 910
   Leu His Asn His His Thr Thr Lys Ser Phe Ser Arg Thr Pro Gly Lys
           915                 920                 925
   Gly Gly Ser His His His His His His His His
       930                 935
<210> 76
   <211> 225
   <212> PRT
   <213> Artificial Sequence
<220>
   <223> Synthetic polypeptide
<400> 76
   Met Ser Arg Ser Val Ala Leu Ala Val Leu Ala Leu Leu Ser Leu Ser
   1               5                   10                  15
   Gly Leu Glu Ala Ala Cys Pro Trp Ala Val Ser Gly Ala Arg Ala Ser
               20                  25                  30
   Pro Gly Ser Ala Ala Ser Pro Arg Leu Arg Glu Gly Pro Glu Leu Ser
           35                  40                  45
   Pro Asp Asp Pro Ala Gly Leu Leu Asp Leu Arg Cys Gly Met Phe Ala
       50                  55                  60
   Gln Leu Val Ala Gln Asn Val Leu Leu Ile Asp Gly Pro Leu Ser Trp
   65                  70                  75                  80
   Tyr Ser Asp Pro Gly Cys Ala Gly Val Ser Leu Thr Gly Gly Leu Ser
                   85                  90                  95
   Tyr Lys Glu Asp Thr Lys Glu Leu Val Val Ala Lys Ala Gly Val Tyr
               100                 105                 110
   Tyr Val Phe Phe Gln Leu Glu Leu Arg Arg Val Val Ala Gly Glu Gly
           115                 120                 125
   Ser Gly Ser Val Ser Leu Ala Leu His Leu Gln Pro Leu Arg Ser Ala
       130                 135                 140
   Ala Gly Ala Ala Ala Leu Ala Leu Thr Val Asp Leu Pro Pro Ala Ser
   145                 150                 155                 160
   Ser Glu Ala Arg Asn Ser Ala Phe Gly Phe Gln Gly Arg Leu Leu His
                   165                 170                 175
   Leu Ser Ala Gly Gln Arg Leu Gly Val His Leu His Thr Glu Ala Arg
               180                 185                 190
   Ala Arg His Ala Trp Gln Leu Thr Gln Gly Ala Thr Val Leu Gly Leu
           195                 200                 205
   Phe Arg Val Thr Pro Glu Ile Cys Ala Gly Leu Pro Ser Pro Arg Ser
       210                 215                 220
   Glu
   225
<210> 77
   <211> 1285
   <212> PRT
   <213> Artificial Sequence
<220>
   <223> Synthetic polypeptide
<400> 77
   Met Ser Arg Ser Val Ala Leu Ala Val Leu Ala Leu Leu Ser Leu Ser
   1               5                   10                  15
   Gly Leu Glu Ala Val Tyr Leu Lys Thr Asn Val Phe Leu Gly Cys Gly
               20                  25                  30
   Ala Ser Gly Gly Gly Gly Ser Gly Gly Gly Gly Ser Met Ile Gln Lys
           35                  40                  45
   Thr Pro Gln Ile Gln Val Tyr Ser Arg His Pro Pro Glu Asn Gly Lys
       50                  55                  60
   Pro Asn Ile Leu Asn Cys Tyr Val Thr Gln Phe His Pro Pro His Ile
   65 70 75 80
   Glu Ile Gln Met Leu Lys Asn Gly Lys Lys Ile Pro Lys Val Glu Met
   85 90 95
   Ser Asp Met Ser Phe Ser Lys Asp Trp Ser Phe Tyr Ile Leu Ala His
   100 105 110
   Thr Glu Phe Thr Pro Thr Glu Thr Asp Thr Tyr Ala Cys Arg Val Lys
   115 120 125
   His Ala Ser Met Ala Glu Pro Lys Thr Val Tyr Trp Asp Arg Asp Met
   130 135 140
   Gly Gly Gly Gly Ser Gly Gly Gly Gly Ser Gly Gly Gly Gly Ser Gly
   145 150 155 160
   Gly Gly Gly Ser Gly Gly Gly Gly Ser Asp Pro Ala Gly Leu Leu Asp
   165 170 175
   Leu Arg Gln Gly Met Phe Ala Gln Leu Val Ala Gln Asn Val Leu Leu
   180 185 190
   Ile Asp Gly Pro Leu Ser Trp Tyr Ser Asp Pro Gly Leu Ala Gly Val
   195 200 205
   Ser Leu Thr Gly Gly Leu Ser Tyr Lys Glu Asp Thr Lys Glu Leu Val
   210 215 220
   Val Ala Lys Ala Gly Val Tyr Tyr Val Phe Phe Gln Leu Glu Leu Arg
   225 230 235 240
   Arg Val Val Ala Gly Glu Gly Ser Gly Ser Val Ser Leu Ala Leu His
   245 250 255
   Leu Gln Pro Leu Arg Ser Ala Ala Gly Ala Ala Ala Leu Ala Leu Thr
   260 265 270
   Val Asp Leu Pro Pro Ala Ser Ser Glu Ala Arg Asn Ser Ala Phe Gly
   275 280 285
   Phe Gln Gly Arg Leu Leu His Leu Ser Ala Gly Gln Arg Leu Gly Val
   290 295 300
   His Leu His Thr Glu Ala Arg Ala Arg His Ala Trp Gln Leu Thr Gln
   305 310 315 320
   Gly Ala Thr Val Leu Gly Leu Phe Arg Val Thr Pro Glu Ile Pro Ala
   325 330 335
   Gly Gly Gly Gly Ser Gly Gly Gly Gly Ser Gly Gly Gly Gly Ser Gly
   340 345 350
   Gly Gly Gly Ser Gly Gly Gly Gly Ser Asp Pro Ala Gly Leu Leu Asp
   355 360 365
   Leu Arg Gln Gly Met Phe Ala Gln Leu Val Ala Gln Asn Val Leu Leu
   370 375 380
   Ile Asp Gly Pro Leu Ser Trp Tyr Ser Asp Pro Gly Leu Ala Gly Val
   385 390 395 400
   Ser Leu Thr Gly Gly Leu Ser Tyr Lys Glu Asp Thr Lys Glu Leu Val
   405 410 415
   Val Ala Lys Ala Gly Val Tyr Tyr Val Phe Phe Gln Leu Glu Leu Arg
   420 425 430
   Arg Val Val Ala Gly Glu Gly Ser Gly Ser Val Ser Leu Ala Leu His
   435 440 445
   Leu Gln Pro Leu Arg Ser Ala Ala Gly Ala Ala Ala Leu Ala Leu Thr
   450 455 460
   Val Asp Leu Pro Pro Ala Ser Ser Glu Ala Arg Asn Ser Ala Phe Gly
   465 470 475 480
   Phe Gln Gly Arg Leu Leu His Leu Ser Ala Gly Gln Arg Leu Gly Val
   485 490 495
   His Leu His Thr Glu Ala Arg Ala Arg His Ala Trp Gln Leu Thr Gln
   500 505 510
   Gly Ala Thr Val Leu Gly Leu Phe Arg Val Thr Pro Glu Ile Pro Ala
   515 520 525
   Gly Gly Gly Gly Ser Gly Gly Gly Gly Ser Gly Gly Gly Gly Ser Gly
   530 535 540
   Gly Gly Gly Ser Gly Gly Gly Gly Ser Asp Pro Ala Gly Leu Leu Asp
   545 550 555 560
   Leu Arg Gln Gly Met Phe Ala Gln Leu Val Ala Gln Asn Val Leu Leu
                   565                 570                 575
   Ile Asp Gly Pro Leu Ser Trp Tyr Ser Asp Pro Gly Leu Ala Gly Val
               580                 585                 590
   Ser Leu Thr Gly Gly Leu Ser Tyr Lys Glu Asp Thr Lys Glu Leu Val
           595                 600                 605
   Val Ala Lys Ala Gly Val Tyr Tyr Val Phe Phe Gln Leu Glu Leu Arg
       610                 615                 620
   Arg Val Val Ala Gly Glu Gly Ser Gly Ser Val Ser Leu Ala Leu His
   625                 630                 635                 640
   Leu Gln Pro Leu Arg Ser Ala Ala Gly Ala Ala Ala Leu Ala Leu Thr
                   645                 650                 655
   Val Asp Leu Pro Pro Ala Ser Ser Glu Ala Arg Asn Ser Ala Phe Gly
               660                 665                 670
   Phe Gln Gly Arg Leu Leu His Leu Ser Ala Gly Gln Arg Leu Gly Val
           675                 680                 685
   His Leu His Thr Glu Ala Arg Ala Arg His Ala Trp Gln Leu Thr Gln
       690                 695                 700
   Gly Ala Thr Val Leu Gly Leu Phe Arg Val Thr Pro Glu Ile Pro Ala
   705                 710                 715                 720
   Ser Gly Ser Gly Ala Thr Asn Phe Ser Leu Leu Lys Gln Ala Gly Asp
                   725                 730                 735
   Val Glu Glu Asn Pro Gly Pro Met Ser Arg Ser Val Ala Leu Ala Val
               740                 745                 750
   Leu Ala Leu Leu Ser Leu Ser Gly Leu Glu Ala Gly Pro His Ser Leu
           755                 760                 765
   Arg Tyr Phe Val Thr Ala Val Ser Arg Pro Gly Leu Gly Glu Pro Arg
       770                 775                 780
   Phe Ile Ala Val Gly Tyr Val Asp Asp Thr Gln Phe Val Arg Phe Asp
   785                 790                 795                 800
   Ser Asp Ala Asp Asn Pro Arg Phe Glu Pro Arg Ala Pro Trp Met Glu
                   805                 810                 815
   Gln Glu Gly Pro Glu Tyr Trp Glu Glu Gln Thr Gln Arg Ala Lys Ser
   820 825 830
   Asp Glu Gln Trp Phe Arg Val Ser Leu Arg Thr Ala Gln Arg Cys Tyr
   835 840 845
   Asn Gln Ser Lys Gly Gly Ser His Thr Phe Gln Arg Met Phe Gly Cys
   850 855 860
   Asp Val Gly Ser Asp Trp Arg Leu Leu Arg Gly Tyr Gln Gln Phe Ala
   865 870 875 880
   Tyr Asp Gly Arg Asp Tyr Ile Ala Leu Asn Glu Asp Leu Lys Thr Trp
   885 890 895
   Thr Ala Ala Asp Thr Ala Ala Leu Ile Thr Arg Arg Lys Trp Glu Gln
   900 905 910
   Ala Gly Asp Ala Glu Tyr Tyr Arg Ala Tyr Leu Glu Gly Glu Cys Val
   915 920 925
   Glu Trp Leu Arg Arg Tyr Leu Glu Leu Gly Asn Glu Thr Leu Leu Arg
   930 935 940
   Thr Asp Ser Pro Lys Ala His Val Thr Tyr His Pro Arg Ser Gln Val
   945 950 955 960
   Asp Val Thr Leu Arg Cys Trp Ala Leu Gly Phe Tyr Pro Ala Asp Ile
   965 970 975
   Thr Leu Thr Trp Gln Leu Asn Gly Glu Asp Leu Thr Gln Asp Met Glu
   980 985 990
   Leu Val Glu Thr Arg Pro Ala Gly Asp Gly Thr Phe Gln Lys Trp Ala
   995 1000 1005
   Ala Val Val Val Pro Leu Gly Lys Glu Gln Asn Tyr Thr Cys His
   1010 1015 1020
   Val His His Lys Gly Leu Pro Glu Pro Leu Thr Leu Arg Trp Ala
   1025 1030 1035
   Ala Ala Gly Gly Pro Arg Gly Pro Thr Ile Lys Pro Cys Pro Pro
   1040 1045 1050
   Cys Lys Cys Pro Ala Pro Asn Leu Leu Gly Gly Pro Ser Val Phe
   1055 1060 1065
   Ile Phe Pro Pro Lys Ile Lys Asp Val Leu Met Ile Ser Leu Ser
   1070 1075 1080
   Pro Ile Val Thr Cys Val Val Val Asp Val Ser Glu Asp Asp Pro
   1085 1090 1095
   Asp Val Gln Ile Ser Trp Phe Val Asn Asn Val Glu Val His Thr
   1100 1105 1110
   Ala Gln Thr Gln Thr His Arg Glu Asp Tyr Asn Ser Thr Leu Arg
   1115 1120 1125
   Val Val Ser Ala Leu Pro Ile Gln His Gln Asp Trp Met Ser Gly
   1130 1135 1140
   Lys Glu Phe Lys Cys Lys Val Asn Asn Lys Asp Leu Pro Ala Pro
   1145 1150 1155
   Ile Glu Arg Thr Ile Ser Lys Pro Lys Gly Ser Val Arg Ala Pro
   1160 1165 1170
   Gln Val Tyr Val Leu Pro Pro Pro Glu Glu Glu Met Thr Lys Lys
   1175 1180 1185
   Gln Val Thr Leu Thr Cys Met Val Thr Asp Phe Met Pro Glu Asp
   1190 1195 1200
   Ile Tyr Val Glu Trp Thr Asn Asn Gly Lys Thr Glu Leu Asn Tyr
   1205 1210 1215
   Lys Asn Thr Glu Pro Val Leu Asp Ser Asp Gly Ser Tyr Phe Met
   1220 1225 1230
   Tyr Ser Lys Leu Arg Val Glu Lys Lys Asn Trp Val Glu Arg Asn
   1235 1240 1245
   Ser Tyr Ser Cys Ser Val Val His Glu Gly Leu His Asn His His
   1250 1255 1260
   Thr Thr Lys Ser Phe Ser Arg Thr Pro Gly Lys Gly Gly Ser His
   1265 1270 1275
   His His His His His His His
   1280 1285
<210> 78
   <211> 119
   <212> PRT
   <213> Homo sapiens
<400> 78
   Met Ser Arg Ser Val Ala Leu Ala Val Leu Ala Leu Leu Ser Leu Ser
   1               5                   10                  15
   Gly Leu Glu Ala Ile Gln Arg Thr Pro Lys Ile Gln Val Tyr Ser Arg
               20                  25                  30
   His Pro Ala Glu Asn Gly Lys Ser Asn Phe Leu Asn Cys Tyr Val Ser
           35                  40                  45
   Gly Phe His Pro Ser Asp Ile Glu Val Asp Leu Leu Lys Asn Gly Glu
       50                  55                  60
   Arg Ile Glu Lys Val Glu His Ser Asp Leu Ser Phe Ser Lys Asp Trp
   65                  70                  75                  80
   Ser Phe Tyr Leu Leu Tyr Tyr Thr Glu Phe Thr Pro Thr Glu Lys Asp
                   85                  90                  95
   Glu Tyr Ala Cys Arg Val Asn His Val Thr Leu Ser Gln Pro Lys Ile
               100                 105                 110
   Val Lys Trp Asp Arg Asp Met
           115
<210> 79
   <211> 119
   <212> PRT
   <213> Pan troglodytes
<400> 79
   Met Ser Arg Ser Val Ala Leu Ala Val Leu Ala Leu Leu Ser Leu Ser
   1               5                   10                  15
   Gly Leu Glu Ala Ile Gln Arg Thr Pro Lys Ile Gln Val Tyr Ser Arg
               20                  25                  30
   His Pro Ala Glu Asn Gly Lys Ser Asn Phe Leu Asn Cys Tyr Val Ser
           35                  40                  45
   Gly Phe His Pro Ser Asp Ile Glu Val Asp Leu Leu Lys Asn Gly Glu
       50                  55                  60
   Arg Ile Glu Lys Val Glu His Ser Asp Leu Ser Phe Ser Lys Asp Trp
   65                  70                  75                  80
   Ser Phe Tyr Leu Leu Tyr Tyr Thr Glu Phe Thr Pro Thr Glu Lys Asp
                   85                  90                  95
   Glu Tyr Ala Cys Arg Val Asn His Val Thr Leu Ser Gln Pro Lys Ile
               100                 105                 110
   Val Lys Trp Asp Arg Asp Met
           115
<210> 80
   <211> 119
   <212> PRT
   <213> Macaca mulatta
<400> 80
   Met Ser Arg Ser Val Ala Leu Ala Val Leu Ala Leu Leu Ser Leu Ser
   1               5                   10                  15
   Gly Leu Glu Ala Ile Gln Arg Thr Pro Lys Ile Gln Val Tyr Ser Arg
               20                  25                  30
   His Pro Pro Glu Asn Gly Lys Pro Asn Phe Leu Asn Cys Tyr Val Ser
           35                  40                  45
   Gly Phe His Pro Ser Asp Ile Glu Val Asp Leu Leu Lys Asn Gly Glu
       50                  55                  60
   Lys Met Gly Lys Val Glu His Ser Asp Leu Ser Phe Ser Lys Asp Trp
   65                  70                  75                  80
   Ser Phe Tyr Leu Leu Tyr Tyr Thr Glu Phe Thr Pro Asn Glu Lys Asp
                   85                  90                  95
   Glu Tyr Ala Cys Arg Val Asn His Val Thr Leu Ser Gly Pro Arg Thr
               100                 105                 110
   Val Lys Trp Asp Arg Asp Met
           115
<210> 81
   <211> 118
   <212> PRT
   <213> Bos taurus
<400> 81
   Met Ala Arg Phe Val Ala Leu Val Leu Leu Gly Leu Leu Ser Leu Ser
   1               5                   10                  15
   Gly Leu Asp Ala Ile Gln Arg Pro Pro Lys Ile Gln Val Tyr Ser Arg
               20                  25                  30
   His Pro Pro Glu Asp Gly Lys Pro Asn Tyr Leu Asn Cys Tyr Val Tyr
           35                  40                  45
   Gly Phe His Pro Pro Gln Ile Glu Ile Asp Leu Leu Lys Asn Gly Glu
       50                  55                  60
   Lys Ile Lys Ser Glu Gln Ser Asp Leu Ser Phe Ser Lys Asp Trp Ser
   65                  70                  75                  80
   Phe Tyr Leu Leu Ser His Ala Glu Phe Thr Pro Asn Ser Lys Asp Gln
                   85                  90                  95
   Tyr Ser Cys Arg Val Lys His Val Thr Leu Glu Gln Pro Arg Ile Val
               100                 105                 110
   Lys Trp Asp Arg Asp Leu
           115
<210> 82
   <211> 119
   <212> PRT
   <213> Mus musculus
<400> 82
   Met Ala Arg Ser Val Thr Leu Val Phe Leu Val Leu Val Ser Leu Thr
   1               5                   10                  15
   Gly Leu Tyr Ala Ile Gln Lys Thr Pro Gln Ile Gln Val Tyr Ser Arg
               20                  25                  30
   His Pro Pro Glu Asn Gly Lys Pro Asn Ile Leu Asn Cys Tyr Val Thr
           35                  40                  45
   Gln Phe His Pro Pro His Ile Glu Ile Gln Met Leu Lys Asn Gly Lys
       50                  55                  60
   Lys Ile Pro Lys Val Glu Met Ser Asp Met Ser Phe Ser Lys Asp Trp
   65                  70                  75                  80
   Ser Phe Tyr Ile Leu Ala His Thr Glu Phe Thr Pro Thr Glu Thr Asp
                   85                  90                  95
   Thr Tyr Ala Cys Arg Val Lys His Ala Ser Met Ala Glu Pro Lys Thr
               100                 105                 110
   Val Tyr Trp Asp Arg Asp Met
   115
<210> 83
   <211> 227
   <212> PRT
   <213> Homo sapiens
<400> 83
   Asp Lys Thr His Thr Cys Pro Pro Cys Pro Ala Pro Glu Leu Leu Gly
   1               5                   10                  15
   Gly Pro Ser Val Phe Leu Phe Pro Pro Lys Pro Lys Asp Thr Leu Met
               20                  25                  30
   Ile Ser Arg Thr Pro Glu Val Thr Cys Val Val Val Asp Val Ser His
           35                  40                  45
   Glu Asp Pro Glu Val Lys Phe Asn Trp Tyr Val Asp Gly Val Glu Val
       50                  55                  60
   His Asn Ala Lys Thr Lys Pro Arg Glu Glu Gln Tyr Asn Ser Thr Tyr
   65                  70                  75                  80
   Arg Val Val Ser Val Leu Thr Val Leu His Gln Asp Trp Leu Asn Gly
                   85                  90                  95
   Lys Glu Tyr Lys Cys Lys Val Ser Asn Lys Ala Leu Pro Ala Pro Ile
               100                 105                 110
   Glu Lys Thr Ile Ser Lys Ala Lys Gly Gln Pro Arg Glu Pro Gln Val
           115                 120                 125
   Tyr Thr Leu Pro Pro Ser Arg Asp Glu Leu Thr Lys Asn Gln Val Ser
       130                 135                 140
   Leu Thr Cys Leu Val Lys Gly Phe Tyr Pro Ser Asp Ile Ala Val Glu
   145                 150                 155                 160
   Trp Glu Ser Asn Gly Gln Pro Glu Asn Asn Tyr Lys Thr Thr Pro Pro
                   165                 170                 175
   Val Leu Asp Ser Asp Gly Ser Phe Phe Leu Tyr Ser Lys Leu Thr Val
               180                 185                 190
   Asp Lys Ser Arg Trp Gln Gln Gly Asn Val Phe Ser Cys Ser Val Met
           195                 200                 205
   His Glu Ala Leu His Asn His Tyr Thr Gln Lys Ser Leu Ser Leu Ser
   210 215 220
   Pro Gly Lys
   225
<210> 84
   <211> 325
   <212> PRT
   <213> Homo sapiens
<400> 84
   Ser Thr Lys Gly Pro Ser Val Phe Pro Leu Ala Pro Cys Ser Arg Ser
   1 5 10 15
   Thr Ser Glu Ser Thr Ala Ala Leu Gly Cys Leu Val Lys Asp Tyr Phe
   20 25 30
   Pro Glu Pro Val Thr Val Ser Trp Asn Ser Gly Ala Leu Thr Ser Gly
   35 40 45
   Val His Thr Phe Pro Ala Val Leu Gln Ser Ser Gly Leu Tyr Ser Leu
   50 55 60
   Ser Ser Val Val Thr Val Pro Ser Ser Asn Phe Gly Thr Gln Thr Tyr
   65 70 75 80
   Thr Cys Asn Val Asp His Lys Pro Ser Asn Thr Lys Val Asp Lys Thr
   85 90 95
   Val Glu Arg Lys Cys Cys Val Glu Cys Pro Pro Cys Pro Ala Pro Pro
   100 105 110
   Val Ala Gly Pro Ser Val Phe Leu Phe Pro Pro Lys Pro Lys Asp Thr
   115 120 125
   Leu Met Ile Ser Arg Thr Pro Glu Val Thr Cys Val Val Val Asp Val
   130 135 140
   Ser His Glu Asp Pro Glu Val Gln Phe Asn Trp Tyr Val Asp Gly Val
   145 150 155 160
   Glu Val His Asn Ala Lys Thr Lys Pro Arg Glu Glu Gln Phe Asn Ser
   165 170 175
   Thr Phe Arg Val Val Ser Val Leu Thr Val Val His Gln Asp Trp Leu
   180 185 190
   Asn Gly Lys Glu Tyr Lys Cys Lys Val Ser Asn Lys Gly Leu Pro Ala
   195 200 205
   Pro Ile Glu Lys Thr Ile Ser Lys Thr Lys Gly Gln Pro Arg Glu Pro
   210 215 220
   Gln Val Tyr Thr Leu Pro Pro Ser Arg Glu Glu Met Thr Lys Asn Gln
   225 230 235 240
   Val Ser Leu Thr Cys Leu Val Lys Gly Phe Tyr Pro Ser Asp Ile Ala
   245 250 255
   Val Glu Trp Glu Ser Asn Gly Gln Pro Glu Asn Asn Tyr Lys Thr Thr
   260 265 270
   Pro Pro Met Leu Asp Ser Asp Gly Ser Phe Phe Leu Tyr Ser Lys Leu
   275 280 285
   Thr Val Asp Lys Ser Arg Trp Gln Gln Gly Asn Val Phe Ser Cys Ser
   290 295 300
   Val Met His Glu Ala Leu His Asn His Tyr Thr Gln Lys Ser Leu Ser
   305 310 315 320
   Leu Ser Pro Gly Lys
   325
<210> 85
   <211> 246
   <212> PRT
   <213> Homo sapiens
<400> 85
   His Lys Pro Ser Asn Thr Lys Val Asp Lys Arg Val Glu Leu Lys Thr
   1 5 10 15
   Pro Leu Gly Asp Thr Thr His Thr Cys Pro Pro Cys Pro Ala Pro Glu
   20 25 30
   Leu Leu Gly Gly Pro Ser Val Phe Leu Phe Pro Pro Lys Pro Lys Asp
   35 40 45
   Thr Leu Met Ile Ser Arg Thr Pro Glu Val Thr Cys Val Val Val Asp
   50 55 60
   Val Ser His Glu Asp Pro Glu Val Lys Phe Asn Trp Tyr Val Asp Gly
   65 70 75 80
   Val Glu Val His Asn Ala Lys Thr Lys Pro Arg Glu Glu Gln Tyr Asn
                   85                  90                  95
   Ser Thr Tyr Arg Val Val Ser Val Leu Thr Val Leu His Gln Asp Trp
               100                 105                 110
   Leu Asn Gly Lys Glu Tyr Lys Cys Lys Val Ser Asn Lys Ala Leu Pro
           115                 120                 125
   Ala Pro Ile Glu Lys Thr Ile Ser Lys Ala Lys Gly Gln Pro Arg Glu
       130                 135                 140
   Pro Gln Val Tyr Thr Leu Pro Pro Ser Arg Asp Glu Leu Thr Lys Asn
   145                 150                 155                 160
   Gln Val Ser Leu Thr Cys Leu Val Lys Gly Phe Tyr Pro Ser Asp Ile
                   165                 170                 175
   Ala Val Glu Trp Glu Ser Asn Gly Gln Pro Glu Asn Asn Tyr Lys Thr
               180                 185                 190
   Thr Pro Pro Val Leu Asp Ser Asp Gly Ser Phe Phe Leu Tyr Ser Lys
           195                 200                 205
   Leu Thr Val Asp Lys Ser Arg Trp Gln Gln Gly Asn Val Phe Ser Cys
       210                 215                 220
   Ser Val Met His Glu Ala Leu His Asn His Tyr Thr Gln Lys Ser Leu
   225                 230                 235                 240
   Ser Leu Ser Pro Gly Lys
                   245
<210> 86
   <211> 383
   <212> PRT
   <213> Homo sapiens
<400> 86
   Pro Thr Lys Ala Pro Asp Val Phe Pro Ile Ile Ser Gly Cys Arg His
   1               5                   10                  15
   Pro Lys Asp Asn Ser Pro Val Val Leu Ala Cys Leu Ile Thr Gly Tyr
               20                  25                  30
   His Pro Thr Ser Val Thr Val Thr Trp Tyr Met Gly Thr Gln Ser Gln
           35                  40                  45
   Pro Gln Arg Thr Phe Pro Glu Ile Gln Arg Arg Asp Ser Tyr Tyr Met
       50                  55                  60
   Thr Ser Ser Gln Leu Ser Thr Pro Leu Gln Gln Trp Arg Gln Gly Glu
   65                  70                  75                  80
   Tyr Lys Cys Val Val Gln His Thr Ala Ser Lys Ser Lys Lys Glu Ile
                   85                  90                  95
   Phe Arg Trp Pro Glu Ser Pro Lys Ala Gln Ala Ser Ser Val Pro Thr
               100                 105                 110
   Ala Gln Pro Gln Ala Glu Gly Ser Leu Ala Lys Ala Thr Thr Ala Pro
           115                 120                 125
   Ala Thr Thr Arg Asn Thr Gly Arg Gly Gly Glu Glu Lys Lys Lys Glu
       130                 135                 140
   Lys Glu Lys Glu Glu Gln Glu Glu Arg Glu Thr Lys Thr Pro Glu Cys
   145                 150                 155                 160
   Pro Ser His Thr Gln Pro Leu Gly Val Tyr Leu Leu Thr Pro Ala Val
                   165                 170                 175
   Gln Asp Leu Trp Leu Arg Asp Lys Ala Thr Phe Thr Cys Phe Val Val
               180                 185                 190
   Gly Ser Asp Leu Lys Asp Ala His Leu Thr Trp Glu Val Ala Gly Lys
           195                 200                 205
   Val Pro Thr Gly Gly Val Glu Glu Gly Leu Leu Glu Arg His Ser Asn
       210                 215                 220
   Gly Ser Gln Ser Gln His Ser Arg Leu Thr Leu Pro Arg Ser Leu Trp
   225                 230                 235                 240
   Asn Ala Gly Thr Ser Val Thr Cys Thr Leu Asn His Pro Ser Leu Pro
                   245                 250                 255
   Pro Gln Arg Leu Met Ala Leu Arg Glu Pro Ala Ala Gln Ala Pro Val
               260                 265                 270
   Lys Leu Ser Leu Asn Leu Leu Ala Ser Ser Asp Pro Pro Glu Ala Ala
           275                 280                 285
   Ser Trp Leu Leu Cys Glu Val Ser Gly Phe Ser Pro Pro Asn Ile Leu
       290                 295                 300
   Leu Met Trp Leu Glu Asp Gln Arg Glu Val Asn Thr Ser Gly Phe Ala
   305 310 315 320
   Pro Ala Arg Pro Pro Pro Gln Pro Arg Ser Thr Thr Phe Trp Ala Trp
   325 330 335
   Ser Val Leu Arg Val Pro Ala Pro Pro Ser Pro Gln Pro Ala Thr Tyr
   340 345 350
   Thr Cys Val Val Ser His Glu Asp Ser Arg Thr Leu Leu Asn Ala Ser
   355 360 365
   Arg Ser Leu Glu Val Ser Tyr Val Thr Asp His Gly Pro Met Lys
   370 375 380
<210> 87
   <211> 276
   <212> PRT
   <213> Homo sapiens
<400> 87
   Val Thr Ser Thr Leu Thr Ile Lys Glx Ser Asp Trp Leu Gly Glu Ser
   1 5 10 15
   Met Phe Thr Cys Arg Val Asp His Arg Gly Leu Thr Phe Gln Gln Asn
   20 25 30
   Ala Ser Ser Met Cys Val Pro Asp Gln Asp Thr Ala Ile Arg Val Phe
   35 40 45
   Ala Ile Pro Pro Ser Phe Ala Ser Ile Phe Leu Thr Lys Ser Thr Lys
   50 55 60
   Leu Thr Cys Leu Val Thr Asp Leu Thr Thr Tyr Asx Ser Val Thr Ile
   65 70 75 80
   Ser Trp Thr Arg Glu Glu Asn Gly Ala Val Lys Thr His Thr Asn Ile
   85 90 95
   Ser Glu Ser His Pro Asn Ala Thr Phe Ser Ala Val Gly Glu Ala Ser
   100 105 110
   Ile Cys Glu Asp Asx Asp Trp Ser Gly Glu Arg Phe Thr Cys Thr Val
   115 120 125
   Thr His Thr Asp Leu Pro Ser Pro Leu Lys Gln Thr Ile Ser Arg Pro
   130 135 140
   Lys Gly Val Ala Leu His Arg Pro Asx Val Tyr Leu Leu Pro Pro Ala
   145                 150                 155                 160
   Arg Glx Glx Leu Asn Leu Arg Glu Ser Ala Thr Ile Thr Cys Leu Val
                   165                 170                 175
   Thr Gly Phe Ser Pro Ala Asp Val Phe Val Glu Trp Met Gln Arg Gly
               180                 185                 190
   Glu Pro Leu Ser Pro Gln Lys Tyr Val Thr Ser Ala Pro Met Pro Glu
           195                 200                 205
   Pro Gln Ala Pro Gly Arg Tyr Phe Ala His Ser Ile Leu Thr Val Ser
       210                 215                 220
   Glu Glu Glu Trp Asn Thr Gly Gly Thr Tyr Thr Cys Val Val Ala His
   225                 230                 235                 240
   Glu Ala Leu Pro Asn Arg Val Thr Glu Arg Thr Val Asp Lys Ser Thr
                   245                 250                 255
   Gly Lys Pro Thr Leu Tyr Asn Val Ser Leu Val Met Ser Asp Thr Ala
               260                 265                 270
   Gly Thr Cys Tyr
           275
<210> 88
   <211> 353
   <212> PRT
   <213> Homo sapiens
<400> 88
   Ala Ser Pro Thr Ser Pro Lys Val Phe Pro Leu Ser Leu Cys Ser Thr
   1               5                   10                  15
   Gln Pro Asp Gly Asn Val Val Ile Ala Cys Leu Val Gln Gly Phe Phe
               20                  25                  30
   Pro Gln Glu Pro Leu Ser Val Thr Trp Ser Glu Ser Gly Gln Gly Val
           35                  40                  45
   Thr Ala Arg Asn Phe Pro Pro Ser Gln Asp Ala Ser Gly Asp Leu Tyr
       50                  55                  60
   Thr Thr Ser Ser Gln Leu Thr Leu Pro Ala Thr Gln Cys Leu Ala Gly
   65                  70                  75                  80
   Lys Ser Val Thr Cys His Val Lys His Tyr Thr Asn Pro Ser Gln Asp
   85 90 95
   Val Thr Val Pro Cys Pro Val Pro Ser Thr Pro Pro Thr Pro Ser Pro
   100 105 110
   Ser Thr Pro Pro Thr Pro Ser Pro Ser Cys Cys His Pro Arg Leu Ser
   115 120 125
   Leu His Arg Pro Ala Leu Glu Asp Leu Leu Leu Gly Ser Glu Ala Asn
   130 135 140
   Leu Thr Cys Thr Leu Thr Gly Leu Arg Asp Ala Ser Gly Val Thr Phe
   145 150 155 160
   Thr Trp Thr Pro Ser Ser Gly Lys Ser Ala Val Gln Gly Pro Pro Glu
   165 170 175
   Arg Asp Leu Cys Gly Cys Tyr Ser Val Ser Ser Val Leu Pro Gly Cys
   180 185 190
   Ala Glu Pro Trp Asn His Gly Lys Thr Phe Thr Cys Thr Ala Ala Tyr
   195 200 205
   Pro Glu Ser Lys Thr Pro Leu Thr Ala Thr Leu Ser Lys Ser Gly Asn
   210 215 220
   Thr Phe Arg Pro Glu Val His Leu Leu Pro Pro Pro Ser Glu Glu Leu
   225 230 235 240
   Ala Leu Asn Glu Leu Val Thr Leu Thr Cys Leu Ala Arg Gly Phe Ser
   245 250 255
   Pro Lys Asp Val Leu Val Arg Trp Leu Gln Gly Ser Gln Glu Leu Pro
   260 265 270
   Arg Glu Lys Tyr Leu Thr Trp Ala Ser Arg Gln Glu Pro Ser Gln Gly
   275 280 285
   Thr Thr Thr Phe Ala Val Thr Ser Ile Leu Arg Val Ala Ala Glu Asp
   290 295 300
   Trp Lys Lys Gly Asp Thr Phe Ser Cys Met Val Gly His Glu Ala Leu
   305 310 315 320
   Pro Leu Ala Phe Thr Gln Lys Thr Ile Asp Arg Leu Ala Gly Lys Pro
   325 330 335
   Thr His Val Asn Val Ser Val Val Met Ala Glu Val Asp Gly Thr Cys
   340 345 350
   Tyr
<210> 89
   <211> 222
   <212> PRT
   <213> Homo sapiens
<400> 89
   Ala Asp Pro Cys Asp Ser Asn Pro Arg Gly Val Ser Ala Tyr Leu Ser
   1               5                   10                  15
   Arg Pro Ser Pro Phe Asp Leu Phe Ile Arg Lys Ser Pro Thr Ile Thr
               20                  25                  30
   Cys Leu Val Val Asp Leu Ala Pro Ser Lys Gly Thr Val Asn Leu Thr
           35                  40                  45
   Trp Ser Arg Ala Ser Gly Lys Pro Val Asn His Ser Thr Arg Lys Glu
       50                  55                  60
   Glu Lys Gln Arg Asn Gly Thr Leu Thr Val Thr Ser Thr Leu Pro Val
   65                  70                  75                  80
   Gly Thr Arg Asp Trp Ile Glu Gly Glu Thr Tyr Gln Cys Arg Val Thr
                   85                  90                  95
   His Pro His Leu Pro Arg Ala Leu Met Arg Ser Thr Thr Lys Thr Ser
               100                 105                 110
   Gly Pro Arg Ala Ala Pro Glu Val Tyr Ala Phe Ala Thr Pro Glu Trp
           115                 120                 125
   Pro Gly Ser Arg Asp Lys Arg Thr Leu Ala Cys Leu Ile Gln Asn Phe
       130                 135                 140
   Met Pro Glu Asp Ile Ser Val Gln Trp Leu His Asn Glu Val Gln Leu
   145                 150                 155                 160
   Pro Asp Ala Arg His Ser Thr Thr Gln Pro Arg Lys Thr Lys Gly Ser
                   165                 170                 175
   Gly Phe Phe Val Phe Ser Arg Leu Glu Val Thr Arg Ala Glu Trp Glu
               180                 185                 190
   Gln Lys Asp Glu Phe Ile Cys Arg Ala Val His Glu Ala Ala Ser Pro
           195                 200                 205
   Ser Gln Thr Val Gln Arg Ala Val Ser Val Asn Pro Gly Lys
       210                 215                 220
<210> 90
   <211> 327
   <212> PRT
   <213> Homo sapiens
<400> 90
   Ala Ser Thr Lys Gly Pro Ser Val Phe Pro Leu Ala Pro Cys Ser Arg
   1               5                   10                  15
   Ser Thr Ser Glu Ser Thr Ala Ala Leu Gly Cys Leu Val Lys Asp Tyr
               20                  25                  30
   Phe Pro Glu Pro Val Thr Val Ser Trp Asn Ser Gly Ala Leu Thr Ser
           35                  40                  45
   Gly Val His Thr Phe Pro Ala Val Leu Gln Ser Ser Gly Leu Tyr Ser
       50                  55                  60
   Leu Ser Ser Val Val Thr Val Pro Ser Ser Ser Leu Gly Thr Lys Thr
   65                  70                  75                  80
   Tyr Thr Cys Asn Val Asp His Lys Pro Ser Asn Thr Lys Val Asp Lys
                   85                  90                  95
   Arg Val Glu Ser Lys Tyr Gly Pro Pro Cys Pro Ser Cys Pro Ala Pro
               100                 105                 110
   Glu Phe Leu Gly Gly Pro Ser Val Phe Leu Phe Pro Pro Lys Pro Lys
           115                 120                 125
   Asp Thr Leu Met Ile Ser Arg Thr Pro Glu Val Thr Cys Val Val Val
       130                 135                 140
   Asp Val Ser Gln Glu Asp Pro Glu Val Gln Phe Asn Trp Tyr Val Asp
   145                 150                 155                 160
   Gly Val Glu Val His Asn Ala Lys Thr Lys Pro Arg Glu Glu Gln Phe
                   165                 170                 175
   Asn Ser Thr Tyr Arg Val Val Ser Val Leu Thr Val Leu His Gln Asp
   180 185 190
   Trp Leu Asn Gly Lys Glu Tyr Lys Cys Lys Val Ser Asn Lys Gly Leu
   195 200 205
   Pro Ser Ser Ile Glu Lys Thr Ile Ser Lys Ala Lys Gly Gln Pro Arg
   210 215 220
   Glu Pro Gln Val Tyr Thr Leu Pro Pro Ser Gln Glu Glu Met Thr Lys
   225 230 235 240
   Asn Gln Val Ser Leu Thr Cys Leu Val Lys Gly Phe Tyr Pro Ser Asp
   245 250 255
   Ile Ala Val Glu Trp Glu Ser Asn Gly Gln Pro Glu Asn Asn Tyr Lys
   260 265 270
   Thr Thr Pro Pro Val Leu Asp Ser Asp Gly Ser Phe Phe Leu Tyr Ser
   275 280 285
   Arg Leu Thr Val Asp Lys Ser Arg Trp Gln Glu Gly Asn Val Phe Ser
   290 295 300
   Cys Ser Val Met His Glu Ala Leu His Asn His Tyr Thr Gln Lys Ser
   305 310 315 320
   Leu Ser Leu Ser Leu Gly Lys
   325
<210> 91
   <211> 365
   <212> PRT
   <213> Homo sapiens
<400> 91
   Met Ala Val Met Ala Pro Arg Thr Leu Leu Leu Leu Leu Ser Gly Ala
   1               5                   10                  15
   Leu Ala Leu Thr Gln Thr Trp Ala Gly Ser His Ser Met Arg Tyr Phe
               20                  25                  30
   Phe Thr Ser Val Ser Arg Pro Gly Arg Gly Glu Pro Arg Phe Ile Ala
           35                  40                  45
   Val Gly Tyr Val Asp Asp Thr Gln Phe Val Arg Phe Asp Ser Asp Ala
       50                  55                  60
   Ala Ser Gln Lys Met Glu Pro Arg Ala Pro Trp Ile Glu Gln Glu Gly
   65 70 75 80
   Pro Glu Tyr Trp Asp Gln Glu Thr Arg Asn Met Lys Ala His Ser Gln
   85 90 95
   Thr Asp Arg Ala Asn Leu Gly Thr Leu Arg Gly Tyr Tyr Asn Gln Ser
   100 105 110
   Glu Asp Gly Ser His Thr Ile Gln Ile Met Tyr Gly Cys Asp Val Gly
   115 120 125
   Pro Asp Gly Arg Phe Leu Arg Gly Tyr Arg Gln Asp Ala Tyr Asp Gly
   130 135 140
   Lys Asp Tyr Ile Ala Leu Asn Glu Asp Leu Arg Ser Trp Thr Ala Ala
   145 150 155 160
   Asp Met Ala Ala Gln Ile Thr Lys Arg Lys Trp Glu Ala Val His Ala
   165 170 175
   Ala Glu Gln Arg Arg Val Tyr Leu Glu Gly Arg Cys Val Asp Gly Leu
   180 185 190
   Arg Arg Tyr Leu Glu Asn Gly Lys Glu Thr Leu Gln Arg Thr Asp Pro
   195 200 205
   Pro Lys Thr His Met Thr His His Pro Ile Ser Asp His Glu Ala Thr
   210 215 220
   Leu Arg Cys Trp Ala Leu Gly Phe Tyr Pro Ala Glu Ile Thr Leu Thr
   225 230 235 240
   Trp Gln Arg Asp Gly Glu Asp Gln Thr Gln Asp Thr Glu Leu Val Glu
   245 250 255
   Thr Arg Pro Ala Gly Asp Gly Thr Phe Gln Lys Trp Ala Ala Val Val
   260 265 270
   Val Pro Ser Gly Glu Glu Gln Arg Tyr Thr Cys His Val Gln His Glu
   275 280 285
   Gly Leu Pro Lys Pro Leu Thr Leu Arg Trp Glu Leu Ser Ser Gln Pro
   290 295 300
   Thr Ile Pro Ile Val Gly Ile Ile Ala Gly Leu Val Leu Leu Gly Ala
   305 310 315 320
   Val Ile Thr Gly Ala Val Val Ala Ala Val Met Trp Arg Arg Lys Ser
                   325                 330                 335
   Ser Asp Arg Lys Gly Gly Ser Tyr Thr Gln Ala Ala Ser Ser Asp Ser
               340                 345                 350
   Ala Gln Gly Ser Asp Val Ser Leu Thr Ala Cys Lys Val
           355                 360                 365
<210> 92
   <211> 362
   <212> PRT
   <213> Homo sapiens
<400> 92
   Met Leu Val Met Ala Pro Arg Thr Val Leu Leu Leu Leu Ser Ala Ala
   1               5                   10                  15
   Leu Ala Leu Thr Glu Thr Trp Ala Gly Ser His Ser Met Arg Tyr Phe
               20                  25                  30
   Tyr Thr Ser Val Ser Arg Pro Gly Arg Gly Glu Pro Arg Phe Ile Ser
           35                  40                  45
   Val Gly Tyr Val Asp Asp Thr Gln Phe Val Arg Phe Asp Ser Asp Ala
       50                  55                  60
   Ala Ser Pro Arg Glu Glu Pro Arg Ala Pro Trp Ile Glu Gln Glu Gly
   65                  70                  75                  80
   Pro Glu Tyr Trp Asp Arg Asn Thr Gln Ile Tyr Lys Ala Gln Ala Gln
                   85                  90                  95
   Thr Asp Arg Glu Ser Leu Arg Asn Leu Arg Gly Tyr Tyr Asn Gln Ser
               100                 105                 110
   Glu Ala Gly Ser His Thr Leu Gln Ser Met Tyr Gly Cys Asp Val Gly
           115                 120                 125
   Pro Asp Gly Arg Leu Leu Arg Gly His Asp Gln Tyr Ala Tyr Asp Gly
       130                 135                 140
   Lys Asp Tyr Ile Ala Leu Asn Glu Asp Leu Arg Ser Trp Thr Ala Ala
   145                 150                 155                 160
   Asp Thr Ala Ala Gln Ile Thr Gln Arg Lys Trp Glu Ala Ala Arg Glu
                   165                 170                 175
   Ala Glu Gln Arg Arg Ala Tyr Leu Glu Gly Glu Cys Val Glu Trp Leu
               180                 185                 190
   Arg Arg Tyr Leu Glu Asn Gly Lys Asp Lys Leu Glu Arg Ala Asp Pro
           195                 200                 205
   Pro Lys Thr His Val Thr His His Pro Ile Ser Asp His Glu Ala Thr
       210                 215                 220
   Leu Arg Cys Trp Ala Leu Gly Phe Tyr Pro Ala Glu Ile Thr Leu Thr
   225                 230                 235                 240
   Trp Gln Arg Asp Gly Glu Asp Gln Thr Gln Asp Thr Glu Leu Val Glu
                   245                 250                 255
   Thr Arg Pro Ala Gly Asp Arg Thr Phe Gln Lys Trp Ala Ala Val Val
               260                 265                 270
   Val Pro Ser Gly Glu Glu Gln Arg Tyr Thr Cys His Val Gln His Glu
           275                 280                 285
   Gly Leu Pro Lys Pro Leu Thr Leu Arg Trp Glu Pro Ser Ser Gln Ser
       290                 295                 300
   Thr Val Pro Ile Val Gly Ile Val Ala Gly Leu Ala Val Leu Ala Val
   305                 310                 315                 320
   Val Val Ile Gly Ala Val Val Ala Ala Val Met Cys Arg Arg Lys Ser
                   325                 330                 335
   Ser Gly Gly Lys Gly Gly Ser Tyr Ser Gln Ala Ala Cys Ser Asp Ser
               340                 345                 350
   Ala Gln Gly Ser Asp Val Ser Leu Thr Ala
           355                 360
<210> 93
   <211> 366
   <212> PRT
   <213> Homo sapiens
<400> 93
   Met Arg Val Met Ala Pro Arg Ala Leu Leu Leu Leu Leu Ser Gly Gly
   1               5                   10                  15
   Leu Ala Leu Thr Glu Thr Trp Ala Cys Ser His Ser Met Arg Tyr Phe
               20                  25                  30
   Asp Thr Ala Val Ser Arg Pro Gly Arg Gly Glu Pro Arg Phe Ile Ser
           35                  40                  45
   Val Gly Tyr Val Asp Asp Thr Gln Phe Val Arg Phe Asp Ser Asp Ala
       50                  55                  60
   Ala Ser Pro Arg Gly Glu Pro Arg Ala Pro Trp Val Glu Gln Glu Gly
   65                  70                  75                  80
   Pro Glu Tyr Trp Asp Arg Glu Thr Gln Asn Tyr Lys Arg Gln Ala Gln
                   85                  90                  95
   Ala Asp Arg Val Ser Leu Arg Asn Leu Arg Gly Tyr Tyr Asn Gln Ser
               100                 105                 110
   Glu Asp Gly Ser His Thr Leu Gln Arg Met Tyr Gly Cys Asp Leu Gly
           115                 120                 125
   Pro Asp Gly Arg Leu Leu Arg Gly Tyr Asp Gln Ser Ala Tyr Asp Gly
       130                 135                 140
   Lys Asp Tyr Ile Ala Leu Asn Glu Asp Leu Arg Ser Trp Thr Ala Ala
   145                 150                 155                 160
   Asp Thr Ala Ala Gln Ile Thr Gln Arg Lys Leu Glu Ala Ala Arg Ala
                   165                 170                 175
   Ala Glu Gln Leu Arg Ala Tyr Leu Glu Gly Thr Cys Val Glu Trp Leu
               180                 185                 190
   Arg Arg Tyr Leu Glu Asn Gly Lys Glu Thr Leu Gln Arg Ala Glu Pro
           195                 200                 205
   Pro Lys Thr His Val Thr His His Pro Leu Ser Asp His Glu Ala Thr
       210                 215                 220
   Leu Arg Cys Trp Ala Leu Gly Phe Tyr Pro Ala Glu Ile Thr Leu Thr
   225                 230                 235                 240
   Trp Gln Arg Asp Gly Glu Asp Gln Thr Gln Asp Thr Glu Leu Val Glu
                   245                 250                 255
   Thr Arg Pro Ala Gly Asp Gly Thr Phe Gln Lys Trp Ala Ala Val Val
               260                 265                 270
   Val Pro Ser Gly Gln Glu Gln Arg Tyr Thr Cys His Met Gln His Glu
           275                 280                 285
   Gly Leu Gln Glu Pro Leu Thr Leu Ser Trp Glu Pro Ser Ser Gln Pro
   290 295 300
   Thr Ile Pro Ile Met Gly Ile Val Ala Gly Leu Ala Val Leu Val Val
   305 310 315 320
   Leu Ala Val Leu Gly Ala Val Val Thr Ala Met Met Cys Arg Arg Lys
   325 330 335
   Ser Ser Gly Gly Lys Gly Gly Ser Cys Ser Gln Ala Ala Cys Ser Asn
   340 345 350
   Ser Ala Gln Gly Ser Asp Glu Ser Leu Ile Thr Cys Lys Ala
   355 360 365
<210> 94
   <211> 290
   <212> PRT
   <213> Homo sapiens
<400> 94
   Met Arg Ile Phe Ala Gly Ile Ile Phe Thr Ala Cys Cys His Leu Leu
   1 5 10 15
   Arg Ala Phe Thr Ile Thr Ala Pro Lys Asp Leu Tyr Val Val Glu Tyr
   20 25 30
   Gly Ser Asn Val Thr Met Glu Cys Arg Phe Pro Val Glu Arg Glu Leu
   35 40 45
   Asp Leu Leu Ala Leu Val Val Tyr Trp Glu Lys Glu Asp Glu Gln Val
   50 55 60
   Ile Gln Phe Val Ala Gly Glu Glu Asp Leu Lys Pro Gln His Ser Asn
   65 70 75 80
   Phe Arg Gly Arg Ala Ser Leu Pro Lys Asp Gln Leu Leu Lys Gly Asn
   85 90 95
   Ala Ala Leu Gln Ile Thr Asp Val Lys Leu Gln Asp Ala Gly Val Tyr
   100 105 110
   Cys Cys Ile Ile Ser Tyr Gly Gly Ala Asp Tyr Lys Arg Ile Thr Leu
   115 120 125
   Lys Val Asn Ala Pro Tyr Arg Lys Ile Asn Gln Arg Ile Ser Val Asp
   130 135 140
   Pro Ala Thr Ser Glu His Glu Leu Ile Cys Gln Ala Glu Gly Tyr Pro
   145 150 155 160
   Glu Ala Glu Val Ile Trp Thr Asn Ser Asp His Gln Pro Val Ser Gly
   165 170 175
   Lys Arg Ser Val Thr Thr Ser Arg Thr Glu Gly Met Leu Leu Asn Val
   180 185 190
   Thr Ser Ser Leu Arg Val Asn Ala Thr Ala Asn Asp Val Phe Tyr Cys
   195 200 205
   Thr Phe Trp Arg Ser Gln Pro Gly Gln Asn His Thr Ala Glu Leu Ile
   210 215 220
   Ile Pro Glu Leu Pro Ala Thr His Pro Pro Gln Asn Arg Thr His Trp
   225 230 235 240
   Val Leu Leu Gly Ser Ile Leu Leu Phe Leu Ile Val Val Ser Thr Val
   245 250 255
   Leu Leu Phe Leu Arg Lys Gln Val Arg Met Leu Asp Val Glu Lys Cys
   260 265 270
   Gly Val Glu Asp Thr Ser Ser Lys Asn Arg Asn Asp Thr Gln Phe Glu
   275 280 285
   Glu Thr
   290
<210> 95
   <211> 290
   <212> PRT
   <213> Homo sapiens
<400> 95
   Met Arg Ile Phe Ala Val Phe Ile Phe Met Thr Tyr Trp His Leu Leu
   1 5 10 15
   Asn Ala Phe Thr Val Thr Val Pro Lys Asp Leu Tyr Val Val Glu Tyr
   20 25 30
   Gly Ser Asn Met Thr Ile Glu Cys Lys Phe Pro Val Glu Lys Gln Leu
   35 40 45
   Asp Leu Ala Ala Leu Ile Val Tyr Trp Glu Met Glu Asp Lys Asn Ile
   50 55 60
   Ile Gln Phe Val His Gly Glu Glu Asp Leu Lys Val Gln His Ser Ser
   65 70 75 80
   Tyr Arg Gln Arg Ala Arg Leu Leu Lys Asp Gln Leu Ser Leu Gly Asn
   85 90 95
   Ala Ala Leu Gln Ile Thr Asp Val Lys Leu Gln Asp Ala Gly Val Tyr
   100 105 110
   Arg Cys Met Ile Ser Tyr Gly Gly Ala Asp Tyr Lys Arg Ile Thr Val
   115 120 125
   Lys Val Asn Ala Pro Tyr Asn Lys Ile Asn Gln Arg Ile Leu Val Val
   130 135 140
   Asp Pro Val Thr Ser Glu His Glu Leu Thr Cys Gln Ala Glu Gly Tyr
   145 150 155 160
   Pro Lys Ala Glu Val Ile Trp Thr Ser Ser Asp His Gln Val Leu Ser
   165 170 175
   Gly Lys Thr Thr Thr Thr Asn Ser Lys Arg Glu Glu Lys Leu Phe Asn
   180 185 190
   Val Thr Ser Thr Leu Arg Ile Asn Thr Thr Thr Asn Glu Ile Phe Tyr
   195 200 205
   Cys Thr Phe Arg Arg Leu Asp Pro Glu Glu Asn His Thr Ala Glu Leu
   210 215 220
   Val Ile Pro Glu Leu Pro Leu Ala His Pro Pro Asn Glu Arg Thr His
   225 230 235 240
   Leu Val Ile Leu Gly Ala Ile Leu Leu Cys Leu Gly Val Ala Leu Thr
   245 250 255
   Phe Ile Phe Arg Leu Arg Lys Gly Arg Met Met Asp Val Lys Lys Cys
   260 265 270
   Gly Ile Gln Asp Thr Asn Ser Lys Lys Gln Ser Asp Thr His Leu Glu
   275 280 285
   Glu Thr
   290
<210> 96
   <211> 254
   <212> PRT
   <213> Homo sapiens
<400> 96
   Met Glu Tyr Ala Ser Asp Ala Ser Leu Asp Pro Glu Ala Pro Trp Pro
   1               5                   10                  15
   Pro Ala Pro Arg Ala Arg Ala Cys Arg Val Leu Pro Trp Ala Leu Val
               20                  25                  30
   Ala Gly Leu Leu Leu Leu Leu Leu Leu Ala Ala Ala Cys Ala Val Phe
           35                  40                  45
   Leu Ala Cys Pro Trp Ala Val Ser Gly Ala Arg Ala Ser Pro Gly Ser
       50                  55                  60
   Ala Ala Ser Pro Arg Leu Arg Glu Gly Pro Glu Leu Ser Pro Asp Asp
   65                  70                  75                  80
   Pro Ala Gly Leu Leu Asp Leu Arg Gln Gly Met Phe Ala Gln Leu Val
                   85                  90                  95
   Ala Gln Asn Val Leu Leu Ile Asp Gly Pro Leu Ser Trp Tyr Ser Asp
               100                 105                 110
   Pro Gly Leu Ala Gly Val Ser Leu Thr Gly Gly Leu Ser Tyr Lys Glu
           115                 120                 125
   Asp Thr Lys Glu Leu Val Val Ala Lys Ala Gly Val Tyr Tyr Val Phe
       130                 135                 140
   Phe Gln Leu Glu Leu Arg Arg Val Val Ala Gly Glu Gly Ser Gly Ser
   145                 150                 155                 160
   Val Ser Leu Ala Leu His Leu Gln Pro Leu Arg Ser Ala Ala Gly Ala
                   165                 170                 175
   Ala Ala Leu Ala Leu Thr Val Asp Leu Pro Pro Ala Ser Ser Glu Ala
               180                 185                 190
   Arg Asn Ser Ala Phe Gly Phe Gln Gly Arg Leu Leu His Leu Ser Ala
           195                 200                 205
   Gly Gln Arg Leu Gly Val His Leu His Thr Glu Ala Arg Ala Arg His
       210                 215                 220
   Ala Trp Gln Leu Thr Gln Gly Ala Thr Val Leu Gly Leu Phe Arg Val
   225                 230                 235                 240
   Thr Pro Glu Ile Pro Ala Gly Leu Pro Ser Pro Arg Ser Glu
               245                 250
<210> 97
   <211> 302
   <212> PRT
   <213> Homo sapiens
<400> 97
   Met Arg Leu Gly Ser Pro Gly Leu Leu Phe Leu Leu Phe Ser Ser Leu
   1               5                   10                  15
   Arg Ala Asp Thr Gln Glu Lys Glu Val Arg Ala Met Val Gly Ser Asp
               20                  25                  30
   Val Glu Leu Ser Cys Ala Cys Pro Glu Gly Ser Arg Phe Asp Leu Asn
           35                  40                  45
   Asp Val Tyr Val Tyr Trp Gln Thr Ser Glu Ser Lys Thr Val Val Thr
       50                  55                  60
   Tyr His Ile Pro Gln Asn Ser Ser Leu Glu Asn Val Asp Ser Arg Tyr
   65                  70                  75                  80
   Arg Asn Arg Ala Leu Met Ser Pro Ala Gly Met Leu Arg Gly Asp Phe
                   85                  90                  95
   Ser Leu Arg Leu Phe Asn Val Thr Pro Gln Asp Glu Gln Lys Phe His
               100                 105                 110
   Cys Leu Val Leu Ser Gln Ser Leu Gly Phe Gln Glu Val Leu Ser Val
           115                 120                 125
   Glu Val Thr Leu His Val Ala Ala Asn Phe Ser Val Pro Val Val Ser
       130                 135                 140
   Ala Pro His Ser Pro Ser Gln Asp Glu Leu Thr Phe Thr Cys Thr Ser
   145                 150                 155                 160
   Ile Asn Gly Tyr Pro Arg Pro Asn Val Tyr Trp Ile Asn Lys Thr Asp
                   165                 170                 175
   Asn Ser Leu Leu Asp Gln Ala Leu Gln Asn Asp Thr Val Phe Leu Asn
               180                 185                 190
   Met Arg Gly Leu Tyr Asp Val Val Ser Val Leu Arg Ile Ala Arg Thr
           195                 200                 205
   Pro Ser Val Asn Ile Gly Cys Cys Ile Glu Asn Val Leu Leu Gln Gln
       210                 215                 220
   Asn Leu Thr Val Gly Ser Gln Thr Gly Asn Asp Ile Gly Glu Arg Asp
   225                 230                 235                 240
   Lys Ile Thr Glu Asn Pro Val Ser Thr Gly Glu Lys Asn Ala Ala Thr
                   245                 250                 255
   Trp Ser Ile Leu Ala Val Leu Cys Leu Leu Val Val Val Ala Val Ala
               260                 265                 270
   Ile Gly Trp Val Cys Arg Asp Arg Cys Leu Gln His Ser Tyr Ala Gly
           275                 280                 285
   Ala Trp Ala Val Ser Pro Glu Thr Glu Leu Thr Gly His Val
       290                 295                 300
<210> 98
   <211> 183
   <212> PRT
   <213> Homo sapiens
<400> 98
   Met Glu Arg Val Gln Pro Leu Glu Glu Asn Val Gly Asn Ala Ala Arg
   1               5                   10                  15
   Pro Arg Phe Glu Arg Asn Lys Leu Leu Leu Val Ala Ser Val Ile Gln
               20                  25                  30
   Gly Leu Gly Leu Leu Leu Cys Phe Thr Tyr Ile Cys Leu His Phe Ser
           35                  40                  45
   Ala Leu Gln Val Ser His Arg Tyr Pro Arg Ile Gln Ser Ile Lys Val
       50                  55                  60
   Gln Phe Thr Glu Tyr Lys Lys Glu Lys Gly Phe Ile Leu Thr Ser Gln
   65                  70                  75                  80
   Lys Glu Asp Glu Ile Met Lys Val Gln Asn Asn Ser Val Ile Ile Asn
                   85                  90                  95
   Cys Asp Gly Phe Tyr Leu Ile Ser Leu Lys Gly Tyr Phe Ser Gln Glu
               100                 105                 110
   Val Asn Ile Ser Leu His Tyr Gln Lys Asp Glu Glu Pro Leu Phe Gln
           115                 120                 125
   Leu Lys Lys Val Arg Ser Val Asn Ser Leu Met Val Ala Ser Leu Thr
   130 135 140
   Tyr Lys Asp Lys Val Tyr Leu Asn Val Thr Thr Asp Asn Thr Ser Leu
   145 150 155 160
   Asp Asp Phe His Val Asn Gly Gly Glu Leu Ile Leu Ile His Gln Asn
   165 170 175
   Pro Gly Glu Phe Cys Val Leu
   180
<210> 99
   <211> 273
   <212> PRT
   <213> Homo sapiens
<400> 99
   Met Ile Phe Leu Leu Leu Met Leu Ser Leu Glu Leu Gln Leu His Gln
   1 5 10 15
   Ile Ala Ala Leu Phe Thr Val Thr Val Pro Lys Glu Leu Tyr Ile Ile
   20 25 30
   Glu His Gly Ser Asn Val Thr Leu Glu Cys Asn Phe Asp Thr Gly Ser
   35 40 45
   His Val Asn Leu Gly Ala Ile Thr Ala Ser Leu Gln Lys Val Glu Asn
   50 55 60
   Asp Thr Ser Pro His Arg Glu Arg Ala Thr Leu Leu Glu Glu Gln Leu
   65 70 75 80
   Pro Leu Gly Lys Ala Ser Phe His Ile Pro Gln Val Gln Val Arg Asp
   85 90 95
   Glu Gly Gln Tyr Gln Cys Ile Ile Ile Tyr Gly Val Ala Trp Asp Tyr
   100 105 110
   Lys Tyr Leu Thr Leu Lys Val Lys Ala Ser Tyr Arg Lys Ile Asn Thr
   115 120 125
   His Ile Leu Lys Val Pro Glu Thr Asp Glu Val Glu Leu Thr Cys Gln
   130 135 140
   Ala Thr Gly Tyr Pro Leu Ala Glu Val Ser Trp Pro Asn Val Ser Val
   145 150 155 160
   Pro Ala Asn Thr Ser His Ser Arg Thr Pro Glu Gly Leu Tyr Gln Val
                   165                 170                 175
   Thr Ser Val Leu Arg Leu Lys Pro Pro Pro Gly Arg Asn Phe Ser Cys
               180                 185                 190
   Val Phe Trp Asn Thr His Val Arg Glu Leu Thr Leu Ala Ser Ile Asp
           195                 200                 205
   Leu Gln Ser Gln Met Glu Pro Arg Thr His Pro Thr Trp Leu Leu His
       210                 215                 220
   Ile Phe Ile Pro Phe Cys Ile Ile Ala Phe Ile Phe Ile Ala Thr Val
   225                 230                 235                 240
   Ile Ala Leu Arg Lys Gln Leu Cys Gln Lys Leu Tyr Ser Ser Lys Asp
                   245                 250                 255
   Thr Thr Lys Arg Pro Val Thr Thr Thr Lys Arg Glu Val Asn Ser Ala
               260                 265                 270
   Ile
<210> 100
   <211> 288
   <212> PRT
   <213> Homo sapiens
<400> 100
   Met Gly His Thr Arg Arg Gln Gly Thr Ser Pro Ser Lys Cys Pro Tyr
   1               5                   10                  15
   Leu Asn Phe Phe Gln Leu Leu Val Leu Ala Gly Leu Ser His Phe Cys
               20                  25                  30
   Ser Gly Val Ile His Val Thr Lys Glu Val Lys Glu Val Ala Thr Leu
           35                  40                  45
   Ser Cys Gly His Asn Val Ser Val Glu Glu Leu Ala Gln Thr Arg Ile
       50                  55                  60
   Tyr Trp Gln Lys Glu Lys Lys Met Val Leu Thr Met Met Ser Gly Asp
   65                  70                  75                  80
   Met Asn Ile Trp Pro Glu Tyr Lys Asn Arg Thr Ile Phe Asp Ile Thr
                   85                  90                  95
   Asn Asn Leu Ser Ile Val Ile Leu Ala Leu Arg Pro Ser Asp Glu Gly
               100                 105                 110
   Thr Tyr Glu Cys Val Val Leu Lys Tyr Glu Lys Asp Ala Phe Lys Arg
           115                 120                 125
   Glu His Leu Ala Glu Val Thr Leu Ser Val Lys Ala Asp Phe Pro Thr
       130                 135                 140
   Pro Ser Ile Ser Asp Phe Glu Ile Pro Thr Ser Asn Ile Arg Arg Ile
   145                 150                 155                 160
   Ile Cys Ser Thr Ser Gly Gly Phe Pro Glu Pro His Leu Ser Trp Leu
                   165                 170                 175
   Glu Asn Gly Glu Glu Leu Asn Ala Ile Asn Thr Thr Val Ser Gln Asp
               180                 185                 190
   Pro Glu Thr Glu Leu Tyr Ala Val Ser Ser Lys Leu Asp Phe Asn Met
           195                 200                 205
   Thr Thr Asn His Ser Phe Met Cys Leu Ile Lys Tyr Gly His Leu Arg
       210                 215                 220
   Val Asn Gln Thr Phe Asn Trp Asn Thr Thr Lys Gln Glu His Phe Pro
   225                 230                 235                 240
   Asp Asn Leu Leu Pro Ser Trp Ala Ile Thr Leu Ile Ser Val Asn Gly
                   245                 250                 255
   Ile Phe Val Ile Cys Cys Leu Thr Tyr Cys Phe Ala Pro Arg Cys Arg
               260                 265                 270
   Glu Arg Arg Arg Asn Glu Arg Leu Arg Arg Glu Ser Val Arg Pro Val
           275                 280                 285
<210> 101
   <211> 329
   <212> PRT
   <213> Homo sapiens
<400> 101
   Met Asp Pro Gln Cys Thr Met Gly Leu Ser Asn Ile Leu Phe Val Met
   1               5                   10                  15
   Ala Phe Leu Leu Ser Gly Ala Ala Pro Leu Lys Ile Gln Ala Tyr Phe
               20                  25                  30
   Asn Glu Thr Ala Asp Leu Pro Cys Gln Phe Ala Asn Ser Gln Asn Gln
           35                  40                  45
   Ser Leu Ser Glu Leu Val Val Phe Trp Gln Asp Gln Glu Asn Leu Val
       50                  55                  60
   Leu Asn Glu Val Tyr Leu Gly Lys Glu Lys Phe Asp Ser Val His Ser
   65                  70                  75                  80
   Lys Tyr Met Gly Arg Thr Ser Phe Asp Ser Asp Ser Trp Thr Leu Arg
                   85                  90                  95
   Leu His Asn Leu Gln Ile Lys Asp Lys Gly Leu Tyr Gln Cys Ile Ile
               100                 105                 110
   His His Lys Lys Pro Thr Gly Met Ile Arg Ile His Gln Met Asn Ser
           115                 120                 125
   Glu Leu Ser Val Leu Ala Asn Phe Ser Gln Pro Glu Ile Val Pro Ile
       130                 135                 140
   Ser Asn Ile Thr Glu Asn Val Tyr Ile Asn Leu Thr Cys Ser Ser Ile
   145                 150                 155                 160
   His Gly Tyr Pro Glu Pro Lys Lys Met Ser Val Leu Leu Arg Thr Lys
                   165                 170                 175
   Asn Ser Thr Ile Glu Tyr Asp Gly Ile Met Gln Lys Ser Gln Asp Asn
               180                 185                 190
   Val Thr Glu Leu Tyr Asp Val Ser Ile Ser Leu Ser Val Ser Phe Pro
           195                 200                 205
   Asp Val Thr Ser Asn Met Thr Ile Phe Cys Ile Leu Glu Thr Asp Lys
       210                 215                 220
   Thr Arg Leu Leu Ser Ser Pro Phe Ser Ile Glu Leu Glu Asp Pro Gln
   225                 230                 235                 240
   Pro Pro Pro Asp His Ile Pro Trp Ile Thr Ala Val Leu Pro Thr Val
                   245                 250                 255
   Ile Ile Cys Val Met Val Phe Cys Leu Ile Leu Trp Lys Trp Lys Lys
               260                 265                 270
   Lys Lys Arg Pro Arg Asn Ser Tyr Lys Cys Gly Thr Asn Thr Met Glu
               275                 280                 285
   Arg Glu Glu Ser Glu Gln Thr Lys Lys Arg Glu Lys Ile His Ile Pro
       290                 295                 300
   Glu Arg Ser Asp Glu Ala Gln Arg Val Phe Lys Ser Ser Lys Thr Ser
   305                 310                 315                 320
   Ser Cys Asp Lys Ser Asp Thr Cys Phe
                   325
<210> 102
   <211> 281
   <212> PRT
   <213> Homo sapiens
<400> 102
   Met Gln Gln Pro Phe Asn Tyr Pro Tyr Pro Gln Ile Tyr Trp Val Asp
   1               5                   10                  15
   Ser Ser Ala Ser Ser Pro Trp Ala Pro Pro Gly Thr Val Leu Pro Cys
               20                  25                  30
   Pro Thr Ser Val Pro Arg Arg Pro Gly Gln Arg Arg Pro Pro Pro Pro
           35                  40                  45
   Pro Pro Pro Pro Pro Leu Pro Pro Pro Pro Pro Pro Pro Pro Leu Pro
       50                  55                  60
   Pro Leu Pro Leu Pro Pro Leu Lys Lys Arg Gly Asn His Ser Thr Gly
   65                  70                  75                  80
   Leu Cys Leu Leu Val Met Phe Phe Met Val Leu Val Ala Leu Val Gly
                   85                  90                  95
   Leu Gly Leu Gly Met Phe Gln Leu Phe His Leu Gln Lys Glu Leu Ala
               100                 105                 110
   Glu Leu Arg Glu Ser Thr Ser Gln Met His Thr Ala Ser Ser Leu Glu
           115                 120                 125
   Lys Gln Ile Gly His Pro Ser Pro Pro Pro Glu Lys Lys Glu Leu Arg
       130                 135                 140
   Lys Val Ala His Leu Thr Gly Lys Ser Asn Ser Arg Ser Met Pro Leu
   145                 150                 155                 160
   Glu Trp Glu Asp Thr Tyr Gly Ile Val Leu Leu Ser Gly Val Lys Tyr
                   165                 170                 175
   Lys Lys Gly Gly Leu Val Ile Asn Glu Thr Gly Leu Tyr Phe Val Tyr
               180                 185                 190
   Ser Lys Val Tyr Phe Arg Gly Gln Ser Cys Asn Asn Leu Pro Leu Ser
           195                 200                 205
   His Lys Val Tyr Met Arg Asn Ser Lys Tyr Pro Gln Asp Leu Val Met
       210                 215                 220
   Met Glu Gly Lys Met Met Ser Tyr Cys Thr Thr Gly Gln Met Trp Ala
   225                 230                 235                 240
   Arg Ser Ser Tyr Leu Gly Ala Val Phe Asn Leu Thr Ser Ala Asp His
                   245                 250                 255
   Leu Tyr Val Asn Val Ser Glu Leu Ser Leu Val Asn Phe Glu Glu Ser
               260                 265                 270
   Gln Thr Phe Phe Gly Leu Tyr Lys Leu
           275                 280